(19) Europäisches Patentamt
European Patent Office
Office européen des brevets

(11) EP 2 006 288 A1

(12) EUROPEAN PATENT APPLICATION
published in accordance with Art. 153(4) EPC

(43) Date of publication:
24.12.2008 Bulletin 2008/52

(21) Application number: 07706978.9

(22) Date of filing: 18.01.2007

(51) Int Cl.:
*C07D 403/04* (2006.01) *A01N 43/54* (2006.01)
*A01P 3/00* (2006.01) *C07D 403/14* (2006.01)

(86) International application number:
PCT/JP2007/050674

(87) International publication number:
WO 2007/083692 (26.07.2007 Gazette 2007/30)

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK RS

(30) Priority: 23.01.2006 JP 2006014392

(71) Applicants:
- KUMIAI CHEMICAL INDUSTRY CO., LTD.
Tokyo 110-0008 (JP)
- IHARA CHEMICAL INDUSTRY CO., LTD.
Taito-ku,
Tokyo 110-0008 (JP)

(72) Inventors:
- BESSHO, Junichiro
Iwata-shi, Shizuoka 437-1213 (JP)
- NAKATANI, Masao
Iwata-shi, Shizuoka 437-1213 (JP)
- HIRANO, Yuuki
Iwata-shi, Shizuoka 437-1213 (JP)
- ARAI, Hirokazu
Iwata-shi, Shizuoka 437-1213 (JP)
- KOGURE, Atsushi
Tokyo 110-0008 (JP)
- YONEKURA, Norihisa
Tokyo 110-0008 (JP)
- HANAI, Ryo
Tokyo 110-0008 (JP)

(74) Representative: Vossius & Partner
Siebertstrasse 4
81675 München (DE)

(54) **AMINOPYRIDINE DERIVATIVE AND PLANT DISEASE CONTROL AGENT FOR AGRICULTURAL OR HORTICULTURAL USE**

(57) [Object] To provide an aminopyrimidine derivative exhibiting a significant effect on a plant disease and a plant disease control agent for agricultural or horticultural use which contains as an active ingredient the aminopyrimidine derivative.

[Solving Means] A plant disease control agent **characterized by** containing as an active ingredient one or more compounds selected from aminopyrimidine derivatives represented by General Formula [I]:

[Chemical Formula 1]

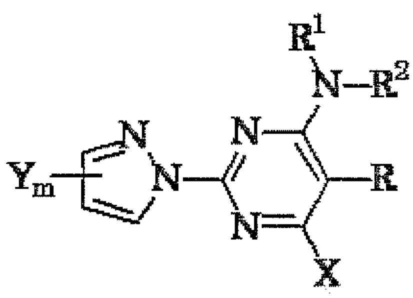

[I]

EP 2 006 288 A1

[wherein R is a substituent such as a $C_{2-10}$ alkyl group; $R^1$ and $R^2$ are each independently a hydrogen atom or a substituent such as an optionally substituted $C_{1-10}$ alkyl group; X is a hydrogen atom or a substituent selected from a predefined substituent group; Y is a substituent selected from a predefined substituent group; and m is an integer from 0 to 3] and agriculturally acceptable salts thereof.

[Effect] The agent solves problems of conventional plant disease control agents and further has excellent control effect, residual efficacy and the like.

## Description

Technical Field

The present invention relates to a novel aminopyrimidine derivative and a plant disease control agent for agricultural or horticultural use.

Background Art

There has been reported in, for example, Patent Document 1, Patent Document 2, Patent Document 3, Patent Document 4 and Patent Document 5, that a certain type of aminopyrimidine derivatives has a disease control effect. However, the aminopyrimidine derivative described in a specification of the present application is not disclosed in these documents. Further, various aminopyrimidine derivatives have been synthesized and reported in Non-Patent Document 1 and the like, but there is no report related to the disease control effect.

Patent Document 1: JP-A No. S54-115384 (Claims and others)
Patent Document 2: JP-A No. S55-036402 (Claims and others)
Patent Document 3: WO 2002/074753 (Claims and others)
Patent Document 4: WO 2004/103978 (Claims and others)
Patent Document 5: JP-A No. 2005-232081 (Claims and others)
Non-Patent Document 1: Revista de Chimie, Vol. 38 No. 8, p. 674-679, 1987

Disclosure of the Invention

Problems to be Solved by the Invention

For growing agricultural and horticultural crops, many control agents have been used against crop diseases. However, with the traditional control agents, there had been cases where the control effect is insufficiently exhibited or its use is limited due to the emergence of pathogenic organism resistance to the drug, or phytotoxicity or contamination to plants is caused, or from the viewpoints of toxicity to man and beast and fishes and effect on environment, there are very few control agents that are satisfactory. Consequently, advent of a disease control agent that has few such defects and can be safely used has been demanded.

An object of the invention is to provide a plant disease control agent that is free from the above-mentioned problems possessed by the traditional plant disease control agent and further has excellent control effect, residual efficacy and the like.

Means for Solving the Problems

Under these circumstances, the present inventors have conducted extensive studies on disease control effect and safety to crops and as a result, they found that a novel aminopyrimidine derivative has excellent disease control effect and safety to crops. Thus, they have completed the invention.

That is, the invention provides the following (1) to (8):

(1) a plant disease control agent for agricultural or horticultural use, which is characterized by containing as an active ingredient one or more compounds selected from aminopyrimidine derivatives represented by General Formula [I]:

[Chemical Formula 1]

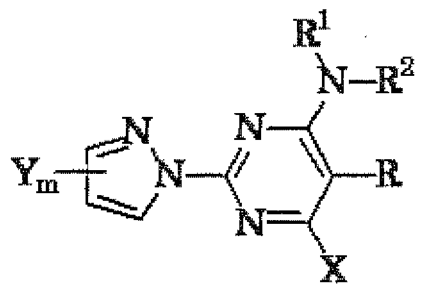

[I]

[wherein

R is a $C_{1-10}$ alkyl group, a $C_{3-8}$ cycloalkyl group, a $C_{3-8}$ cycloalkyl $C_{1-3}$ alkyl group, a $C_{1-6}$ haloalkyl group, a $C_{2-6}$ alkenyl group, a $C_{2-6}$ alkynyl group, a $C_{3-8}$ cycloalkenyl group, a $C_{1-6}$ acyl group, a $C_{1-6}$ hydroxylalkyl group, a $C_{1-6}$ alkoxy $C_{1-6}$ alkyl group, a 1,3-dioxolan-2-yl group or a 1,3-dioxan-2-yl group;

$R^1$ and $R^2$ are each independently a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono($C_{1-6}$ alkyl) aminocarbonyl group, a di($C_{1-6}$ alkyl) aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group, or a di($C_{1-6}$ alkyl) aminosulfonyl group,

while $R^1$ and $R^2$ may form a 5-membered or 6-membered ring (the 5-membered or 6-membered ring may be substituted with one or more substituents selected from Substituent Group α) with an atom arbitrarily selected from the group consisting of a carbon atom, an oxygen atom, a nitrogen atom and a sulfur atom, together with the nitrogen atom to which $R^1$ and $R^2$ are bonded;

X is a hydrogen atom or a substituent selected from Substituent Group α;

Y is a substituent selected from Substituent Group α; and

m is an integer from 0 to 3,

while Substituent Group α being defined as follows:

"Substituent Group α":

a halogen atom, a $C_{1-10}$ alkyl group, a $C_{3-8}$ cycloalkyl group, a $C_{3-8}$ cycloalkyl $C_{1-3}$ alkyl group, a $C_{2-6}$ alkynyl group, a $C_{2-6}$ alkenyl group, a $C_{1-10}$ alkoxy group, a $C_{1-6}$ alkoxy $C_{1-3}$ alkyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkynyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, an amino group, a nitro group, a hydroxyl group, a $C_{1-6}$ hydroxylalkyl group, a mono($C_{1-6}$ alkyl)amino group, a di($C_{1-6}$ alkyl) amino group, a $C_{1-6}$ acyl group, a carboxyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a thiol group, a thiocyanate group, a tri($C_{1-6}$ alkyl)silyl group, an optionally substituted benzyloxy group, a hydroxy-iminomethyl group, a $C_{1-6}$ alkoxyiminomethyl group and an optionally substituted phenyl group]

and agriculturally acceptable salts thereof;

(2) an aminopyrimidine derivative represented by General Formula [I]:

[Chemical Formula 2]

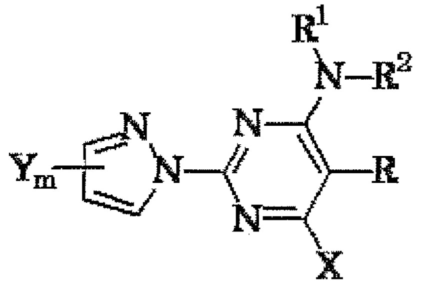

[I]

[wherein

R is a $C_{2-10}$ alkyl group, a $C_{3-8}$ cycloalkyl group, a $C_{3-8}$ cycloalkyl $C_{1-3}$ alkyl group, a $C_{1-6}$ haloalkyl group, a $C_{2-6}$ alkenyl group, a $C_{2-6}$ alkynyl group, a $C_{3-8}$ cycloalkenyl group, a $C_{1-6}$ acyl group, a $C_{1-6}$ hydroxylalkyl group, a $C_{1-6}$ alkoxy $C_{1-6}$ alkyl group, a 1,3-dioxolan-2-yl group or a 1,3-dioxan-2-yl group;

$R^1$ and $R^2$ are each independently a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group, or a di($C_{1-6}$ alkyl)aminosulfonyl group,

while $R^1$ and $R^2$ may form a 5-membered or 6-membered ring (the 5-membered or 6-membered ring may be substituted with one or more substituents selected from Substituent Group α) with an atom arbitrarily selected from the group consisting of a carbon atom, an oxygen atom, a nitrogen atom and a sulfur atom, together with the nitrogen atom to which $R^1$ and $R^2$ are bonded;

X is a hydrogen atom or a substituent selected from Substituent Group α;

Y is a substituent selected from Substituent Group α; and

m is an integer from 0 to 3,

while Substituent Group α being defined as follows:

"Substituent Group α":

a halogen atom, a $C_{1-10}$ alkyl group, a $C_{3-8}$ cycloalkyl group, a $C_{3-8}$ cycloalkyl $C_{1-3}$ alkyl group, a $C_{2-6}$ alkynyl group, a $C_{2-6}$ alkenyl group, a $C_{1-10}$ alkoxy group, a $C_{1-6}$ alkoxy $C_{1-3}$ alkyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkynyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, an amino group, a nitro group, a hydroxyl group, a $C_{1-6}$ hydroxylalkyl group, a mono($C_{1-6}$ alkyl)amino group, a di($C_{1-6}$ alkyl)amino group, a $C_{1-6}$ acyl group, a carboxyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a thiol group, a thiocyanate group, a tri($C_{1-6}$ alkyl)silyl group, an optionally substituted benzyloxy group, a hydroxy-iminomethyl group, a $C_{1-6}$ alkoxyiminomethyl group and an optionally substituted phenyl group]

or an agriculturally acceptable salt thereof;

(3) the aminopyrimidine derivative or an agriculturally acceptable salt thereof as described in (2), wherein, in General Formula [I],

$R^1$ is a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group and

$R^2$ is a $C_{1-10}$ alkyl group which may be substituted with one or more substituents selected from Substituent Group α, a

$C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl) aminocarbonyl group, a di ($C_{1-6}$ alkyl) aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring may be substituted with one or more substituents selected from Substituent Group α) with an atom arbitrarily selected from the group consisting of a carbon atom, an oxygen atom, a nitrogen atom and a sulfur atom, together with the nitrogen atom to which $R^1$ and $R^2$ are bonded;

(4) the aminopyrimidine derivative or an agriculturally acceptable salt thereof as described in (2),
wherein, in General Formula [I],
$R^1$ is a $C_{1-10}$ alkyl group which may be substituted with one or more substituents selected from Substituent Group β, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl) aminocarbonyl group, a $C_{1-6}$ alkylsulfonyl group, a $C_{1-6}$ haloalkylsulfonyl group, a sulfamoyl group, a mono($C_{1-6}$ alkyl) aminosulfonyl group or a di($C_{1-6}$ alkyl)aminosulfonyl group and
$R^2$ is a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl) aminocarbonyl group, a $C_{1-6}$ alkylsulfonyl group, a $C_{1-6}$ haloalkylsulfonyl group, a sulfamoyl group, a mono ($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring is independently substituted with 1 to 4 halogen atoms or/and a $C_{1-6}$ haloalkyl group) with an atom arbitrarily selected from the group consisting of a carbon atom, an oxygen atom, a nitrogen atom and a sulfur atom, together with the nitrogen atom to which $R^1$ and $R^2$ are bonded,
where Substituent Group β being defined as follows:

"Substituent Group β":

a halogen atom, a $C_{2-6}$ alkynyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkynyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, a nitro group, a $C_{1-6}$ acyl group, a carboxyl group, a $C_{1-6}$ alkoxycarbonyl group, a carbamoyl group, a mono($C_{1-6}$ alkyl) aminocarbonyl group, a di($C_{1-6}$ alkyl)aminocarbonyl group and a tri($C_{1-6}$ alkyl)silyl group;

(5) the aminopyrimidine derivative or an agriculturally acceptable salt thereof as described in (2),
wherein, in General Formula [I],
$R^1$ is a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono($C_{1-6}$ alkyl) aminocarbonyl group, a di($C_{1-6}$ alkyl)aminocarbonyl group a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group and
$R^2$ is a hydrogen atom, a $C_{1-10}$ alkyl group which may be substituted with one or more substituents selected from Substituent Group β, a $C_{1-10}$ alkoxy group which may be substituted with one or more substituents selected from Substituent Group α, a $C_{2-6}$ alkynyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring is independently substituted with 1 to 4 halogen atoms or/and a $C_{1-6}$ haloalkyl group) with an atom arbitrarily selected from the group consisting of a carbon atom, an oxygen atom, a nitrogen atom and a sulfur atom, together with the nitrogen atom to which $R^1$ and $R^2$ are bonded,
where Substituent Group β being defined as follows:

"Substituent Group β":

a halogen atom, a $C_{2-6}$ alkynyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkynyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, a nitro group, a $C_{1-6}$ acryl group, a carboxyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group and a tri($C_{1-6}$ alkyl)silyl group;

(6) the aminopyrimidine derivative or an agriculturally acceptable salt thereof as described in (5), wherein, in General Formula [I],
$R^2$ is a $C_{1-10}$ alkyl group which may be substituted with one or more substituents selected from Substituent Group β, a $C_{1-10}$ alkoxy group which may be substituted with one or more substituents selected from Substituent Group α, a $C_{2-6}$ alkynyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring is independently substituted with 1 to 4 halogen atoms or/and a $C_{1-6}$ haloalkyl group) with an atom arbitrarily selected from the group consisting of a carbon atom, an oxygen atom, a nitrogen atom and a sulfur atom, together with the nitrogen atom to which $R^1$ and $R^2$ are bonded;
(7) a plant disease control agent for agricultural or horticultural use, which is characterized by containing as an active ingredient one or more compounds selected from the aminopyrimidine derivative as described in any one of (2) to (6) and an agriculturally acceptable salt thereof; and
(8) a method of using an agent, which includes applying an effective amount of one or more compounds selected from the aminopyrimidine derivative as described in any one of (2) to (6) and an agriculturally acceptable salt thereof to target useful crops or soil, for protecting the useful crops from plant disease.

Advantage of the Invention

The aminopyrimidine derivative of the invention (hereinafter, referred to as 'compound of present application') is a novel compound known in literatures.
The plant disease control agent for agricultural or horticultural use according to the invention has a high control effect on Pyricularia oryzae, Rhizoctonia solani, Erysiphe graminis, Septoria nodorum, Septoria tritici, Puccinia recondite, Pseudoperonospora cubensis, Botrytis cinerea, Colletotrichum lagenarium, Venturia inaequalis, Physalospora piricola, Plasmopara viticola and the like and further has a characteristic of exhibiting excellent residual efficacy and rain resistance without causing a crop damage. Thus, the agent is useful as a plant disease control agent for agricultural or horticultural use.

Best Mode for Carrying out the Invention

Definition of symbols and terms used in the present specification are shown below.
The halogen atom represents a fluorine atom, a chlorine atom, a bromine atom, or an iodine atom.
A notation such as $C_{1-6}$ refers to a number of carbon atoms of the following substituent, which is from 1 to 6 in this case.
The $C_{1-6}$ alkyl group represents, unless otherwise particularly defined, a linear or branched chain alkyl group having 1 to 6 carbon atoms. Examples thereof may include groups such as methyl, ethyl, n-propyl, isopropyl, n-butyl, sec-butyl, isobutyl, tert-butyl, n-pentyl, 1-methylbutyl, 2-methylbutyl, 3-methylbutyl, 1-ethylpropyl, 1,1-dimethylpropyl, 1,2-dimethylpropyl, neopentyl, n-hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1, 3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethyl-1-methylpropyl and 1-ethyl-2-methylpropyl,
**[0025]** The $C_{1-10}$ alkyl group represents, unless otherwise particularly defined, a linear or branched chain alkyl group having 1 to 10 carbon atoms. Examples thereof may include groups such as heptyl, 1-methylhexyl, 5-methylhexyl, 1,1-dimethylpentyl, 2,2-dimethylpentyl, 4,4-dimethylpentyl, 1-ethylpentyl, 2-ethylpentyl, 1,1,3-trimethylbutyl, 1,2,2-trimethylbutyl, 1,3,3-trimethylbutyl, 2,2,3-trimethylbutyl, 2,3,3-trimethylbutyl, 1-propylbutyl, 1,1,2,2-tetramethylpropyl, octyl, 1-methylheptyl, 3-methylheptyl, 6-methylheptyl, 2-ethylhexyl, 5,5-dimethylhexyl, 2,4,4-trimethylpentyl, 1-ethyl-1-methylpentyl, n-nonyl, 1-methyloctyl, 2-methyloctyl, 3-methyloctyl, 7-methyloctyl, 1-ethylheptyl, 1,1-dimethylheptyl, 6,6-dimethylheptyl, decyl, 1-methylnonyl, 2-methylnonyl, 6-methylnonyl, 1-ethyloctyl, 1-propylheptyl and n-decyl, in addition to the above examples of $C_{1-6}$ alkyl group.

The $C_{3-8}$ cycloalkyl group represents, unless otherwise particularly defined, a cycloalkyl group having 3 to 8 carbon atoms. Examples thereof may include groups such as cyclopropyl, cyclobutyl, cyclopentyl, cyclohexyl, cycloheptyl and cyclooctyl.

The $C_{3-8}$ cycloalkyl $C_{1-3}$ alkyl group represents, unless otherwise particularly defined, a cycloalkyl-alkyl group where the cycloalkyl moiety has the same meanings as defined above and the alkyl moiety is a linear or branched chain alkyl group having 1 to 3 carbon atoms. Examples may include groups such as cyclopropylmethyl, 1-cyclopropylethyl, 2-cyclopropylethyl, 1-cyclopropylpropyl, 2-cyclopropylpropyl, 3-cyclopropylpropyl, cyclobutylmethyl, cyclopentylmethyl and cyclohexylmethyl.

The $C_{1-6}$ haloalkyl group represents, unless otherwise particularly defined, a linear or branched chain alkyl group having 1 to 6 carbon atoms while the group is substituted with 1 to 13 halogen atoms that may be the same with or different from each other. Examples thereof may include groups such as fluoromethyl, chloromethyl, bromomethyl, difluoromethyl, dichloromethyl, trifluoromethyl, trichloromethyl, chlorodifluoromethyl, bromodifluoromethyl, 2-fluoroethyl, 1-chloroethyl, 2-chloroethyl, 1-bromoethyl, 2-bromoethyl, 2,2-difluoroethyl, 2-chloro-2,2-difluoroethyl, 1,2-dichloroethyl, 2,2-dichloroethyl, 2,2,2-trifluoroethyl, 2,2,2-trichloroethyl, 1,1,2,2-tetrafluoroethyl, pentafluoroethyl, 2-bromo-2-chloroethyl, 2-chloro-1,1,2,2-tetrafluoroethyl, 1-chloro-1,2,2,2-tetrafluoroethyl, 1-chloropropyl, 2-chloropropyl, 3-chloropropyl, 2-bromopropyl, 3-bromopropyl, 2-bromo-1-methylethyl, 3-iodopropyl, 2,3-dichloropropyl, 2,3-dibromopropyl, 3,3,3-trifluoropropyl, 3,3,3-trichloropropyl, 3-bromo-3,3-difluoropropyl, 3,3-dichloro-3-fluoropropyl, 2,2,3,3-tetrafluoropropyl, 1-bromo-3,3,3-trifluoropropyl, 2,2,3,3,3-pentafluoropropyl, 2,2,2-trifluoro-1-trifluoromethylethyl, heptafluoropropyl, 1,2,2,2-tetrafluoro-1-trifluoromethylethyl, 2,3-dichloro-1,1,2,3,3-pentafluoropropyl, 1-fluoro-1-methylethyl, 1-methyl-2,2,2-trifluoroethyl, 2-chlorobutyl, 3-chlorobutyl, 4-chlorobutyl, 2-chloro-1,1-dimethylethyl, 4-bromobutyl, 3-bromo-2-methylpropyl, 2-bromo-1,1-dimethylethyl, 2,2-dichloro-1,1-dimethylethyl, 2-chloro-1-chloromethyl-2-methylethyl, 4,4,4-trifluorobutyl, 3,3,3-trifluoro-1-methylpropyl, 3,3,3-trifluoro-2-methylpropyl, 2,3,4-trichlorobutyl, 2,2,2-trichloro-1,1-dimethylethyl, 4-chloro-4,4-difluorobutyl, 4,4-dichloro-4-fluorobutyl, 4-bromo-4,4-difluorobutyl, 2,4-dibromo-4,4-difluorobutyl, 3,4-dichloro-3,4,4-trifluorobutyl, 3,3-dichloro-4,4,4-trifluorobutyl, 4-bromo-3,3,4,4-tetrafluorobutyl, 4-bromo-3-chloro-3,4,4-trifluorobutyl, 2,2,3,3,4,4-hexafluorobutyl, 2,2,3,4,4,4-hexafluorobutyl, 2,2,2-trifluoro-1-methyl-1-trifluoromethylethyl, 3,3,3-trifluoro-2-trifluoromethylpropyl, 2,2,3,3,4,4,4-heptafluorobutyl, 2,3,3,3-tetrafluoro-2-trifluoromethylpropyl, 1,1,2,2,3,3,4,4-octafluorobutyl, nonafluorobutyl, 4-chloro-1,1,2,2,3,3,4,4-octafluorobutyl, 5-fluoropentyl, 5-chloropentyl, 5,5-difluoropentyl, 5,5-dichloropentyl, 5,5,5-trifluoropentyl, 6,6,6-trifluorohexyl and 5,5,6,6,6-pentafluorohexyl.

The $C_{2-6}$ alkenyl group represents, unless otherwise particularly defined, a linear or branched chain alkenyl group having 2 to 6 carbon atoms. Examples thereof may include groups such as vinyl, 1-propenyl, isopropenyl, 2-propenyl, 1-butenyl, 1-methyl-1-propenyl, 2-butenyl, 1-methyl-2-propenyl, 3-butenyl, 2-methyl-1-propenyl, 2-methyl-2-propenyl, 1,3-butadienyl, 1-pentenyl, 1-ethyl-2-propenyl, 2-pentenyl, 1-methyl-1-butenyl, 3-pentenyl, 1-methyl-2-butenyl, 4-pentenyl, 1-methyl-3-butenyl, 3-methyl-1-butenyl, 1,2-dimethyl-2-propenyl, 1,1-dimethyl-2-propenyl, 2-methyl-2-butenyl, 3-methyl-2-butenyl, 1,2-dimethyl-1-propenyl, 2-methyl-3-butenyl, 3-methyl-3-butenyl, 1,3-pentadienyl, 1-vinyl-2-propenyl, 1-hexenyl, 1-propyl-2-propenyl, 2-hexenyl, 1-methyl-1-pentenyl, 1-ethyl-2-butenyl, 3-hexenyl, 4-hexenyl, 5-hexenyl, 1-methyl-4-pentenyl, 1-ethyl-3-butenyl, 1-(isobutyl)vinyl, 1-ethyl-1-methyl-2-propenyl, 1-ethyl-2-methyl-2-propenyl, 1-(isopropyl)-2-propenyl, 2-methyl-2-pentenyl, 3-methyl-3-pentenyl, 4-methyl-3-pentenyl, 1,3-dimethyl-2-butenyl, 1,1-dimethyl-3-butenyl, 3-methyl-4-pentenyl, 4-methyl-4-pentenyl, 1,2-dimethyl-3-butenyl, 1,3-dimethyl-3-butenyl, 1,1,2-trimethyl-2-propenyl, 1,5-hexadienyl, 1-vinyl-3-butenyl and 2,4-hexadienyl.

The $C_{3-8}$ cycloalkenyl group represents, unless otherwise particularly defined, a cyclic alkenyl group having 3 to 8 carbon atoms. Examples thereof may include groups such as 1-cyclopentenyl, 2-cyclopentenyl, 3-cyclopentenyl, 1-cyclohexenyl, 2-cyclohexenyl, 3-cyclohexenyl, 2-cycloheptenyl and 2-cyclooctenyl.

The $C_{2-6}$ alkynyl group represents, unless otherwise particularly defined, a linear or branched chain alkynyl group having 2 to 6 carbon atoms. Examples thereof may include groups such as ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 1-(n-propyl)-2-propynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, 1-ethyl-3-butynyl, 1-ethyl-1-methyl-2-propynyl, 1-(isopropyl)-2-propynyl, 1,1-dimethyl-2-butynyl and 2,2-dimethyl-3-butynyl.

The $C_{1-10}$ alkoxy group represents, unless otherwise particularly defined, a ($C_{1-10}$ alkyl)-O- group, where the alkyl moiety has the same meanings as defined above. Examples thereof may include groups such as methoxy, ethoxy, propoxy, n-propoxy, isopropoxy, butoxy, pentyloxy and hexyloxy.

The $C_{1-6}$ alkoxy $C_{1-3}$ alkyl group represents, unless otherwise particularly defined, a ($C_{1-6}$ alkyl)-O-($C_{1-3}$ alkyl) group, where the alkyl moiety has the same meaning as defined above. Examples may include groups such as methoxymethyl, ethoxymethyl, n-propoxymethyl, iso-propoxymethyl, n-butoxymethyl, iso-butoxymethyl, sec-butoxymethyl, n-pentyloxymethyl, 2-pentyloxymethyl, 3-pentyloxymethyl, n-hexyloxymethyl, 1-methoxyethyl, 1-ethoxyethyl, 1-methoxypropyl, 1-ethoxypropyl, 2-methoxyethyl, 2-ethoxyethyl, 3-methoxypropyl, 3-ethoxypropyl, 2-methoxypropyl, 2-ethoxypropyl, 2-methoxy-1-methylethyl and 2-ethoxy-1-methylethyl.

The $C_{1-6}$ haloalkoxy group represents, unless otherwise particularly defined, a ($C_{1-6}$ alkyl)-O- group, where the alkyl moiety has the same meaning as defined above. The alkyl moiety represents a substituent substituted with 1 to 13 halogen atoms that may be the same with or different from each other. Examples of the group may include groups such as chloromethoxy, difluoromethoxy, chlorodifluoromethoxy, trifluoromethoxy, 2,2-difluoroethoxy and 2,2,2-trifluoroethoxy.

The $C_{3-8}$ cycloalkyloxy group represents, unless otherwise particularly defined, a ($C_{3-8}$ cycloalkyl)-O- group, where the cycloalkyl moiety has the same meaning as defined above. Examples may include groups such as cyclopropyloxy, cyclobutyloxy, cyclopentyloxy and cyclohexyloxy.

The $C_{3-8}$ cycloalkyl $C_{1-3}$ alkyloxy group represents, unless otherwise particularly defined, a ($C_{3-8}$ cycloalkyl-$C_{1-3}$ alkyl)-O- group, where the cycloalkylalkyl moiety has the same meanings as defined above. Examples may include groups such as cyclopropylmethoxy, 1-cyclopropylethoxy, 2-cyclopropylethoxy, 1-cyclopropylpropoxy, 2-cyclopropylpropoxy, 3-cyclopropylpropoxy, cyclobutylmethoxy, cyclopentylmethoxy and cyclohexylmethoxy.

The $C_{2-6}$ alkenyloxy group and the $C_{2-6}$ alkynyloxy group represent, unless otherwise particularly defined, a ($C_{2-6}$ alkenyl)-O- group and a ($C_{2-6}$ alkynyl)-O- group, respectively, where the alkenyl moiety and the alkynyl moiety have the same meanings as defined above. Examples may include groups such as 2-propenyloxy and 2-propynyloxy.

The $C_{1-6}$ hydroxyalkyl group represents, unless otherwise particularly defined, a $C_{1-6}$ alkyl group while one hydroxyl group is substituted. Examples may include groups such as hydroxymethyl, 1-hydroxyethyl, 1-hydroxypropyl, 1-hydroxy-1-methylethyl and 1-hydroxy-2-methylpropyl.

The $C_{1-6}$ alkylthio group, the $C_{1-6}$ alkylsulfinyl group and the $C_{1-6}$ alkylsulfonyl group represent, unless otherwise particularly defined, a ($C_{1-6}$ alkyl)-S- group, a ($C_{1-6}$ alkyl)-SO- group and a ($C_{1-6}$ alkyl)-$SO_2$- group, respectively, where the alkyl moiety has the same meaning as defined above. Examples thereof may include groups such as methylthio, ethylthio, n-propylthio, isopropylthio, methylsulfinyl, methylsulfonyl, ethylsulfonyl, n-propylsulfonyl and isopropylsulfonyl.

The mono($C_{1-6}$ alkyl)amino group represents, unless otherwise particularly defined, a ($C_{1-6}$ alkyl)-NH- group, where the alkyl moiety has the same meaning as defined above. Examples thereof may include groups such as methylamino, ethylamino, n-propylamino and isopropylamino.

The di($C_{1-6}$ alkyl)amino group represents, unless otherwise particularly defined, a di($C_{1-6}$ alkyl)N- group where the alkyl moiety has the same meaning as defined above. Examples thereof may include groups such as dimethylamino, diethylamino, methylethylamino, dipropylamino and dibutylamino.

The mono($C_{1-6}$ alkyl)aminocarbonyl group represents, unless otherwise particularly defined, a ($C_{1-6}$ alkyl)-NH-CO-group where the alkyl moiety has the same meaning as defined above. Examples thereof may include groups such as methylaminocarbonyl and ethylaminocarbonyl.

The di($C_{1-6}$ alkyl) aminocarbonyl group represents, unless otherwise particularly defined, a di($C_{1-6}$ alkyl)NCO-group where the alkyl moiety has the same meaning as defined above. Examples thereof may include groups such as dimethylaminocarbonyl, diethylaminocarbonyl, methylethylaminocarbonyl, dipropylaminocarbonyl and dibutylaminocarbonyl.

The mono($C_{1-6}$ alkyl) aminosulfonyl group represents, unless otherwise particularly defined, a ($C_{1-6}$ alkyl)-$NHSO_2$-group where the alkyl moiety has the same meaning as defined above. Examples thereof may include groups such as methylaminosulfonyl and ethylaminosulfonyl.

The di($C_{1-6}$ alkyl)aminosulfonyl group represents, unless otherwise particularly defined, a di($C_{1-6}$ alkyl) $NSO_2$-group where the alkyl moiety has the same meaning as defined above. Examples thereof may include groups such as dimethylaminosulfonyl, diethylaminosulfonyl, methylethylaminosulfonyl, dipropylaminosulfonyl and dibutylaminosulfonyl.

The $C_{1-6}$ alkoxycarbonyl group represents, unless otherwise particularly defined, a ($C_{1-6}$ alkyl)-O(C=O)- group, where the alkyl moiety has the same meaning as defined above. Examples thereof may include groups such as methoxycarbonyl, ethoxycarbonyl, n-propoxycarbonyl, isopropoxycarbonyl and t-butoxycarbonyl.

The $C_{1-6}$ acyl group represents, unless otherwise particularly defined, a linear or branched chain aliphatic acyl group having 1 to 6 carbon atoms. Examples thereof may include groups such as formyl, acetyl, propionyl, isopropionyl, butyryl and pivaloyl.

The $C_{1-6}$ haloalkylthio group, the $C_{1-6}$ haloalkylsulfinyl group and the $C_{1-6}$ haloalkylsulfonyl group represent, unless otherwise particularly defined, a ($C_{1-6}$ haloalkyl)-S- group, a ($C_{1-6}$ haloalkyl)-SO- group and a ($C_{1-6}$ haloalkyl)-$SO_2$-group, respectively, where the haloalkyl moiety has the same meaning as defined above, respectively. Examples thereof may include groups such as difluoromethylthio, trifluoromethylthio, chloromethylsulfinyl, difluoromethylsulfinyl, trifluoromethylsulfinyl, chloromethylsulfonyl, difluoromethylsulfonyl and trifluoromethylsulfonyl.

The $C_{1-6}$ haloalkylcarbonyl group represents, unless otherwise particularly defined, a ($C_{1-6}$ haloalkyl)-CO-group, where the haloalkyl moiety has the same meanings as defined above. Examples thereof may include groups such as chloroacetyl, trifluoroacetyl and pentafluoropropionyl.

The tri($C_{1-6}$ alkyl) silyl group represents, unless otherwise particularly defined, a tri($C_{1-6}$ alkyl)silyl group, where the alkyl moiety has the same meanings as defined above. Examples thereof may include groups such as trimethylsilyl,

triethylsilyl, tri(n-propyl)silyl, tri(n-butyl)silyl and tri(n-hexyl)silyl.

The $C_{1-6}$ alkoxyiminomethyl group represents, unless otherwise particularly defined, a ($C_{1-6}$ alkyl)-O-N=CH-group, where the alkoxy moiety has the same meanings as defined above. Examples thereof may include groups such as methoxy-iminomethyl and ethoxyiminomethyl.

The optionally substituted phenyl group represents, unless otherwise particularly defined, groups such as phenyl, 2-chlorophenyl, 3-chlorophenyl, 4-chlorophenyl, 2-fluorophenyl, 3-fluorophenyl, 4-fluorophenyl, 2-methylphenyl, 3-methylphenyl, 4-methylphenyl, 2-methoxyphenyl, 3-methoxyphenyl, 4-methoxyphenyl, 2-nitrophenyl, 3-nitrophenyl and 4-nitrophenyl.

The optionally substituted benzyloxy group represents, unless otherwise particularly defined, a (phenyl)-$CH_2$-O-groups, where the phenyl moiety has the same meaning as defined above. Examples thereof may include groups such as benzyloxy, 2-chlorobenzyloxy, 3-chlorobenzyloxy, 4-chlorobenzyloxy, 2-fluorobenzyloxy, 3-fluorobenzyloxy, 4-fluorobenzyloxy, 2-methylbenzyloxy, 3-methylbenzyloxy, 4-methylbenzyloxy, 2-methoxybenzyloxy, 3-methoxybenzyloxy, 4-methoxybenzyloxy, 2-nitrobenzyloxy, 3-nitrobenzyloxy and 4-nitrobenzyloxy.

The optionally substituted phenylthio group represents, unless otherwise particularly defined, a (phenyl)-S-groups, where the phenyl moiety has the same meaning as defined above. Examples thereof may include groups such as phenylthio, 2-chlorophenylthio, 3-chlorophenylthio, 4-chlorophenylthio, 2-fluorophenylthio, 3-fluorophenylthio, 4-fluorophenylthio, 2-methylphenylthio, 3-methylphenylthio, 4-methylphenylthio, 2-methoxyphenylthio, 3-methoxyphenylthio, 4-methoxyphenylthio, 2-nitrophenylthio, 3-nitrophenylthio and 4-nitrophenylthio.

The optionally substituted benzylthio group represents, unless otherwise particularly defined, a (phenyl)-$CH_2$-S-groups, where the phenyl moiety has the same meaning as defined above. Examples thereof may include groups such as benzylthio, 2-chlorobenzylthio, 3-chlorobenzylthio, 4-chlorobenzylthio, 2-fluorobenzylthio, 3-fluorobenzylthio, 4-fluorobenzylthio, 2-methylbenzylthio, 3-methylbenzylthio, 4-methylbenzylthio, 2-methoxybenzylthio, 3-methoxybenzylthio, 4-methoxybenzyloxy, 2-nitrobenzylthio, 3-nitrobenzylthio and 4-nitrobenzylthio.

The term '$R^1$ and $R^2$ may form a 5-membered or 6-membered ring with an atom arbitrarily selected from the group consisting of a carbon atom, an oxygen atom, a nitrogen atom and a sulfur atom, together with the nitrogen atom to which $R^1$ and $R^2$ are bonded' may include, unless otherwise particularly defined, a case of forming a ring such as pyrrolidine, pyrazolidine, oxazolidine, thiazolidine, imidazolidine, isoxazolidine, isothiazolidine, piperidine, piperazine, hexahydropyridazine, morpholine, or thiomorpholine.

In regard to the term '$R^1$ and $R^2$ may form a 5-membered or 6-membered ring (the 5-membered or 6-membered ring is independently substituted with 1 to 4 halogen atoms or/and a $C_{1-6}$ haloalkyl group) with an atom arbitrarily selected from the group consisting of a carbon atom, an oxygen atom, a nitrogen atom and a sulfur atom, together with the nitrogen atom to which $R^1$ and $R^2$ are bonded', unless otherwise particularly defined, for example, 2-trifluoromethylpyrrolidin-1-yl, 3,3-difluoropyrrolidin-1-yl, 4-trifluoromethylpiperidin-1-yl, or 3,3,4,4-tetrafluoropyrrolidin-1-yl, may be mentioned.

When for the compound of Formula [I] a hydroxyl group, a carboxyl group, an amino group, or the like is present in its structure, or alternatively when the nitrogen atom of the compound of Formula [I] where the nitrogen atom forms a pyrazole ring or a pyrimidine ring shows a basic property, the agriculturally acceptable salt refers to a salt of the compound with metal or organic base or a salt of the compound with mineral acid or organic acid. As the metal, there are alkali metals such as sodium and potassium; and alkaline-earth metals such as magnesium and calcium. As the organic base, there are triethylamine, diisopropylamine and the like. As the mineral acid, there are hydrochloric acid, sulfuric acid and the like. As the organic acid, there are acetic acid, methanesulfonic acid, p-toluenesulfonic acid and the like.

In regard to the compound that can be included in the invention, there may be a case where a geometric isomer of E-form and Z-form exists according to the type of a substituent. The invention includes all of E-form, Z-form and a mixture including E-form and Z-form in an arbitrary proportion. Further, in regard to the compound that can be included in the invention, there exists an optically active substance caused by the presence of one or more asymmetric carbon atoms. The invention includes all of optically active substances as well as racemate. Moreover, when the compound that can be included in the invention has a hydroxyl group as a substituent, there may be a compound with a keto-enol isomer and the invention also includes the conformation thereof.

Next, representative examples of the compound of the present application represented by Formula [1] will be shown in Tables 1 to 40. However, the compound of the present application is not limited to those compounds. For the compound Nos., refer to the descriptions below.

The following abbreviations in Tables in the present specification represent groups as shown below, respectively.

Me: methyl group
Et: ethyl group
n-Pr: n-propyl group

iso-Pr: isopropyl group
c-Pr: cyclopropyl group
n-Bu: n-butyl group
sec-Bu: sec-butyl group
iso-Bu: iso-butyl group
tert-Bu: tert-butyl group
n-Pen: n-pentyl group
2-Pen: 2-pentyl group
3-Pen: 3-pentyl group
c-Pen: cyclopentyl group
c-Hex: cyclohexyl group

In addition, the following abbreviations refer to corresponding meanings, respectively.

3-Cl: a chlorine atom is substituted at 3rd position
3-$CF_3$-4-COOMe: a trifluoromethyl group is substituted at 3rd position and a methoxycarbonyl group is substituted at 4th position
3,5-$(Me)_2$: methyl groups are substituted at 3rd and 5th positions, respectively

[Table 1]

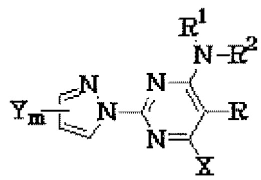

| Compound No | R | $R^1$ | $R^2$ | X | Ym |
|---|---|---|---|---|---|
| 0001 | Me | *iso*-Pr | H | H | - |
| 0002 | Me | *iso*-Pr | H | Cl | - |
| 0003 | Me | *iso*-Pr | H | CN | - |
| 0004 | Me | *iso*-Pr | H | Me | - |
| 0005 | Me | $CH_2CF_3$ | H | H | - |
| 0006 | Me | $CH_2CF_3$ | H | Cl | - |
| 0007 | Me | $CH_2CF_3$ | H | CN | - |
| 0008 | Me | $CH_2CF_3$ | H | Me | - |
| 0009 | Me | Et | Et | H | - |
| 0010 | Me | Et | Et | Cl | - |
| 0011 | Me | Et | Et | CN | - |
| 0012 | Me | Et | Et | Me | - |
| 0013 | Me | $-(CH_2)_2CH(Me)(CH_2)_3-$ | | H | - |
| 0014 | Me | $-(CH_2)_2CH(Me)(CH_2)_3-$ | | Cl | - |
| 0015 | Me | $-(CH_2)_2CH(Me)(CH_2)_3-$ | | CN | - |
| 0016 | Me | $-(CH_2)_2CH(Me)(CH_2)_3-$ | | Me | - |
| 0017 | Et | *iso*-Pr | H | H | - |
| 0018 | Et | *iso*-Pr | H | Cl | - |
| 0019 | Et | *iso*-Pr | H | CN | - |
| 0020 | Et | *iso*-Pr | H | Me | - |
| 0021 | Et | $CH_2CF_3$ | H | H | - |
| 0022 | Et | $CH_2CF_3$ | H | Cl | - |
| 0023 | Et | $CH_2OF_3$ | H | CN | - |
| 0024 | Et | $CH_2CF_3$ | H | Me | - |

(continued)

| Compound No | R | $R^1$ | $R^2$ | X | Ym |
|---|---|---|---|---|---|
| 0025 | Et | Et | Et | H | - |
| 0026 | Et | Et | Et | Cl | - |
| 0027 | Et | Et | Et | CN | - |
| 0028 | Et | Et | Et | Me | - |
| 0029 | Et | $-(CH_2)_2CH(Me)(CH_2)_2-$ | | H | - |
| 0030 | Et | $-(CH_2)_2CH(Me)(CH_2)_2-$ | | Cl | - |
| 0031 | Et | $-(CH_2)_2CH(Me)(CH_2)_2-$ | | CN | - |
| 0032 | Et | $-(CH_2)_2CH(Me)(CH_2)_2-$ | | Me | - |
| 0033 | *iso*-Pr | *iso*-Pr | H | H | - |
| 0034 | *iso*-Pr | *iso*-Pr | H | F | - |
| 0035 | *iso*-Pr | *iso*-Pr | H | Cl | - |
| 0036 | *iso*-Pr | *iso*-Pr | H | Br | - |
| 0037 | *iso*-Pr | *iso*-Pr | H | CN | - |
| 0038 | *iso*-Pr | *iso*-Pr | H | Me | - |
| 0039 | *iso*-Pr | *iso*-Pr | H | $CF_3$ | - |
| 0040 | *iso*-Pr | *tert*-Bu | H | H | - |
| 0041 | *iso*-Pr | *tert*-Bu | H | F | - |
| 0042 | *iso*-Pr | *tert*-Bu | H | Cl | - |
| 0043 | *iso*-Pr | *tert*-Bu | H | Br | - |
| 0044 | *iso*-Pr | *tert*-Bu | H | CN | - |
| 0045 | *iso*-Pr | *tert*-Bu | H | Me | - |

[Table 2]

| Compound No | R | $R^1$ | $R^2$ | X | Ym |
|---|---|---|---|---|---|
| 0046 | *iso*-Pr | *tert*-Bu | H | $CF_3$ | - |
| 0047 | *iso*-Pr | $CH_2CF_3$ | H | H | - |
| 0048 | *iso*-Pr | $CH_2CF_3$ | H | F | - |
| 0049 | *iso*-Pr | $CH_2CF_3$ | H | Cl | - |
| 0050 | *iso*-Pr | $CH_2CF_3$ | H | Br | - |
| 0051 | *iso*-Pr | $CH_2CF_3$ | H | I | - |
| 0052 | *iso*-Pr | $CH_2CF_3$ | H | OH | - |
| 0053 | *iso*-Pr | $CH_2CF_3$ | H | OMe | - |
| 0054 | *iso*-Pr | $CH_2CF_3$ | H | OEt | - |
| 0055 | *iso*-Pr | $CH_2CF_3$ | H | $OCH_3$*c*-Pr | - |
| 0056 | *iso*-Pr | $CH_2CF_3$ | H | $OCHF_3$ | - |
| 0057 | *iso*-Pr | $CH_2CF_3$ | H | $OCF_3$ | - |
| 0058 | *iso*-Pr | $CH_2CF_3$ | H | $OCH_3CHF_2$ | - |
| 0059 | *iso*-Pr | $CH_2CF_3$ | H | $OCH_2CF_3$ | - |
| 0060 | *iso*-Pr | $CH_2CF_3$ | H | SMe | - |
| 0061 | *iso*-Pr | $CH_2CF_3$ | H | SOMe | - |
| 0062 | *iso*-Pr | $CH_2CF_3$ | H | $SO_2Me$ | - |
| 0063 | *iso*-Pr | $CH_2CF_3$ | H | $SCF_3$ | - |
| 0064 | *iso*-Pr | $CH_2CF_3$ | H | $SOCF_3$ | - |
| 0065 | *iso*-Pr | $CH_2CF_3$ | H | $SO_2CF_3$ | - |
| 0066 | *iso*-Pr | $CH_2CF_3$ | H | $NH_2$ | - |
| 0067 | *iso*-Pr | $CH_2CF_3$ | H | HHMe | - |

(continued)

| Compound No | R | $R^1$ | $R^2$ | X | Ym |
|---|---|---|---|---|---|
| 0068 | *iso*-Pr | $CH_2CF_3$ | H | NH*iso*-Pr | - |
| 0069 | *iso*-Pr | $CH_2CF_3$ | H | $N(Me)_2$ | - |
| 0070 | *iso*-Pr | $CH_2CF_3$ | H | $N(Et)_2$ | - |
| 0071 | *iso*-Pr | $CH_2CF_3$ | H | CN | - |
| 0072 | *iso*-Pr | $CH_2CF_3$ | H | CHO | - |
| 0073 | *iso*-Pr | $CH_2CF_3$ | H | COMe | - |
| 0074 | *iso*-Pr | $CH_2CF_3$ | H | COEt | - |
| 0075 | *iso*-Pr | $CH_2CF_3$ | H | $CO_2H$ | - |
| 0076 | *iso*-Pr | $CH_2CF_3$ | H | $CO_2Me$ - | |
| 0077 | *iso*-Pr | $CH_2CF_3$ | H | $CO_2Et$ | - |
| 0078 | *iso*-Pr | $CH_2CF_3$ | H | $CONH_2$ | - |
| 0079 | *iso*-Pr | $CH_2CF_3$ | H | CONHMe | - |
| 0080 | *iso*-Pr | $CH_2CF_3$ | H | $CON(Me)_2$ | - |
| 0081 | *iso*-Pr | $CH_2CF_3$ | H | Me | - |
| 0082 | *iso*-Pr | $CH_2CF_3$ | H | Et | - |
| 0083 | *iso*-Pr | $CH_2CF_3$ | H | *iso*-Pr | |
| 0084 | *iso*-Pr | $CH_2CF_3$ | H | *c*-Pr | - |
| 0085 | *iso*-Pr | $CH_2CF_3$ | H | $CH_2F$ | - |
| 0086 | *iso*-Pr | $H_2CF_3$ 11 | H | $CH_2Cl$ | - |
| 0087 | *iso*-Pr | $CH_3CF_3$ | H | $CH_2Br$ | - |
| 0088 | *iso*-Pr | $CH_2CF_3$ | H | $CHF_3$ | - |
| 0089 | *iso*-Pr | $CH_2CF_3$ | H | $CF_3$ | - |
| 0090 | *iso*-Pr | $CH(Me)CF_3$ | H | H | - |
| 0091 | *iso*-Pr | $CH(Me)CF_3$ | H | F | - |
| 0092 | *iso*-Pr | $CH(Me)CF_3$ | H | Cl | - |
| 0093 | *iso*-Pr | $CH(Me)CF_3$ | H | Br | - |
| 0094 | *iso*-Pr | $CH(Me)CF_3$ | H | I | - |
| 0095 | *iso*-Pr | $CH(Me)CF_3$ | H | OH | - |
| 0096 | *iso*-Pr | $CH(Me)CF_3$ | H | OMe | - |

[Table 3]

| Compound No | R | $R^1$ | $R^1$ | X | Ym |
|---|---|---|---|---|---|
| 0097 | *iso*-Pr | $CH(Me)CF_3$ | H | OEt | - |
| 0098 | *iso*-Pr | $CH(Me)CF_3$ | H | $0CH_2$*c*-Pr | - |
| 0099 | *iso*-Pr | $CH(Me)CF_3$ | H | $OCHF_3$ | - |
| 0100 | *iso*-Pr | $CH(Me)CF_3$ | H | $OCF_3$ | - |
| 0101 | *iso*-Pr | $CH(Me)CF_3$ | H | $OCH_2CHF_2$ | - |
| 0102 | *iso*-Pr | $CH(Me)CF_3$ | H | $OCH_2CF_3$ | - |
| 0103 | *iso*-Pr | $CH(Me)CF_3$ | H | SMe | - |
| 0104 | *iso*-Pr | $CH(Me)CF_3$ | H | SOMe | - |
| 0105 | *iso*-Pr | $CH(Me)CF_3$ | H | $SO_2Me$ | - |
| 0106 | *iso*-Pr | $CH(Me)CF_3$ | H | $SCF_3$ | - |
| 0107 | *iso*-Pr | $CH(Me)CF_3$ | H | $SOCF_3$ | - |
| 0108 | *iso*-Pr | $CH(Me)CF_3$ | H | $SO_2CF_3$ | - |
| 0109 | *iso*-Pr | $CH(Me)CF_3$ | H | $NH_3$ | - |
| 0110 | *iso*-Pr | $CH(Me)CF_3$ | H | NHMe | - |
| 0111 | *iso*-Pr | $CH(Me)CF_3$ | H | NH*iso*-Pr | - |

(continued)

| Compound No | R | $R^1$ | $R^1$ | X | Ym |
|---|---|---|---|---|---|
| 0112 | *iso*-Pr | $CH(Me)CF_3$ | H | $N(Me)_2$ | |
| 0113 | *iso*-Pr | $CH(Me)CF_3$ | H | $N(Et)_2$ | - |
| 0114 | *iso*-Pr | $CH(Me)CF_3$ | H | CN | - |
| 0115 | *iso*-Pr | $CH(Me)CF_3$ | H | CHO | - |
| 0116 | *iso*-Pr | $CH(Me)CF_3$ | H | COMe | - |
| 0117 | *iso*-Pr | $CH(Me)CF_3$ | H | COEt | - |
| 0118 | *iso*-Pr | $CH(Me)CF_3$ | H | $CO_2H$ | - |
| 0119 | *iso*-Pr | $CH(Me)CF_3$ | H | $CO_2Me$ | |
| 0120 | *iso*-Pr | $CH(Me)CF_3$ | H | $CO_2Et$ | - |
| 0121 | *iso*-Pr | $CH(Me)CF_3$ | H | $CONH_2$ | - |
| 0122 | *iso*-Pr | $CH(Me)CF_3$ | H | COMHMe | - |
| 0123 | *iso*-Pr | $CH(Me)CF_3$ | H | $CON(Me)_2$ | - |
| 0124 | *iso*-Pr | $CH(Me)CF_3$ | H | Me | - |
| 0125 | *iso*-Pr | $CH(Me)CF_3$ | H | Et | - |
| 0126 | *iso*-Pr | $CH(Me)CF_3$ | H | *iso*-Pr | |
| 0127 | *iso*-Pr | $CH(Me)CF_3$ | H | *c*-Pr | - |
| 0128 | *iso*-Pr | $CH(Me)OF_3$ | H | $CH_2F$ | - |
| 0129 | *iso*-Pr | $CH(Me)CF_3$ | H | $CH_2Cl$ | - |
| 0130 | *iso*-Pr | $CH(Me)CF_3$ | H | $CH_2Br$ | - |
| 0131 | *iso*-Pr | $CH(Me)CF_3$ | H | $CHF_2$ | - |
| 0132 | *iso*-Pr | $CH(Me)CF_3$ | H | $CF_3$ | - |
| 0133 | *iso*-Pr | Et | Et | H | - |
| 0134 | *iso*-Pr | Et | Et | F | - |
| 0135 | *iso*-Pr | Et | Et | Cl | - |
| 0136 | *iso*-Pr | Et | Et | Br | - |
| 0137 | *iso*-Pr | Et | Et | CN | - |
| 0138 | *iso*-Pr | Et | Et | Me | - |
| 0139 | *iso*-Pr | Et | Et | $CF_3$ | - |
| 0140 | *iso*-Pr | $-CH(CF_3)CH_2)_3-$ | | H | - |
| 0141 | *iso*-Pr | $-CH(CF_3)CH_2)_3-$ | | | - |
| 0142 | *iso*-Pr | $-CH(CF_3)CH_2)_3-$ | | Cl | - |
| 0143 | *iso*-Pr | $-CH(CF_3)CH_2)_3-$ | | Br | - |
| 0144 | *iso*-Pr | $-CH(CF_3)CH_2)_3-$ | | CN | - |
| 0145 | *iso*-Pr | $-CH(CF_3)CH_2)_3-$ | | Me | - |
| 0146 | *iso*-Pr | $-CH(CF_3)CH_2)_3-$ | | $CF_3$ | - |

[Table 4]

| Compound No | R | $R^1$ | $R^2$ | X | Ym |
|---|---|---|---|---|---|
| 0147 | *iso*-Pr | $-(CH_2)_3CH(Me)(CH_2)_3-$ | | H | - |
| 0148 | *iso*-Pr | $-(CH_2)_3CH(Me)(CH_2)_3-$ | | F | - |
| 0149 | *iso*-Pr | $-(CH_2)_3CH(Me)(CH_2)_3-$ | | Cl | - |
| 0150 | *iso*-Pr | $-(CH_2)_3CH(Me)(CH_2)_3-$ | | Br | - |
| 0151 | *iso*-Pr | $-(CH_2)_3CH(Me)(CH_2)_3-$ | | CN | - |
| 0152 | *iso*-Pr | $-(CH_2)_3CH(Me)(CH_2)_3-$ | | Me | - |
| 0153 | *iso*-Pr | $-(CH_2)_3CH(Me)(CH_2)_3-$ | | $CF_3$ | - |
| 0154 | *n*-Fr | *iso*-Pr | H | H | - |
| 0155 | *n*-Pr | *iso*-Pr | H | Cl | - |

(continued)

| Compound No | R | $R^1$ | $R^2$ | X | Ym |
|---|---|---|---|---|---|
| 0156 | *n*-Pr | *iso*-Pr | H | CN | - |
| 0157 | *n*-Pr | *iso*-Pr | H | Me | - |
| 0158 | *n*-Pr | $CH_2CF_3$ | H | H | - |
| 0159 | *n*-Pr | $CH_2CF_3$ | H | Cl | - |
| 0160 | *n*-Pr | $CH_2CF_3$ | H | CN | - |
| 0161 | *n*-Pr | $CH_2CF_3$ | H | Me | - |
| 0162 | *n*-Pr | Et | Et | H | - |
| 0163 | *n*-Pr | Et | Et | Cl | - |
| 0164 | *n*-Pr | Et | Et | CN | - |
| 0165 | *n*-Pr | Et | Et | Me | - |
| 0166 | *n*-Pr | $-(CH_2)_3CH(Me)(CH_2)_3-$ | | H | - |
| 0167 | *n*-Pr | $-(CH_2)_3CH(Me)(CH_2)_3-$ | | Cl | - |
| 0168 | *n*-Pr | $-(CH_2)_3CH(Me)(CH_2)_3-$ | | CN | - |
| 0169 | *n*-Pr | $-(CH_2)_3CH(Me)(CH_2)_3-$ | | Me | - |
| 0170 | *n*-Bu | *iso*-Pr | H | H | - |
| 0171 | *n*-Bu | *iso*-Pr | H | Cl | - |
| 0172 | *n*-Bu | *iso*-Pr | H | CN | - |
| 0173 | *n*-Bu | *iso*-Pr | H | Me | - |
| 0174 | *n*-Bu | $CH_2CF_3$ | H | H | - |
| 0175 | *n*-Bu | $CH_2CF_3$ | H | Cl | - |
| 0176 | *n*-Bu | $CH_2CF_3$ | H | CN | - |
| 0177 | *n*-Bu | $CH_2CF_3$ | H | Me | - |
| 0178 | *n*-Bu | Et | Et | H | - |
| 0179 | *n*-Bu | Et | Et | Cl | - |
| 0180 | *n*-Bu | Et | Et | CN | - |
| 0181 | *n*-Bu | Et | Et | Me | - |
| 0182 | *n*-Bu | $-(CH_2)_3CH(Me)(CH_2)_3-$ | | H | - |
| 0183 | *n*-Bu | $-(CH_2)_3CH(Me)(CH_2)_3-$ | | Cl | - |
| 0184 | *n*-Bu | $-(CH_2)_3CH(Me)(CH_2)_3-$ | | CN | - |
| 0185 | *n*-Bu | $-(CH_2)_3CH(Me)(CH_2)_3-$ | | Me | - |
| 0186 | *sec*-Bu | H | H | H | - |
| 0187 | *sec*-Bu | H | H | Cl | - |
| 0188 | *sec*-Bu | H | H | CN | - |
| 0189 | *sec*-Bu | H | H | Me | - |
| 0190 | *sec*-Bu | Me | H | H | - |
| 0191 | *sec*-Bu | Me | H | Cl | - |
| 0192 | *sec*-Bu | Me | H | CN | - |
| 0193 | *sec*-Bu | Me | H | Me | - |
| 0194 | *sec*-Bu | Et | H | H | - |
| 0195 | *sec*-Bu | Et | H | Cl | - |
| 0196 | *sec*-Bu | Et | H | CN | - |
| 0197 | *sec*-Bu | Et | H | Me | - |

[Table 5]

| Compound No | R | $R^1$ | $R^2$ | X | Ym |
|---|---|---|---|---|---|
| 0198 | *sec*-Bu | *iso*-Pr | H | H | - |

(continued)

| Compound No | R | $R^1$ | $R^2$ | X | Ym |
|---|---|---|---|---|---|
| 0199 | *sec*-Bu | *iso*-Pr | H | F | - |
| 0200 | *sec*-Bu | *iso*-Pr | H | a | - |
| 0201 | *sec*-Bu | *iso*-Pr | H | Br | - |
| 0202 | *sec*-Bu | *iso*-Pr | H | I | - |
| 0203 | *sec*-Bu | *iso*-Pr | H | OH | - |
| 0204 | *sec*-Bu | *iso*-Pr | H | OMe | - |
| 0205 | *sec*-Bu | *iso*-Pr | H | OEt | - |
| 0206 | *sec*-Bu | *iso*-Pr | H | $OCH_3$*c*-Pr | - |
| 0307 | *sec*-Bu | *iso*-Pr | H | $OCHF_3$ | - |
| 0208 | *sec*-Bu | *iso*-Pr | H | $OCF_3$ | - |
| 0209 | *sec*-Bu | *iso*-Pr | H | $OCH_3CF_2$ | - |
| 0210 | *sec*-Bu | *iso*-Pr | H | $OCH_2CF_3$ | - |
| 0211 | *sec*-Bu | *iso*-Pr | H | SMe | - |
| 0212 | *sec*-Bu | *iso*-Pr | H | SOMe | - |
| 0213 | *sec*-Bu | *iso*-Pr | H | $SO_2Me$ | - |
| 0214 | *sec*-Bu | *iso*-Pr | H | $SCF_3$ | - |
| 0215 | *sec*-Bu | *iso*-Pr | H | $SOCF_3$ | - |
| 0216 | *sec*-Bu | *iso*-Pr | H | $SO_2CF_3$ | - |
| 0217 | *sec*-Bu | *iso*-Pr | H | $NH_3$ | - |
| 0218 | *sec*-Bu | *iso*-Pr | H | NHMe | - |
| 0219 | *sec*-Bu | *iso*-Pr | H | NH*iso*-Pr | - |
| 0220 | *sec*-Bu | *iso*-Pr | H | $N(Me)_2$ | - |
| 0221 | *sec*-Bu | *iso*-Pr | H | $N(Et)_3$ | - |
| 0222 | *sec*-Bu | *iso*-Pr | H | CN | - |
| 0223 | *sec*-Bu | *iso*-Pr | H | CHO | - |
| 0224 | *sec*-Bu | *iso*-Pr | H | COMe | - |
| 0225 | *sec*-Bu | *iso*-Pr | H | COEt | - |
| 0226 | *sec*-Bu | *iso*-Pr | H | $CO_2H$ | - |
| 0227 | *sec*-Bu | *iso*-Pr | H | COMe - | |
| 0228 | *sec*-Bu | *iso*-Pr | H | $CO_3Et$ | - |
| 0229 | *sec*-Bu | *iso*-Pr | H | $CONH_3$ | - |
| 0230 | *sec*-Bu | *iso*-Pr | H | CONHMe | - |
| 0231 | *sec*-Bu | *iso*-Pr | H | $CON(Me)_2$ | - |
| 0232 | *sec*-Bu | *iso*-Pr | H | Me | - |
| 0233 | *sec*-Bu | *iso*-Pr | H | Et | - |
| 0234 | *sec*-Bu | *iso*-Pr | H | *iso*-Pr | - |
| 0235 | *sec*-Bu | *iso*-Pr | H | *c*-Pr | - |
| 0236 | *sec*-Bu | *iso*-Pr | H | $CH_2F$ | - |
| 0237 | *sec*-Bu | *iso*-Pr | H | $CH_2Cl$ | - |
| 0238 | *sec*-Bu | *iso*-Pr | H | $CH_3Br$ | - |
| 0239 | *sec*-Bu | *iso*-Pr | H | $CHF_3$ | - |
| 0240 | *sec*-Bu | *iso*-Pr | H | $CF_3$ | - |
| 0241 | *sec*-Bu | *n*-Pr | H | H | - |
| 0242 | *sec*-Bu | *n*-Pr | H | Cl | - |
| 0243 | *sec*-Bu | *n*-Pr | H | CN | - |
| 0244 | *sec*-Bu | *n*-Pr | H | Me | - |
| 0245 | *sec*-Bu | *n*-Bu | H | H | - |
| 0246 | *sec*-Bu | *n*-Bu | H | Cl | - |
| 0247 | *sec*-Bu | *n*-Bu | H | CN | - |
| 0248 | *sec*-Bu | *n*-Bu | H | Me | - |

[Table 6]

| Compound No | R | $R^1$ | $R^2$ | X | Ym |
|---|---|---|---|---|---|
| 0249 | *sec*-Bu | *iso*-Bu | H | H | - |
| 0250 | *sec*-Bu | *iso*-Bu | H | Cl | - |
| 0251 | *sec*-Bu | *iso*-Bu | H | CN | - |
| 0252 | *sec*-Bu | *iso*-Bu | H | Me | - |
| 0253 | *sec*-Bu | *sec*-Bu | H | H | - |
| 0254 | *sec*-Bu | *sec*-Bu | H | a | - |
| 0255 | *sec*-Bu | *sec*-Bu | H | CN | - |
| 0256 | *sec*-Bu | *sec*-Bu | H | Me | - |
| 0257 | *sec*-Bu | *tert*-Bu | H | H | - |
| 0258 | *sec*-Bu | *tert*-Bu | H | F | - |
| 0259 | *sec*-Bu | *tert*-Bu | H | Cl | - |
| 0260 | *sec*-Bu | *tert*-Bu | H | Br | - |
| 0261 | *sec*-Bu | *tert*-Bu | H | I | - |
| 0262 | *sec*-Bu | *tert*-Bu | H | OH | - |
| 0263 | *sec*-Bu | *tert*-Bu | H | OMe | - |
| 0264 | *sec*-Bu | *tert*-Bu | H | OEt | - |
| 0265 | *sec*-Bu | *tert*-Bu | H | $OCH_2$*c*-Pr | - |
| 0266 | *sec*-Bu | *tert*-Bu | H | $OCHF_2$ | - |
| 0267 | *sec*-Bu | *tert*-Bu | H | $OCF_3$ | - |
| 0268 | *sec*-Bu | *tert*-Bu | H | $OCH_2CHF_3$ | - |
| 0269 | *sec*-Bu | *tert*-Bu | H | $OCH_2CF_3$ | - |
| 0270 | *sec*-Bu | *tert*-Bu | H | SMe | - |
| 0271 | *sec*-Bu | *tert*-Bu | H | SOMe | - |
| 0272 | *sec*-Bu | *tert*-Bu | H | $SO_2Me$ | - |
| 0273 | *sec*-Bu | *tert*-Bu | H | $SCF_3$ | - |
| 0274 | *sec*-Bu | *tert*-Bu | H | $SOCF_3$ | - |
| 0275 | *sec*-Bu | *tert*-Bu | H | $SOCF_3$ | - |
| 0276 | *sec*-Bu | *tert*-Bu | H | $NH_2$ | - |
| 0277 | *sec*-Bu | *tert*-Bu | H | NHMe | - |
| 0278 | *sec*-Bu | *tert*-Bu | H | NH*iso*-Pr | - |
| 0279 | *sec*-Bu | *tert*-Bu | H | $N(Me)_2$ | - |
| 0280 | *sec*-Bu | *tert*-Bu | H | $N(Et)_2$ | - |
| 0281 | *sec*-Bu | *tert*-Bu | H | CN | - |
| 0282 | *sec*-Bu | *tert*-Bu | H | CHO | - |
| 0283 | *sec*-Bu | *tert*-Bu | H | COMe | - |
| 0284 | *sec*-Bu | *tert*-Bu | H | COEt | - |
| 0285 | *sec*-Bu | *tert*-Bu | H | $CO_3H$ | - |
| 0286 | *sec*-Bu | *tert*-Bu | H | $CO_2Me$ - | |
| 0287 | *sec*-Bu | *tert*-Bu | H | $CO_3Et$ | - |
| 0288 | *sec*-Bu | *tert*-Bu | H | $CONH_3$ | - |
| 0289 | *sec*-Bu | *tert*-Bu | H | CONHMe | - |
| 0290 | *sec*-Bu | *tert*-Bu | H | $OON(Me)_2$ | - |
| 0291 | *sec*-Bu | *tert*-Bu | H | Me | - |
| 0292 | *sec*-Bu | *tert*-Bu | H | Et | - |
| 0293 | *sec*-Bu | *tert*-Bu | H | *iso*-Pr | - |
| 0294 | *sec*-Bu | *tert*-Bu | H | *c*-Pr | - |
| 0295 | *sec*-Bu | *tert*-Bu | H | $CH_3F$ | - |
| 0296 | *sec*-Bu | *tert*-Bu | H | $CH_2Cl$ | - |

(continued)

| Compound No | R | $R^1$ | $R^2$ | X | Ym |
|---|---|---|---|---|---|
| 0297 | *sec*-Bu | *tert*-Bu | H | $CH_3Br$ | - |
| 0298 | *sec*-Bu | *tert*-Bu | H | $CHF_2$ | - |
| 0299 | *sec*-Bu | *tert*-Bu | H | $CF_3$ | - |

[Table 7]

| Compound No | R | $R^1$ | $R^2$ | X | Ym |
|---|---|---|---|---|---|
| 0300 | *sec*-Bu | *n*-Pen | H | H | - |
| 0301 | *sec*-Bu | *n*-Pen | H | Cl | - |
| 0302 | *sec*-Bu | *n*-Pen | H | CN | - |
| 0303 | *sec*-Bu | *n*-Pen | H | Me | - |
| 0304 | *sec*-Bu | CH(Me)*n*-Pr | H | H | - |
| 0305 | *sec*-Bu | CH(Me)*n*-Pr | H | Cl | - |
| 0306 | *sec*-Bu | CH(Me)*n*-Pr | H | CN | - |
| 0307 | *sec*-Bu | CH(Me)*n*-Pr | H | Me | - |
| 0308 | *sec*-Bu | $CH(Et)_2$ | H | H | - |
| 0309 | *sec*-Bu | $CH(Et)_2$ | H | Cl | - |
| 0310 | *sec*-Bu | $CH(Et)_2$ | H | CN | - |
| 0311 | *sec*-Bu | $CH(Et)_2$ | H | Me | - |
| 0312 | *sec*-Bu | $CH(Me)CH(Me)_2$ | H | H | - |
| 0313 | *sec*-Bu | $CH(Me)CH(Me)_2$ | H | Cl | - |
| 0314 | *sec*-Bu | $CH(Me)CH(Me)_2$ | H | CN | - |
| 0315 | *sec*-Bu | $CH(Me)CH(Me)_2$ | H | Me | - |
| 0316 | *sec*-Bu | $CH_2C(Me)_3$ | H | H | - |
| 0317 | *sec*-Bu | $CH_2C(Me)_3$ | H | Cl | - |
| 0318 | *sec*-Bu | $CH_2C(Me)_3$ | H | CN | - |
| 0319 | *sec*-Bu | $CH_2C(Me)_3$ | H | Me | - |
| 0320 | *sec*-Bu | $CH(Me)C(Me)_3$ | H | H | - |
| 0321 | *sec*-Bu | $CH(Me)C(Me)_3$ | H | Cl | - |
| 0322 | *sec*-Bu | $CH(Me)C(Me)_3$ | H | CN | - |
| 0323 | *sec*-Bu | $CH(Me)C(Me)_3$ | H | Me | - |
| 0324 | *sec*-Bu | *n*-Hex | H | H | - |
| 0325 | *sec*-Bu | *n*-Hex | H | Cl | - |
| 0326 | *sec*-Bu | *n*-Hex | H | CN | - |
| 0327 | *sec*-Bu | *n*-Hex | H | Me | - |
| 0328 | *sec*-Bu | *c*-Pr | H | H | - |
| 0329 | *sec*-Bu | *c*-Pr | H | Cl | - |
| 0330 | *sec*-Bu | *c*-Pr | H | CN | - |
| 0331 | *sec*-Bu | *c*-Pr | H | Me | - |
| 0332 | *sec*-Bu | *c*-Pen | H | H | - |
| 0333 | *sec*-Bu | *c*-Pen | H | Cl | - |
| 0334 | *sec*-Bu | *c*-Pen | H | CN | - |
| 0335 | *sec*-Bu | *c*-Pen | H | Me | - |
| 0336 | *sec*-Bu | *c*-Hex | H | H | - |
| 0337 | *sec*-Bu | *c*-Hex | H | Cl | - |
| 0338 | *sec*-Bu | *c*-Hex | H | CN | - |
| 0339 | *sec*-Bu | *c*-Hex | H | Me | - |
| 0340 | *sec*-Bu | $CH_2CHF_3$ | H | H | - |

(continued)

| Compound No | R | $R^1$ | $R^2$ | X | Ym |
|---|---|---|---|---|---|
| 0341 | *sec*-Bu | $CH_2CHF_3$ | H | Cl | - |
| 0342 | *sec*-Bu | $CH_2CHF_3$ | H | CN | - |
| 0343 | *sec*-Bu | $CH_2CHF_3$ | H | Me | - |
| 0344 | *sec*-Bu | $CH_2CClF_3$ | H | H | - |
| 0345 | *sec*-Bu | $CH_2CClF_3$ | H | Cl | - |
| 0346 | *sec*-Bu | $CH_2CClF_3$ | H | CN | - |
| 0347 | *sec*-Bu | $CH_2CClF_3$ | H | Me | - |
| 0348 | *sec*-Bu | $CH_2CF_3$ | H | H | - |
| 0349 | *sec*-Bu | $CH_2CF_3$ | H | F | - |
| 0350 | *sec*-Bu | $CH_2CF_3$ | H | Cl | - |

[Table 8]

| Compound No | R | $R^1$ | $R^2$ | X | Ym |
|---|---|---|---|---|---|
| 0351 | *sec*-Bu | $CH_2CF_3$ | H | Br | - |
| 0352 | *sec*-Bu | $CH_2CF_3$ | H | I | - |
| 0353 | *sec*-Bu | $CH_2CF_3$ | H | OH | - |
| 0354 | *sec*-Bu | $CH_2CF_3$ | H | OMe | - |
| 0355 | *sec*-Bu | $CH_2CF_3$ | H | OEt | - |
| 0356 | *sec*-Bu | $CH_2CF_3$ | H | $OCH_3$*c*-Pr | - |
| 0357 | *sec*-Bu | $CH_2CF_3$ | H | $OCH_2Ph$ | - |
| 0358 | *sec*-Bu | $CH_2CF_3$ | H | $OCHF_3$ | - |
| 0359 | *sec*-Bu | $CH_2CF_3$ | H | $OCH_2CHF_2$ | - |
| 0360 | *sec*-Bu | $CH_2CF_3$ | H | $OCH_2CF_3$ | - |
| 0361 | *sec*-Bu | $CH_2CF_3$ | H | SMe | - |
| 0362 | *sec*-Bu | $CH_2CF_3$ | H | SOMe | - |
| 0363 | *sec*-Bu | $CH_2CF_3$ | H | $SO_3Me$ | - |
| 0364 | *sec*-Bu | $CH_2CF_3$ | H | $SCF_3$ | - |
| 0365 | *sec*-Bu | $CH_2CF_3$ | H | $OOCF_3$ | - |
| 0366 | *sec*-Bu | $CH_2CF_3$ | H | $SO_2CF_3$ | - |
| 0367 | *sec*-Bu | $CH_2CF_3$ | H | $NH_2$ | - |
| 0368 | *sec*-Bu | $CH_2CF_3$ | H | NHMe - | |
| 0369 | *sec*-Bu | $CH_2CF_3$ | H | NH*iso*-Pr | - |
| 0370 | *sec*-Bu | $CH_2CF_3$ | H | $N(Me)_2$ | - |
| 0371 | *sec*-Bu | $CH_2CF_3$ | H | $N(Et)_2$ | - |
| 0372 | *sec*-Bu | $CH_2CF_3$ | H | CN | - |
| 0373 | *sec*-Bu | $CH_2CF_3$ | H | CHO | - |
| 0374 | *sec*-Bu | $CH_2CF_3$ | H | COMe | - |
| 0375 | *sec*-Bu | $CH_2CF_3$ | H | COEt | - |
| 0376 | *sec*-Bu | $CH_2CF_3$ | H | $CO_3H$ | - |
| 0377 | *sec*-Bu | $CH_2CF_3$ | H | $CO_3ME$ - | |
| 0378 | *sec*-Bu | $CH_2CF_3$ | H | $CO_3Et$ | - |
| 0319 | *sec*-Bu | $CH_2CF_3$ | H | $OONH_2$ | - |
| 0380 | *sec*-Bu | $CH_2CF_3$ | H | CONHMe | - |
| 0381 | *sec*-Bu | $CH_2CF_3$ | H | $CON(Me)_2$ | - |
| 0382 | *sec*-Bu | $CH_2CF_3$ | H | CH=NOH | - |
| 0383 | *sec*-Bu | $CH_2CF_3$ | H | CH=NOMe | - |
| 0384 | *sec*-Bu | $CH_2CF_3$ | H | Me | - |

(continued)

| Compound No | R | $R^1$ | $R^2$ | X | Ym |
|---|---|---|---|---|---|
| 0385 | *sec*-Bu | $CH_2CF_3$ | H | Et | - |
| 0386 | *sec*-Bu | $CH_2CF_3$ | H | *iso*-Pr | - |
| 0387 | *sec*-Bu | $CH_2CF_3$ | H | *c*-Pr | - |
| 0388 | *sec*-Bu | $CH_2CF_3$ | H | $CH_3F$ | - |
| 0389 | *sec*-Bu | $CH_2CF_3$ | H | $CH_3Cl$ | - |
| 0390 | *sec*-Bu | $CH_2CF_3$ | H | $CH_2Br$ | - |
| 0391 | *sec*-Bu | $CH_2CF_3$ | H | $CHF_2$ | - |
| 0392 | *sec*-Bu | $CH_2CF_3$ | H | $CF_3$ | - |
| 0393 | *sec*-Bu | $CH_2CF_3$ | H | $CH_3OH$ | - |
| 0334 | *sec*-Bu | $CH_2CF_3$ | H | CH(OH)Me | - |
| 0396 | *sec*-Bu | $CH_2CF_3$ | H | $CH_3Me$ | - |
| 0396 | *sec*-Bu | $CH_2CF_3$ | H | CH(OMe)Me | - |
| 0397 | *sec*-Bu | $CH_2CF_3$ | H | Cl | 3-F |
| 0398 | *sec*-Bu | $CH_2CF_3$ | H | Cl | 3-Cl |
| 0399 | *sec*-Bu | $CH_2CF_3$ | H | Cl | 3-Br |
| 0400 | *sec*-Bu | $CH_2CF_3$ | H | Cl | 3-I |
| 0401 | *sec*-Bu | $CH_2CF_3$ | H | Cl | 3-Me |

[Table 9]

| Compound No | R | $R^1$ | $R^2$ | X | Ym |
|---|---|---|---|---|---|
| 0402 | *sec*-Bu | $CH_2CF_3$ | H | Cl | 3-Et |
| 0403 | *sec*-Bu | $CH_2CF_3$ | H | Cl | 3-*iso*-Pr |
| 0404 | *sec*-Bu | $CH_2CF_3$ | H | Cl | 3-*c*-Pr |
| 0405 | *sec*-Bu | $CH_2CF_3$ | H | Cl | 3-C≡CH |
| 0406 | *sec*-Bu | $CH_2CF_3$ | H | Cl | 3-$CH_3OH$ |
| 0407 | *sec*-Bu | $CH_2CF_3$ | H | Cl | 3-$CH_3OMe$ |
| 0408 | *sec*-Bu | $CH_2CF_3$ | H | Cl | 3-$CH_2Cl$ |
| 0409 | *sec*-Bu | $CH_2CF_3$ | H | Cl | 3-$CHF_2$ |
| 0410 | *sec*-Bu | $CH_2CF_3$ | H | Cl | 3-$CF_3$ |
| 0411 | *sec*-Bu | $CH_2CF_3$ | H | Cl | 3-OH |
| 0412 | *sec*-Bu | $CH_2CF_3$ | H | Cl | 3-OMe |
| 0413 | *sec*-Bu | $CH_2CF_3$ | H | a | 3-$OCHF_2$ |
| 0414 | *sec*-Bu | $CH_2CF_3$ | H | Cl | 3-SH |
| 0415 | *sec*-Bu | $CH_2CF_3$ | H | Cl | 3-SMe |
| 0416 | *sec*-Bu | $CH_2CF_3$ | H | Cl | 3-SOMe |
| 0417 | *sec*-Bu | $CH_2CF_3$ | H | Cl | 3-$SO_3Me$ |
| 0418 | *sec*-Bu | $CH_2CF_3$ | H | Cl | 3-$SCF_3$ |
| 0419 | *sec*-Bu | $CH_2CF_3$ | H | Cl | 3-$SOCF_3$ |
| 0420 | *sec*-Bu | $CH_2CF_3$ | H | Cl | 3-$SO_3CF_3$ |
| 0421 | *sec*-Bu | $CH_2CF_3$ | H | Cl | 3-$NO_2$ |
| 0422 | *sec*-Bu | $CH_2CF_3$ | H | Cl | 3-$NH_2$ |
| 0423 | *sec*-Bu | $CH_2CF_3$ | H | Cl | 3-NHMe |
| 0424 | *sec*-Bu | $CH_2CF_3$ | H | Cl | 3-$N(Me)_2$ |
| 0425 | *sec*-Bu | $CH_2CF_3$ | H | Cl | 3-CN |
| 0426 | *sec*-Bu | $CH_2CF_3$ | H | Cl | 3-CHO |
| 0427 | *sec*-Bu | $CH_2CF_3$ | H | Cl | 3-OOMe |
| 0428 | *sec*-Bu | $CH_2CF_3$ | H | Cl | 3-$CO_3H$ |

(continued)

| Compound No | R | $R^1$ | $R^2$ | X | Ym |
|---|---|---|---|---|---|
| 0429 | *sec*-Bu | $CH_2CF_3$ | H | Cl | 3-$CO_3$Et |
| 0430 | *sec*-Bu | $CH_2CF_3$ | H | Cl | 3-$CONH_3$ |
| 0431 | *sec*-Bu | $CH_2CF_3$ | H | Cl | 3-CONHMe |
| 0432 | *sec*-Bu | $CH_2CF_3$ | H | Cl | 3-CON$(Me)_3$ |
| 0433 | *sec*-Bu | $CH_2CF_3$ | H | Cl | 4-F |
| 0434 | *sec*-Bu | $CH_2CF_3$ | H | Cl | 4-Cl |
| 0435 | *sec*-Bu | $CH_2CF_3$ | H | Cl | 4-Br |
| 0436 | *sec*-Bu | $CH_2CF_3$ | H | Cl | 4-I |
| 0437 | *sec*-Bu | $CH_2CF_3$ | H | Cl | 4-Me |
| 0438 | *sec*-Bu | $CH_2CF_3$ | H | Cl | 4-$CH_3OH$ |
| 0439 | *sec*-Bu | $CH_2CF_3$ | H | Cl | 4-$CH_3$OMe |
| 0440 | *sec*-Bu | $CH_2CF_3$ | H | Cl | 4-$CH_3Cl$ |
| 0441 | *sec*-Bu | $CH_2CF_3$ | H | Cl | 4-$CHF_3$ |
| 0442 | *sec*-Bu | $CH_2CF_3$ | H | Cl | 4-$CF_3$ |
| 0443 | *sec*-Bu | $CH_2CF_3$ | H | Cl | 4-OH |
| 0444 | *sec*-Bu | $CH_2CF_3$ | H | Cl | 4-Me |
| 0445 | *sec*-Bu | $CH_2CF_3$ | H | Cl | 4-$OCHF_3$ |
| 0446 | *sec*-Bu | $CH_2CF_3$ | H | Cl | 4-SH |
| 0447 | *sec*-Bu | $CH_2CF_3$ | H | Cl | 4-SMe |
| 0448 | *sec*-Bu | $CH_2CF_3$ | H | Cl | 4-SOMe |
| 0449 | *sec*-Bu | $CH_2CF_3$ | H | Cl | 4-$SO_3$Me |
| 0450 | *sec*-Bu | $CH_2CF_3$ | H | Cl | 4-$SCF_3$ |
| 0451 | *sec*-Bu | $CH_2CF_3$ | H | Cl | 4-$SOCF_3$ |
| 0452 | *sec*-Bu | $CH_2CF_3$ | H | Cl | 4-$SO_2CF_3$ |

[Table 10]

| Compound No | R | $R^1$ | $R^3$ | X | Ym |
|---|---|---|---|---|---|
| 0453 | *sec*-Bu | $CH_3CF_3$ | H | Cl | 4-SCN |
| 0454 | *sec*-Bu | $CH_3CF_3$ | H | Cl | 4-$NO_3$ |
| 0455 | *sec*-Bu | $CH_3CF_3$ | H | Cl | 4-$NH_3$ |
| 0456 | *sec*-Bu | $CH_3CF_3$ | H | Cl | 4-NHMe |
| 0457 | *sec*-Bu | $CH_3CF_3$ | H | Cl | 4-N$(Me)_3$ |
| 0458 | *sec*-Bu | $CH_3CF_3$ | H | Cl | 4-CN |
| 0459 | *sec*-Bu | $CH_3CF_3$ | H | Cl | 4-CHO |
| 0460 | *sec*-Bu | $CH_3CF_3$ | H | Cl | 4-COMe |
| 0461 | *sec*-Bu | $CH_3CF_3$ | H | Cl | 4-$CO_3H$ |
| 0462 | *sec*-Bu | $CH_3CF_3$ | H | Cl | 4-$CO_3$Et |
| 0463 | *sec*-Bu | $CH_3CF_3$ | H | Cl | 4-$CONH_3$ |
| 0464 | *sec*-Bu | $CH_3CF_3$ | H | Cl | 4-CONHMe |
| 0465 | *sec*-Bu | $CH_3CF_3$ | H | Cl | 4-CON$(Me)_3$ |
| 0466 | *sec*-Bu | $CH_3CF_3$ | H | Cl | 5-Cl |
| 0467 | *sec*-Bu | $CH_3CF_3$ | H | Cl | 5-Me |
| 0468 | *sec*-Bu | $CH_3CF_3$ | H | Cl | 5-$CF_3$ |
| 0469 | *sec*-Bu | $CH_3CF_3$ | H | Cl | 5-OH |
| 0470 | *sec*-Bu | $CH_3CF_3$ | H | Cl | 5-OMe |
| 0471 | *sec*-Bu | $CH_3CF_3$ | H | Cl | 5-$OCHF_2$ |
| 0472 | *sec*-Bu | $CH_3CF_3$ | H | Cl | 5-$NH_2$ |

(continued)

| Compound No | R | $R^1$ | $R^3$ | X | Ym |
|---|---|---|---|---|---|
| 0473 | *sec*-Bu | $CH_3CF_3$ | H | Cl | 3-Me-4-F |
| 0474 | *sec*-Bu | $CH_3CF_3$ | H | Cl | 3-Me-4-Cl |
| 0475 | *sec*-Bu | $CH_3CF_3$ | H | Cl | 3,4-$(Me)_3$ |
| 0476 | *sec*-Bu | $CH_3CF_3$ | H | Cl | 3-Me-4-OH |
| 0477 | *sec*-Bu | $CH_3CF_3$ | H | Cl | 3-Me-4-OMe |
| 0478 | *sec*-Bu | $CH_3CF_3$ | H | Cl | 3-Me-4-$OCHF_3$, |
| 0479 | *sec*-Bu | $CH_3CF_3$ | H | Cl | 3-Et-4-Cl |
| 0480 | *sec*-Bu | $CH_3CF_3$ | H | Cl | 3-Et-4-OH |
| 0481 | *sec*-Bu | $CH_3CF_3$ | H | Cl | 3-*iso*-Pr-4-Cl |
| 0482 | *sec*-Bu | $CH_3CF_3$ | H | Cl | 3-*iso*-Pr-4-OH |
| 0483 | *sec*-Bu | $CH_2CH_3$ | H | Cl | 3-*c*-Pr-4-Cl |
| 0484 | *sec*-Bu | $CH_2CH_3$ | H | Cl | 3-*c*-Pr-4-OH |
| 0485 | *sec*-Bu | $CH_3CH_3$ | H | Cl | 3-Me-4-CN |
| 0486 | *sec*-Bu | $CH_3CH_3$ | H | Cl | 3-Et-4-CN |
| 0487 | *sec*-Bu | $CH_2CH_3$ | H | Cl | 3-*c*-Pr-4-CN |
| 0488 | *sec*-Bu | $CH_2CH_3$ | H | Cl | 3-$GF_3$-4-Cl |
| 0489 | *sec*-Bu | $CH_3CH_3$ | H | Cl | 3-$CF_3$-4-$CO_2$Et |
| 0490 | *sec*-Bu | $CH_3CH_3$ | H | Cl | 3-$CF_3$-4-$CO_2$H |
| 0491 | *sec*-Bu | $CH_3CH_3$ | H | Cl | 3-$CF_3$-4-$NH_2$ |
| 0492 | *sec*-Bu | $CH_3CH_3$ | H | Cl | 3-$CF_3$-4-$CONH_3$ |
| 0493 | *sec*-Bu | $CH_3CH_3$ | H | Cl | 3-$GF_3$-4-CONHMe |
| 0494 | *sec*-Bu | $CH_3CH_3$ | H | Cl | 3-$CF_3$-4-$CON(Me)_2$ |
| 0495 | *sec*-Bu | $CH_3CH_3$ | H | Cl | 3-$CF_3$-4-Me |
| 0496 | *sec*-Bu | $CH_3CH_3$ | H | Cl | 3-$CF_3$-4-Cl |
| 0497 | *sec*-Bu | $CH_3CH_3$ | H | Cl | 3-$CF_3$-4-CN |
| 0498 | *sec*-Bu | $CH_3CH_3$ | H | Cl | 3-$CF_3$-4-$CO_3$Me |
| 0499 | *sec*-Bu | $CH_3CH_3$ | H | Cl | 3-$CF_3$-4-$CO_3$H |
| 0500 | *sec*-Bu | $CH_2CH_3$ | H | Cl | 3-$CF_3$-4-$CONH_2$ |
| 0501 | *sec*-Bu | $CH_3CH_3$ | H | Cl | 3-$GF_3$-4-CONHMe |
| 0503 | *sec*-Bu | $CH_3CH_3$ | H | Cl | 3-$CF_3$-4-$CON(Me)_2$ |
| 0503 | *sec*-Bu | $CH_3CH_3$ | H | Cl | 3-$CF_3$-4-$NH_2$ |

[Table 11]

| Compound No | R | $R^1$ | $R^2$ | X | Ym |
|---|---|---|---|---|---|
| 0504 | *sec*-Bu | $CH_2OF_3$ | H | Cl | 3-$NH_3$-4-Br |
| 0505 | *sec*-Bu | $CH_2CF_3$ | H | Cl | 3-$NH_3$-4-Cl |
| 0506 | *sec*-Bu | $CH_2CF_3$ | H | Cl | 3$NH_2$-4-F |
| 0507 | *sec*-Bu | $CH_2CF_3$ | H | Cl | 3-Cl-4-Br |
| 0508 | *sec*-Bu | $CH_2CF_3$ | H | Cl | 3,4-$Br_3$ |
| 0509 | *sec*-Bu | $CH_2CF_3$ | H | Cl | 3,4-$Cl_2$ |
| 0510 | *sec*-Bu | $CH_2CF_3$ | H | Cl | 3-$NH_3$-4-$CO_3$Et |
| 0511 | *sec*-Bu | $CH_2CF_3$ | H | Cl | 3-Cl-4-$CO_3$Et |
| 0512 | *sec*-Bu | $CH_2CF_3$ | H | Cl | 3-$NH_3$-4-$CO_3$H |
| 0513 | *sec*-Bu | $CH_2CF_3$ | H | Cl | 3-Cl-4-$CO_3$H |
| 0514 | *sec*-Bu | $CH_2CF_3$ | H | Cl | 3,4-$(NH_2)_2$ |
| 0515 | *sec*-Bu | $CH_2CF_3$ | H | Cl | 3-Cl-4-$NH_2$ |
| 0516 | *sec*-Bu | $CH_2CF_3$ | H | Cl | 3-$NH_2$-4-$CONH_3$ |

(continued)

| Compound No | R | $R^1$ | $R^2$ | X | Ym |
|---|---|---|---|---|---|
| 0517 | *sec*-Bu | $CH_2CF_3$ | H | Cl | 3-Cl-4-$CONH_3$ |
| 0518 | *sec*-Bu | $CH_2CF_3$ | H | Cl | 3-$NH_2$-4-CONHMe |
| 0519 | *sec*-Bu | $CH_2CF_3$ | H | Cl | 3-Cl-4-CONHMe |
| 0520 | *sec*-Bu | $CH_2CF_3$ | H | Cl | 3-$NH_2$-4-CON$(Me)_3$ |
| 0521 | *sec*-Bu | $CH_2CF_3$ | H | Cl | 3-Cl-4-CON$(Me)_3$ |
| 0522 | *sec*-Bu | $CH_2CF_3$ | H | Cl | 3-$CO_3$Et-4-Cl |
| 0523 | *sec*-Bu | $CH_2CF_3$ | H | Cl | 3-$CO_3$H-4-Cl |
| 0524 | *sec*-Bu | $CH_2CF_3$ | H | Cl | 3-$CONH_3$-4-Cl |
| 0525 | *sec*-Bu | $CH_2CF_3$ | H | Cl | 3-CONHMe-4-Cl |
| 0526 | *sec*-Bu | $CH_2CF_3$ | H | Cl | 3-CON$(Me)_3$-4-Cl |
| 0697 | *sec*-Bu | $CH_2CF_3$ | H | Cl | 3-CN-4-Cl |
| 0528 | *sec*-Bu | $CH_2CF_3$ | H | Cl | 3-$NH_3$-4-CN |
| 0529 | *sec*-Bu | $CH_2CF_3$ | H | Cl | 3-Cl-4-CN |
| 0530 | *sec*-Bu | $CH_2CF_3$ | H | Cl | 3-CN-4-CN |
| 0531 | *sec*-Bu | $CH_2CF_3$ | H | Cl | 3-SMe-4-CN |
| 0532 | *sec*-Bu | $CH_2CF_3$ | H | Cl | 3-SOMe-4-CN |
| 0533 | *sec*-Bu | $CH_2CF_3$ | H | Cl | 3-$SO_3$Me-4-CN |
| 0534 | *sec*-Bu | $CH_2CF_3$ | H | Cl | 3-SH-4-CN |
| 0535 | *sec*-Bu | $CH_2CF_3$ | H | Cl | 3-$SCF_3$-4-CN |
| 0536 | *sec*-Bu | $CH_2CF_3$ | H | Cl | 3,5-$(Me)_2$ |
| 0537 | *sec*-Bu | $CH_2CF_3$ | H | Cl | 3-Me-5-OH |
| 0538 | *sec*-Bu | $CH_2CF_3$ | H | Cl | 3-Me-5-OMe |
| 0539 | *sec*-Bu | $CH_2CF_3$ | H | Cl | 3-Me-5-$OCHF_2$ |
| 0540 | *sec*-Bu | $CH_2CF_3$ | H | Cl | 3-OH-5-Me |
| 0541 | *sec*-Bu | $CH_2CF_3$ | H | Cl | 3-OMe-5-Me |
| 0542 | *sec*-Bu | $CH_2CF_3$ | H | Cl | 3-$OCHF_2$-5-Me |
| 0543 | *sec*-Bu | $CH_2CF_3$ | H | Cl | 3-Et-5-OH |
| 0544 | *sec*-Bu | $CH_2CF_3$ | H | Cl | 3-*c*-Pr-5-OH |
| 0545 | *sec*-Bu | $CH_2CF_3$ | H | Cl | 3-OH-5-Et |
| 0546 | *sec*-Bu | $CH_2CF_3$ | H | Cl | 3-OH-5-*c*-Pr |
| 0547 | *sec*-Bu | $CH_2CF_3$ | H | Cl | 3-Me-5-$NH_2$ |
| 0548 | *sec*-Bu | $CH_2CF_3$ | H | Cl | 3-Me-5-$CO_2$Et |
| 0549 | *sec*-Bu | $CH_2CF_3$ | H | Cl | 3-Et-5-$CO_2$Et |
| 0550 | *sec*-Bu | $CH_2CF_3$ | H | Cl | 3-Me-5-Cl |
| 0551 | *sec*-Bu | $CH_2CF_3$ | H | Cl | 3-Et-5-$NH_2$ |
| 0552 | *sec*-Bu | $CH_2CF_3$ | H | Cl | 3-Et-5-Cl |
| 0553 | *sec*-Bu | $CH_3CF_3$ | H | Cl | 3-*c*-Pr-5-$NH_2$ |
| 0554 | *sec*-Bu | $CH_3CF_3$ | H | Cl | 3-*c*-Pr-5-Cl |

[Table 12]

| Compound No | R | $R^1$ | $R^2$ | X | Ym |
|---|---|---|---|---|---|
| 0555 | *sec*-Bu | $CH_3CF_3$ | H | Cl | 3-$CF_3$-5-OH |
| 0556 | *sec*-Bu | $CH_3CF_3$ | H | Cl | 3-$CF_3$-5-OMe |
| 0557 | *sec*-Bu | $CH_3CF_3$ | H | Cl | 3-$CF_3$-5-$OCHF_3$ |
| 0558 | *sec*-Bu | $CH_3CF_3$ | H | Cl | 3-$CF_3$-5-$NH_3$ |
| 0559 | *sec*-Bu | $CH_3CF_3$ | H | Cl | 3-$CO_3$Et-5-OH |

(continued)

| Compound No | R | $R^1$ | $R^2$ | X | Ym |
|---|---|---|---|---|---|
| 0560 | *sec*-Bu | $CH_3CF_3$ | H | Cl | 3-$CO_3$Et-5-OMe |
| 0561 | *sec*-Bu | $CH_3CF_3$ | H | Cl | 3-$CO_3$Et-5-$NH_3$ |
| 0562 | *sec*-Bu | $CH_3CF_3$ | H | Cl | 3-$CO_4$Et-5-Cl |
| 0563 | *sec*-Bu | $CH_3CF_3$ | H | Cl | 3-$CO_2$H-5-$NH_3$ |
| 0564 | *sec*-Bu | $CH_3CF_3$ | H | Cl | 3-$CO_3$H-5-Cl |
| 0565 | *sec*-Bu | $CH_3CF_3$ | H | Cl | 3-$CONH_2$-5-$NH_3$ |
| 0566 | *sec*-Bu | $CH_3CF_3$ | H | Cl | 3-$CONH_3$-5-Cl |
| 0567 | *sec*-Bu | $CH_3CF_3$ | H | Cl | 3-CONHMe-5-$NH_3$ |
| 0568 | *sec*-Bu | $CH_3CF_3$ | H | Cl | 3-CONHMe-5-Cl |
| 0569 | *sec*-Bu | $CH_3CF_3$ | H | Cl | 3-$CON(Me)_2$-5-$NH_3$ |
| 0570 | *sec*-Bu | $CH_3CF_3$ | H | Cl | 3-$CON(Me)_2$-5-Cl |
| 0571 | *sec*-Bu | $CH_3CF_3$ | H | Cl | 3-CN-5-$NH_2$ |
| 0572 | *sec*-Bu | $CH_3CF_3$ | H | Cl | 3-CN-5-Cl |
| 0573 | *sec*-Bu | $CH_3CF_3$ | H | Cl | 3,5-$(NH_3)_3$ |
| 0574 | *sec*-Bu | $CH_3CF_3$ | H | Cl | 3-$CF_3$-5-SH |
| 0575 | *sec*-Bu | $CH_3CF_3$ | H | Cl | 3-$CF_3$-5-SMe |
| 0576 | *sec*-Bu | $CH_3CF_3$ | H | Cl | 3-SMe-5-$NH_3$ |
| 0577 | *sec*-Bu | $CH_3CF_3$ | H | Cl | 3-SOMe-5-$NH_3$ |
| 0578 | *sec*-Bu | $CH_3CF_3$ | H | Cl | 3-$SO_3$Me-5-$NH_3$ |
| 0579 | *sec*-Bu | $CH_3CF_3$ | H | Cl | 3-SMe-5-Cl |
| 0580 | *sec*-Bu | $CH_3CF_3$ | H | Cl | 3-SOMe-5-Cl |
| 0581 | *sec*-Bu | $CH_3CF_3$ | H | Cl | 3-$SO_3$Me-6-Cl |
| 0582 | *sec*-Bu | $CH_3CF_3$ | H | Cl | 4-Me-5-OH |
| 0583 | *sec*-Bu | $CH_3CF_3$ | H | Cl | 4-Me-5-OMe |
| 0584 | *sec*-Bu | $CH_3CF_3$ | H | Cl | 4-Me-5-$OCHF_3$ |
| 0585 | *sec*-Bu | $CH_3CF_3$ | H | Cl | 4-Et-5-OH |
| 0586 | *sec*-Bu | $CH_3CF_3$ | H | Cl | 4-*c*-Pr-5-OH |
| 0587 | *sec*-Bu | $CH_3CF_3$ | H | Cl | 4-$CO_3$Et-5-Me |
| 0588 | *sec*-Bu | $CH_3CF_3$ | H | Cl | 4-$CO_3$H-5-Me |
| 0589 | *sec*-Bu | $CH_3CF_3$ | H | Cl | 4-$NH_3$-5-Me |
| 0690 | *sec*-Bu | $CH_3CF_3$ | H | Cl | 4-Cl-5-Me |
| 0591 | *sec*-Bu | $CH_3CF_3$ | H | Cl | 4-$CONH_3$-5-Me |
| 0592 | *sec*-Bu | $CH_3CF_3$ | H | Cl | 4-CONHMe-5-Me |
| 0593 | *sec*-Bu | $CH_3CF_3$ | H | Cl | 4-$CON(Me)_3$-5-Me |
| 0594 | *sec*-Bu | $CH_3CF_3$ | H | Cl | 4-$CO_3$-Et-5-$CF_3$ |
| 0595 | *sec*-Bu | $CH_3CF_3$ | H | Cl | 4-$CO_3$-H-5-$CF_3$ |
| 0596 | *sec*-Bu | $CH_3CF_3$ | H | Cl | 4-Cl-5-$CF_3$ |
| 0597 | *sec*-Bu | $CH_3CF_3$ | H | Cl | 4-$NH_3$-5-$CF_3$ |

(continued)

| Compound No | R | $R^1$ | $R^2$ | X | Ym |
|---|---|---|---|---|---|
| 0598 | *sec*-Bu | $CH_3CF_3$ | H | Cl | 4-$CONH_3$-5-$CF_3$ |
| 0599 | *sec*-Bu | $CH_3CF_3$ | H | Cl | 4-CONHMe-5-$CF_3$ |
| 0600 | *sec*-Bu | $CH_3CF_3$ | H | Cl | 4-$CONMe_3$-5-$CF_3$ |
| 0601 | *sec*-Bu | $CH_3CF_3$ | H | Cl | 4-$CO_3$Et-5-$NH_3$ |
| 0602 | *sec*-Bu | $CH_3CF_3$ | H | Cl | 4-$CO_3$Et-5-Cl |
| 0603 | *sec*-Bu | $CH_3CF_3$ | H | Cl | 4-$CO_3$H-5-$NH_3$ |
| 0604 | *sec*-Bu | $CH_3CF_3$ | H | Cl | 4-$CO_3$H-5-Cl |
| 0605 | *sec*-Bu | $CH_3CF_3$ | H | Cl | 4-$CONH_3$-5-$NH_3$ |

[Table 13]

| Componund No | R | $R^1$ | $R^3$ | X | Ym |
|---|---|---|---|---|---|
| 0606 | *sec*-Bu | $CH_2CF_3$ | H | Cl | 4-$CONH_3$-5-Cl |
| 0607 | *sec*-Bu | $CH_2CF_3$ | H | Cl | 4-CONHMe-5-$NH_3$ |
| 0608 | *sec*-Bu | $CH_3CF_3$ | H | Cl | 4-CONHMe-5-Cl |
| 0609 | *sec*-Bu | $CH_3CF_3$ | H | Cl | 4-$CON(Me)_3$-5-$NH_3$ |
| 0610 | *sec*-Bu | $CH_2CF_3$ | H | Cl | 4-$CON(Me)_3$-5-Cl |
| 0611 | *sec*-Bu | $CH_3CF_3$ | H | Cl | 4-F-5-$NH_3$ |
| 0612 | *sec*-Bu | $CH_3CF_3$ | H | Cl | 4-Cl-5-$NH_3$ |
| 0613 | *sec*-Bu | $CH_3CF_3$ | H | Cl | 4,5-$Cl_3$ |
| 0614 | *sec*-Bu | $CH_3CF_3$ | H | Cl | 4-CN-5-$NH_2$ |
| 0615 | *sec*-Bu | $CH_3CF_3$ | H | Cl | 4-CN-5-Cl |
| 0616 | *sec*-Bu | $CH_3CF_3$ | H | Cl | 4-CHO-5-$NH_3$ |
| 0617 | *sec*-Bu | $CH_3CF_3$ | H | Cl | 4-CHO-5-Cl |
| 0618 | *sec*-Bu | $CH_3CF_3$ | H | Cl | 4-$CHF_3$-5-$NH_2$ |
| 0619 | *sec*-Bu | $CH_3CF_3$ | H | Cl | 4-$CHF_3$-5-Cl |
| 0620 | *sec*-Bu | $CH_2CF_3$ | H | Cl | 4-$CH_3OH$-5-$NH_3$ |
| 0621 | *sec*-Bu | $CH_3CF_3$ | H | Cl | 4-$CH_3OH$-5-Cl |
| 0622 | *sec*-Bu | $CH_3CF_3$ | H | Cl | 4-$CH_3$-Cl-5-$NH_3$ |
| 0623 | *sec*-Bu | $CH_3CF_3$ | H | Cl | 4-$CH_3$-Cl-5-Cl |
| 0624 | *sec*-Bu | $CH_3CF_3$ | H | Cl | 4-$CH_3OMe$-5-$NH_3$ |
| 0625 | *sec*-Bu | $CH_3CF_3$ | H | Cl | 4-$CH_3OMe$-5-Cl |
| 0626 | *sec*-Bu | $CH_3CF_3$ | H | Cl | 4-$NO_3$-5-$NH_3$ |
| 0627 | *sec*-Bu | $CH_3CF_3$ | H | Cl | 4-$NO_3$-5-Cl |
| 0628 | *sec*-Bu | $CH_3CF_3$ | H | Cl | 4-SCN-5-$NH_3$ |
| 0629 | *sec*-Bu | $CH_3CF_3$ | H | Cl | 4-SCN-5-Cl |
| 0630 | *sec*-Bu | $CH_3CF_3$ | H | Cl | 4-SH-5-$NH_3$ |
| 0631 | *sec*-Bu | $CH_3CF_3$ | H | Cl | 4-SH-5-Cl |

(continued)

| Componund No | R | $R^1$ | $R^3$ | X | Ym |
|---|---|---|---|---|---|
| 0632 | *sec*-Bu | $CH_3CF_3$ | H | Cl | 4-SMe-5-$NH_2$ |
| 0633 | *sec*-Bu | $CH_3CF_3$ | H | Cl | 4-SMe-5-Cl |
| 0634 | *sec*-Bu | $CH_3CF_3$ | H | Cl | 4-$SCF_3$-5-$NH_3$ |
| 0635 | *sec*-Bu | $CH_3CF_3$ | H | Cl | 4-$SCF_3$-5-Cl |
| 0636 | *sec*-Bu | $CH_3CF_3$ | H | Cl | 3-Me-4-Cl-5-$NH_3$ |
| 0637 | *sec*-Bu | $CH_3CF_3$ | H | Cl | 3-Me-4-F-5-$NH_3$ |
| 0638 | *sec*-Bu | $CH_3CF_3$ | H | Cl | 3-Me-4-Cl-5-Cl |
| 0639 | *sec*-Bu | $CH_3CF_3$ | H | Cl | 3-Et-4-Cl-5-$NH_3$ |
| 0640 | *sec*-Bu | $CH_3CF_3$ | H | Cl | 3-Et-4-Cl-5-Cl |
| 0641 | *sec*-Bu | $CH_3CF_3$ | H | Cl | 3-*c*-Pr-4-Cl-5-$NH_2$ |
| 0642 | *sec*-Bu | $CH_3CF_3$ | H | Cl | 3-*c*-Pr-4-Cl-5-Cl |
| 0643 | *sec*-Bu | $CH_3CF_3$ | H | Cl | 3-Me-4-CHO-5-OH |
| 0644 | *sec*-Bu | $CH_3CF_3$ | H | Cl | -Me-4-CHO-5-Cl |
| 0645 | *sec*-Bu | $CH_3CF_3$ | H | Cl | 3-Me-4-CHO-5-F |
| 0646 | *sec*-Bu | $CH_3CF_3$ | H | Cl | 3-Me-4-CH=NOH-5-Cl |
| 0647 | *sec*-Bu | $CH_3CF_3$ | H | Cl | -Me-4-CH=NOH-5-F |
| 0648 | *sec*-Bu | $CH_3CF_3$ | H | Cl | 3-Me-4-CH=NOMe-5-Cl |
| 0649 | *sec*-Bu | $CH_3CF_3$ | H | Cl | 3-Me-4-CH=NOMe-5-F |
| 0650 | *sec*-Bu | $CH_2CF_3$ | H | Cl | 3-Me-4-CN-5-$NH_3$ |
| 0651 | *sec*-Bu | $CH_2CF_3$ | H | Cl | 3-Me-4-CN-5-Cl |
| 0652 | *sec*-Bu | $CH_3CF_3$ | H | Cl | 3-Et-4-CN-5-$NH_3$ |
| 0653 | *sec*-Bu | $CH_3CF_3$ | H | Cl | 3-Et-4-CN-5-Cl |
| 0654 | *sec*-Bu | $CH_3CF_3$ | H | Cl | 3-Me-4-Cl-5-$CO_2$Et |
| 0655 | *sec*-Bu | $CH_3CF_3$ | H | Cl | 3-Et-4-Cl-5-$CO_2$Et |
| 0656 | *sec*-Bu | $CH_3CF_3$ | H | Cl | 3-*c*-Pr-4-CN-5-$NH_3$ |

[Table 14]

| Compound No | R | $R^1$ | $R^2$ | X | Ym |
|---|---|---|---|---|---|
| 0657 | *sec*-Bu | $CH_2CF_3$ | H | Cl | 3-*c*-Pr-4-CN-5-Cl |
| 0658 | *sec*-Bu | $CH_3CF_3$ | H | Cl | 3-$CF_3$-4-Me-5-$NH_2$ |
| 0659 | *sec*-Bu | $CH_2CF_3$ | H | Cl | 3-$CF_3$-4-Me-5-Cl |
| 0660 | *sec*-Bu | $CH_2CF_3$ | H | Cl | 3-$CF_3$-4-Cl-5-$NH_2$ |
| 0661 | *sec*-Bu | $CH_3CF_3$ | H | Cl | 3-$CF_3$-4,5-$Cl_2$ |
| 0662 | *sec*-Bu | $CH_3CF_3$ | H | Cl | 3-$CF_3$-4CHO-5-OH |
| 0663 | *sec*-Bu | $CH_2CF_3$ | H | Cl | 3-$CF_3$-4-CHO-5-Cl |
| 0664 | *sec*-Bu | $CH_2CF_3$ | H | Cl | 3-$CF_3$-4-CHO-5-F |
| 0665 | *sec*-Bu | $CH_2CF_3$ | H | Cl | 3-$CF_3$-4-CH=NOH-5-Cl |

(continued)

| Compound No | R | $R^1$ | $R^2$ | X | Ym |
|---|---|---|---|---|---|
| 0666 | *sec*-Bu | $CH_2CF_3$ | H | Cl | $3-CF_3-4-CH=NOH-5-F$ |
| 0667 | *sec*-Bu | $CH_2CF_3$ | H | Cl | $3-CF_3-4-CH=NOMe-5-Cl$ |
| 0668 | *sec*-Bu | $CH_2CF_3$ | H | Cl | $3-CF_3-4-CH=NOMe-5-F$ |
| 0669 | *sec*-Bu | $CH_2CF_3$ | H | Cl | $3-CF_3-4-CN-5-NH_2$ |
| 0670 | *sec*-Bu | $CH_2CF_3$ | H | Cl | $3-CF_3-4-CN-5-Cl$ |
| 0671 | *sec*-Bu | $CH_2CF_3$ | H | Cl | $3-CF_3-4-CO_2Me-5-NH_2$ |
| 0672 | *sec*-Bu | $CH_2CF_3$ | H | Cl | $3-CF_3-4-CN-5-F$ |
| 0673 | *sec*-Bu | $CH_2CF_3$ | H | Cl | $3-CF_3-4-CO_3Me-5-Cl$ |
| 0674 | *sec*-Bu | $CH_2CF_3$ | H | Cl | $3-CF_3-4-CO_2H-5-NH_2$ |
| 0675 | *sec*-Bu | $CH_2CF_3$ | H | Cl | $3-CF_3-4-CO_2H-5-Cl$ |
| 0676 | *sec*-Bu | $CH_2CF_3$ | H | Cl | $3-CF_3-4-CONH_2-5-NH_2$ |
| 0677 | *sec*-Bu | $CH_2CF_3$ | H | Cl | $3-CF_3-4-CONH_2-5-Cl$ |
| 0678 | *sec*-Bu | $CH_2CF_3$ | H | Cl | $3-CF_3-4-CONHMe-5-NH_2$ |
| 0679 | *sec*-Bu | $CH_2CF_3$ | H | Cl | $3-CF_3-4-CONHMe-5-Cl$ |
| 0680 | *sec*--Bu | $CH_2CF_3$ | H | Cl | $3-CF_3-4-CON(Me)_2-5-Cl$ |
| 0681 | *sec*-Bu | $CH_2CF_3$ | H | Cl | $3-CF_3-4-CON(Me)_2-5-Cl$ |
| 0682 | *sec*-Bu | $CH_2CF_3$ | H | Cl | $3-CF_3-4,5-(NH_3)_2$ |
| 0683 | *sec*--Bu | $CH_2CF_3$ | H | Cl | $3-CF_3-4-NH_3-5-Cl$ |
| 0684 | *sec*-Bu | $CH_2CF_3$ | H | Cl | $3-CO_2Et-4-Cl-5-NH_2$ |
| 0685 | *sec*-Bu | $CH_2CF_3$ | H | Cl | $3-CO_2Et-4,5-Cl_2$ |
| 0686 | *sec*-Bu | $CH_2CF_3$ | H | Cl | $3-CO_3H-4-Cl-5-NH_3$ |
| 0687 | *sec*-Bu | $CH_2CF_3$ | H | Cl | $3-CO_2H-4,5-Cl_2$ |
| 0688 | *sec*-Bu | $CH_2CF_3$ | H | Cl | $3-CONH_3-4-Cl-5-NH_2$ |
| 0689 | *sec*-Bu | $CH_2CF_3$ | H | Cl | $3-CONH_3-4,5-Cl_2$ |
| 0690 | *sec*-Bu | $CH_2CF_3$ | H | Cl | $3-CONHMe-4-Cl-5-NH_2$ |
| 0691 | *sec*-Bu | $CH_2CF_3$ | H | Cl | $3-CONHMe-4,5-Cl_2$ |
| 0692 | *sec*-Bu | $CH_2CF_3$ | H | Cl | $3-CON(Me)_2-4-Cl-5-NH_2$ |
| 0693 | *sec*-Bu | $CH_2CF_3$ | H | Cl | $3-CON(Me)_2-4,5-Cl_2$ |
| 0694 | *sec*-Bu | $CH_2CF_3$ | H | Cl | $3-CN-4-Cl-5-NH_2$ |
| 0695 | *sec*-Bu | $CH_2CF_3$ | H | Cl | $3-CN-4,5-Cl_2$ |
| 0696 | *sec*-Bu | $CH_2CF_3$ | H | Cl | $3.5-(CH_3)_3-4-Cl$ |
| 0697 | *sec*-Bu | $CH_2CF_3$ | H | Cl | $3,4,5-Cl_3$ |
| 0698 | *sec*-Bu | $CH_2CF_3$ | H | Cl | $3-SMe4CN5-NH_2$ |
| 0699 | *sec*-Bu | $CH_2CF_3$ | H | Cl | 3-SMe-4-CN-5-Cl |
| 0700 | *sec*-Bu | $CH_2CF_3$ | H | Cl | $3-SOMe-4-CN-5-NH_2$ |
| 0701 | *sec*-Bu | $CH_2CF_3$ | H | Cl | 3-SOMe-4-CN-5-Cl |
| 0702 | *sec*-Bu | $CH_2CF_3$ | H | Cl | $3-SO_2Me-4-CN-5-NH_3$ |
| 0703 | *sec*-Bu | $CH_2CF_3$ | H | Cl | $3-SO_2Me-4-CN-5-Cl$ |

(continued)

| Compound No | R | $R^1$ | $R^2$ | X | Ym |
|---|---|---|---|---|---|
| 0704 | *sec*-Bu | $CH_2CF_3$ | H | Cl | 3-SH-4-CN-5-NHE |
| 0705 | *sec*-Ru | $CH_2CF_3$ | H | Cl | 3-SH-4-CN-5-Cl |
| 0706 | *sec*-Bu | $CH_2CF_3$ | H | Cl | 3-$SCF_3$-4-CN-5-$NH_2$ |
| 0707 | *sec*-Bu | $CH_2CF_3$ | H | Cl | 3-$SCF_3$-4-CN-5-Cl |

[Table 15]

| Compound No | R | $R^1$ | $R^2$ | X | Ym |
|---|---|---|---|---|---|
| 0708 | *sec*-Bu | $CH(Me)CF_3$ | H | H | - |
| 0709 | *sec*-Bu | $CH(Me)CF_3$ | H | F | - |
| 0710 | *sec*-Bu | $CH(Me)CF_3$ | H | Cl | - |
| 0711 | *sec*-Bu | $CH(Me)CF_3$ | H | Br | - |
| 0712 | *sec*-Bu | $CH(Me)CF_3$ | H | I | - |
| 0713 | *sec*-Bu | $CH(Me)CF_3$ | H | OH | - |
| 0714 | *sec*-Bu | $CH(Me)CF_3$ | H | OMe | - |
| 0715 | *sec*-Bu | $CH(Me)CF_3$ | H | OEt | - |
| 0716 | *sec*-Bu | $CH(Me)CF_3$ | H | $OCH_3$c-Pr | - |
| 0717 | *sec*-Bu | $CH(Me)CF_3$ | H | $OCHF_2$ | - |
| 0718 | *sec*-Bu | $CH(Me)CF_3$ | H | $OCF_3$ | - |
| 0719 | *sec*-Bu | $CH(Me)CF_3$ | H | $OCH_2CF_3$ | - |
| 0720 | *sec*-Bu | $CH(Me)CF_3$ | H | $OCH_2CF_3$ | - |
| 0721 | *sec*-Bu | $CH(Me)CF_3$ | H | SMe | - |
| 0722 | *sec*-Bu | $CH(Me)CF_3$ | H | SOMe | - |
| 0723 | *sec*-Bu | $CH(Me)CF_3$ | H | $SO_2Me$ | - |
| 0724 | *sec*-Bu | $CH(Me)CF_3$ | H | $SCF_3$ | - |
| 0725 | *sec*-Bu | $CH(Me)CF_3$ | H | $SOCF_3$ | - |
| 0726 | *sec*-Bu | $CH(Me)CF_3$ | H | $SO_2CF_3$ | - |
| 0727 | *sec*-Bu | $CH(Me)CF_3$ | H | $NH_2$ | - |
| 0728 | *sec*-Bu | $CH(Me)CF_3$ | H | NHMe | - |
| 0729 | *sec*-Bu | $CH(Me)CF_3$ | H | NHiso-Pr | - |
| 0730 | *sec*-Bu | $CH(Me)CF_3$ | H | $N(Me)_3$ | - |
| 0731 | *sec*-Bu | $GH(Me)CF_3$ | H | $N(Et)_2$ | - |
| 0732 | *sec*-Bu | $CH(Me)CF_3$ | H | CN | - |
| 0733 | *sec*-Bu | $CH(Me)CF_3$ | H | CHO | - |
| 0734 | *sec*-Bu | $CH(Me)CF_3$ | H | OOMe | - |
| 0735 | *sec*-Bu | $CH(Me)CF_3$ | H | COEt | - |
| 0736 | *sec*-Bu | $CH(Me)CF_3$ | H | $CO_3H$ | - |
| 0737 | *sec*-Bu | $CH(Me)CF_3$ | H | $CC_2Me$ | - |

(continued)

| Compound No | R | $R^1$ | $R^2$ | X | Ym |
|---|---|---|---|---|---|
| 0738 | *sec*-Bu | $CH(Me)CF_3$ | H | $CO_2Et$ | - |
| 0739 | *sec*-Bu | $CH(Me)CF_3$ | H | $CONH_2$ | - |
| 0740 | *sec*-Bu | $CH(Me)CF_3$ | H | CONHMe | - |
| 0741 | *sec*-Bu | $CH(Me)CF_3$ | H | $CON(Me)_2$ | - |
| 0742 | *sec*-Bu | $CH(Me)CF_3$ | H | Me | - |
| 0743 | *sec*-Bu | $CH(Me)CF_3$ | H | Et | - |
| 0744 | *sec*-Bu | $CH(Me)CF_3$ | H | *iso*-Pr | - |
| 0745 | *sec*-Bu | $CH(Me)CF_3$ | H | *c*-Pr | - |
| 0746 | *sec*-Bu | $CH(Me)CF_3$ | H | $CH_2F$ | - |
| 0747 | *sec*-Bu | $CH(Me)CF_3$ | H | $CH_2Cl$ | - |
| 0748 | *sec*-Bu | $CH(Me)CF_3$ | H | $CH_2Br$ | - |
| 0749 | *sec*-Bu | $CH(Me)CF_3$ | H | $CHF_3$ | - |
| 0750 | *sec*-Bu | $CH(Me)CF_3$ | H | $CF_3$ | - |
| 0751 | *sec*-Bu | $CH(Me)CF_3$ | H | H | - |
| 0752 | *sec*-Bu | $GH(Et)CF_3$ | H | F | - |
| 0753 | *sec*-Bu | $CH(Et)CF_3$ | H | Cl | - |
| 0754 | *sec*-Bu | $CH(Et)CF_3$ | H | Br | - |
| 0755 | *sec*-Bu | $CH(Et)CF_3$ | H | OMe | - |
| 0756 | *sec*-Bu | $CH(Et)CF_3$ | H | CN | - |
| 0757 | *sec*-Bu | $CH(Et)CF_3$ | H | Me | - |
| 0758 | *sec*-Bu | $CH(Et)CF_3$ | H | $CF_3$ | - |

[Table 16]

| Compound | R | $R^1$ | $R^2$ | X | Ym |
|---|---|---|---|---|---|
| 0759 | *sec*-Bu | $CH_2CH_2CF_3$ | H | H | - |
| 0760 | *sec*-Bu | $CH_2CH_2CF_3$ | H | Cl | - |
| 0761 | *sec*-Bu | $CH_2CH_2CF_3$ | H | CN | - |
| 0762 | *sec*-Bu | $CH_2CH_2CF_3$ | H | Me | - |
| 0763 | *sec*-Bu | $CH_3CH_3CF_3$ | H | H | - |
| 0764 | *sec*-Bu | $CH_3CH_3CF_3$ | H | Cl | - |
| 0765 | *sec*-Bu | $CH_3CH_3CF_3$ | H | CN | - |
| 0766 | *sec*-Bu | $CH_3CH_3CF_3$ | H | Me | - |
| 0767 | *sec*-Bu | $CH_3CH(Me)CF_3$ | H | H | - |
| 0768 | *sec*-Bu | $CH_3CH(Me)CF_3$ | H | Cl | - |
| 0769 | *sec*-Bu | $CH_3CH(Me)CF_3$ | H | CN | - |
| 0770 | *sec*-Bu | $CH_3CH(Me)CF_3$ | H | Me | - |
| 0771 | *sec*-Bu | $CH_3CH_3CF_3$ | H | H | - |

(continued)

| Compound | R | $R^1$ | $R^2$ | X | Ym |
|---|---|---|---|---|---|
| 0772 | *sec*-Bu | $CH_3CH_3CF_3$ | H | Cl | - |
| 0773 | *sec*-Bu | $CH_3CH_3CF_3$ | H | CN | - |
| 0774 | *sec*-Bu | $CH_3CH_3CF_3$ | H | Me | - |
| 0775 | *sec*-Bu | $CH_3$*c*-Pr | H | H | - |
| 0776 | *sec*-Bu | $CH_3$*c*-Pr | H | Cl | - |
| 0777 | *sec*-Bu | $CH_3$*c*-Pr | H | CN | - |
| 0778 | *sec*-Bu | $CH_3$*c*-Pr | H | Me | - |
| 0779 | *sec*-Bu | $CH_2$*c*-Pen | H | H | - |
| 0780 | *sec*-Bu | $CH_2$*c*-Pen | H | Cl | - |
| 0781 | *sec*-Bu | $CH_2$*c*-Pen | H | CN | - |
| 0782 | *sec*-Bu | $CH_2$*c*-Pen | H | Me | - |
| 0783 | *sec*-Bu | $CH_2$*c*-Hex | H | H | - |
| 0784 | *sec*-Bu | $CH_2$*c*-Hex | H | Cl | - |
| 0785 | *sec*-Bu | $CH_2$*c*-Hex | H | CN | - |
| 0786 | *sec*-Bu | $CH_2$*c*-Hex | H | Me | - |
| 0787 | *sec*-Bu | $CH_2$*c*-Hex | H | H | - |
| 0788 | *sec*-Bu | $CH_2$*c*-Hex | H | Cl | - |
| 0789 | *sec*-Bu | $CH_3CH_3OH$ | H | CN | - |
| 0790 | *sec*-Bu | $CH_3CH_3OH$ | H | Me | - |
| 0791 | *sec*-Bu | $CH_3CH_3OMe$ | H | H | - |
| 0792 | *sec*-Bu | $CH_3CH_3OMe$ | H | Cl | - |
| 0793 | *sec*-Bu | $CH_3CH_3OMe$ | H | CN | - |
| 0794 | *sec*-Bu | $CH_3CH_3OMe$ | H | Me | - |
| 0795 | *sec*-Bu | $CH_3CH_3OEt$ | H | H | - |
| 0796 | *sec*-Bu | $CH_3CH_3OEt$ | H | Cl | - |
| 0797 | *sec*-Bu | $CH_3CH_3OEt$ | H | CN | - |
| 0798 | *sec*-Bu | $CH_3CH_3OEt$ | H | Me | - |
| 0799 | *sec*-Bu | $CH_3CH_3CH_3OMe$ | H | H | - |
| 0800 | *sec*-Bu | $CH_2CH_2CH_2OMe$ | H | Cl | - |
| 0801 | *sec*-Bu | $CH_2CH_2CH_2OMe$ | H | CN | - |
| 0802 | *sec*-Bu | $CH_2CH_2CH_2OMe$ | H | Me | - |
| 0803 | *sec*-Bu | $CH_2CH_2NHMe$ | H | H | - |
| 0804 | *sec*-Bu | $CH_2CH_2NHMe$ | H | Cl | - |
| 0805 | *sec*-Bu | $CH_2CH_2NHMe$ | H | CN | - |
| 0806 | *sec*-Bu | $CH_2CH_2NHMe$ | H | Me | - |
| 0807 | *sec*-Bu | $CH_2CH_2N(Me)_2$ | H | H | - |
| 0808 | *sec*-Bu | $CH_2CH_2N(Me)_2$ | H | Cl | - |
| 0809 | *sec*-Bu | $CH_2CH_2N(Me)_2$ | H | CN | - |

[Table 17]

| Compound No | R | $R^1$ | $R^2$ | X | Ym |
|---|---|---|---|---|---|
| 0810 | *sec*-Bu | $CH_2CH_2N(Me)_2$ | H | Me | - |
| 0811 | *sec*-Bu | $CH_2CH_2SMe$ | H | H | - |
| 0812 | *sec*-Bu | $CH_2CH_2SMe$ | H | Cl | - |
| 0813 | *sec*-Bu | $CH_2CH_2SMe$ | H | CN | - |
| 0814 | *sec*-Bu | $CH_2CH_2SMe$ | H | Me | - |
| 0815 | *sec*-Bu | $CH_2CH_2SOMe$ | H | H | - |
| 0816 | *sec*-Bu | $CH_2CH_2SOMe$ | H | Cl | - |
| 0817 | *sec*-Bu | $CH_2CH_2SOMe$ | H | CN | - |
| 0818 | *sec*-Bu | $CH_2CH_2SOMe$ | H | Me | - |
| 0819 | *sec*-Bu | $CH_2CH_2SO_2Me$ | H | H | - |
| 0820 | *sec*-Bu | $CH_2CH_2SO_2Me$ | H | Cl | - |
| 0821 | *sec*-Bu | $CH_2CH_2SO_2Me$ | H | CN | - |
| 0822 | *sec*-Bu | $CH_2CH_2SO_2Me$ | H | Me | - |
| 0823 | *sec*-Bu | $CH_2CN$ | H | H | - |
| 0824 | *sec*-Bu | $CH_2CN$ | H | Cl | - |
| 0825 | *sec*-Bu | $CH_2CN$ | H | CN | - |
| 0826 | *sec*-Bu | $CH_2CN$ | H | Me | - |
| 0827 | *sec*-Bu | $CH_2CH_2CN$ | H | H | - |
| 0828 | *sec*-Bu | $CH_2CH_2CN$ | H | Cl | - |
| 0829 | *sec*-Bu | $CH_2CH_2CN$ | H | CN | - |
| 0830 | *sec*-Bu | $CH_2CH_2CN$ | H | Ne | - |
| 0831 | *sec*-Bu | $CH_2COMe$ | H | H | - |
| 0832 | *sec*-Bu | $CH_2COMe$ | H | Cl | - |
| 0833 | *sec*-Bu | $CH_2COMe$ | H | CN | - |
| 0834 | *sec*-Bu | $CH_2COMe$ | H | Me | - |
| 0835 | *sec*-Bu | $CH_2CO_2Et$ | H | H | - |
| 0836 | *sec*-Bu | $CH_2CO_2Et$ | H | Cl | - |
| 0837 | *sec*-Bu | $CH_2CO_2Et$ | H | CN | - |
| 0838 | *sec*-Bu | $CH_2CO_2Et$ | H | Me | - |
| 0839 | *sec*-Bu | $CH(Me)CO_2Et$ | H | H | - |
| 0840 | *sec*-Bu | $CH(Me)CO_2Et$ | H | Cl | - |
| 0841 | *sec*-Bu | $CH(Me)CO_2Et$ | H | CN | - |
| 0842 | *sec*-Bu | $CH(Me)CO_2Et$ | H | Me | - |
| 0843 | *sec*-Bu | CH(*iso*-Pr)$CO_2Et$ | H | H | - |
| 0844 | *sec*-Bu | CH(*iso*-Pr)$CO_2Et$ | H | Cl | - |
| 0845 | *sec*-Bu | CH(*iso*-Pr)$CO_2Et$ | H | CN | - |
| 0846 | *sec*-Bu | CH(*iso*-Pr)$CO_2Et$ | H | Me | - |
| 0847 | *sec*-Bu | $CH_2CONH_2$ | H | H | - |
| 0848 | *sec*-Bu | $CH_2CONH_2$ | H | Cl | - |
| 0849 | *sec*-Bu | $CH_2CONH_2$ | H | CN | - |
| 0850 | *sec*-Bu | $CH_2CONH_2$ | H | Me | - |
| 0851 | *sec*-Bu | $CH_2CONHMe$ | H | H | - |
| 0852 | *sec*-Bu | $CH_2CONHMe$ | H | Cl | - |
| 0853 | *sec*-Bu | $CH_2CONHMe$ | H | CN | - |
| 0854 | *sec*-Bu | $CH_2CONHMe$ | H | Me | - |
| 0855 | *sec*-Bu | $CH_2CON(Me)_3$ | H | H | - |
| 0856 | *sec*-Bu | $CH_2CON(Me)_3$ | H | Cl | - |
| 0857 | *sec*-Bu | $CH_2CON(Me)_2$ | H | CN | - |

(continued)

| Compound No | R | $R^1$ | $R^2$ | X | Ym |
|---|---|---|---|---|---|
| 0858 | *sec*-Bu | $CH_2CON(Me)_2$ | H | Me | - |
| 0859 | *sec*-Bu | $CH_2CH=CH_2$ | H | H | - |
| 0860 | *sec*-Bu | $CH_2CH=CH_2$ | H | Cl | - |

[Table 18]

| Compound No | R | $R^1$ | $R^2$ | X | Ym |
|---|---|---|---|---|---|
| 0861 | *sec*-Bu | $CH_2CH=CH_2$ | H | CN | - |
| 0862 | *sec*-Bu | $CH_2CH=CH_2$ | H | Me | - |
| 0863 | *sec*-Bu | $CH_2C(Me)=CH_2$ | H | H | - |
| 0864 | *sec*-Bu | $CH_2C(Me)=CH_2$ | H | Cl | - |
| 0865 | *sec*-Bu | $CH_2C(Me)=CH_2$ | H | CN | - |
| 0866 | *sec*-Bu | $CH_2C(Me)=CH_2$ | H | Me | - |
| 0887 | *sec*-Bu | $CH_2C≡CH$ | H | H | - |
| 0868 | *sec*-Bu | $CH_2C≡CH$ | H | Cl | - |
| 0869 | *sec*-Bu | $CH_2C≡CH$ | H | CN | - |
| 0870 | *sec*-Bu | $CH_2C≡CH$ | H | Me | - |
| 0871 | *sec*-Bu | COMe | H | H | - |
| 0872 | *sec*-Bu | COMe | H | Cl | - |
| 0873 | *sec*-Bu | COMe | H | CN | - |
| 0874 | *sec*-Bu | COMe | H | Me | - |
| 0875 | *sec*-Bu | $CO_2Me$ | H | H | - |
| 0876 | *sec*-Bu | $CO_2Me$ | H | Cl | - |
| 0877 | *sec*-Bu | $CO_2Me$ | H | CN | - |
| 0878 | *sec*-Bu | $CO_2Me$ | H | Me | - |
| 0879 | *sec*-Bu | $SO_2Me$ | H | H | - |
| 0880 | *sec*-Bu | $SO_2Me$ | H | Cl | - |
| 0881 | *sec*-Bu | $SO_2Me$ | H | CN | - |
| 0882 | *sec*-Bu | $SO_2Me$ | H | Me | - |
| 0883 | *sec*-Bu | $SO_3CH_3$ | H | H | - |
| 0884 | *sec*-Bu | $SO_3CH_3$ | H | Cl | - |
| 0885 | *sec*-Bu | $SO_3CH_3$ | H | CN | - |
| 0886 | *sec*-Bu | $SO_3CH_3$ | H | Me | - |
| 0887 | *sec*-Bu | $SO_3CH_3$ | H | H | - |
| 0888 | *sec*-Bu | $SO_3CH_3$ | H | Cl | - |
| 0889 | *sec*-Bu | $SO_3CH_3$ | H | CN | - |
| 0890 | *sec*-Bu | $SO_3CH_3$ | H | Me | - |
| 0891 | *sec*-Bu | $SO_3NHMe$ | H | H | - |
| 0892 | *sec*-Bu | $SO_3NHMe$ | H | Cl | - |
| 0893 | *sec*-Bu | $SO_3NHMe$ | H | CN | - |
| 0894 | *sec*-Bu | $SO_3NHMe$ | H | Me | - |
| 0896 | *sec*-Bu | $SO_3N(Me)_3$ | H | H | - |
| 0896 | *sec*-Bu | $SO_3N(Me)_3$ | H | Cl | - |
| 0897 | *sec*-Bu | $SO_3N(Me)_3$ | H | CN | - |
| 0898 | *sec*-Bu | $SO_3N(Me)_3$ | H | Me | - |
| 0899 | *sec*-Bu | OH | H | Cl | - |
| 0900 | *sec*-Bu | OMe | H | Cl | - |
| 0901 | *sec*-Bu | OEt | H | Cl | - |

(continued)

| Compound No | R | $R^1$ | $R^2$ | X | Ym |
|---|---|---|---|---|---|
| 0902 | *sec*-Bu | $OCH_3C_H=CH_2$ | H | Cl | - |
| 0903 | *sec*-Bu | $CH_2Ph$ | H | H | - |
| 0904 | *sec*-Bu | $CH_2Ph$ | H | Cl | - |
| 0905 | *sec*-Bu | $CH_2Si(Me)_3$ | H | H | - |
| 0906 | *sec*-Bu | $CH_2Si(Me)_3$ | H | Cl | - |
| 0907 | *sec*-Bu | Me | Me | H | - |
| 0908 | *sec*-Bu | Me | Me | Cl | - |
| 0909 | *sec*-Bu | Me | Me | CN | - |
| 0910 | *sec*-Bu | Me | Me | Me | - |
| 0911 | *sec*-Bu | Me | COMe | Cl | - |

[Table 19]

| Compound No | R | $R^1$ | $R^2$ | X | Ym |
|---|---|---|---|---|---|
| 0912 | *sec*-Bu | Me | COOMe | Cl | - |
| 0913 | *sec*-Bu | Me | $SO_2Me$ | Cl | - |
| 0914 | *sec*-Bu | Me | $SO_2CF_3$ | Cl | - |
| 0915 | *sec*-Bu | Et | Me | H | - |
| 0916 | *sec*-Bu | Et | Me | Cl | - |
| 0917 | *sec*-Bu | Et | Me | CN | - |
| 0918 | *sec*-Bu | Et | Me | Me | - |
| 0919 | *sec*-Bu | Et | Et | H | - |
| 0920 | *sec*-Bu | Et | Et | F | - |
| 0921 | *sec*-Bu | Et | Et | Cl | - |
| 0922 | *sec*-Bu | Et | Et | Br | - |
| 0923 | *sec*-Bu | Et | Et | I | - |
| 0924 | *sec*-Bu | Et | Et | OH | - |
| 0925 | *sec*-Bu | Et | Et | OMe | - |
| 0926 | *sec*-Bu | Et | Et | OEt | - |
| 0927 | *sec*-Bu | Et | Et | $OCH_3$*c*-Pr | - |
| 0928 | *sec*-Bu | Et | Et | $OCHF_3$ | - |
| 0929 | *sec*-Bu | Et | Et | $OCF_3$ | - |
| 0930 | *sec*-Bu | Et | Et | $OCH_2CHF_3$ | - |
| 0931 | *sec*-Bu | Et | Et | $OCH_2CF_3$ | - |
| 0932 | *sec*-Bu | Et | Et | SMe | - |
| 0933 | *sec*-Bu | Et | Et | SOMe | - |
| 0934 | *sec*-Bu | Et | Et | $SO_2Me$ | - |
| 0935 | *sec*-Bu | Et | Et | $SCF_3$ | - |
| 0936 | *sec*-Bu | Et | Et | $SOCF_3$ | - |
| 0937 | *sec*-Bu | Et | Et | $SO_2CF_3$ | - |
| 0938 | *sec*-Bu | Et | Et | $NH_2$ | - |
| 0939 | *sec*-Bu | Et | Et | NHMe | - |
| 0940 | *sec*-Bu | Et | Et | NHM*iso*-Pr | - |
| 0941 | *sec*-Bu | Et | Et | $N(Me)_2$ | - |
| 0942 | *sec*-Bu | Et | Et | $N(Et)_3$ | - |
| 0943 | *sec*-Bu | Et | Et | CN | - |
| 0944 | *sec*-Bu | Et | Et | CHO | - |
| 0945 | *sec*-Bu | Et | Et | COMe | - |

(continued)

| Compound No | R | $R^1$ | $R^2$ | X | Ym |
|---|---|---|---|---|---|
| 0946 | *sec*-Bu | Et | Et | COEt | - |
| 0947 | *sec*-Bu | Et | Et | $CO_2H$ | - |
| 0948 | *sec*-Bu | Et | Et | $CO_3Me$ | - |
| 0949 | *sec*-Bu | Et | Et | $CO_2Et$ | - |
| 0950 | *sec*-Bu | Et | Et | $CONH_2$ | - |
| 0951 | *sec*-Bu | Et | Et | CONHMe | - |
| 0952 | *sec*-Bu | Et | Et | $CON(Me)_2$ | - |
| 0953 | *sec*-Bu | Et | Et | Me | - |
| 0954 | *sec*-Bu | Et | Et | Et | - |
| 0955 | *sec*-Bu | Et | Et | *iso*-Pr | - |
| 0956 | *sec*-Bu | Et | Et | *c*-Pr | - |
| 0957 | *sec*-Bu | Et | Et | $CH_2F$ | - |
| 0958 | *sec*-Bu | Et | Et | $CH_2Cl$ | - |
| 0959 | *sec*-Bu | Et | Et | $CH_2Br$ | - |
| 0960 | *sec*-Bu | Et | Et | $CHF_2$ | - |
| 0961 | *sec*-Bu | Et | Et | $CF_3$ | - |
| 0962 | *sec*-Bu | n-Pr | Me | H | - |

[Table 20]

| Compound No | R | $R^1$ | $R^2$ | X | Ym |
|---|---|---|---|---|---|
| 0963 | *sec*-Bu | *n*-Pr | Me | Cl | - |
| 0964 | *sec*-Bu | *n*-Pr | Me | CN | - |
| 0965 | *sec*-Bu | *n*-Pr | Me | Me | - |
| 0966 | *sec*-Bu | *n*-Pr | Et | H | - |
| 0967 | *sec*-Bu | *n*-Pr | Et | Cl | - |
| 0968 | *sec*-Bu | *n*-Pr | Et | CN | - |
| 0969 | *sec*-Bu | *n*-Pr | Et | Me | - |
| 0970 | *sec*-Bu | *n*-Pr | *n*-Pr | H | - |
| 0971 | *sec*-Bu | *n*-Pr | *n*-Pr | Cl | - |
| 0972 | *sec*-Bu | *n*-Pr | *n*-Pr | CN | - |
| 0973 | *sec*-Bu | *n*-Pr | *n*-Pr | Me | - |
| 0974 | *sec*-Bu | *iso*-Pr | Me | H | - |
| 0975 | *sec*-Bu | *iso*-Pr | Me | Cl | - |
| 0976 | *sec*-Bu | *iso*-Pr | Me | CN | - |
| 0977 | *sec*-Bu | *iso*-Pr | Me | Me | - |
| 0978 | *sec*-Bu | *iso*-Pr | Et | H | - |
| 0979 | *sec*-Bu | *iso*-Pr | Et | Cl | - |
| 0980 | *sec*-Bu | *iso*-Pr | Et | CN | - |
| 0981 | *sec*-Bu | *iso*-Pr | Et | Me | - |
| 0982 | *sec*-Bu | *iso*-Pr | *iso*-Pr | H | - |
| 0983 | *sec*-Bu | *iso*-Pr | *iso*-Pr | Cl | - |
| 0984 | *sec*-Bu | *iso*-Pr | *iso*-Pr | CN | - |
| 0985 | *sec*-Bu | *iso*-Pr | *iso*-Pr | Me | - |
| 0986 | *sec*-Bu | *n*-Bu | Me | H | - |
| 0987 | *sec*-Bu | *n*-Bu | Me | Cl | - |
| 0988 | *sec*-Bu | *n*-Bu | Me | CN | - |
| 0989 | *sec*-Bu | *n*-Bu | Me | Me | - |

(continued)

| Compound No | R | $R^1$ | $R^2$ | X | Ym |
|---|---|---|---|---|---|
| 0990 | *sec*-Bu | *n*-Bu | Et | H | - |
| 0991 | *sec*-Bu | *n*-Bu | Et | Cl | - |
| 0992 | *sec*-Bu | *n*-Bu | Et | CN | - |
| 0993 | *sec*-Bu | *n*-Bu | Et | Me | - |
| 0994 | *sec*-Bu | *iso*-Bu | Me | H | - |
| 0995 | *sec*-Bu | *iso*-Bu | Me | Cl | - |
| 0996 | *sec*-Bu | *iso*-Bu | Me | CN | - |
| 0997 | *sec*-Bu | *iso*-Bu | Me | Me | - |
| 0998 | *sec*-Bu | $CH_3CF_3$ | Me | H | - |
| 0999 | *sec*-Bu | $CH_3CF_3$ | Me | F | - |
| 1000 | *sec*-Bu | $CH_3CF_3$ | Me | Cl | - |
| 1001 | *sec*-Bu | $CH_3CF_3$ | Me | Br | - |
| 1002 | *sec*-Bu | $CH_3CF_3$ | Me | OMe | - |
| 1003 | *sec*-Bu | $CH_3CF_3$ | Me | CN | - |
| 1004 | *sec*-Bu | $CH_3CF_3$ | Me | Me | - |
| 1005 | *sec*-Bu | $CH_3CF_3$ | Me | CF3 | - |
| 1006 | *sec*-Bu | $CH_3CF_3$ | Et | H | - |
| 1007 | *sec*-Bu | $CH_3CF_3$ | Et | F | - |
| 1008 | *sec*-Bu | $CH_3CH_3$ | Et | Cl | - |
| 1009 | *sec*-Bu | $CH_3CF_3$ | Et | Br | - |
| 1010 | *sec*-Bu | $CH_3CF_3$ | Et | OMe | - |
| 1011 | *sec*-Bu | $CH_3CF_3$ | Et | CN | - |
| 1012 | *sec*-Bu | $CH_3CF_3$ | Et | Me | - |
| 1013 | *sec*-Bu | $CH_3CF_3$ | Et | $CF_3$ | - |

[Table 21]

| Compound No | R | $R^1$ | $R^2$ | X | Ym |
|---|---|---|---|---|---|
| 1014 | *sec*-Bu | $CH_3CF_3$ | $CH_2OMe$ | H | - |
| 1015 | *sec*-Bu | $CH_3CF_3$ | $CH_3OMe$ | Cl | - |
| 1016 | *sec*-Bu | $CH_3CF_3$ | $CH_2OMe$ | CN | - |
| 1017 | *sec*-Bu | $CH_2CF_3$ | $CH_2OMe$ | Me | - |
| 1018 | *sec*-Bu | $CH_2CF_3$ | $CH_2O(Me){=}CH_2$ | H | - |
| 1019 | *sec*-Bu | $CH_2CF_3$ | $CH_2C(Me){=}CH_2$ | Cl | - |
| 1020 | *sec*-Bu | $CH_2CF_3$ | $CH_2C(Me){=}CH_2$ | CN | - |
| 1021 | *sec*-Bu | $CH_2CF_3$ | $CH_2O(Me){=}CH_2$, | Me | - |
| 1022 | *sec*-Bu | $CH_2CF_3$ | COMe | H | - |
| 1023 | *sec*-Bu | $CH_2CF_3$ | COMe | Cl | - |
| 1024 | *sec*-Bu | $CH_2CF_3$ | COMe | CN | - |
| 1025 | *sec*-Bu | $CH_2CF_3$ | COMe | Me | - |
| 1026 | *sec*-Bu | $CH_2CF_3$ | $CO_2$*tert*-Bu | H | - |
| 1027 | *sec*-Bu | $CH_2CF_3$ | $CO_2$*tert*-Bu | Cl | - |
| 1028 | *sec*-Bu | $CH_2CF_3$ | $CO_2$*tert*-Bu | CN | - |
| 1029 | *sec*-Bu | $CH_2CF_3$ | $CO_2$*tert*-Bu | Me | - |
| 1030 | *sec*-Bu | $CH_2CF_3$ | $SO_2Me$ | H | - |
| 1031 | *sec*-Bu | $CH_3CF_3$ | $SO_2ME$ | Cl | - |
| 1032 | *sec*-Bu | $CH_2F_3$ | $SO_3Me$ | CN | - |
| 1033 | *sec*-Bu | $CH_2CF_3$ | $SO_2Me$ | Me | - |

(continued)

| Compound No | R | $R^1$ | $R^2$ | X | Ym |
|---|---|---|---|---|---|
| 1034 | *sec*-Bu | $CH_2CF_3$ | $SO_2CF_3$ | H | - |
| 1035 | *sec*-Bu | $CH_2CF_3$ | $SO_2CF_3$ | Cl | - |
| 1036 | *sec*-Bu | $CH_2CF_3$ | $SO_2CF_3$ | CN | - |
| 1037 | *sec*-Bu | $CH_2CF_3$ | $SO_2CF_3$ | Me | - |
| 1038 | *sec*-Bu | $CH(Me)CF_3$ | Me | H | - |
| 1039 | *sec*-Bu | $CH(Me)CF_3$ | Me | Cl | - |
| 1040 | *sec*-Bu | $CH(Me)CF_3$ | Me | CN | - |
| 1041 | *sec*-Bu | $CH(Me)CF_3$ | Me | Me | - |
| 1042 | *sec*-Bu | $CH(Me)CF_3$ | Et | H | - |
| 1043 | *sec*-Bu | $CH(Me)CF_3$ | Et | Cl | - |
| 1044 | *sec*-Bu | $CH(Me)CF_3$ | Et | CN | - |
| 1045 | *sec*-Bu | $CH(Me)CF_3$ | Et | Me | - |
| 1046 | *sec*-Bu | $CH(Me)CF_3$ | $CH_2C(Me)=CH_2$ | H | - |
| 1047 | *sec*-Bu | $CH(Me)CF_3$ | $CH_2C(Me)=CH_2$ | Cl | - |
| 1048 | *sec*-Bu | $CH(Me)CF_3$ | $CH_2(Me)=CH_2$ | CN | - |
| 1049 | *sec*-Bu | $CH(Me)CF_3$ | $CH_2C(Me)=CH_2$ | Me | - |
| 1050 | *sec*-Bu | $CH(Me)CF_3$ | COMe | H | - |
| 1051 | *sec*-Bu | $CH(Me)CF_3$ | COMe | Cl | - |
| 1052 | *sec*-Bu | $CH(Me)CF_3$ | COMe | CN | - |
| 1053 | *sec*-Bu | $CH(Me)CF_3$ | COMe | Me | - |
| 1054 | *sec*-Bu | $CH(Me)CF_3$ | COO*tert*-Bu | H | - |
| 1055 | *sec*-Bu | $CH(Me)CF_3$ | COO*tert*-Bu | Cl | - |
| 1056 | *sec*-Bu | $CH(Me)CF_3$ | COO*tert*-Bu | CN | - |
| 1057 | *sec*-Bu | $CH(Me)CF_3$ | COO*tert*-Bu | Me | - |
| 1058 | *sec*-Bu | $CH_2CH=CH_2$ | Me | Cl | - |
| 1059 | *sec*-Bu | $CH_3CH=CH_2$ | Et | Cl | - |
| 1060 | *sec*-Bu | $CH_2C(Me)=CH_2$ | Me | Cl | - |
| 1061 | *sec*-Bu | $CH_2C(Me)=CH_3$ | Et | Cl | - |
| 1062 | *sec*-Bu | $CH_3C{\equiv}CH$ | Me | Cl | - |
| 1063 | *sec*-Bu | $CH_3{\equiv}CH$ | Et | Cl | - |
| 1064 | *sec*-Bu | $CH_2CH_2OMe$ | Me | H | - |

[Table 22]

| Compound No | R | $R^1$ | $R^2$ | X | Ym |
|---|---|---|---|---|---|
| 1065 | *sec*-Bu | $CH_3CH_2OMe$ | Me | Cl | - |
| 1066 | *sec*-Bu | $CH_2CH_2OEt$ | Et | H | - |
| 1067 | *sec*-Bu | $CH_2CH_2OEt$ | Et | Cl | - |
| 1068 | *sec*-Bu | $CH_2CN$ | Me | Cl | - |
| 1069 | *sec*-Bu | $CH_2CN$ | Et | Cl | - |
| 1070 | *sec*-Bu | $CH_3CH_2CN$ | Me | H | - |
| 1071 | *sec*-Bu | $CH_2CH_2CN$ | Me | Cl | - |
| 1072 | *sec*-Bu | $CH_2CH_2CN$ | Et | H | - |
| 1073 | *sec*-Bu | $CH_2CH_2CN$ | Et | Cl | - |
| 1074 | *sec*-Bu | $CH_2CO_2Et$ | Me | H | - |
| 1075 | *sec*-Bu | $CH_2CO_2Et$ | Me | Cl | - |
| 1076 | *sec*-Bu | $CH_2CO_2Et$ | Et | H | - |
| 1077 | *sec*-Bu | $CH_2CO_2Et$ | Et | Cl | - |

(continued)

| Compound No | R | $R^1$ | $R^2$ | X | Ym |
|---|---|---|---|---|---|
| 1078 | *sec*-Bu | OH | Me | Cl | - |
| 1079 | *sec*-Bu | OMe | Me | Cl | - |
| 1080 | *sec*-Bu | OEt | Me | Cl | - |
| 1081 | *sec*-Bu | $-(CH_2)_4-$ | | H | - |
| 1082 | *sec*-Bu | $-(CH_2)_4-$ | | Cl | - |
| 1083 | *sec*-Bu | $-(CH_2)_4-$ | | CN | - |
| 1084 | *sec*-Bu | $-(CH_2)_4-$ | | Me | - |
| 1085 | *sec*-Bu | $-CH(Me)(CH_2)_3-$ | | H | - |
| 1086 | *sec*-Bu | $-CH(Me)(CH_2)_3-$ | | F | - |
| 1087 | *sec*-Bu | $-CH(Me)(CH_2)_3-$ | | Cl | - |
| 1088 | *sec*-Bu | $-CH(Me)(CH_2)_3-$ | | Br | - |
| 1089 | *sec*-Bu | $-CH(Me)(CH_2)_3-$ | | OMe | - |
| 1090 | *sec*-Bu | $-CH(Me)(CH_2)_3-$ | | CN | - |
| 1091 | *sec*-Bu | $-CH(Me)(CH_2)_3-$ | | Me | - |
| 1092 | *sec*-Bu | $-CH(Me)(CH_2)_3-$ | | $CF_3$ | - |
| 1093 | *sec*-Bu | $-CH(Me)(CH_2)_2-CH(Me)-$ | | H | - |
| 1094 | *sec*-Bu | $-CH(Me)(CH_2)_2-CH(Me)-$ | | Cl | |
| 1095 | *sec*-Bu | $-CH(Me)(CH_2)_2-CH(Me)-$ | | CN | - |
| 1096 | *sec*-Bu | $-CH(Me)(CH_2)_2-CH(Me)-$ | | Me | - |
| 1097 | *sec*-Bu | $CH_2CH(Me)(CH_2)_3-$ | | H | - |
| 1098 | *sec*-Bu | $CH_2CH(Me)(CH_2)_2-$ | | Cl | - |
| 1099 | *sec*-Bu | $CH_2CH(Me)(CH_2)_2-$ | | CN | - |
| 1100 | *sec*-Bu | $CH_2CH(Me)(CH_2)_2-$ | | Me | - |
| 1101 | *sec*-Bu | $-CH_2C(Me)_2(CH_2)_2-$ | | H | - |
| 1102 | *sec*-Bu | $-CH_2C(Me)_2(CH_2)_2-$ | | Cl | - |
| 1103 | *sec*-Bu | $-CH_2C(Me)_2(CH_2)_2-$ | | CN | - |
| 1104 | *sec*-Bu | $-CH_2C(Me)_2(CH_2)_2-$ | | Me | - |
| 1105 | *sec*-Bu | $-CH_2CH(OH)(CH_2)_2-$ | | H | - |
| 1106 | *sec*-Bu | $-CH_2CH(OH)(CH_2)_2-$ | | Cl | - |
| 1107 | *sec*-Bu | $-CH_2CH(OH)(CH_2)_2-$ | | CN | - |
| 1108 | *sec*-Bu | $-CH_2CH(OH)(CH_2)_2-$ | | Me | - |
| 1109 | *sec*-Bu | $CH_2CHF(CH_2)_2-$ | | H | - |
| 1110 | *sec*-Bu | $CH_2CHF(CH_2)_2-$ | | Cl | - |
| 1111 | *sec*-Bu | $CH_2CHF(CH_2)_2-$ | | CN | - |
| 1112 | *sec*-Bu | $CH_2CHF(CH_2)_2-$ | | Me | - |
| 1113 | *sec*-Bu | $-CH(CF_3)(CH_2)_3-$ | | H | - |
| 1114 | *sec*-Bu | $-CH(CF_3)(CH_2)_3-$ | | F | - |
| 1115 | *sec*-Bu | $-CH(CF_3)(CH_2)_3-$ | | Cl | - |

[Table 23]

| Compound No | R | $R^1$ | $R^2$ | X | Ym |
|---|---|---|---|---|---|
| 1116 | *sec*-Bu | $-CH(CF_3)(CH_2)_3-$ | | Br | - |
| 1117 | *sec*-Bu | $OH(CF_3(CH_2)_3-$ | | I | - |
| 1118 | *sec*-Bu | $-CH(CF_3)(CH_2)_3-$ | | OH | - |
| 1119 | *sec*-Bu | $-CH(CF_3)(CH_2)_3-$ | | OMe | - |

(continued)

| Compound No | R | $R^1$ | $R^2$ | X | Ym |
|---|---|---|---|---|---|
| 1120 | *sec*-Bu | $-CH(CF_3)(CH_2)_3-$ | | OEt | - |
| 1121 | *sec*-Bu | $-CH(CF_3)(CH_2)_3-$ | | $OCHF_2$ | - |
| 1122 | *sec*-Bu | $-CH(CF_3)(CH_2)_3-$ | | $OCF_3$ | - |
| 1123 | *sec*-Bu | $-CH(CF_3)(CH_2)_3-$ | | $OCH_2CHF_3$ | - |
| 1124 | *sec*-Bu | $-CH(CF_3)(CH_2)_3-$ | | $OCH_2CF_3$ | - |
| 1125 | *sec*-Bu | $-CH(CF_3)(CH_2)_3-$ | | SMe | - |
| 1126 | *sec*-Bu | $-CH(CF_3)(CH_2)_3-$ | | SOMe | - |
| 1127 | *sec*-Bu | $-CH(CF_3)(CH_2)_3-$ | | $SO_2Me$ | - |
| 1128 | *sec*-Bu | $-CH(CF_3)(CH_2)_3-$ | | $SCF_3$ | - |
| 1129 | *sec*-Bu | $-CH(CF_3)(CH_2)_3-$ | | $SOCF_3$ | - |
| 1130 | *sec*-Bu | $-CH(CF_3)(CH_2)_3-$ | | $SO_2CF_3$ | - |
| 1131 | *sec*-Bu | $-CH(CF_3)(CH_2)_3-$ | | $NH_2$ | - |
| 1132 | *sec*-Bu | $-CH(CF_3)(CH_2)_3-$ | | NHMe | - |
| 1133 | *sec*-Bu | $-CH(CF_3)(CH_2)_3-$ | | NH*iso*-Pr | - |
| 1134 | *sec*-Bu | $-CH(CF_3)(CH_2)_3-$ | | $N(Me)_2$ | - |
| 1135 | *sec*-Bu | $-CH(CF_3)(CH_2)_3-$ | | N(Et), | - |
| 1136 | *sec*-Bu | $-CH(CF_3)(CH_2)_3-$ | | CN | - |
| 1137 | *sec*-Bu | $-CH(CF_3)(CH_2)_3-$ | | CHO | - |
| 1138 | *sec*-Bu | $-CH(CF_3)(CH_2)_3-$ | | COMe | - |
| 1139 | *sec*-Bu | $-CH(CF_3)(CH_2)_3-$ | | COEt | - |
| 1140 | *sec*-Bu | $-CH(CF_3)(CH_2)_3-$ | | $CO_2H$ | - |
| 1141 | *sec*-Bu | $-CH(CF_3)(CH_2)_3-$ | | $CO_2Me$ | - |
| 1142 | *sec*-Bu | $-CH(CF_3)(CH_2)_3-$ | | $CO_2Et$ | - |
| 1143 | *sec*-Bu | $-CH(CF_3)(CH_2)_3-$ | | $CONH_2$ | - |
| 1144 | *sec*-Bu | $-CH(CF_3)(CH_2)_3-$ | | ONHMe | - |
| 1145 | *sec*-Bu | $-CH(CF_3)(CH_2)_3-$ | | $CON(Me)_2$ | - |
| 1146 | *sec*-Bu | $-CH(CF_3)(CH_2)_3-$ | | Me | - |
| 1147 | *sec*-Bu | $-CH(CF_3)(CH_2)_3-$ | | Et | - |
| 1148 | *sec*-Bu | $-CH(CF_3)(CH_2)_3-$ | | *iso*-Pr | - |
| 1149 | *sec*-Bu | $-CH(CF_3)(CH_2)_3-$ | | *c*-Pr | - |
| 1150 | *sec*-Bu | $-CH(CF_3)(CH_2)_3-$ | | $CH_2F$ | - |
| 1151 | *sec*-Bu | $-CH(CF_3)(CH_2)_3-$ | | $CH_3Cl$ | - |
| 1152 | *sec*-Bu | $-CH(CF_3)(CH_2)_3-$ | | $CH_3Br$ | - |
| 1153 | *sec*-Bu | $-CH(CF_3)(CH_2)_3-$ | | $CHF_2$ | - |
| 1154 | *sec*-Bu | $-CH(CF_3)(CH_2)_3-$ | | $CF_3$ | - |
| 1155 | *sec*-Bu | $-CH_2CF_2(CH_2)_2-$ | | H | - |
| 1156 | *sec*-Bu | $-CH_2CF_2(CH_2)_2-$ | | Cl | - |
| 1157 | *sec*-Bu | $-CH_2CF_2(CH_2)_2-$ | | CN | - |
| 1158 | *sec*-Bu | $-CH_2CF_2(CH_2)_2-$ | | Me | - |
| 1159 | *sec*-Bu | $-CH_2CF_2(CH_2)_2-$ | | H | - |
| 1160 | *sec*-Bu | $-CH_2CF_2(CH_2)_2-$ | | Cl | - |
| 1161 | *sec*-Bu | $-CH_2CF_2(CH_2)_2-$ | | SMe | - |
| 1162 | *sec*-Bu | $-CH_2CF_2(CH_2)_2-$ | | SOMe | - |
| 1163 | *sec*-Bu | $-CH_2CF_2(CH_2)_2-$ | | $SO_2Me$ | - |
| 1164 | *sec*-Bu | $-CH_2CF_2(CH_2)_2-$ | | CN | - |
| 1165 | *sec*-Bu | $-CH_2CF_2(CH_2)_2-$ | | Me | - |
| 1166 | *sec*-Bu | $-CH_2CH_2(CO_2Me)(CH_2)_2-$ | | H | - |

[Table 24]

| Compound No | R | $R^1$ | $R^2$ | X | Ym |
|---|---|---|---|---|---|
| 1167 | *sec*-Bu | $-CH_2CH(CO_2Me)(CH_2)_2-$ | | Cl | - |
| 1168 | *sec*-Bu | $-CH_2CH(CO_2Me)(CH_2)_2-$ | | CN | - |
| 1169 | *sec*-Bu | $-CH_2CH(CO_2Me)(CH_2)_2-$ | | Me | - |
| 1170 | *sec*-Bu | $-CH_3OCH_3CH_3-$ | | H | - |
| 1171 | *sec*-Bu | $-CH_3OCH_3CH_3-$ | | Cl | - |
| 1172 | *sec*-Bu | $-CH_3OCH_3CH_3-$ | | CN | - |
| 1173 | *sec*-Bu | $-CH_3OCH_3CH_3-$ | | Me | - |
| 1174 | *sec*-Bu | $-CH_3SCH_3CH_3-$ | | H | - |
| 1175 | *sec*-Bu | $-CH_3SCH_3CH_3-$ | | Cl | - |
| 1176 | *sec*-Bu | $-CH_2SCH_2CH_2-$ | | CN | - |
| 1177 | *sec*-Bu | $-CH_2SCH_2CH_2-$ | | Me | - |
| 1178 | *sec*-Bu | $-(CH_2)_5-$ | | H | - |
| 1179 | *sec*-Bu | $-(CH_2)_5-$ | | Cl | - |
| 1180 | *sec*-Bu | $-(CH_2)_5-$ | | CN | - |
| 1181 | *sec*-Bu | $-(CH_2)_5-$ | | Me | - |
| 1182 | *sec*-Bu | $-CH(Me)(CH_3)_4-$ | | H | - |
| 1183 | *sec*-Bu | $-CH(Me)(CH_3)_4-$ | | Cl | - |
| 1184 | *sec*-Bu | $-CH(Me)(CH_2)_4-$ | | CN | - |
| 1185 | *sec*-Bu | $-CH(Me)(CH_2)_4-$ | | Me | - |
| 1186 | *sec*-Bu | $-CH_2CH(Me)(CH_2)_3-$ | | H | - |
| 1187 | *sec*-Bu | $-CH_2CH(Me)(CH_2)_3-$ | | Cl | - |
| 1188 | *sec*-Bu | $-CH_2CH(Me)(CH_2)_3-$ | | CN | - |
| 1189 | *sec*-Bu | $-CH_2CH(Me)(CH_2)_3-$ | | Me | - |
| 1190 | *sec*-Bu | $-(CH_2)_2CH(Me)(CH_2)_2-$ | | H | - |
| 1191 | *sec*-Bu | $-(CH_2)_2CH(Me)(CH_2)_2-$ | | F | - |
| 1192 | *sec*-Bu | $-(CH_2)_2CH(Me)(CH_2)_2-$ | | Cl | - |
| 1193 | *sec*-Bu | $-(CH_2)_2CH(Me)(CH_2)_2-$ | | Br | - |
| 1194 | *sec*-Bu | $-(CH_2)_2CH(Me)(CH_2)_2-$ | | I | - |
| 1195 | *sec*-Bu | $-(CH_2)_2CH(Me)(CH_2)_2-$ | | OH | - |
| 1196 | *sec*-Bu | $-(CH_2)_2CH(Me)(CH_2)_2-$ | | OMe | - |
| 1197 | *sec*-Bu | $-(CH_2)_2CH(Me)(CH_2)_2-$ | | OEt | - |
| 1198 | *sec*-Bu | $-(CH_2)_2CH(Me)(CH_2)_2-$ | | $OCH_2$*c*-Pr | - |
| 1199 | *sec*-Bu | $-(CH_2)_2CH(Me)(CH_2)_2-$ | | $OCH_2Ph$ | - |
| 1200 | *sec*-Bu | $-(CH_2)_2CH(Me)(CH_2)_2-$ | | $OCHF_2$ | - |
| 1201 | *sec*-Bu | $-(CH_2)_2CH(Me)(CH_2)_2-$ | | $OCF_3$ | - |
| 1202 | *sec*-Bu | $-(CH_2)_2CH(Me)(CH_2)_2-$ | | $OCH_2CHF_2$ | - |
| 1203 | *sec*-Bu | $-(CH_2)_2CH(Me)(CH_2)_2-$ | | $OCH_3CF_3$ | - |
| 1204 | *sec*-Bu | $-(CH_2)_2CH(Me)(CH_2)_2-$ | | SMe | - |
| 1205 | *sec*-Bu | $-(CH_2)_2CH(Me)(CH_2)_2-$ | | SOMe | - |
| 1206 | *sec*-Bu | $-(CH_2)_2CH(Me)(CH_2)_2-$ | | $SO_3Me$ | - |
| 1207 | *sec*-Bu | $-(CH_2)_2CH(Me)(CH_2)_2-$ | | $SCF_3$ | - |
| 1208 | *sec*-Bu | $-(CH_2)_2CH(Me)(CH_2)_2-$ | | $SOCF_3$ | - |
| 1209 | *sec*-Bu | $-(CH_2)_2CH(Me)(CH_2)_2-$ | | $SO_2CF_3$ | - |
| 1210 | *sec*-Bu | $-(CH_2)_2CH(Me)(CH_2)_2-$ | | $NH_2$ | - |
| 1211 | *sec*-Bu | $-(CH_2)_2CH(Me)(CH_2)_2-$ | | NHMe | - |
| 1212 | *sec*-Bu | $-(CH_2)_2CH(Me)(CH_2)_2-$ | | NH*iso*-Pr | - |
| 1213 | *sec*-Bu | $-(CH_2)_2CH(Me)(CH_2)_2-$ | | $N(Me)_2$ | - |
| 1214 | *sec*-Bu | $-(CH_2)_2CH(Me)(CH_2)_2-$ | | $N(Et)_2$ | - |
| 1215 | *sec*-Bu | $-(CH_2)_2CH(Me)(CH_2)_2-$ | | CN | - |

(continued)

| Compound No | R | $R^1$ | $R^2$ | X | Ym |
|---|---|---|---|---|---|
| 1216 | *sec*-Bu | $-(CH_2)_2CH(Me)(CH_2)_2-$ | | CHO | - |
| 1217 | *sec*-Bu | $-(CH_2)_2CH(Me)(CH_2)_2-$ | | COMe | - |

[Table 25]

| Compound No | R | $R^1$ | $R^2$ | X | Ym |
|---|---|---|---|---|---|
| 1218 | *sec*-Bu | $-(CH_2)_2CH(Me)(CH_2)_2-$ | | COEt | - |
| 1219 | *sec*-Bu | $-(CH_2)_2CH(Me)(CH_2)_2-$ | | $CO_2H$ | - |
| 1220 | *sec*-Bu | $-(CH_2)_2CH(Me)(CH_2)_2-$ | | $CO_2Me$ | - |
| 1221 | *sec*-Bu | $-(CH_2)_2CH(Me)(CH_2)_2-$ | | $CO_2Et$ | - |
| 1222 | *sec*-Bu | $-(CH_2)_2CH(Me)(CH_2)_2-$ | | $CONH_2$ | - |
| 1223 | *sec*-Bu | $-(CH_2)_2CH(Me)(CH_2)_2-$ | | ONHMe | - |
| 1224 | *sec*-Bu | $-(CH_2)_2CH(Me)(CH_2)_2-$ | | $CON(Me)_2$ | - |
| 1225 | *sec*-Bu | $-(CH_2)_2CH(Me)(CH_2)_2-$ | | Me | - |
| 1226 | *sec*-Bu | $-(CH_2)_2CH(Me)(CH_2)_2-$ | | Et | - |
| 1227 | *sec*-Bu | $-(CH_2)_2CH(Me)(CH_2)_2-$ | | *iso*-Pr | - |
| 1238 | *sec*-Bu | $-(CH_2)_2CH(Me)(CH_2)_2-$ | | *c*-Pr | - |
| 1229 | *sec*-Bu | $-(CH_2)_2CH(Me)(CH_2)_2-$ | | $CH_2F$ | - |
| 1230 | *sec*-Bu | $-(CH_2)_2CH(Me)(CH_2)_2-$ | | $CH_2Cl$ | - |
| 1231 | *sec*-Bu | $-(CH_2)_2CH(Me)(CH_2)_2-$ | | $CH_2Br$ | - |
| 1232 | *sec*-Bu | $-(CH_2)_2CH(Me)(CH_2)_2-$ | | $CHF_2$ | - |
| 1233 | *sec*-Bu | $-(CH_2)_2CH(Me)(CH_2)_2-$ | | $CF_3$ | - |
| 1234 | *sec*-Bu | $-(CH_2)_2CH(Me)(CH_2)_2-$ | | H | 3-Cl |
| 1235 | *sec*-Bu | $-(CH_2)_2CH(Me)(CH_2)_2-$ | | Cl | 3-Cl |
| 1236 | *sec*-Bu | $-(CH_2)_2CH(Me)(CH_2)_2-$ | | CN | 3-Cl |
| 1237 | *sec*-Bu | $-(CH_2)_2CH(Me)(CH_2)_2-$ | | Me | 3-Cl |
| 1338 | *sec*-Bu | $-(CH_2)_2CH(Me)(CH_2)_2-$ | | H | 3-Me |
| 1339 | *sec*-Bu | $-(CH_2)_2CH(Me)(CH_2)_2-$ | | Cl | 3-Me |
| 1240 | *sec*-Bu | $-(CH_2)_2CH(Me)(CH_2)_2-$ | | CN | 3-Me |
| 1241 | *sec*-Bu | $-(CH_2)_2CH(Me)(CH_2)_2-$ | | Me | 3-Me |
| 1242 | *sec*-Bu | $-(CH_2)_2CH(Me)(CH_2)_2-$ | | H | 3-Pr-i |
| 1243 | *sec*-Bu | $-(CH_2)_2CH(Me)(CH_2)_2-$ | | Cl | 3-Pr-i |
| 1244 | *sec*-Bu | $-(CH_2)_2CH(Me)(CH_2)_2-$ | | CN | 3-Pr-i |
| 1245 | *sec*-Bu | $-(CH_2)_2CH(Me)(CH_2)_2-$ | | Me | 3-Pr-i |
| 1246 | *sec*-Bu | $-(CH_2)_2CH(Me)(CH_2)_2-$ | | H | $3-CF_3-4-CO_2Et$ |
| 1247 | *sec*-Bu | $-(CH_2)_2CH(Me)(CH_2)_2-$ | | Cl | $3-CF_3-4-CO_2Et$ |
| 1248 | *sec*-Bu | $-(CH_2)_2CH(Me)(CH_2)_2-$ | | CN | $3-CF_3-4-CO_2Et$ |
| 1249 | *sec*-Bu | $-(CH_2)_2CH(Me)(CH_2)_2-$ | | Me | $3-CF_3-4-CO_2Et$ |
| 1250 | *sec*-Bu | $-(CH_2)_2CH(Me)(CH_2)_2-$ | | H | $3,5-(Me)_2$ |
| 1251 | *sec*-Bu | $-(CH_2)_2CH(Me)(CH_2)_2-$ | | Cl | $3,5-(Me)_2$ |
| 1252 | *sec*-Bu | $-(CH_2)_2CH(Me)(CH_2)_2-$ | | CN | $3,5-(Me)_2$ |
| 1253 | *sec*-Bu | $-(CH_2)_2CH(Me)(CH_2)_2-$ | | Me | $3,5-(Me)_2$ |
| 1254 | *sec*-Bu | $-(CH_2)_2O(CH_2)_2-$ | | H | - |
| 1255 | *sec*-Bu | $-(CH_2)_2O(CH_2)_2-$ | | Cl | - |
| 1256 | *sec*-Bu | $-(CH_2)_2O(CH_2)_2-$ | | CN | - |
| 1257 | *sec*-Bu | $-(CH_2)_2O(CH_2)_2-$ | | Me | - |
| 1258 | *sec*-Bu | $-CH_2CH(Me)OCH(Me)CH_2-$ | | H | - |
| 1259 | *sec*-Bu | $-CH_2CH(Me)OCH(Me)CH_2-$ | | Cl | - |

(continued)

| Compound No | R | $R^1$ | $R^2$ | X | Ym |
|---|---|---|---|---|---|
| 1260 | *sec*-Bu | $-CH_2CH(Me)OCH(Me)CH_2-$ | | CN | - |
| 1261 | *sec*-Bu | $-CH_2CH(Me)OCH(Me)CH_2-$ | | Me | - |
| 1262 | *sec*-Bu | $-(CH_2)_2S(CH_2)_2-$ | | H | - |
| 1263 | *sec*-Bu | $-(CH_2)_2S(CH_2)_2-$ | | Cl | - |
| 1264 | *sec*-Bu | $-(CH_2)_2S(CH_2)_2-$ | | CN | - |
| 1265 | *sec*-Bu | $-(CH_2)_2S(CH_2)_2-$ | | Me | - |
| 1266 | *sec*-Bu | $-(CH_2)_2S(CH_2)_2-$ | | H | - |
| 1267 | *sec*-Bu | $-(CH_2)_2S(CH_2)_2-$ | | Cl | - |
| 1268 | *sec*-Bu | $-(CH_2)_2S(CH_2)_2-$ | | CN | - |

[Table 26]

| Compound No | R | $R^1$ | $R^2$ | X | Ym |
|---|---|---|---|---|---|
| 1269 | *sec*-Bu | $-(CH_2)_2S(O)(CH_2)_2-$ | | Me | - |
| 1270 | *sec*-Bu | $-(CH_2)_2S(O)(CH_2)_2-$ | | H | - |
| 1271 | *sec*-Bu | $-(CH_2)_2S(O)(CH_2)_2-$ | | Cl | - |
| 1272 | *sec*-Bu | $-(CH_2)_2S(O)(CH_2)_2-$ | | CN | - |
| 1273 | *sec*-Bu | $-(CH_2)_2S(O)(CH_2)_2-$ | | Me | - |
| 1274 | *sec*-Bu | $-(CH_2)_2S(O)(CH_2)_2-$ | | H | - |
| 1275 | *sec*-Bu | $-(CH_2)_2S(O)(CH_2)_2-$ | | Cl | - |
| 1276 | *sec*-Bu | $-(CH_2)_2S(O)(CH_2)_2-$ | | CN | - |
| 1277 | *sec*-Bu | $-(CH_2)_2S(O)(CH_2)_2-$ | | Me | - |
| 1278 | *iso*-Bu | *iso*-Pr | H | H | - |
| 1279 | *iso*-Bu | *iso*-Pr | H | Cl | - |
| 1280 | *iso*-Bu | *iso*-Pr | H | CN | - |
| 1281 | *iso*-Bu | *iso*-Pr | H | Me | - |
| 1282 | *iso*-Bu | $CH_2CF_3$ | H | H | - |
| 1283 | *iso*-Bu | $CH_2CF_3$ | H | Cl | - |
| 1284 | *iso*-Bu | $CH_2CF_3$ | H | CN | - |
| 1285 | *iso*-Bu | $CH_2CF_3$ | H | Me | - |
| 1286 | *iso*-Bu | Et | Et | H | - |
| 1287 | *iso*-Bu | Et | Et | Cl | - |
| 1388 | *iso*-Bu | Et | Et | CN | - |
| 1289 | *iso*-Bu | Et | Et | Me | - |
| 1290 | *iso*-Bu | $-(CH_2)_2CH(Me)(CH_3)_2-$ | | H | - |
| 1291 | *iso*-Bu | $-(CH_2)_2CH(Me)(CH_3)_2-$ | | Cl | - |
| 1292 | *iso*-Bu | $-(CH_2)_2CH(Me)(CH_3)_2-$ | | CN | - |
| 1293 | *iso*-Bu | $-(CH_2)_2CH(Me)(CH_3)_2-$ | | Me | - |
| 1294 | *n*-Pen | *iso*-Pr | H | H | - |
| 1295 | *n*-Pen | *iso*-Pr | H | Cl | - |
| 1296 | *n*-Pen | *iso*-Pr | H | CN | - |
| 1297 | *n*-Pen | *iso*-Pr | H | Me | - |
| 1298 | *n*-Pen | $CH_2CF_3$ | H | H | - |
| 1299 | *n*-Pen | $CH_2CF_3$ | H | Cl | - |
| 1300 | *n*-Pen | $CH_2CF_3$ | H | CN | - |
| 1301 | *n*-Pen | $CH_2CF_3$ | H | Me | - |
| 1302 | *n*-Pen | Et | Et | H | - |

(continued)

| Compound No | R | $R^1$ | $R^2$ | X | Ym |
|---|---|---|---|---|---|
| 1303 | *n*-Pen | Et | Et | Cl | - |
| 1304 | *n*-Pen | Et | Et | CN | - |
| 1305 | *n*-Pen | Et | Et | Me | - |
| 1306 | *n*-Pen | $-(CH_2)_2CH(Me)(CH_2)_2-$ | | H | - |
| 1307 | *n*-Pen | $-(CH_2)_2CH(Me)(CH_2)_2-$ | | Cl | - |
| 1308 | *n*-Pen | $-(CH_2)_2CH(Me)(CH_2)_2-$ | | CN | - |
| 1309 | *n*-Pen | $-(CH_2)_2CH(Me)(CH_2)_2-$ | | Me | - |
| 1310 | 2-Pen | *iso*-Pr | H | H | - |
| 1311 | 2-Pen | *iso*-Pr | H | Cl | - |
| 1312 | 2-Pen | *iso*-Pr | H | CN | - |
| 1313 | 2-Pen | *iso*-Pr | H | Me | - |
| 1314 | 2-Pen | $CH_2CF_3$ | H | H | - |
| 1315 | 2-Pen | $CH_2CF_3$ | H | Cl | - |
| 1316 | 2-Pen | $CH_2CF_3$ | H | CN | - |
| 1317 | 2-Pen | $CH_2CF_3$ | H | Me | - |
| 1318 | 2-Pen | Et | Et | H | - |
| 1319 | 2-Pen | Et | Et | Cl | - |

[Table 27]

| Compound No | R | $R^1$ | $R^2$ | X | Ym |
|---|---|---|---|---|---|
| 1320 | 2-Pen | Et | Et | CN | - |
| 1321 | 2-Pen | Et | Et | Me | - |
| 1322 | 2-Pen | $-(CH_2)_2CH(Me)(CH_2)_2-$ | | H | - |
| 1323 | 2-Pen | $-(CH_2)_2CH(Me)(CH_2)_2-$ | | Cl | - |
| 1324 | 2-Pen | $-(CH_2)_2CH(Me)(CH_2)_2-$ | | CN | - |
| 1325 | 2-Pen | $-(CH_2)_2CH(Me)(CH_2)_2-$ | | Me | - |
| 1326 | 3-Pen | *iso*-Pr | H | H | - |
| 1327 | 3-Pen | *iso*-Pr | H | F | - |
| 1328 | 3-Pen | *iso*-Pr | H | Cl | - |
| 1329 | 3-Pen | *iso*-Pr | H | Br | - |
| 1330 | 3-Pen | *iso*-Pr | H | CN | - |
| 1331 | 3-Pen | *iso*-Pr | H | Me | - |
| 1332 | 3-Pen | *iso*-Pr | H | $CF_3$ | - |
| 1333 | 3-Pen | *tert*-Bu | H | H | - |
| 1334 | 3-Pen | *tert*-Bu | H | F | - |
| 1335 | 3-Pen | *tert*-Bu | H | Cl | - |
| 1336 | 3-Pen | *tert*-Bu | H | Br | - |
| 1337 | 3-Pen | *tert*-Bu | H | CN | - |
| 1338 | 3-Pen | *tert*-Bu | H | Me | - |
| 1339 | 3-Pen | *tert*-Bu | H | $CF_3$ | - |
| 1340 | 3-Pen | $CH_2CF_3$ | H | H | - |
| 1341 | 3-Pen | $CH_2CF_3$ | H | F | - |
| 1342 | 3-Pen | $CH_2CF_3$ | H | Cl | - |
| 1343 | 3-Pen | $CH_2CF_3$ | H | Br | - |
| 1344 | 3-Pen | $CH_2CF_3$ | H | I | - |
| 1345 | 3-Pen | $CH_2CF_3$ | H | OH | - |

(continued)

| Compound No | R | $R^1$ | $R^2$ | X | Ym |
|---|---|---|---|---|---|
| 1346 | 3-Pen | $CH_2CF_3$ | H | OMe | - |
| 1347 | 3-Pen | $CH_2CF_3$ | H | OEt | - |
| 1348 | 3-Pen | $CH_2CF_3$ | H | $OCH_2$*c*-Pr | - |
| 1349 | 3-Pen | $CH_2CF_3$ | H | $OCHF_2$ | - |
| 1350 | 3-Pen | $CH_2CF_3$ | H | $OCF_3$ | - |
| 1351 | 3-Pen | $CH_2CF_3$ | H | $OCH_2CHF_2$ | - |
| 1352 | 3-Pen | $CH_2CF_3$ | H | $OCH_3CF_3$ | - |
| 1353 | 3-Pen | $CH_2CF_3$ | H | SMe | - |
| 1354 | 3-Pen | $CH_2CF_3$ | H | SOMe | - |
| 1355 | 3-Pen | $CH_2CF_3$ | H | $SO_2Me$ | - |
| 1356 | 3-Pen | $CH_2CF_3$ | H | $SCF_3$ | - |
| 1357 | 3-Pen | $CH_2CF_3$ | H | $SOCF_3$ | - |
| 1358 | 3-Pen | $CH_2CF_3$ | H | $SO_2CF_3$ | - |
| 1359 | 3-Pen | $CH_2CF_3$ | H | $NH_2$ | - |
| 1360 | 3-Pen | $CH_2CF_3$ | H | NHMe | - |
| 1361 | 3-Pen | $CH_2CF_3$ | H | NH*iso*-Pr | - |
| 1362 | 3-Pen | $CH_2CF_3$ | H | $N(Me)_2$ | - |
| 1363 | 3-Pen | $CH_2CF_3$ | H | N(Et), | - |
| 1364 | 3-Pen | $CH_2CF_3$ | H | CN | - |
| 1365 | 3-Pen | $CH_2CF_3$ | H | CHO | - |
| 1366 | 3-Pen | $CH_2CF_3$ | H | COMe | - |
| 1367 | 3-Pen | $CH_2CF_3$ | H | COEt | - |
| 1368 | 3-Pen | $CH_2CF_3$ | H | $CO_2H$ | - |
| 1369 | 3-Pen | $CH_2CF_3$ | H | $CO_2Me$ | - |
| 1370 | 3-Pen | $CH_2CF_3$ | H | $CO_2Et$ | - |

[Table 28]

| Compound No | R | $R^1$ | $R^2$ | X | Ym |
|---|---|---|---|---|---|
| 1371 | 3-Pen | $CH_2CF_3$ | H | $CONH_3$ | - |
| 1372 | 3-Pen | $CH_2CF_3$ | H | ODNHMe | - |
| 1373 | 3-Pen | $CH_2CF_3$ | H | $CON(Me)_3$ | - |
| 1374 | 3-Pen | $CH_2CF_3$ | H | Me | - |
| 1375 | 3-Pen | $CH_2CF_3$ | H | Et | - |
| 1376 | 3-Pen | $CH_2CF_3$ | H | *iso*-Pr | - |
| 1377 | 3-Pen | $CH_2CF_3$ | H | *c*-Pr | - |
| 1378 | 3-Pen | $CH_2CH_3$ | H | $CH_3F$ | - |
| 1379 | 3-Pen | $CH_2CF_3$ | H | $CH_2Cl$ | - |
| 1380 | 3-Pen | $CH_2CF_3$ | H | $CH_3Br$ | - |
| 1381 | 3-Pen | $CH_2CF_3$ | H | $CHF_3$ | - |
| 1382 | 3-Pen | $CH_2CF_3$ | H | $CF_3$ | - |
| 1383 | 3-Pen | $CH(Me)CF_3$ | H | H | - |
| 1384 | 3-Pen | $CH(Me)CF_3$ | H | F | - |
| 1385 | 3-Pen | $CH(Me)OF_3$ | H | Cl | - |
| 1386 | 3-Pen | $CH(Me)CF_3$ | H | Br | - |
| 1387 | 3-Pen | $CH(Me)CF_3$ | H | I | - |
| 1388 | 3-Pen | $CH(Me)CF_3$ | H | OH | - |
| 1389 | 3-Pen | $CH(Me)CF_3$ | H | OMe | - |

(continued)

| Compound No | R | $R^1$ | $R^2$ | X | Ym |
|---|---|---|---|---|---|
| 1390 | 3-Pen | $CH(Me)CF_3$ | H | OEt | - |
| 1391 | 3-Pen | $CH(Me)CF_3$ | H | $OCH_3$-Pr | - |
| 1392 | 3-Pen | $CH(Me)CF_3$ | H | $OCHF_3$ | - |
| 1393 | 3-Pen | $CH(Me)CF_3$ | H | $OCF_3$ | - |
| 1394 | 3-Pen | $CH(Me)CF_3$ | H | $OCH_2CHF_3$ | - |
| 1395 | 3-Pen | $CH(Me)CF_3$ | H | $OCH_2CF_3$ | - |
| 1396 | 3-Pen | $CH(Me)CF_3$ | H | SMe | - |
| 1397 | 3-Pen | $CH(Me)CF_3$ | H | SOMe | - |
| 1398 | 3-Pen | $CH(Me)CF_3$ | H | $SO_3Me$ | - |
| 1399 | 3-Pen | $CH(Me)CF_3$ | H | $SCF_3$ | - |
| 1400 | 3-Pen | $CH(Me)CF_3$ | H | $SOCF_3$ | - |
| 1401 | 3-Pen | $CH(Me)CF_3$ | H | $SO_3CF_3$ | - |
| 1402 | 3-Pen | $CH(Me)CF_3$ | H | $NH_3$ | - |
| 1403 | 3-Pen | $CH(Me)CF_3$ | H | NHMe | - |
| 1404 | 3-Pen | $CH(Me)CF_3$ | H | NH*iso*-Pr | - |
| 1405 | 3-Pen | $CH(Me)CF_3$ | H | $N(Me)_3$ | - |
| 1406 | 3-Pen | $CH(Me)CF_3$ | H | $N(Et)_3$ | - |
| 1407 | 3-Pen | $CH(Me)CF_3$ | H | CN | - |
| 1408 | 3-Pen | $CH(Me)CF_3$ | H | CHO | - |
| 1409 | 3-Pen | $CH(Me)CF_3$ | H | COMe | - |
| 1410 | 3-Pen | $CH(Me)CF_3$ | H | COEt | - |
| 1411 | 3-Pen | $CH(Me)CF_3$ | H | $CO_3H$ | - |
| 1412 | 3-Pen | $CH(Me)CF_3$ | H | $CO_3Me$ | - |
| 1413 | 3-Pen | $CH(Me)CF_3$ | H | $CO_3Et$ | - |
| 1414 | 3-Pen | $CH(Me)CF_3$ | H | $VONH_3$ | - |
| 1415 | 3-Pen | $CH(Me)CF_3$ | H | CONHMe | - |
| 1416 | 3-Pen | $CH(Me)CF_3$ | H | $CON(Me)_3$ | - |
| 1417 | 3-Pen | $CH(Me)CF_3$ | H | Me | - |
| 1418 | 3-Pen | $CH(Me)CF_3$ | H | Et | - |
| 1419 | 3-Pen | $CH(Me)CF_3$ | H | *iso*-Pr | - |
| 1420 | 3-Pen | $CH(Me)CF_3$ | H | *c*-Pr | - |
| 1421 | 3-Pen | $CH(Me)CF_3$ | H | $CH_3F$ | - |

[Table 29]

| Compound No | R | $R^1$ | $R^2$ | X | Ym |
|---|---|---|---|---|---|
| 1422 | 3-Pen | $CH(Me)CF_3$ | H | $CH_3Cl$ | - |
| 1423 | 3-Pen | $CH(Me)CF_3$ | H | $CH_3Br$ | - |
| 1424 | 3-Pen | $CH(Me)CF_3$ | H | $CHF_3$ | - |
| 1425 | 3-Pen | $CH(Me)CF_3$ | H | $CF_3$ | - |
| 1426 | 3-Pen | Et | Et | H | - |
| 1427 | 3-Pen | Et | Et | F | - |
| 1428 | 3-Pen | Et | Et | Cl | |
| 1429 | 3-Pen | Et | Et | Br | - |
| 1430 | 3-Pen | Et | Et | CN | - |
| 1431 | 3-Pen | Et | Et | Me | - |
| 1432 | 3-Pen | Et | Et | $CF_3$ | - |
| 1433 | 3-Pen | $-CH(CF_3)(CH_2)_3-$ | | H | - |

(continued)

| Compound No | R | $R^1$ | $R^2$ | X | Ym |
|---|---|---|---|---|---|
| 1434 | 3-Pen | $-CH(CF_3)(CH_2)_3-$ | | F | - |
| 1435 | 3-Pen | $-CH(CF_3)(CH_2)_3-$ | | Cl | - |
| 1436 | 3-Pen | $-CH(CF_3)(CH_2)_3-$ | | Br | - |
| 1437 | 3-Pen | $-CH(CF_3)(CH_2)_3-$ | | CN | - |
| 1438 | 3-Pen | $-CH(CF_3)(CH_2)_3-$ | | Me | - |
| 1439 | 3-Pen | $-CH(CF_3)(CH_2)_3-$ | | $CF_3$ | - |
| 1440 | 3-Pen | $-(CH_2)_3CH(Me)(CH_2)_3-$ | | H | - |
| 1441 | 3-Pen | $-(CH_2)_3CH(Me)(CH_2)_3-$ | | F | - |
| 1442 | 3-Pen | $-(CH_2)_3CH(Me)(CH_2)_3-$ | | Cl | - |
| 1443 | 3-Pen | $-(CH_2)_3CH(Me)(CH_2)_3-$ | | Br | - |
| 1444 | 3-Pen | $-(CH_2)_3CH(Me)(CH_2)_3-$ | | CN | - |
| 1445 | 3-Pen | $-(CH_2)_3CH(Me)(CH_2)_3-$ | | Me | - |
| 1446 | 3-Pen | $-(CH_2)_3CH(Me)(CH_2)_3-$ | | $CF_3$ | - |
| 1447 | *tert*-Bu | *iso*-Pr | H | H | - |
| 1448 | *tert*-Bu | *iso*-Pr | H | Cl | - |
| 1449 | *tert*-Bu | *iss*-Pr | H | CN | - |
| 1450 | *tert*-Bu | *iso*-Pr | H | Me | - |
| 1451 | *tert*-Bu | $CH_2CF_3$ | H | H | - |
| 1452 | *tert*-Bu | $CH_2CF_3$ | H | Cl | - |
| 1453 | *tert*-Bu | $CH_2CF_3$ | H | CN | - |
| 1454 | *tert*-Bu | $CH_2CF_3$ | H | Me | - |
| 1455 | tert-Bu | Et | Et | H | - |
| 1456 | *tert*-Bu | Et | Et | Cl | - |
| 1457 | *tert*-Bu | Et | Et | CN | - |
| 1458 | *tert*-Bu | Et | Et | Me | - |
| 1459 | *tert*-Bu | $-(CH_2)_3CH(Me)(CH_2)_3-$ | | H | - |
| 1460 | *tert*-Eu | $-(CH_2)_3CH(Me)(CH_2)_3-$ | | Cl | - |
| 1461 | *tert*-Bu | $-(CH_2)_3CH(Me)(CH_2)_3-$ | | CN | - |
| 1462 | *tert*-Bu | $-(CH_2)_3CH(Me)(CH_2)_3-$ | | Me | - |
| 1463 | $C(Me)_3Et$ | *iso*-Pr | H | H | - |
| 1464 | $C(Me)_3Et$ | *iso*-Pr | H | Cl | - |
| 1465 | $C(Me)_3Et$ | *iso*-Pr | H | CN | - |
| 1466 | $C(Me)_3Et$ | *iso*-Pr | H | Me | - |
| 1467 | $C(Me)_3Et$ | $CH_2CF_3$ | H | H | - |
| 1468 | $C(Me)_3Et$ | $CH_2CF_3$ | H | Cl | - |
| 1469 | $C(Me)_3Et$ | $CH_2CF_3$ | H | CN | - |
| 1470 | $C(Me)_3Et$ | $CH_2CF_3$ | H | Me | - |
| 1471 | $C(Me)_3Et$ | Et | Et | H | - |
| 1472 | $C(Me)_2Et$ | Et | Et | Cl | - |

[Table 30]

| Compound No | R | $R^1$ $R^2$ | | X | Ym |
|---|---|---|---|---|---|
| 1473 | $C(Me)_2Et$ | Et | Et | CN | - |
| 1474 | $C(Me)_3Et$ | Et | Et | Me | - |
| 1475 | $C(Me)_3Et$ | $-(CH_2)_3CH(Me)(CH_2)_3-$ | | H | - |
| 1476 | $C(Me)_3Et$ | $-(CH_2)_3CH(Me)(CH_2)_3-$ | | Cl | - |

(continued)

| Compound No | R | $R^1$ | $R^2$ | X | Ym |
|---|---|---|---|---|---|
| 1477 | C(Me)-Et | $-(CH_2)_3CH(Me)(CH_2)_3-$ | | CN | - |
| 1478 | $C(Me)_3Et$ | $-(CH_2)_3CH(Me)(CH_2)_3-$ | | Me | |
| 1479 | *c*-Pr | *iso*-Pr | H | H | - |
| 1480 | *c*-Pr | *iso*-Pr | H | Cl | - |
| 1481 | *c*-Pr | *iso*-Pr | H | CN | - |
| 1482 | *c*-Pr | *iso*-Pr | H | Me | - |
| 1483 | *c*-Pr | $CH_2CF_3$ | H | H | - |
| 1484 | *c*-Pr | $CH_2CF_3$ | H | Cl | - |
| 1485 | *c*-Pr | $CH_2CF_3$ | H | CN | - |
| 1486 | *c*-Pr | $CH_2CF_3$ | H | Me | - |
| 1487 | *c*-Pr | Et | Et | H | - |
| 1488 | *c*-Pr | Et | Et | Cl | - |
| 1489 | *c*-Pr | Et | Et | CN | - |
| 1490 | *c*-Pr | Et | Et | Me | - |
| 1491 | *c*-Pr | $-(CH_2)_3CH(Me)(CH_2)_3-$ | | H | - |
| 1492 | *c*-Pr | $-(CH_2)_3CH(Me)(CH_2)_3-$ | | Cl | |
| 1493 | *c*-Pr | $-(CH_2)_3CH(Me)(CH_2)_3-$ | | CN | - |
| 1494 | *c*-Pr | $-(CH_2)_3CH(Me)(CH_2)_3-$ | | Me | - |
| 1495 | *c*-Pen | *iso*-Pr | H | H | - |
| 1496 | *c*-Pen | *iso*-Pr | H | F | - |
| 1497 | *c*-Pen | *iso*-Pr | H | Cl | - |
| 1498 | *c*-Pen | *iso*-Pr | H | Br | - |
| 1499 | *c*-Pen | *iso*-Pr | H | CN | - |
| 1500 | *c*-Pan | *iso*-Pr | H | Me | - |
| 1501 | *c*-Pen | *iso*-Pr | H | $CF_3$ | - |
| 1502 | *c*-Pen | *tert*-Bu | H | H | - |
| 1503 | *c*-Pen | *tert*-Bu | H | F | - |
| 1504 | *c*-Pen | *tert*-Bu | H | Cl | - |
| 1505 | *c*-Pen | *tert*-Bu | H | Br | - |
| 1506 | *c*-Pen | *tert*-Bu | H | CN | - |
| 1507 | *c*-Pon | *tert*-Bu | H | Me | - |
| 1508 | *c*-Pen | *tert*-Bu | H | $CF_3$ | - |
| 1509 | *c*-Pen | $CH_2CF_3$ | H | H | - |
| 1510 | *c*-Pen | $CH_2CF_3$ | H | F | - |
| 1511 | *c*-Pen | $CH_2CF_3$ | H | Cl | - |
| 1512 | *c*-Pen | $CH_2CF_3$ | H | Br | - |
| 1513 | *c*-Pen | $CH_2CF_3$ | H | I | - |
| 1514 | *c*-Pen | $CH_2CF_3$ | H | OH | - |
| 1515 | *c*-Pen | $CH_2CF_3$ | H | OMe | - |
| 1516 | *c*-Pen | $CH_2CF_3$ | H | OEt | - |
| 1517 | *c*-Pen | $CH_2CF_3$ | H | $OCH_3$ *c*-Pr | - |
| 1518 | *c*-Pon | $CH_2CF_3$ | H | $OCHF_3$ | - |
| 1519 | *c*-Pen | $CH_2CF_3$ | H | $OCF_3$ | - |
| 1520 | *c*-Pen | $CH_2CF_3$ | H | $OCH_2CHF_3$ | - |
| 1521 | *c*-Pen | $CH_2CF_3$ | H | $OCH_2CF_3$ | - |
| 1522 | *c*-Pen | $CH_2CF_3$ | H | SMe | - |
| 1523 | *c*-Pen | $CH_2CF_3$ | H | SOMe | - |

[Table 31]

| Compound No | R | $R^1$ | $R^2$ | X | Ym |
|---|---|---|---|---|---|
| 1624 | *c*-Pen | $CH_2CF_3$ | H | $SO_2Me$ | - |
| 1525 | *c*-Pen | $CH_2CF_3$ | H | $SCF_3$ | - |
| 1526 | *c*-Pen | $CH_2CF_3$ | H | $SOCF_3$ | - |
| 1527 | *c*-Pen | $CH_2CF_3$ | H | $SO_2CF_3$ | - |
| 1528 | *c*-Pen | $CH_2CF_3$ | H | $NH_3$ | - |
| 1529 | *c*-Pen | $CH_2CF_3$ | H | NHMe | - |
| 1530 | *c*-Pen | $CH_2CF_3$ | H | NH*iso*-Pr | - |
| 1531 | *c*-Pen | $CH_2CF_3$ | H | $N(Me)_3$ | - |
| 1532 | *c*-Pen | $CH_2CF_3$ | H | $N(Et)_2$ | - |
| 1533 | *c*-Pen | $CH_2CF_3$ | H | CN | - |
| 1534 | *c*-Pen | $CH_2CF_3$ | H | CHO | - |
| 1535 | *c*-Pen | $CH_2CF_3$ | H | COMe | - |
| 1536 | *c*-Pen | $CH_2CF_3$ | H | COEt | - |
| 1637 | *c*-Pen | $CH_2CF_3$ | H | $CO_3H$ | - |
| 1538 | *c*-Pen | $CH_2CF_3$ | H | $CO_3Me$ | - |
| 1539 | *c*-Pen | $CH_2CF_3$ | H | $CO_3Et$ | - |
| 1540 | *c*-Pen | $CH_2CF_3$ | H | $CONH_3$, | - |
| 1541 | *c*-Pen | $CH_2CF_3$ | H | CONHMe | - |
| 1542 | *c*-Pen | $CH_2CF_3$ | H | $CON(Me)_2$ | - |
| 1543 | *c*-Pen | $CH_2CF_3$ | H | Me | - |
| 1544 | *c*-Pen | $CH_2CF_3$ | H | Et | - |
| 1545 | *c*-Pen | $CH_2CF_3$ | H | *iso*-Pr | - |
| 1546 | *c*-Pen | $CH_2CF_3$ | H | *c*-Pr | - |
| 1547 | *c*-Pen | $CH_2CF_3$ | H | $CH_3F$ | - |
| 1548 | *c*-Pen | $CH_2CF_3$ | H | $CH_2Gl$ | - |
| 1549 | *c*-Pen | $CH_2CF_3$ | H | $CH_3Br$ | - |
| 1550 | *c*-Pen | $CH_2CF_3$ | H | $CHF_3$ | - |
| 1551 | *c*-Pen | $CH_2CF_3$ | H | $CF_3$ | - |
| 1552 | *c*-Pen | $CH(Me)CF_3$ | H | H | - |
| 1553 | *c*-Pen | $CH(Me)CF_3$ | H | F | - |
| 1554 | *c*-Pen | $CH(Me)CF_3$ | H | Cl | - |
| 1555 | *c*-Pen | $CH(Me)CF_3$ | H | Br | - |
| 1556 | *c*-Pen | $CH(Me)CF_3$ | H | I | - |
| 1557 | *c*-Pen | $CH(Me)CF_3$ | H | OH | - |
| 1558 | *c*-Pen | $CH(Me)CF_3$ | H | CMe | - |
| 1559 | *c*-Pen | $CH(Me)CF_3$ | H | OEt | - |
| 1560 | *c*-Pen | $CH(Me)CF_3$ | H | $OCH_3$*c*-Pr | - |
| 1561 | *c*-Pen | $CH(Me)CF_3$ | H | $OCHF_2$ | - |
| 1562 | *c*-Pen | $CH(Me)CF_3$ | H | $OCF_3$ | - |
| 1563 | *c*-Pen | $CH(Me)CF_3$ | H | $OOH_2CHF_3$ | - |
| 1564 | *c*-Pen | $CH(Me)CF_3$ | H | $OCH_2CF_3$ | - |
| 1565 | *c*-Pen | $CH(Me)CF_3$ | H | SMe | - |
| 1566 | *c*-Pen | $CH(Me)CF_3$ | H | SOMe | - |
| 1567 | *c*-Pen | $CH(Me)CF_3$ | H | $SO_3Me$ | - |
| 1568 | *c*-Pen | $CH(Me)CF_3$ | H | $SCF_3$ | - |
| 1569 | *c*-Pen | $CH(Me)CF_3$ | H | $SOCF_3$ | - |
| 1579 | *c*-Pen | $CH(Me)CF_3$ | H | $SO_2CF_3$ | - |
| 1571 | *c*-Pen | $CH(Me)CF_3$ | H | $NH_3$ | - |
| 1572 | *c*-Pen | $CH(Me)CF_3$ | H | NHMe | - |

(continued)

| Compound No | R | $R^1$ | $R^2$ | X | Ym |
|---|---|---|---|---|---|
| 1573 | *c*-Pen | $CH(Me)CF_3$ | H | NH*iso*-Pr | - |
| 1574 | *c*-Pen | $CH(Me)CF_3$ | H | $N(Me)_2$ | - |

[Table 32]

| Compound No | R | $R^1$ | $R^3$ | X | Ym |
|---|---|---|---|---|---|
| 1575 | *c*-Pen | $CH(Me)CF_3$ | H | $N(Et)_2$ | - |
| 1576 | *c*-Pen | $CH(Me)CF_3$ | H | CN | - |
| 1577 | *c*-pen | $CH(Me)CF_3$ | H | CHO | - |
| 1578 | *c*-Pen | $CH(Me)CF_3$ | H | COMe | - |
| 1579 | *c*-Pen | $CH(Me)CF_3$ | H | COEt | - |
| 1580 | *c*-pen | $CH(Me)CF_3$ | H | $CO_3H$ | - |
| 1581 | *c*-Pen | $CH(Me)CF_3$ | H | $CC_3Me$ - | |
| 1582 | *c*-pen | $CH(Me)CF_3$ | H | $CC_3Et$ | - |
| 1583 | *c*-Pen | $CH(Me)CF_3$ | H | $CONH_3$ | - |
| 1584 | *c*-Pen | $CH(Me)OF_3$ | H | CONHMe | - |
| 1585 | *c*-Pen | $CH(Me)CF_3$ | H | $CON(Me)_3$ | - |
| 1586 | *c*-Pen | $CH(Me)CF_3$ | H | Me - | |
| 1587 | *c*-Pen | $CH(Me)CF_3$ | H | Et | - |
| 1588 | *c*-Pen | $CH(Me)CF_3$ | H | *iso*-Pr | - |
| 1589 | *c*-Pen | $CH(Me)CF_3$ | H | *c*-Pr | - |
| 1590 | *c*-Pen | $CH(Me)CF_3$ | H | $CH_3F$ | - |
| 1591 | *c*-Pen | $CH(Me)CF_3$ | H | $CH_3Cl$ | - |
| 1592 | *c*-Pen | $CH(Me)CF_3$ | H | $CH_3Br$ | - |
| 1593 | *c*-Pen | $CH(Me)CF_3$ | H | $CHF_3$ | - |
| 1594 | *c*-Pen | $CH(Me)CF_3$ | H | $CF_3$ | - |
| 1595 | *c*-Pen | Et | Et | H | - |
| 1596 | *c*-Pen | Et | Et | F | - |
| 1597 | *c*-Pen | Et | Et | Cl | - |
| 1598 | *c*-Pen | Et | Et | Br | - |
| 1599 | *c*-Pen | Et | Et | CN | - |
| 1600 | *c*-Pen | Et | Et | Me | - |
| 1601 | *c*-Pen | Et | Et | $CF_3$ | - |
| 1602 | *c*-Pen | $CH(CF_3)(CH_3)_3$- | | H | - |
| 1603 | *c*-Pen | $CH(CF_3)(CH_3)_3$- | | F | - |
| 1604 | *c*-Pen | $CH(CF_3)(CH_3)_3$- | | Cl | - |
| 1605 | *c*-Pen | $CH(CF_3)(CH_3)_3$- | | Br | - |
| 1606 | *c*-Pen | $CH(CF_3)(CH_3)_3$- | | CN | - |
| 1607 | *c*-Pen | $CH(CF_3)(CH_3)_3$- | | Me | - |
| 1608 | *c*-Pen | $CH(CF_3)(CH_3)_3$- | | $CF_3$ | - |
| 1609 | *c*-Pen | $CH(CF_3)(CH_3)_3$- | | H | - |
| 1610 | *c*-Pen | $CH(CF_3)(CH_3)_3$- | | F | - |
| 1611 | *c*-Pen | $CH(CF_3)(CH_3)_3$- | | Cl - | |
| 1612 | *c*-Pen | $CH(CF_3)(CH_3)_3$- | | Br | - |
| 1613 | *c*-Pen | $CH(CF_3)(CH_3)_3$- | | CN | - |
| 1614 | *c*-Pen | $CH(CF_3)(CH_3)_3$- | | Me | - |
| 1615 | *c*-Pen | $CH(CF_3)(CH_3)_3$- | | $CF_3$ | - |

(continued)

| Compound No | R | $R^1$ | $R^3$ | X | Ym |
|---|---|---|---|---|---|
| 1616 | *c*-Hex | *iso*-Pr | H | H | - |
| 1617 | *c*-Hex | *iso*-Pr | H | Cl | - |
| 1618 | *c*-Hex | *iso*-Pr | H | CN | - |
| 1619 | *c*-Hex | *iso*-Pr | H | Me | - |
| 1620 | *c*-Hex | $CH_3CF_3$ | H | H | - |
| 1621 | *c*-Hex | $CH_3CF_3$ | H | Cl | - |
| 1622 | *c*-Hex | $CH_3CF_3$ | H | CN | - |
| 1623 | *c*-Hex | $CH_3CF_3$ | H | Me | - |
| 1624 | *c*-Hex | Et | Et | H | - |
| 1625 | *c*-Hex | Et | Et | Cl | - |

[Table 33]

| Compound No | R | $R^1$ | $R^3$ | X | Ym |
|---|---|---|---|---|---|
| 1626 | *c*-Hex | Et | Et | CN | - |
| 1627 | *c*-Hex | Et | Et | Me | - |
| 1628 | *c*-Hex | $-(CH_2)_2CH(Me)(CH_2)_2-$ | | H | - |
| 1629 | *c*-Hex | $-(CH_2)_2CH(Me)(CH_2)_2-$ | | Cl - | |
| 1630 | c-Hex | $-(CH_2)_2CH(Me)(CH_2)_2-$ | | CN | - |
| 1631 | *c*-Hex | $-(CH_2)_2CH(Me)(CH_2)_2-$ | | Me | - |
| 1632 | $CH_3$*c*-Pr | *iso*-Pr | H | H | - |
| 1633 | $CH_3$*c*-Pr | *iso*-Pr | H | Cl | - |
| 1634 | $CH_3$*c*-Pr | *iso*-Pr | H | CN | - |
| 1635 | $CH_3$*c*-Pr | *iso*-Pr | H | Me | - |
| 1636 | $CCH_3$*c*-Pr | $CH_2CF_3$ | H | H | - |
| 1637 | $CH_3$*c*-Pr | $CH_2CF_3$ | H | Cl | - |
| 1638 | $CH_3$*c*-Pr | $CH_2CF_3$ | H | CN | - |
| 1639 | $CH_3$*c*-Pr | $CH_2CF_3$ | H | Me | - |
| 1640 | $CH_3$*c*-Pr | Et | Et | H | - |
| 1641 | $CH_3$*c*-Pr | Et | Et | Cl | - |
| 1642 | $CH_3$*c*-Pr | Et | Et | CN | - |
| 1643 | $CH_3$*c*-Pr | Et | Et | Me | - |
| 1644 | $CH_3$*c*-Pr | $-(CH_2)_2CH(Me)(CH_2)_2-$ | | H | - |
| 1645 | $CH_3$*c*-Pr | $-(CH_2)_2CH(Me)(CH_2)_2-$ | | Cl | - |
| 1646 | $CH_3$*c*-Pr | $-(CH_2)_2CH(Me)(CH_2)_2-$ | | CN | - |
| 1647 | $CH_3$*c*-Pr | $-(CH_2)_2CH(Me)(CH_2)_2-$ | | Me | - |
| 1648 | $CH_3$*c*-Pr | *iso*-Pr | H | H | - |
| 1649 | $CH_3CF_3$ | *iso*-Pr | H | Cl | - |
| 1650 | $CH_3CF_3$ | *iso*-Pr | H | CN | - |
| 1651 | $CH_3CF_3$ | *iso*-Pr | H | Me | - |
| 1652 | $CH_2CF_3$ | $CH_2CF_3$ | H | H | - |
| 1653 | $CH_2CF_3$ | $CH_2CF_3$ | H | Cl | - |
| 1654 | $CH_2CF_3$ | $CH_2CF_3$ | H | CN | - |
| 1655 | $CH_2CF_3$ | $CH_2CF_3$ | H | Me | - |
| 1656 | $CH_2CF_3$ | Et | Et | H | - |
| 1657 | $CH_2CF_3$ | Et | Et | Cl | - |
| 1658 | $CH_2CF_3$ | Et | Et | CN | - |

(continued)

| Compound No | R | $R^1$ | $R^3$ | X | Ym |
|---|---|---|---|---|---|
| 1659 | $CH_2CF_3$ | Et | Et | Me | - |
| 1660 | $CH_2CF_3$ | $-(CH_2)_2CH(Me)(CH_2)_2-$ | | H | - |
| 1661 | $CH_2CF_3$ | $-(CH_2)_2CH(Me)(CH_2)_2-$ | | Cl | - |
| 1662 | $CH_2CF_3$ | $-(CH_2)_2CH(Me)(CH_2)_2-$ | | CN | - |
| 1663 | $CH_2CF_3$ | $-(CH_2)_2CH(Me)(CH_2)_2-$ | | Me | - |
| 1664 | $CF(Me)_3$ | *iso*-Pr | H | H | - |
| 1666 | $CF(Me)_3$ | *iso*-Pr | H | Cl | - |
| 1666 | $CF(Me)_3$ | *iso*-Pr | H | CN | - |
| 1667 | $CF(Me)_3$ | *iso*-Pr | H | Me | - |
| 1668 | $CF(Me)_3$ | $CH_3CF_3$ | | H | - |
| 1669 | $CF(Me)_3$ | $CH_3CF_3$ | H | Cl | - |
| 1670 | $CF(Me)_3$ | $CH_3CF_3$ | H | CN | - |
| 1671 | $CF(Me)_3$ | $CH_3CF_3$ | H | Me | - |
| 1672 | $CF(Me)_3$ | Et | Et | H | - |
| 1673 | $CF(Me)_3$ | Et | Et | Cl | - |
| 1674 | $CF(Me)_3$ | Et | Et | CN | - |
| 1675 | $CF(Me)_3$ | Et | Et | Me | - |
| 1676 | $CF(Me)_3$ | $-(CH_2)_2CH(Me)(CH_2)_2-$ | | H | - |

[Table 34]

| Compound No | R | $R^1$ | $R^3$ | X | Ym |
|---|---|---|---|---|---|
| 1677 | $CF(Me)_3$ | $-(CH_2)_2CH(Me)(CH_2)_2-$ | | Cl | - |
| 1678 | $CF(Me)_3$ | $-(CH_2)_2CH(Me)(CH_2)_2-$ | | CN | - |
| 1679 | $CF(Me)_3$ | $-(CH_2)_2CH(Me)(CH_2)_2-$ | | Me | - |
| 1680 | CF(Me)Et | *iso*-Pr | H | H | - |
| 1681 | CF(Me)Et | *iso*-Pr | H | Cl | - |
| 1682 | CF(Me)Et | *iso*-Pr | H | CN | - |
| 1683 | CF(Me)Et | *iso*-Pr | H | Me | - |
| 1684 | CF(Me)Et | $CH_2CF_3$ | H | H | - |
| 1685 | CF(Me)Et | $CH_2CF_3$ | H | Cl | - |
| 1686 | CF(Me)Et | $CH_2CF_3$ | H | CN | - |
| 1687 | CF(Me)Et | $CH_2CF_3$ | H | Me | - |
| 1688 | CF(Me)Et | Et | Et | H | - |
| 1689 | CF(Me)Et | Et | Et | Cl | - |
| 1690 | CF(Me)Et | Et | Et | CN | - |
| 1691 | CF(Me)Et | Et | Et | Me | - |
| 1692 | CF(Me)Et | $-(CH_2)_2CH(Me)(CH_2)_2-$ | | H | - |
| 1693 | CF(Me)Et | $-(CH_2)_2CH(Me)(CH_2)_2-$ | | Cl | - |
| 1694 | CF(Me)Et | $-(CH_2)_2CH(Me)(CH_2)_2-$ | | CN | - |
| 1695 | CF(Me)Et | $-(CH_2)_2CH(Me)(CH_2)_2-$ | | Me | - |
| 1696 | $CF(CF_3)_2$ | *iso*-Pr | H | H | - |
| 1697 | $CF(CF_3)_2$ | *iso*-Pr | H | Cl | - |
| 1698 | $CF(CF_3)_2$ | *iso*-Pr | H | CN | - |
| 1699 | $CF(CF_3)_2$ | *iso*-Pr | H | Me | - |
| 1700 | $CF(CF_3)_2$ | $CH_2CF_3$ | H | H | - |

(continued)

| Compound No | R | $R^1$ | $R^3$ | X | Ym |
|---|---|---|---|---|---|
| 1701 | $CF(CF_3)_2$ | $CH_2CF_3$ | H | Cl | |
| 1702 | $CF(CF_3)_2$ | $CH_2CF_3$ | H | CN | - |
| 1703 | $CF(CF_3)_2$ | $CH_2CF_3$ | H | Me | - |
| 1704 | $CF(CF_3)_2$ | Et | Et | H | - |
| 1705 | $CF(CF_3)_2$ | Et | Et | Cl | - |
| 1706 | $CF(CF_3)_2$ | Et | Et | CN | - |
| 1707 | $CF(CF_3)_2$ | Et | Et | Me | - |
| 1708 | $CF(CF_3)_2$ | $-(CH_2)_2CH(Me)(CH_2)_2-$ | | H | - |
| 1709 | $CF(CF_3)_2$ | $-(CH_2)_2CH(Me)(CH_2)_2-$ | | Cl | - |
| 1710 | $CF(CF_3)_2$ | $-(CH_2)_2CH(Me)(CH_2)_2-$ | | CN | - |
| 1711 | $CF(CF_3)_2$ | $-(CH_2)_2CH(Me)(CH_2)_2-$ | | Me | - |
| 1712 | $CH_3OH$ | *iso*-Pr | H | H | - |
| 1713 | $CH_3OH$ | *iso*-Pr | H | Cl | - |
| 1714 | $CH_3OH$ | *iso*-Pr | H | CN | - |
| 1715 | $CH_3OH$ | *iso*-Pr | H | Me | - |
| 1716 | $CH_3OH$ | $CH_2CF_3$ | H | H | - |
| 1717 | $CH_3OH$ | $CH_2CF_3$ | H | Cl | - |
| 1718 | $CH_3OH$ | $CH_2CF_3$ | H | CN | - |
| 1719 | $CH_3OH$ | $CH_2CF_3$ | H | Me | - |
| 1720 | $CH_3OH$ | Et | Et | H | - |
| 1721 | $CH_3OH$ | Et | Et | Cl | - |
| 1722 | $CH_3OH$ | Et | Et | CN | - |
| 1723 | $CH_3OH$ | Et | Et | Me | - |
| 1724 | $CH_3OH$ | $-(CH_2)_2CH(Me)(CH_2)_2-$ | | H | - |
| 1725 | $CH_3OH$ | $-(CH_2)_2CH(Me)(CH_2)_2-$ | | Cl | - |
| 1726 | $CH_3OH$ | $-(CH_2)_2CH(Me)(CH_2)_2-$ | | CN | - |
| 1727 | $CH_3OH$ | $-(CH_2)_2CH(Me)(CH_2)_2-$ | | Me | - |

[Table 35]

| Compound No | R | $R^1$ | $R^2$ | X | Ym |
|---|---|---|---|---|---|
| 1728 | CH(OH)Me | *iso*-Pr | H | H | - |
| 1729 | CH(OH)Me | *iso*-Pr | H | Cl | - |
| 1730 | CH(OH)Me | *iso*-Pr | H | CN | - |
| 1731 | CH(OH)Me | *iso*-Pr | H | Me | - |
| 1732 | CH(OH)Me | $CH_3CF_3$ | H | H | - |
| 1733 | CH(OH)Me | $CH_3CF_3$ | H | Cl | - |
| 1734 | CH(OH)Me | $CH_3CF_3$ | H | CN | - |
| 1735 | CH(OH)Me | $CH_3CF_3$ | H | Me | - |
| 1736 | CH(OH)Me | Et | Et | H | - |
| 1737 | CH(OH)Me | Et | Et | Cl | - |
| 1738 | CH(OH)Me | Et | Et | CN | |
| 1739 | CH(OH)Me | Et | Et | Me | - |
| 1740 | CH(OH)Me | $-(CH_2)_2CH(Me)(CH_2)_2-$ | | H | - |
| 1741 | CH(OH)Me | $-(CH_2)_2CH(Me)(CH_2)_2-$ | | Cl | - |
| 1742 | CH(OH)Me | $-(CH_2)_2CH(Me)(CH_2)_2-$ | | CN | - |
| 1743 | CH(OH)Me | $-(CH_2)_2CH(Me)(CH_2)_2-$ | | Me | - |

(continued)

| Compound No | R | $R^1$ | $R^2$ | X | Ym |
|---|---|---|---|---|---|
| 1744 | CH(OH)Me | *iso*-Pr | H | H | - |
| 1745 | CH(OH)Et | *iso*-Pr | H | Cl | - |
| 1746 | CH(OH)Et | *iso*-Pr | H | CN | - |
| 1747 | CH(OH)Et | *iso*-Pr | H | Me | - |
| 1748 | CH(OH)Et | $CH_2CF_3$ | H | H | - |
| 1749 | CH(OH)Et | $CH_2CF_3$ | H | Cl | - |
| 1750 | CH(OH)Et | $CH_2CF_3$ | H | CN | - |
| 1751 | CH(OH)Et | $CH_2CF_3$ | H | Me | - |
| 1752 | CH(OH)Et | Et | Et | H | - |
| 1753 | CH(OH)Et | Et | Et | Cl | - |
| 1754 | CH(OH)Et | Et | Et | CN | - |
| 1755 | CH(OH)Et | Et | Et | Me | - |
| 1756 | CH(OH)Et | $-(CH_2)_2CH(Me)(CH_2)_2-$ | | H | - |
| 1757 | CH(OH)Et | $-(CH_2)_2CH(Me)(CH_2)_2-$ | | Cl | - |
| 1758 | CH(OH)Et | $-(CH_2)_2CH(Me)(CH_2)_2-$ | | CN | - |
| 1759 | CH(OH)Et | $-(CH_2)_2CH(Me)(CH_2)_2-$ | | Me | - |
| 1760 | CH(OH)*iso*-Pr | *iso*-Pr | H | H | - |
| 1761 | CH(OH)*iso*-Pr | *iso*-Pr | H | Cl | - |
| 1762 | CH(OH)*iso*-Pr | *iso*-Pr | H | CN | - |
| 1763 | CH(OH)*iso*-Pr | *iso*-Pr | H | Me | - |
| 1764 | CH(OH)*iso*-Pr | $CH_2CF_3$ | H | H | - |
| 1765 | CH(OH)*iso*-Pr | $CH_2CF_3$ | H | Cl | - |
| 1766 | CH(OH)*iso*-Pr | $CH_2CF_3$ | H | CN | - |
| 1767 | CH(OH)*iso*-Pr | $CH_2CF_3$ | H | Me | - |
| 1768 | CH(OH)*iso*-Pr | Et | Et | H | - |
| 1769 | CH(OH)*iso*-Pr | Et | Et | Cl | - |
| 1770 | CH(OH)*iso*-Pr | Et | Et | CN | - |
| 1771 | CH(OH)*iso*-Pr | Et | Et | Me | - |
| 1172 | CH(OH)*iso*-Pr | $-(CH_2)_2CH(Me)(CH_2)_2-$ | | H | - |
| 1773 | CH(OH)*iso*-Pr | $-(CH_2)_2CH(Me)(CH_2)_2-$ | | Cl | - |
| 1774 | CH(OH)*iso*-Pr | $-(CH_2)_2CH(Me)(CH_2)_2-$ | | CN | - |
| 1775 | CH(OH)*iso*-Pr | $-(CH_2)_2CH(Me)(CH_2)_2-$ | | Me | - |
| 1776 | CH(OMe)Me | *iso*-Pr | H | H | - |
| 1777 | CH(OMe)Me | *iso*-Pr | H | Cl | - |
| 1778 | CH(OMe)Me | *iso*-Pr | H | CN | - |

[Table 36]

| Compound No | R | $R^1$ | $R^2$ | X | Ym |
|---|---|---|---|---|---|
| 1779 | CH(OMe)Me | *iso*-Pr | H | Me | - |
| 1780 | CH(OMe)Me | $CH_3CF_3$ | H | H | - |
| 1781 | CH(OMe)Me | $CH_3CF_3$ | H | Cl | - |
| 1782 | CH(OMe)Me | $CH_3CF_3$ | H | CN | - |
| 1783 | CH(OMe)Me | $CH_3CF_3$ | H | Me | - |
| 1784 | CH(OMe)Me | Et | Et | H | - |
| 1785 | CH(OMe)Me | Et | Et | Cl | - |
| 1786 | CH(OMe)Me | Et | Et | CN | - |

(continued)

| Compound No | R | $R^1$ | $R^2$ | X | Ym |
|---|---|---|---|---|---|
| 1787 | CH(OMe)Me | Et | Et | Me | - |
| 1788 | CH(OMe)Me | $-(CH_2)_2CH(Me)(CH_2)_2-$ | | H | - |
| 1789 | CH(OMe)Me | $-(CH_2)_2CH(Me)(CH_2)_2-$ | | Cl | - |
| 1790 | CH(OMe)Me | $-(CH_2)_2CH(Me)(CH_2)_2-$ | | CN | - |
| 1791 | CH(OMe)Me | $-(CH_2)_2CH(Me)(CH_2)_2-$ | | Me | - |
| 1792 | CH(OEt)Me | *iso*-Pr | H | H | - |
| 1793 | CH(OEt)Me | *iso*-Pr | H | Cl | - |
| 1794 | CH(OEt)Me | *iso*-Pr | H | CN | - |
| 1795 | CH(OEt)Me | *iso*-Pr | H | Me | - |
| 1796 | CH(OEt)Me | $CH_2CF_3$ | H | H | - |
| 1797 | CH(OEt)Me | $CH_2CF_3$ | H | Cl | - |
| 1798 | CH(OEt)Me | $CH_2CF_3$ | H | CN | - |
| 1799 | CH(OEt)Me | $CH_2CF_3$ | H | Me | - |
| 1800 | CH(OEt)Me | Et | Et | H | - |
| 1801 | CH(OEt)Me | Et | Et | Cl | |
| 1802 | CH(OEt)Me | Et | Et | CN | - |
| 1803 | CH(OEt)Me | Et | Et | Me | - |
| 1804 | CH(OEt)Me | $-(CH_2)_2CH(Me)(CH_2)_2-$ | | H | - |
| 1805 | CH(OEt)Me | $-(CH_2)_2CH(Me)(CH_2)_2-$ | | Cl | - |
| 1806 | CH(OEt)Me | $-(CH_2)_2CH(Me)(CH_2)_2-$ | | CN | - |
| 1807 | CH(OEt)Me | $-(CH_2)_2CH(Me)(CH_2)_2-$ | | Me | - |
| 1808 | CH(OMe)Et | *iso*-Pr | H | H | - |
| 1809 | CH(OMe)Et | *iso*-Pr | H | Cl | - |
| 1810 | CH(OMe)Et | *iso*-Pr | H | CN | - |
| 1811 | CH(OMe)Et | *iso*-Pr | H | Me | - |
| 1812 | CH(OMe)Et | $CH_2CF_3$ | H | H | - |
| 1813 | CH(OMe)Et | $CH_2CF_3$ | H | Cl | - |
| 1814 | CH(OMe)Et | $CH_2CF_3$ | H | CN | - |
| 1815 | CH(OMe)Et | $CH_2CF_3$ | H | Me | - |
| 1816 | CH(OMe)Et | Et | Et | H | - |
| 1817 | CH(OMe)Et | Et | Et | Cl | - |
| 1818 | CH(OMe)Et | Et | Et | CN | - |
| 1819 | CH(OMe)Et | Et | Et | Me | - |
| 1820 | CH(OMe)Et | $-(CH_2)_2CH(Me)(CH_2)_2-$ | | H | - |
| 1821 | CH(OMe)Et | $-(CH_2)_2CH(Me)(CH_2)_2-$ | | Cl | - |
| 1822 | CH(OMe)Et | $-(CH_2)_2CH(Me)(CH_2)_2-$ | | CN | - |
| 1823 | CH(OMe)Et | $-(CH_2)_2CH(Me)(CH_2)_2-$ | | Me | - |
| 1824 | O(=O)H | *iso*-Pr | H | H | - |
| 1825 | O(=O)H | *iso*-Pr | H | Cl | - |
| 1826 | O(=O)H | *iso*-Pr | H | CN | - |
| 1827 | O(=O)H | *iso*-Pr | H | Me | - |
| 1828 | O(=O)H | $CH_2CF_3$ | H | H | - |
| 1829 | O(=O)H | $CH_2CF_3$ | H | Cl | - |

[Table 37]

| Compound No | R | $R^1$ | $R^2$ | X | Ym |
|---|---|---|---|---|---|
| 1830 | C(=O)H | $CH_3CF_3$ | H | CN | - |
| 1831 | C(=O)H | $CH_3CF_3$ | H | Me | - |
| 1632 | C(=O)H | Et | Et | H | - |
| 1833 | C(=O)H | Et | Et | Cl | - |
| 1834 | C(=O)H | Et | Et | CN | - |
| 1835 | C(=O)H | Et | Et | Me | - |
| 1836 | C(=O)H | $-(CH_3)_3CH(Me)(CH_3)_3-$ | | H | - |
| 1837 | C(=O)H | $-(CH_3)_3CH(Me)(CH_3)_3-$ | | Cl | - |
| 1838 | C(=O)H | $-(CH_3)_3CH(Me)(CH_3)_3-$ | | CN | - |
| 1839 | C(=O)H | $-(CH_3)_3CH(Me)(CH_3)_3-$ | | Me | - |
| 1840 | C(=O)Me | *iso*-Pr | H | H | - |
| 1841 | C(=O)Me | *iso*-Pr | H | Cl | - |
| 1842 | C(=O)Me | *iso*-Pr | H | CN | - |
| 1843 | C(=O)Me | *iso*-Pr | H | Me | - |
| 1844 | C(=O)Me | $CH_3CF_3$ | H | H | - |
| 1845 | C(=O)Me | $CH_3CF_3$ | H | Cl | - |
| 1846 | C(=O)Me | $CH_3CF_3$ | H | CN | - |
| 1847 | C(=O)Me | $CH_3CF_3$ | H | Me | - |
| 1848 | C(=O)Me | Et | Et | H | - |
| 1849 | C(=O)Me | Et | Et | Cl | - |
| 1850 | C(=O)Me | Et | Et | $SO_3Me$ | - |
| 1851 | C(=O)Me | Et | Et | CN | - |
| 1852 | C(=O)Me | Et | Et | Me | - |
| 1853 | C(=O)Me | $-(CH_3)_3CH(Me)(CH_3)_3-$ | | H | - |
| 1854 | C(=O)Me | $-(CH_3)_3CH(Me)(CH_3)_3$ | | Cl | - |
| 1855 | C(=O)Me | $-(CH_3)_3CH(Me)(CH_3)_3-$ | | CN | - |
| 1856 | C(=O)Me | $(CH_3)_3CH(Me)(CH_3)_3-$ | | Me | - |
| 1857 | C(=O)Et | *iso*-Pr | H | H | - |
| 1858 | C(=O)Et | *iso*-Pr | H | Cl | - |
| 1859 | C(=O)Et | *iso*-Pr | H | CN | - |
| 1860 | C(=O)Et | *iso*-Pr | H | Me | - |
| 1861 | C(=O)Et | $CH_2CF_3$ | H | H | - |
| 1862 | C(=O)Et | $CH_2OF_3$ | H | Cl | - |
| 1863 | C(C=O)Et | $OH_2CF_3$ | H | CN | - |
| 1864 | C(=O)Et | $CH_2CF_3$ | H | Me | - |
| 1865 | C(=O)Et | Et | Et | H | - |
| 1866 | C(=O)Et | Et | Et | Cl | - |
| 1867 | C(=O)Et | Et | Et | CN | - |
| 1868 | C(=O)Et | Et | Et | Me | - |
| 1869 | C(=O)Et | $-(CH_3)_3CH(Me)(CH_3)_3-$ | | H | - |
| 1870 | C(=O)Et | $-(CH_3)_3CH(Me)(CH_3)_3-$ | | Cl | - |
| 1871 | C(=O)Et | $-(CH_3)_3CH(Me)(CH_3)_3-$ | | CN | - |
| 1872 | C(=O)Et | $-(CH_3)_3CH(Me)(CH_3)_3-$ | | Me | - |
| 1873 | C(=O)*iso*-Pr | *iso*-Pr | H | H | - |
| 1874 | C(=O)*iso*-Pr | *iso*-Pr | H | Cl | - |
| 1875 | C(=O)*iso*-Pr | *iso*-Pr | H | CN | - |
| 1876 | C(=O)*iso*-Pr | *iso*-Pr | H | Me | - |
| 1877 | C(=O)*iso*-Pr | $CH_2CF_3$ | H | H | - |

(continued)

| Compound No | R | $R^1$ | $R^2$ | X | Ym |
|---|---|---|---|---|---|
| 1878 | C(=O)*iso*-Pr | $CH_2CF_3$ | H | Cl | - |
| 1879 | C(=O)*iso*-Pr | $CH_3CF_3$ | H | CN | - |
| 1880 | C(=O)*iso*-Pr | $CH_2CF_3$ | H | Me | - |

[Table 381

| Compound No | R | $R^1$ | $R^2$ | X | Ym |
|---|---|---|---|---|---|
| 1881 | C(=O)*iso*-Pr | Et | Et | H | - |
| 1882 | C(=O)*iso*-Pr | Et | Et | Cl | - |
| 1883 | C(=O)*iso*-Pr | Et | Et | CN | - |
| 1884 | C(=O)*iso*-Pr | Et | Et | Me | - |
| 1885 | C(=O)*iso*-Pr | $-(CH_3)_3CH(Me)(CH_3)_3-$ | | H | - |
| 1886 | C(=O)*iso*-Pr | $-(CH_3)_3CH(Me)(CH_3)_3-$ | | Cl | - |
| 1887 | C(=O)*iso*-Pr | $-(CH_3)_3CH(Me)(CH_3)_3$ | | CN | - |
| 1888 | C(=O)*iso*-Pr | $-(CH_3)_3CH(Me)(CH_3)_3-$ | | Me | - |
| 1889 | $CH=CH_3$ | *iso*-Pr | H | H | - |
| 1890 | $CH=CH_3$ | *iso*-Pr | H | Cl | - |
| 1891 | $CH=CH_3$ | *iso*-Pr | H | CN | - |
| 1892 | $CH=CH_3$ | *iso*-Pr | H | Me | - |
| 1893 | $CH=CH_3$ | $CH_3CF_3$ | H | H | - |
| 1894 | $CH=CH_3$ | $CH_3CF_3$ | H | Cl | - |
| 1895 | $CH=CH_3$ | $CH_2CF_3$ | H | CN | - |
| 1896 | $CH=CH_3$ | $CH_3CF_3$ | H | Me | - |
| 1897 | $CH=CH_3$ | Et | Et | H | - |
| 1898 | $CH=CH_3$ | Et | Et | Cl | - |
| 1899 | $CH=CH_3$ | Et | Et | CN | - |
| 1900 | $CH=CH_3$ | Et | Et | Me | - |
| 1901 | $CH=CH_3$ | $-(CH_3)_3CH(Me)(CH_3)_3-$ | | H | - |
| 1902 | $CH=CH_3$ | $-(CH_3)CH(Me)(CH_3)_3-$ | | Cl | - |
| 1903 | $CH=CH_3$ | $(CH_3)_3CH(Me)(CH_3)_3.$ | | CN | - |
| 1904 | $CH=CH_3$ | $-(CH_3)_3CH(Me)(CH_3)_3-$ | | Me | - |
| 1905 | $CH_3CH=CH_3$ | *iso*-Pr | H | H | - |
| 1906 | $CH_3CH=CH_3$ | *iso*-Pr | H | Cl | - |
| 1907 | $CH_3CH=CH_3$ | *iso*-Pr | H | CN | - |
| 1908 | $CH_3CH=CH_3$ | *iso*-Pr | H | Me | - |
| 1909 | $CH_3CH=CH_3$ | $CH_3CF_3$ | H | H | - |
| 1910 | $CH_3CH=CH_3$ | $CH_3CF_3$ | H | Cl | - |
| 1911 | $CH_3CH=CH_3$ | $CH_3GF_3$ | H | CN | - |
| 1912 | $CH_3CH=CH_3$ | $CH_3CF_3$ | H | Me | - |
| 1913 | $CH_3CH=CH_3$ | Et | Et | H | - |
| 1914 | $CH_3CH=CH_3$ | Et | Et | Cl | - |
| 1915 | $CH_3CH=CH_3$ | Et | Et | CN | - |
| 1916 | $CH_2CH=CH_3$ | Et | Et | Me | - |
| 1917 | $CH_3CH=CH_3$ | $-(CH_3)_3CH(Me)(CH_3)_3-$ | | H | - |
| 1918 | $CH_3CH=CH_3$ | $-(CH_3)_3CH(Me)(CH_3)_3-$ | | Cl | - |
| 1919 | $CH_3CH=CH_3$ | $-(CH_3)_3CH(Me)(CH_3)_3-$ | | CN | - |
| 1920 | $CH_3CH=CH_3$ | $-(CH_3)_3CH(Me)(CH_3)_3-$ | | Me | - |

(continued)

| Compound No | R | $R^1$ | $R^2$ | X | Ym |
|---|---|---|---|---|---|
| 1921 | $CH(Me)CH=CH_3$ | *iso*-Pr | H | H | - |
| 1922 | $CH(Me)CH=CH_3$ | *iso*-Pr | H | Cl | - |
| 1923 | $CH(Me)CH=CH_3$ | *iso*-Pr | H | CN | - |
| 1924 | $CH(Me)CH=CH_3$ | *iso*-Pr | H | Me | - |
| 1925 | $CH(Me)CH=CH_3$ | $CH_3CF_3$ | H | H | - |
| 1926 | $CH(Me)CH=CH_3$ | $CH_3CF_3$ | H | Cl | - |
| 1927 | $CH(Me)CH=CH_3$ | $CH_3CF_3$ | H | CN | - |
| 1928 | $CH(Me)CH=CH_3$ | $CH_3CF_3$ | H | Me | - |
| 1929 | $CH(Me)CH=CH_3$ | Et | Et | H | - |
| 1930 | $CH(Me)CH=CH_3$ | Et | Et | Cl | - |
| 1931 | $CH(Me)CH=CH_3$ | Et | Et | CN | - |

[Table 39]

| Compound No. | R | $R^1$ | $R^2$ | X | Ym |
|---|---|---|---|---|---|
| 1932 | $CH(Me)CH=CH_3$ | Et | Et | Me | - |
| 1933 | $CH(Me)CH=CH_3$ | $-(CH_3)_3CH(Me)(CH_3)_3-$ | | H | - |
| 1934 | $CH(Me)CH=CH_3$ | $-(CH_3)_3CH(Me)(CH_3)_3-$ | | Cl | - |
| 1935 | $CH(Me)CH=CH_3$ | $-(CH_3)_3CH(Me)(CH_3)_3-$ | | CN | - |
| 1936 | $CH(Me)CH=CH_3$ | $-(CH_3)_3CH(Me)(CH_3)_3-$ | | Me | - |
| 1937 | C(Me)=CHMe | *iso*-Pr | H | H | - |
| 1938 | C(Me)=CHMe | *iso*-Pr | H | Cl | - |
| 1939 | C(Me)=CHMe | *iso*-Pr | H | CN | - |
| 1940 | C(Me)=CHMe | *iso*-Pr | H | Me | - |
| 1941 | C(Me)=CHMe | $CH_2CF_3$ | H | H | - |
| 1942 | C(Me)=CHMe | $CH_2CF_3$ | H | Cl | - |
| 1943 | C(Me)=CHMe | $CH_2CF_3$ | H | CN | - |
| 1944 | C(Me)=OHMe | $CH_3CF_3$ | H | Me | - |
| 1945 | C(Me)=CHMe | Et | Et | H | - |
| 1946 | C(Me)=CHMe | Et | Et | Cl | - |
| 1947 | C(Me)=CHMe | Et | Et | CN | - |
| 1948 | C(Me)=CHMe | Et | Et | Me | - |
| 1949 | C(Me)=CHMe | $-(CH_3)_3CH(Me)(CH_3)_3-$ | | H | - |
| 1950 | C(Me)=CHMe | $-(CH_3)_3CH(Me)(CH_3)_3-$ | | Cl | - |
| 1951 | C(Me)=CHMe | $-(CH_3)_3CH(Me)(CH_3)_3-$ | | CN | - |
| 1962 | C(Me)=CHMe | $-(CH_3)_3CH(Me)(CH_3)_3-$ | | Me | - |
| 1953 | 2-cyclopenten-1-yl | *iso*-Pr | H | H | - |
| 1954 | 2-cyclopenten-1-yl | *iso*-Pr | H | Cl | - |
| 1955 | 2-cyclopenten-1-yl | *iso*-Pr | H | CN | - |
| 1956 | 2-cyclopenten-1-yl | *iso*-Pr | H | Me | - |
| 1957 | 2-cyclopenten-1-yl | $CH_2CF_3$ | H | H | - |
| 1958 | 2-cyclopenten-1-yl | $CH_2CF_3$ | H | Cl | - |
| 1959 | 2-cyclopenten-1-yl | $CH_2CF_3$ | H | CN | - |
| 1960 | 2-cyclopenten-1-yl | $CH_2CF_3$ | H | Me | - |
| 1961 | 2-cyclopenten-1-yl | Et | Et | H | - |
| 1962 | 2-cyclopenten-1-yl | Et | Et | Cl | - |
| 1963 | 2-cyclopenten-1-yl | Et | Et | CN | - |

(continued)

| Compound No. | R | $R^1$ | $R^2$ | X | Ym |
|---|---|---|---|---|---|
| 1964 | 2-cyclopenten-1-yl | Et | Et | Me | - |
| 1965 | 2-cyclopenten-1-yl | $-(CH_3)_3$-CH(Me)$(CH_3)_3-$ | | H | - |
| 1966 | 2-cyclopenten-1-yl | $-(CH_3)_3$-CH(Me)$(CH_3)_3-$ | | Cl | - |
| 1967 | 2-cyclopenten-1-yl | $-(CH_3)_3$-CH(Me)$(CH_3)_3-$ | | CN | - |
| 1968 | 2-cyclopenten-1-yl | $-(CH_3)_3$-CH(Me)$(CH_3)_3-$ | | Me | - |
| 1969 | 2-cyclopenten-1-yl | *iso*-Pr | H | H | - |
| 1970 | 2-cyclopenten-1-yl | *iso*-Pr | H | Cl | - |
| 1971 | 2-cyclopenten-1-yl | *iso*-Pr | H | CN | - |
| 1972 | 2-cyclopenten-1-yl | *iso*-Pr | H | Me | - |
| 1973 | 2-cyclopenten-1-yl | $CH_2CF_3$ | H | H | - |
| 1974 | 2-cyclopenten-1-yl | $CH_2CF_3$ | H | Cl | - |
| 1975 | 2-cyclopenten-1-yl | $CH_2CF_3$ | H | CN | - |
| 1976 | 2-cyclopenten-1-yl | $CH_2CF_3$ | H | Me | - |
| 1977 | 2-cyclopenten-1-yl | Et | Et | H | - |
| 1978 | 2-cyclopenten-1-yl | Et | Et | Cl | - |
| 1979 | 2-cyclopenten-1-yl | Et | Et | CN | - |
| 1980 | 2-cyclopenten-1-yl | Et | Et | Me | - |
| 1981 | 2-cyclopenten-1-yl | $-(CH_3)_3CH(Me)(CH_3)_3-$ | | H | - |
| 1982 | 2-cyclopenten-1-yl | $-(CH_3)_3CH(Me)(CH_3)_3-$ | | Cl | - |

[Table 40]

| Compound No | R | $R^1$ | $R^2$ | X | Ym |
|---|---|---|---|---|---|
| 1983 | 2-cyclopenten-1-yl | $-(CH_3)_3CH(Me)(CH_3)_3-$ | | CN | - |
| 1984 | 2-cyclopenten-1-yl | $-(CH_3)_3CH(Me)(CH_3)_3-$ | | Me | - |
| 1985 | 1,3-dioxolan-2-yl | *iso*-Pr | H | H | - |
| 1986 | 1,3-dioxolan-2-yl | *iso*-Pr | H | Cl | - |
| 1987 | 1,3-dioxolan-2-yl | *iso*-Pr | H | CN | - |
| 1988 | 1,3-dioxolan-2-yl | *iso*-Pr | H | Me | - |
| 1989 | 1,3-dioxolan-2-yl | $CH_2CF_3$ | H | H | - |
| 1990 | 1,3-dioxolan-2-yl | $CH_2CF_3$ | H | Cl | - |
| 1991 | 1,3-dioxolan-2-yl | $CH_2CF_3$ | H | CN | - |
| 1992 | 1,3-dioxolan-2-yl | $CH_2CF_3$ | H | Me | - |
| 1993 | 1,3-dioxolan-2-yl | Et | Et | H | - |
| 1994 | 1,3-dioxolan-2-yl | Et | Et | Cl | - |
| 1995 | 1,3-dioxolan-2-yl | Et | Et | $SO_2Me$ | - |
| 1996 | 1,3-dioxolan-2-yl | Et | Et | CN | - |
| 1991 | 1,3-dioxolan-2-yl | Et | Et | Me | - |
| 1998 | 1,3-dioxolan-2-yl | $-(CH_3)_3CH(Me)(CH_3)_3-$ | | H | - |
| 1999 | 1,3-dioxolan-2-yl | $-(CH_3)_3CH(Me)(CH_3)_3-$ | | Cl | - |
| 2000 | 1,3-dioxolan-2-yl | $-(CH_3)_3CH(Me)(CH_3)_3-$ | | CN | - |
| 2001 | 1,3-dioxolan-2-yl | $-(CH_3)_3CH(Me)(CH_3)_3-$ | | Me | - |
| 2002 | 1,3-dioxan-2-yl | *iso*-Pr | H | H | - |
| 2003 | 1,3-dioxan-2-yl | *iso*-Pr | H | Cl | - |
| 2004 | 1,3-dioxan-2-yl | *iso*-Pr | H | CN | - |
| 2005 | 1,3-dioxan-2-yl | *iso*-Pr | H | Me | - |

(continued)

| Compound No | R | $R^1$ | $R^2$ | X | Ym |
|---|---|---|---|---|---|
| 2006 | 1,3-dioxan-2-yl | $CH_2CF_3$ | H | H | - |
| 2007 | 1,3-dioxan-2-yl | $CH_2CF_3$ | H | Cl | |
| 2008 | 1,3-dioxan-2-yl | $CH_2CF_3$ | H | CN | - |
| 2009 | 1,3-dioxan-2-yl | $CH_2CF_3$ | H | Me | - |
| 2010 | 1,3-dioxan-2-yl | Et | Et | H | - |
| 2011 | 1,3-dioxan-2-yl | Et | Et | Cl | - |
| 2012 | 1,3-dioxan-2-yl | Et | Et | CN | - |
| 2013 | 1,3-dioxan-2-yl | Et | Et | Me | - |
| 2014 | 1,3-dioxan-2-yl | $-(CH_3)_3CH(Me)(CH_2)_3-$ | | H | - |
| 2015 | 1,3-dioxan-2-yl | $-(CH_3)_3CH(Me)(CH_2)_3-$ | | Cl | - |
| 2016 | 1,3-dioxan-2-yl | $-(CH_3)_3CH(Me)(CH_2)_3-$ | | CN | - |
| 2017 | 1,3-dioxan-2-yl | $-(CH_3)_3CH(Me)(CH_2)_3-$ | | Me | - |

The compound of the present application represented by Formula [I] can be produced according to Production Methods shown below, but not limited by these methods. The production methods are described in detail for every process.

<Production Method 1>

(Process 1)

[Chemical Formula 3]

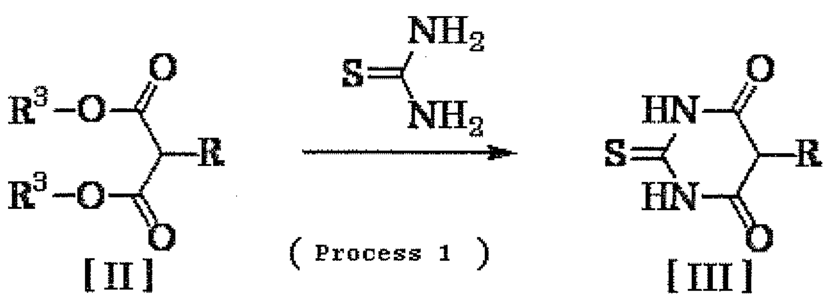


[II] [III]

(wherein R has the same meaning as defined above and $R^3$s are each a leaving group such as a $C_{1-6}$ alkyl group, an optionally substituted phenyl group or an optionally substituted benzyl group).

The compound represented by Formula [III] can be produced by allowing the compound represented by Formula [II] to react with thiourea in a suitable solvent in the presence of a suitable base.

The reaction temperature of the present reaction is in the arbitrarily range of from 0˚C to reflux temperature in the reaction system, preferably from 10 to 150˚C.

The reaction time of the present reaction varies according to a reaction temperature, a reactant, a reaction amount, or the like, but is between 0.5 and 120 hours.

For the amount of agents to be provided in the present reaction, 1 to 2 equivalents of the thiourea and 1 to 5 equivalents of the base are used with respect to 1 equivalent of the compound represented by Formula [II]. In addition, the amount of solvent to be used is from 0 to 50 L (liter), preferably from 0.1 to 3.0 L, with respect to 1 mole of the compound represented by Formula [II].

The solvent for use in the present process may be any solvent as long as it is an inert solvent not inhibiting the process of the present reaction. Examples thereof may include ethers such as 1,2-dimethoxyethane and tetrahydrofuran; amides such as N,N-dimethylacetoamide, N,N-dimethylformamide, 1,3-dimethyl-2-imidazolidinone and N-methyl-2-pyrrolidinone; sulfur compounds such as dimethylsulfoxide and sulfolane; aromatic hydrocarbons such as benzene, toluene and xylene; alcohols such as methanol, ethanol, propanol, isopropanol, butanol, 2-methoxyethanol and tert-butanol; water; and a mixture thereof.

Examples of the base that can be used in the present process may include organic bases such as pyridine, triethylamine, tributylamine and 1,8-diazabicyclo[5.4.0]-7-undecene; inorganic bases such as alkali metal hydroxide, for example, sodium hydroxide or potassium hydroxide, alkaline-earth metal hydroxide, for example, calcium hydroxide or magnesium hydroxide, alkali metal carbonates, for example, sodium carbonate or potassium carbonate, alkali metal acetates, for example, sodium acetate or potassium acetate and alkali metal bicarbonates, for example, sodium bicarbonate or potassium bicarbonate; and alcohol metal salts such as sodium methoxide, sodium ethoxide and potassium tert-butoxide.
After completion of the reaction, the compound represented by Formula [III] that is a desired product of the present reaction can be used in the subsequent process without being isolated and purified, but can be collected from the reaction system by a usual method and purified by a manipulation such as column chromatography or recrystallization, as the case requires.

(Process 2)

[Chemical Formula 4]

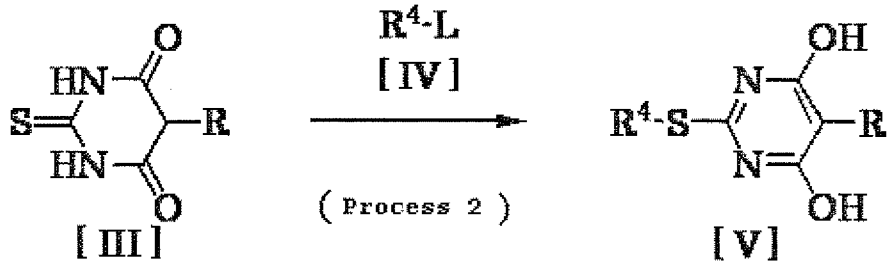


(wherein R has the same meaning as defined above, $R^4$ is a $C_{1-6}$ alkyl group, an optionally substituted phenyl group, or an optionally substituted benzyl group and L is a halogen atom, an optionally substituted alkylsulfonyloxy group, an optionally substituted phenylsulfonyloxy group, or an optionally substituted benzylsulfonyloxy group).
The compound represented by Formula [V] can be produced by allowing the compound represented by Formula [III] to react with the compound represented by Formula [IV] in a suitable solvent, in the presence of a suitable base.
The reaction temperature of the present reaction is in the arbitrarily range of from 0°C to reflux temperature in the reaction system, preferably from 10 to 150°C.
The reaction time of the present reaction varies according to a reaction temperature, a reactant, a reaction amount, or the like, but is between 1 and 72 hours.
For the amount of agents to be provided in the present reaction, 1 to 3 equivalents of the compound represented by Formula [IV] and 1 to 3 equivalents of the base are used with respect to 1 equivalent of the compound represented by Formula [III]. In addition, the amount of solvent to be used is from 0 to 50 L, preferably from 0.1 to 3.0 L, with respect to 1 mole of the compound represented by Formula [III].
As the solvent and base that can be used in the present process, the same ones mentioned in Process 1 of Production Method 1 can be exemplified.
After completion of the reaction, the compound represented by Formula [V] that is a desired product of the present reaction can be collected from the reaction system by a usual method and, as the case requires, purified by a manipulation such as column chromatography or recrystallization.

(Process 3)

[Chemical Formula 5]

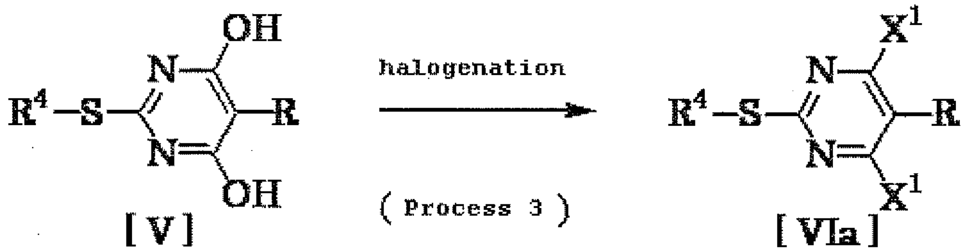


(wherein R and $R^4$ have the same meanings as defined above and $X^1$ is a chlorine atom or a bromine atom).

The compound represented by Formula [VIa] can be produced by allowing the compound represented by Formula [V] to react with a halogenating agent in a suitable solvent or in the absence of a solvent. Also, a suitable catalyst can be added for the production.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 72 hours.

For the amount of agents to be provided in the present reaction, 1 to 5 equivalents of the halogenating agent and 0.01 to 1.0 equivalent of the catalyst are used with respect to 1 equivalent of the compound represented by Formula [V]. In addition, the amount of solvent to be used is from 0 to 50 L, preferably from 0 to 3.0 L, with respect to 1 mole of the compound represented by Formula [V].

The solvent for use in the present process may be any solvent as long as it is an inert solvent not inhibiting the process of the present reaction. Examples thereof may include nitriles such as acetonitrile; aromatic hydrocarbons such as toluene and xylene; halogenated hydrocarbons such as monochlorobenzene and 1,2-dichloroethane; and the like.

As the halogenating agent that can be used in the present process, phosphorus oxychloride, phosphorus oxybromide, thionyl chloride, thionyl bromide, or the like can be exemplified.

As the catalyst that can be used in the present process, triethylamine, N,N-dimethylformamide, N,N-dimethylaniline, N,N-diethylaniline, or the like can be exemplified.

After completion of the reaction, the compound represented by Formula [VIa] that is a desired product of the present reaction can be collected from the reaction system by a usual method and, as the case requires, purified by a manipulation such as column chromatography or recrystallization.

(Process 4)

[Chemical Formula 6]

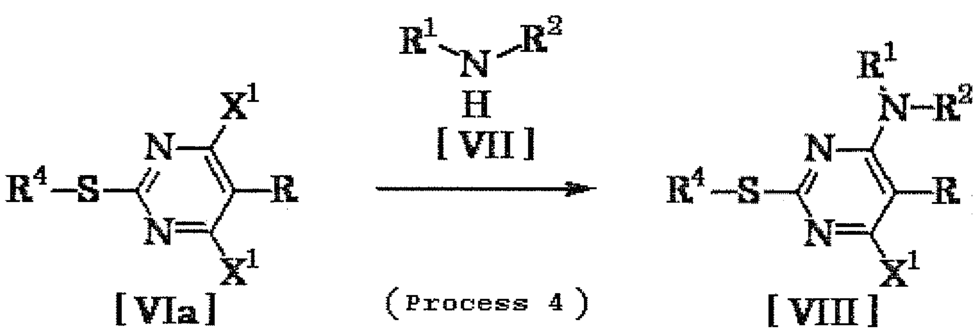


(wherein R, $R^1$, $R^2$, $R^4$ and $X^1$ have the same meanings as defined above).

The compound represented by Formula [VIII] can be produced by allowing the compound represented by Formula [VIa] to react with the compound represented by Formula [VII] in a suitable solvent or in the absence of a

solvent, in the presence of a suitable base. Also, a suitable catalyst can be added for the production.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 120 hours.

For the amount of agents to be provided in the present reaction, 1 to 3, preferably 1 to 1.5 equivalents of the compound represented by Formula [VII], 1 to 3, preferably 1 to 1.5 equivalents of the base and 0.001 to 0.5 equivalent of the catalyst are used, with respect to 1 equivalent of the compound represented by Formula [VIa]. In addition, the amount of solvent to be used is from 0 to 50 L, preferably from 0.1 to 3.0 L, with respect to 1 mole of the compound represented by Formula [VIa].

The solvent for use in the present process may be any solvent as long as it is an inert solvent not inhibiting the process of the present reaction. Examples thereof may include ethers such as 1,2-dimethoxyethane and tetrahydrofuran; halogenated hydrocarbons such as dichloroethane, carbon tetrachloride, chlorobenzene and dichlorobenzene; amides such as N,N-dimethylacetoamide, N,N-dimethylformamide, 1,3-dimethyl-2-imidazolidinone and N-methyl-2-pyrrolidinone; sulfur compounds such as dimethylsulfoxide and sulfolane; aromatic hydrocarbons such as benzene, toluene and xylene; alcohols such as methanol, ethanol, n-propanol, 2-propanol, n-butanol and 2-methyl-2-propanol; nitriles such as acetonitrile; carboxylic acids such as formic acid and acetic acid; water; and a mixture thereof.

Examples of the base that can be used in the present process may include organic bases such as pyridine, triethylamine, tributylamine and 1,8-diazabicyclo[5.4.0]-7-undecene; inorganic bases such as alkali metal hydroxide, for example, sodium hydroxide or potassium hydroxide, alkaline-earth metal hydroxide, for example, calcium hydroxide or magnesium hydroxide, alkali metal carbonates, for example, sodium carbonate or potassium carbonate and alkali metal bicarbonates, for example, sodium bicarbonate or potassium bicarbonate; alcohol metal salts such as sodium methoxide, sodium ethoxide and potassium tert-butoxide; and alkali metal hydrides such as sodium hydride.

As the catalyst that can be used in the present process, for example, sodium p-toluenesulfinate, sodium methane-sulfinate, or sodium benzenesulfinate can be exemplified.

After completion of the reaction, the compound represented by Formula [VIII] that is a desired product of the present reaction can be collected from the reaction system by a usual method and, as the case requires, purified by a manipulation such as column chromatography or recrystallization.

Further, in place of the compounds represented by Formulae [VIa] and [VIII], a compound in which any one of $X^1$ is X (X has the same meaning as defined above, but is an atom other than a chlorine atom and a bromine atom) can also be used as a raw material as in the present process and can be produced.

(Process 5)

[Chemical Formula 7]

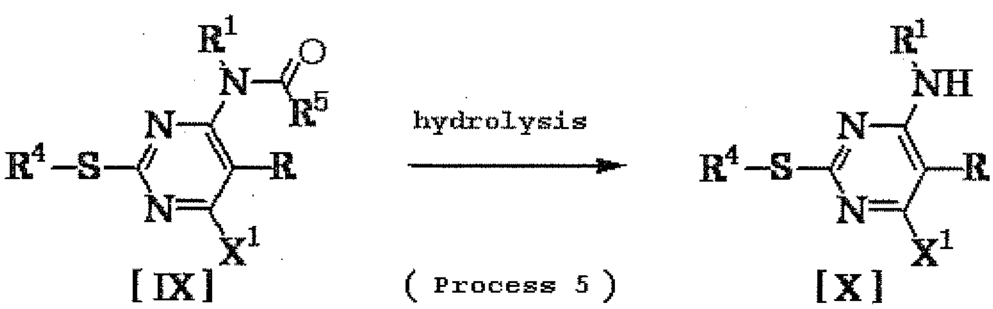


(wherein R, $R^1$, $R^4$ and $X^1$ have the same meanings as defined above and $R^5$ is a $C_{1-6}$ alkyl group, a $C_{1-6}$ haloalkyl group, or a $C_{1-6}$ alkykoxy group).

The compound represented by Formula [X] can be produced by hydrolyzing the compound represented by Formula [IX] in a suitable solvent or in the absence of a solvent with the use of acid or base.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 120 hours.

For the amount of agent to be provided in the present reaction, 1 to 10, preferably 1 to 3 equivalents of acid

or base can be used with respect to 1 equivalent of the compound represented by Formula [IX]. An amount of the solvent to be used is from 0 to 50 L, preferably from 0.1 to 3.0 L, with respect to 1 mole of the compound represented by Formula [IX].

The solvent for use in the present process may be any solvent as long as it is an inert solvent not inhibiting the process of the present reaction. Examples thereof may include ethers such as 1,2-dimethoxyethane and tetrahydrofuran; halogenated hydrocarbons such as dichloroethane, carbon tetrachloride, chlorobenzene and dichlorobenzene; amides such as N,N-dimethylacetoamide, N,N-dimethylformamide, 1,3-dimethyl-2-imidazolidinone and N-methyl-2-pyrrolidinone; sulfur compounds such as dimethylsulfoxide and sulfolane; aromatic hydrocarbons such as benzene, toluene and xylene; alcohols such as methanol, ethanol, n-propanol, 2-propanol, n-butanol and 2-methyl-2-propanol; nitrites such as acetonitrile; carboxylic acids such as formic acid and acetic acid; water; and a mixture thereof.

As the acid that can be used in the present process, inorganic acids such as hydrochloric acid, sulfuric acid, phosphoric acid, or nitric acid; organic acids such as formic acid, acetic acid, trifluoroacetic acid, methanesulfonic acid, tartaric acid, citric acid, or succinic acid; or a mixture thereof can be exemplified.

Examples of the base that can be used in the present process may include organic bases such as pyridine, triethylamine, tributylamine and 1,8-diazabicyclo[5.4.0]-7-undecene; inorganic bases such as alkali metal hydroxide, for example, sodium hydroxide or potassium hydroxide, alkaline-earth metal hydroxide, for example, calcium hydroxide or magnesium hydroxide, alkali metal carbonates, for example, sodium carbonate or potassium carbonate and alkali metal bicarbonates, for example, sodium bicarbonate or potassium bicarbonate; alcohol metal salts such as sodium methoxide, sodium ethoxide and potassium tert-butoxide; and alkali metal hydrides such as sodium hydride.

After completion of the reaction, the compound represented by Formula [X] that is a desired product of the present reaction can be collected from the reaction system by a usual method and, as the case requires, purified by a manipulation such as column chromatography or recrystallization.

Further, in place of the compounds represented by Formulae [IX] and [X], a compound in which $X^1$ is X (X has the same meaning as defined above, but is an atom other than a chlorine atom and a bromine atom) can also be used as a raw material as in the present process and can be produced.

(Process 6)

[Chemical Formula 8]

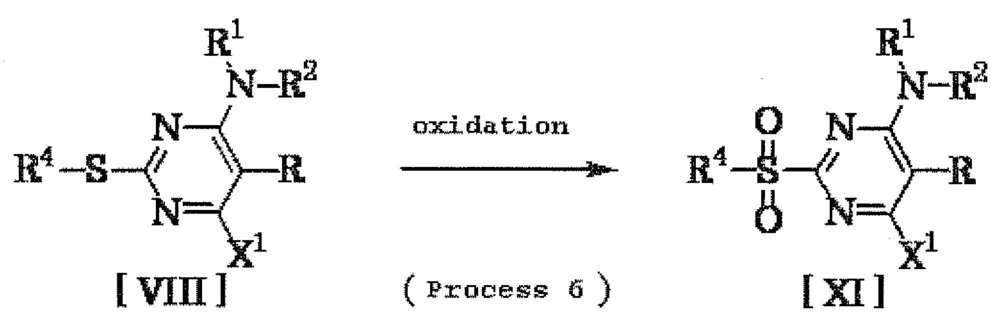


(wherein R, $R^1$, $R^2$, $R^4$ and $X^1$ have the same meanings as defined above).

The compound represented by Formula [XI] can be produced by allowing the compound represented by Formula [VIII] to react with an oxidizing agent in a suitable solvent. Also, a suitable catalyst can be added for the production.

The reaction temperature of the present reaction is in the arbitrarily range of from -30˚C to reflux temperature in the reaction system, preferably from 0 to 100˚C.

The reaction time of the present reaction varies according to a reaction temperature, a reactant, a reaction amount, or the like, but is between 0.5 and 48 hours.

For the amount of agents to be provided in the present reaction, 0.5 to 5 equivalents of an oxidizing agent and 0.01 to 0.5 equivalent of the catalyst are used, with respect to 1 equivalent of the compound represented by Formula [VIII]. In addition, the amount of solvent to be used is from 0 to 50 L, preferably from 0.1 to 3.0 L, with respect to 1 mole of the compound represented by Formula [VIII].

The solvent for use in the present process may be any solvent as long as it is an inert solvent not inhibiting the process of the present reaction. Examples thereof may include halogenated hydrocarbons such as dichloromethane, chloroform, 1,2-dichloroethane, carbon tetrachloride, chlorobenzene and dichlorobenzene; alcohols such as methanol,

ethanol, propanol, isopropanol, butanol and tert-butanol; ketones such as acetone and 2-butanone; nitriles such as acetonitrile; acetic acid; water; and a mixture thereof.

Examples of the oxidizing agent that can be used in the present process may include organic peroxides such as m-chloroperbenzoate, peroxyformic acid and peracetic acid; and inorganic peroxides such as OXONE (trade name, produced by Du Pont, $2KHSO_5 \cdot KHSO_4 \cdot K_2SO_4$), hydrogen peroxide, potassium permanganate and sodium periodate.

As the catalyst that can be used in the present process, for example, sodium tungstate can be exemplified.

After completion of the reaction, the compound represented by Formula [XI] that is a desired product of the present reaction can be collected from the reaction system by a usual method and, as the case requires, purified by a manipulation such as column chromatography or recrystallization.

Further, in place of the compounds represented by Formulae [VIII] and [XI], a compound in which $X^1$ is X (X has the same meaning as defined above, but is an atom other than a chlorine atom and a bromine atom) can also be used as a raw material as in the present process and can be produced.

(Process 7)

[Chemical Formula 9]

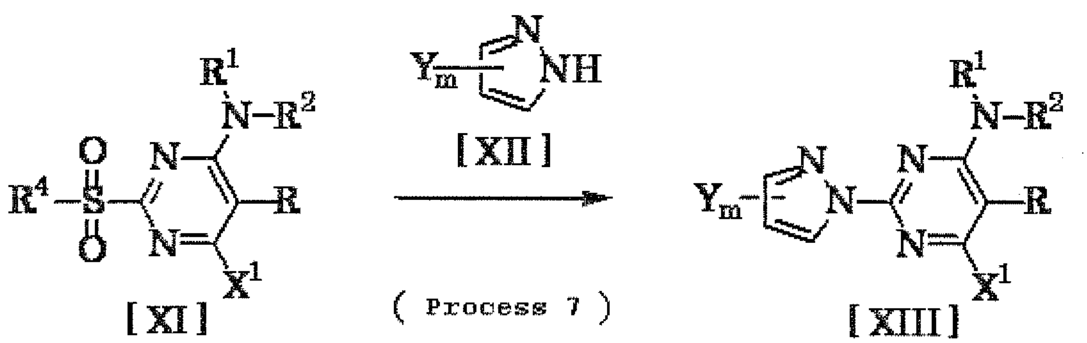


(wherein R, $R^1$, $R^2$, $R^4$, $X^1$, Y and m have the same meanings as defined above).

The compound represented by Formula [XIII] can be produced by allowing the compound represented by Formula [XI] to react with the compound represented by Formula [XII] in a suitable solvent in the presence of a suitable base.

The reaction temperature of the present reaction is in the arbitrarily range of from -30˚C to reflux temperature in the reaction system, preferably from 0 to 150˚C.

The reaction time of the present reaction varies according to a reaction temperature, a reactant, a reaction amount, or the like, but is between 0.5 and 48 hours.

For the amount of agents to be provided in the present reaction, 1 to 3 equivalents of the compound represented by Formula [XII] and 1 to 3 equivalents of the base are used, with respect to 1 equivalent of the compound represented by Formula [XI]. In addition, the amount of solvent to be used is from 0 to 50 L, preferably from 0.1 to 3.0 L, with respect to 1 mole of the compound represented by Formula [XI].

As the solvent and the base that can be used in the present process, the same ones mentioned in Process 4 of Production Method 1 can be exemplified.

After completion of the reaction, the compound represented by Formula [XIII] that is a desired product of the present reaction can be collected from the reaction system by a usual method and, as the case requires, purified by a manipulation such as column chromatography or recrystallization.

Further, in place of the compounds represented by Formulae [XI] and [XIII], a compound in which $X^1$ is X (X has the same meaning as defined above, but is an atom other than a chlorine atom and a bromine atom) can also be used as a raw material as in the present process or can be produced.

<Production Method 2>

(Process 8)

[Chemical Formula 10]

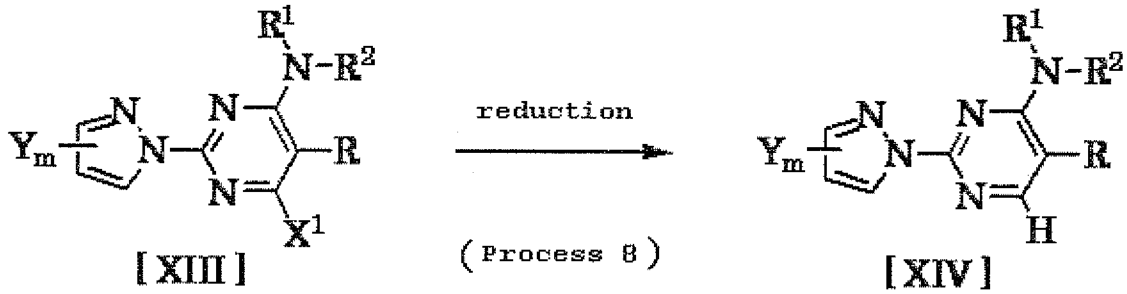


[XIII] [XIV]

(wherein R, $R^1$, $R^2$, $X^1$, Y and m have the same meanings as defined above).

The compound represented by Formula [XIV] can be produced by reducing the compound represented by Formula [XIII] in a suitable solvent in the presence of a suitable base.

As the reduction reaction, for example, a catalytic reduction method which employs the use of hydrogen gas and a suitable catalyst can be mentioned. This catalytic reduction method can be carried out in a hydrogen atmosphere under any conditions of normal pressure and applying pressure.

For the amount of agents to be provided in the present reaction, 0.001 to 0.5 equivalent of the catalyst and 0.1 to 5.0 equivalent of the base are used, with respect to 1 equivalent of the compound represented by Formula [XIII]. In addition, the amount of solvent to be used is from 0 to 50 L, preferably from 0.1 to 3.0 L, with respect to 1 mole of the compound represented by Formula [XIII].

The reaction temperature of the present reaction is in the arbitrarily range of from 0˚C to reflux temperature in the reaction system, preferably from 0 to 100˚C.

The reaction time of the present reaction varies according to a reaction temperature, a reactant, a reaction amount, or the like, but is between 0.5 and 72 hours.

The solvent for use in the present process may be any solvent as long as it is an inert solvent not inhibiting the process of the present reaction. Examples thereof may include aromatic hydrocarbons such as toluene and xylene; halogenated aliphatic hydrocarbons such as dichloromethane and chloroform; acetic acid esters such as methyl acetate, ethyl acetate and butyl acetate; aprotic polar solvents such as N,N-dimethylformamide, N,N-dimethylacetoamide, N-methylpyrrolidone, tetramethyl urea and hexamethylphosphoric triamide; ether-type solvents such as diethylether, tetrahydrofuran and dioxane; aliphatic hydrocarbons such as pentane and n-hexane; fatty alcohols such as methanol, ethanol, n-propanol, isopropanol, 1-butanol, sec-butanol and tert-butanol; water; and the like. Preferred are fatty alcohols such as methanol, ethanol, n-propanol, isopropanol, n-butanol, sec-butanol and tert-butanol and water.

As the catalyst that can be used in the present process, platinum, Raney nickel, platinum black, palladium-carbon, ruthenium complex, or the like can be exemplified.

Examples of the base that can be used in the present process may include organic bases such as pyridine, triethylamine, tributylamine and 1,8-diazabicyclo[5.4.0]-7-undecene; inorganic bases such as alkali metal hydroxide, for example, sodium hydroxide or potassium hydroxide, alkaline-earth metal hydroxide, for example, calcium hydroxide or magnesium hydroxide, alkali metal carbonates, for examples, sodium carbonate or potassium carbonate, alkali metal acetates, for example, sodium acetate or potassium acetate and alkali metal bicarbonates, for example, sodium bicarbonate or potassium bicarbonate; and alcohol metal salts such as sodium methoxide, sodium ethoxide and potassium tert-butoxide.

After completion of the reaction, the compound represented by Formula [XIV] that is a desired product of the present reaction can be collected from the reaction system by a usual method and, as the case requires, purified by a manipulation such as column chromatography or recrystallization.

<Production Method 3>

(Process 9)

[Chemical Formula 11]

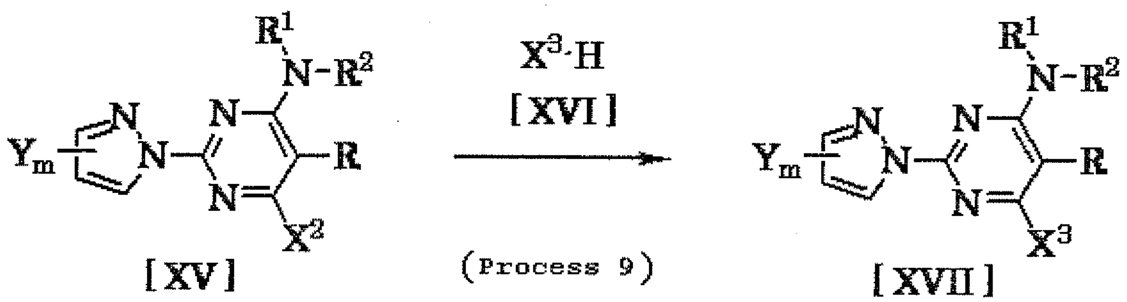


[XV] [XVII]

(wherein R, $R^1$, $R^2$, Y and m have the same meanings as defined above; $X^2$ is a halogen atom, a $C_{1-6}$ alkylsulfonyl group, a $C_{1-6}$ haloalkylsulfonyl group, an optionally substituted benzenesulfonyl group, or an optionally substituted benzylsulfonyl group; and $X^3$ is a $C_{1-6}$ alkoxy group, a $C_{1-3}$ haloalkoxy group, a $C_{1-6}$ alkynyloxy group, a $C_{2-6}$ alkenyloxy group, a $C_{3-8}$ cycloalkyloxy group, a $C_{3-8}$ cycloalkyl $C_{1-3}$ alkyloxy group, a $C_{1-6}$ alkylthio group, an optionally substituted phenylthio group, an optionally substituted benzylthio group, an amino- group, a hydroxyl group, an optionally substituted benzyloxy group, a mono $C_{1-6}$ alkylamino group, or a di($C_{1-6}$ alkyl) amino group).

The compound represented by Formula [XVII] can be produced by allowing the compound represented by Formula [XV] to react with the compound represented by Formula [XVI] in a suitable solvent in the presence of a suitable base. Also, a suitable catalyst can be added for the production. Among compounds represented by Formula [XV], compounds in which $X^2$ is other than halogen can be produced according to Processes 13, 14 and 15 of Production Method 6 shown below.

The reaction temperature of the present reaction is in the arbitrarily range of from -30˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agents to be provided in the present reaction, 1 to 3 equivalents of the compound represented by Formula [XVI] and 1 to 3 equivalents of the base are used, with respect to 1 equivalent of the compound represented by Formula [XV]. In addition, the amount of solvent to be used is from 0 to 50 L, preferably from 0.1 to 3.0 L, with respect to 1 mole of the compound represented by Formula [XV].

The solvent for use in the present process may be any solvent as long as it is an inert solvent not inhibiting the process of the present reaction. Examples thereof may include ethers such as 1,2-dimethoxyethane and tetrahydrofuran; halogenated hydrocarbons such as dichloroethane, carbon tetrachloride, chlorobenzene and dichlorobenzene; amides such as N,N-dimethylacetoamide, N,N-dimethylformamide, 1,3-dimethyl-2-imidazoladinane and N-methyl-2-pyrrolidinone; sulfur compounds such as dimethylsulfoxide and sulfolane; aromatic hydrocarbons such as benzene, toluene and xylene; alcohols such as methanol, ethanol, n-propanol, 2-propanol, n-butanol and 2-methyl-2-propanol; nitriles such as acetonitrile; carboxylic acids such as formic acid and acetic acid; water; and a mixture thereof.

Examples of the base that can be used in the present process may include organic bases such as pyridine, triethylamine, tributylamine and 1,8-diazabicyclo[5.4.0]-7-undecene; inorganic bases such as alkali metal hydroxide, for example, sodium hydroxide or potassium hydroxide, alkaline-earth metal hydroxide, for example, calcium hydroxide or magnesium hydroxide, alkali metal carbonates, for example, sodium carbonate or potassium carbonate and alkali metal bicarbonates, for example, sodium bicarbonate or potassium bicarbonate; alcohol metal salts such as sodium methoxide, sodium ethoxide and potassium tert-butoxide; and alkali metal hydrides such as sodium hydride.

As the catalyst that can be used in the present process, for example, sodium p-toluenesulfinate, sodium methane-sulfinate, or sodium benzenesulfinate can be exemplified.

After completion of the reaction, the compound represented by Formula [XVII] that is a desired product of the present reaction can be collected from the reaction system by a usual method and, as the case requires, purified by a manipulation such as column chromatography or recrystallization.

<Production Method 4>

(Process 10)

[Chemical Formula 12]

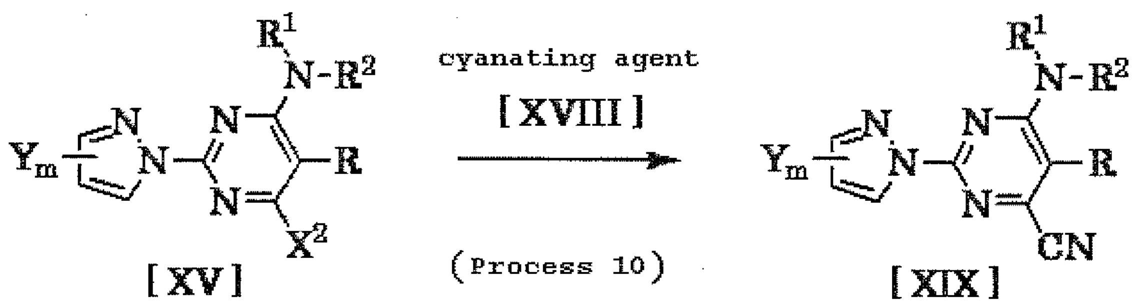


(wherein R, $R^1$, $R^2$, $X^2$, Y and m have the same meanings as defined above).

The compound represented by Formula [XIX] can be produced by allowing the compound represented by Formula [XV] to react with a cyanating agent [XVIII] in a suitable solvent.

The reaction temperature of the present reaction is in the arbitrarily range of from -30˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agent to be provided in the present reaction, 1 to 3 equivalents of the compound represented by Formula [XVIII] is used with respect to 1 equivalent of the compound represented by Formula [XV]. In addition, the amount of solvent to be used is from 0 to 50 L, preferably from 0.1 to 3.0 L, with respect to 1 mole of the compound represented by Formula [XV].

The solvent for use in the present process may be any solvent as long as it is an inert solvent not inhibiting the process of the present reaction. Examples thereof may include ethers such as 1,2-dimethoxyethane and tetrahydrofuran; halogenated hydrocarbons such as dichloroethane, carbon tetrachloride, chlorobenzene and dichlorobenzene; amides such as N,N-dimethylacetoamide, N,N-dimethylformamide, 1,3-dimethyl-2-imidazolidinone and N-methyl-2-pyrrolidinone; sulfur compounds such as dimethylsulfoxide and sulfolane; aromatic hydrocarbons such as benzene, toluene and xylene; alcohols such as methanol, ethanol, n-propanol, 2-propanol, n-butanol and 2-methyl-2-propanol; nitriles such as acetonitrile; carboxylic acids such as formic acid and acetic acid; water; and a mixture thereof.

Examples of the cyanating agent [XVIII] that can be used in the present process may include sodium cyanide, potassium cyanide, zinc cyanide and copper cyanide.

After completion of the reaction, the compound represented by Formula [XIX] that is a desired product of the present reaction can be collected from the reaction system by a usual method and, as the case requires, purified by a manipulation such as column chromatography or recrystallization.

<Production Method 5>

[Chemical Formula 13]

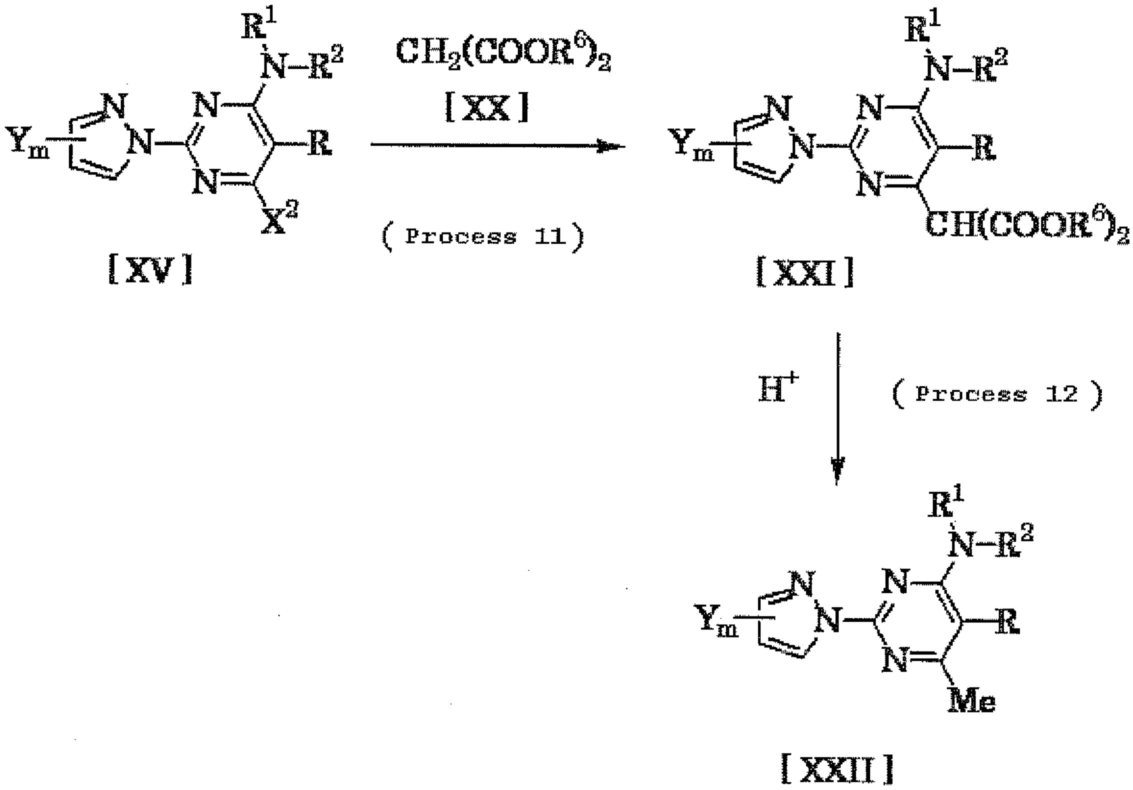


(wherein R, $R^1$, $R^2$, $X^2$, Y and m have the same meanings as defined above and $R^6$ is a $C_{1-6}$ alkyl group).

(Process 11)

The compound represented by Formula [XXI] can be produced by allowing the compound represented by Formula [XV] to react with the compound represented by Formula [XX] in a suitable solvent in the presence of a suitable base.

The reaction temperature of the present reaction is in the arbitrarily range of from -30˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agents to be provided in the present reaction, 1 to 3 equivalents of the compound represented by Formula [XX] and 1 to 3 equivalents of the base are used, with respect to 1 equivalent of the compound represented by Formula [XV]. In addition, the amount of solvent to be used is from 0 to 50 L, preferably from 0.1 to 3.0 L, with respect to 1 mole of the compound represented by Formula [XV].

The solvent for use in the present process may be any solvent as long as it is an inert solvent not inhibiting the process of the present reaction. Examples thereof may include ethers such as 1,2-dimethoxyethane and tetrahydrofuran; halogenated hydrocarbons such as dichloroethane, carbon tetrachloride, chlorobenzene and dichlorobenzene; amides such as N,N-dimethylacetoamide, N,N-dimethylformamide, 1,3-dimethyl-2-imidazolidinone and N-methyl-2-pyrrolidinone; sulfur compounds such as dimethylsulfoxide and sulfolane; aromatic hydrocarbons such as benzene, toluene and xylene; alcohols such as methanol, ethanol, n-propanol, 2-propanol, n-butanol and 2-methyl-2-propanol; nitriles such as acetonitrile; carboxylic acids such as formic acid and acetic acid; water; and a mixture thereof.

Examples of the base that can be used in the present process may include organic bases such as pyridine, triethylamine, tributylamine and 1,8-diazabicyclo[5.4.0]-7-undecene; inorganic bases such as alkali metal hydroxide, for example, sodium hydroxide or potassium hydroxide, alkaline-earth metal hydroxide, for example, calcium hydroxide or magnesium hydroxide, alkali metal carbonates, for example, sodium carbonate or potassium carbonate and alkali metal bicarbonates, for example, sodium bicarbonate or potassium bicarbonate; alcohol metal salts such as sodium methoxide, sodium ethoxide and potassium tert-butoxide; and alkali metal hydrides such as sodium hydride.

After completion of the reaction, the compound represented by Formula [XXI] that is a desired product of the present reaction can be collected from the reaction system by a usual method and, as the case requires, purified by a manipulation such as column chromatography or recrystallization.

(Process 12)

The compound represented by Formula [XXII] can be produced by applying the compound represented by Formula [XXI] to a decarboxylation reaction in a suitable solvent or in the absence of a solvent, in the presence of a suitable acid.
The reaction temperature of the present reaction is in the arbitrarily range of from 0˚C to reflux temperature in the reaction system, preferably from 0 to 150˚C.
The reaction time of the present reaction varies according to a reaction temperature, a reactant, a reaction amount, or the like, but is between 1 and 120 hours.
For the amount of agent to be provided in the present reaction, 0.1 to 10 equivalents of acid is used with respect to 1 equivalent of the compound represented by Formula [XXI]. In addition, the amount of solvent to be used is from 0 to 50 L, preferably from 0.1 to 3.0 L, with respect to 1 mole of the compound represented by Formula [XXI].
The solvent for use in the present process may be any solvent as long as it is an inert solvent not inhibiting the process of the present reaction. Examples thereof may include aliphatic hydrocarbons such as pentane, hexane, cyclohexane and petroleum ether; aromatic hydrocarbons such as toluene and xylene; halogenated hydrocarbons such as methylene chloride, chloroform and chlorobenzene; ethers such as diethylether, diisopropylether, tert-butylmethylether, dioxane, anisole and tetrahydrofuran; nitriles such as acetonitrile and propionitrile; ketones such as acetone, methyl ethyl ketone, diethyl ketone and tert-butyl methyl ketone; alcohols such as methanol, ethanol, 1-propanol, 2-propanol, 1-butanol and tert-butanol; amides such as N,N-dimethylacetoamide, N,N-dimethylformamide, 1,3-dimethyl-2-imidazolidinone and N-methyl-2-pyrrolidinone; sulfur compounds such as dimethylsulfoxide and sulfolane; carboxylic acids such as formic acid and acetic acid; water; and a mixture thereof.
Examples of the acid that can be used in the present process may include hydrochloric acid, sulfuric acid, phosphoric acid, formic acid, acetic acid, methanesulfonic acid, benzenesulfonic acid and p-toluenesulfonic acid.
After completion of the reaction, the compound represented by Formula [XXII] that is a desired product of the present reaction can be collected from the reaction system by a usual method and, as the case requires, purified by a manipulation such as column chromatography or recrystallization.

<Production Method 6>

[Chemical Formula 14]

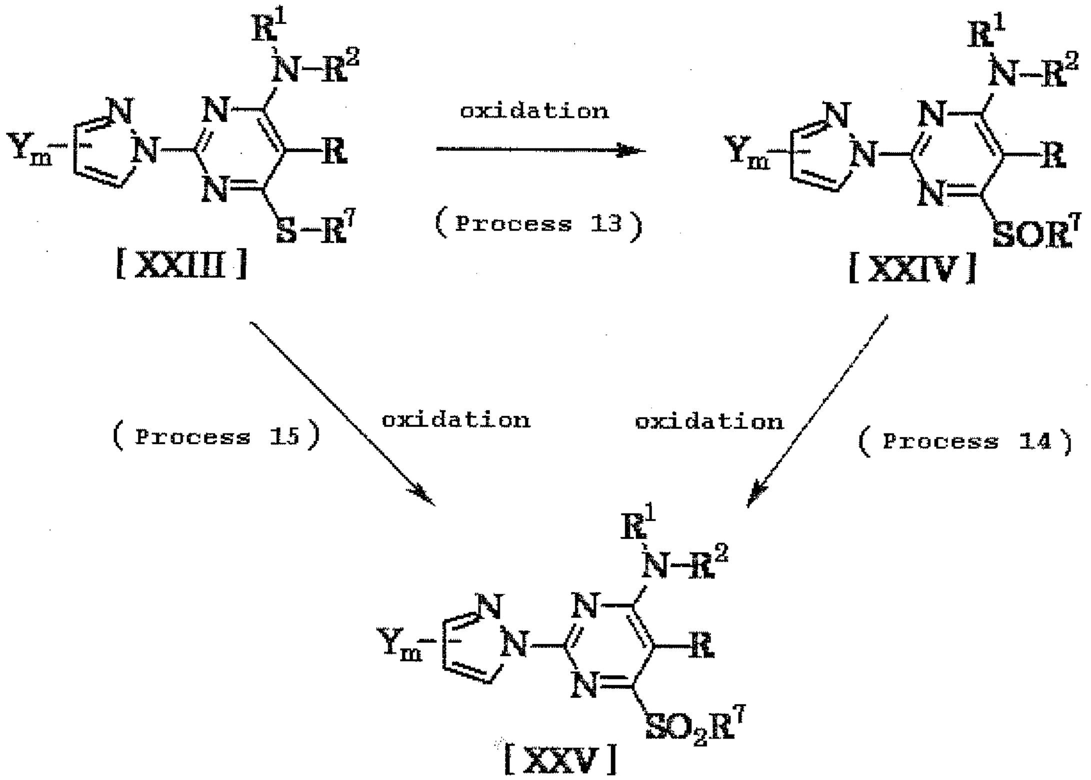


(wherein R, $R^1$, $R^2$, Y and m have the same meanings as defined above and $R^7$ is a $C_{1-6}$ alkyl group, a $C_{1-6}$ haloalkyl group, an optionally substituted phenyl group, or an optionally substituted benzyl group).

(Process 13)

The compound represented by Formula [XXIV] can be produced by allowing the compound represented by Formula [XXIII] to react with an oxidizing agent in a suitable solvent. Also, a suitable catalyst can be added for the production. Herein, the compound represented by Formula [XXIII] can be produced by allowing a compound in which $X^2$ in Formula [XV] is a halogen atom to react with a thiol compound $R^7$-SH, in accordance with Process 9 of Production Method 3.

The reaction temperature of the present reaction is in the arbitrarily range of from 0˚C to reflux temperature in the reaction system, preferably from 10 to 100˚C.

The reaction time of the present reaction varies according to a reaction temperature, a reactant, a reaction amount, or the like, but is between 0.5 and 48 hours.

For the amount of agents to be provided in the present reaction, 0.5 to 5 equivalents of the oxidizing agent and 0.01 to 0.5 equivalent of the catalyst are used, with respect to 1 equivalent of the compound represented by Formula [XXIII]. In addition, the amount of solvent to be used is from 0 to 50 L, preferably from 0.1 to 3.0 L, with respect to 1 mole of the compound represented by Formula [XXIII].

As the solvent, the oxidizing agent, the base and the catalyst, for use in the present process, the same ones mentioned in Process 6 of Production Method 1 can be exemplified.

After completion of the reaction, the compound represented by Formula [XXIV] that is a desired product of the present reaction can be collected from the reaction system by a usual method and, as the case requires, purified by a manipulation such as column chromatography or recrystallization.

(Process 14)

The compound represented by Formula [XXV] can be produced by allowing the compound represented by Formula [XXIV] to react with an oxidizing agent in a suitable solvent. Also, a suitable catalyst can be added for the production.

The reaction temperature of the present reaction is in the arbitrarily range of from 0˚C to reflux temperature in the reaction system, preferably from 10 to 100˚C.

The reaction time of the present reaction varies according to a reaction temperature, a reactant, a reaction amount, or the like, but is between 0.5 and 48 hours.

For the amount of agents to be provided in the present reaction, 0.5 to 5 equivalents of the oxidizing agent and 0.01 to 0.5 equivalent of the catalyst are used, with respect to 1 equivalent of the compound represented by Formula [XXIV]. In addition, the amount of solvent to be used is from 0 to 50 L, preferably from 0.1 to 3.0 L, with respect to 1 mole of the compound represented by Formula [XXIV].

As the solvent, the oxidizing agent, the base and the catalyst, for use in the present process, the same ones mentioned in Process 6 of Production Method 1 can be exemplified.

After completion of the reaction, the compound represented by Formula [XXV] that is a desired product of the present reaction can be collected from the reaction system by a usual method and, as the case requires, purified by a manipulation such as column chromatography or recrystallization.

(Process 15)

The compound represented by Formula [XXV] can be produced without obtaining the compound represented by Formula [XXIV] by allowing the compound represented by Formula [XXIII] to react with an oxidizing agent in a suitable solvent. Also, a suitable catalyst can be added for the production.

The reaction temperature of the present reaction is in the arbitrarily range of from 0˚C to reflux temperature in the reaction system, preferably from 10 to 100˚C.

The reaction time of the present reaction varies according to a reaction temperature, a reactant, a reaction amount, or the like, but is between 0.5 and 48 hours.

For the amount of agents to be provided in the present reaction, 0.5 to 5 equivalents of the oxidizing agent and 0.01 to 0.5 equivalent of the catalyst are used, with respect to 1 equivalent weight of the compound represented by Formula [XXIII]. In addition, the amount of solvent to be used is from 0 to 50 L, preferably from 0.1 to 3.0 L, with respect to 1 mole of the compound represented by Formula [XXIII].

As the solvent, the oxidizing agent, the base and the catalyst, for use in the present process, the same ones mentioned in Process 6 of Production Method 1 can be exemplified.

After completion of the reaction, the compound represented by Formula [XXV] that is a desired product of the present reaction can be collected from the reaction system by a usual method and, as the case requires, purified by a manipulation such as column chromatography or recrystallization.

<Production Method 7>

(Process 16)

[Chemical Formula 15]

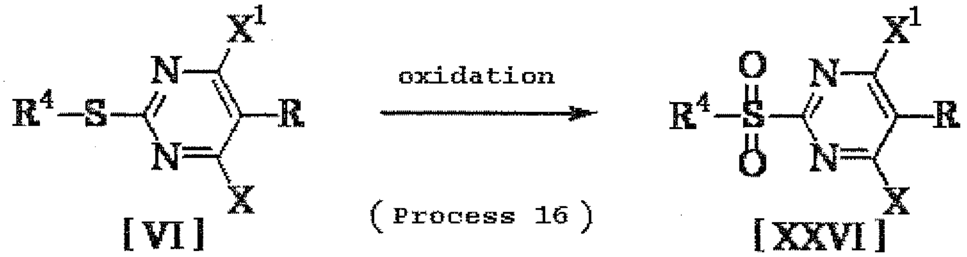


[VI] [XXVI]

(wherein R, $R^4$, X and $X^1$ have the same meanings as defined above).

The compound represented by Formula [XXVI] can be produced by allowing the compound represented by Formula [VI] to react with an oxidizing agent in a suitable solvent. Also, a suitable catalyst can be added for the production.

The reaction temperature of the present reaction is in the arbitrarily range of from -100˚C to reflux temperature in the reaction system, preferably from -10 to 100˚C.

The reaction time of the present reaction varies according to a reaction temperature, a reactant, a reaction amount, or the like, but is between 0.5 and 48 hours.

For the amount of agents to be provided in the present reaction, 0.5 to 5 equivalents of the oxidizing agent and 0.01 to 0.5 equivalent of the catalyst are used, with respect to 1 equivalent of the compound represented by Formula [VI]. In addition, the amount of solvent to be used is from 0.01 to 50 L, preferably from 0.1 to 3.0 L, with respect to 1 mole of the compound represented by Formula [VI].
As the solvent, the oxidizing agent, the base and the catalyst, for use in the present process, the same ones mentioned in Process 6 of Production Method 1 can be exemplified.
After completion of the reaction, the compound represented by Formula [XXVI] that is a desired product of the present reaction can be collected from the reaction system by a usual method and, as the case requires, purified by a manipulation such as column chromatography or recrystallization.

(Process 17)

[Chemical Formula 16]

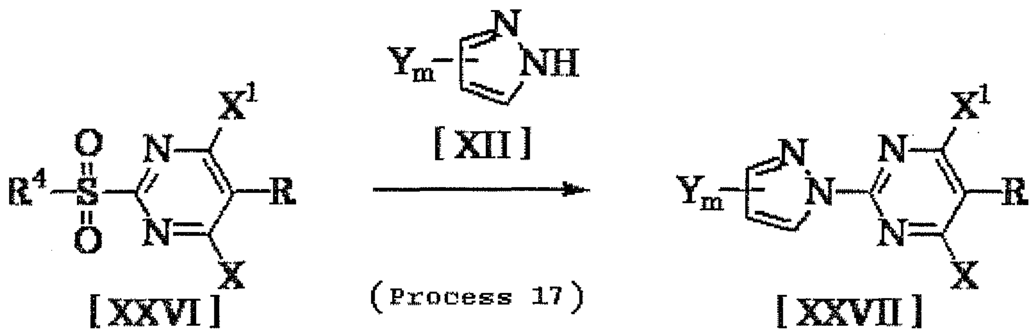


(wherein R, $R^4$, X, $X^1$, Y and m have the same meanings as defined above)
The compound represented by Formula [XXVII] can be produced by allowing the compound represented by Formula [XXVI] to react with the compound represented by Formula [XII] in a suitable solvent, in the presence of a suitable base.
The reaction temperature of the present reaction is in the arbitrarily range of from -100˚C to reflux temperature in the reaction system, preferably from -78 to 50˚C.
The reaction time of the present reaction varies according to a reaction temperature, a reactant, a reaction amount, or the like, but is between 0.5 and 48 hours.
For the amount of agents to be provided in the present reaction, 1 to 3 equivalents of the compound represented by Formula [XII] and 1 to 3 equivalents of the base are used, with respect to 1 equivalent of the compound represented by Formula [XXVI]. In addition, the amount of solvent to be used is from 0.01 to 50 L, preferably from 0.1 to 3.0 L, with respect to 1 mole of the compound represented by Formula [XXVI].
As the solvent and the base for use in the present process, the same ones mentioned in Process 4 of Production Method 1 can be exemplified.
After completion of the reaction, the compound represented by Formula [XXVII] that is a desired product of the present reaction can be collected from the reaction system by a usual method and, as the case requires, purified by a manipulation such as column chromatography or recrystallization.

(Process 18)

[Chemical Formula 17]

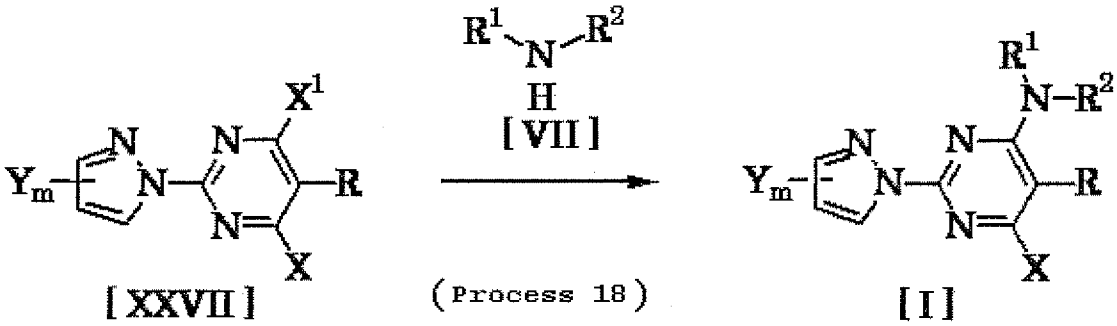


(wherein R, $R^1$, $R^2$, X, $X^1$, Y and m have the same meanings as defined above).

The compound represented by Formula [I] can be produced by allowing the compound represented by Formula [XXVII] to react with the compound represented by Formula [VII] in a suitable solvent or in the absence of a solvent, in the presence of a suitable base or absence of a base. Also, a suitable catalyst can be added for the production.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 120 hours.

For the amount of agents to be provided in the present reaction, 1 to 3, preferably 1 to 1.5 equivalents of the compound represented by Formula [VII]; 0 to 3, preferably 1 to 1.5 equivalents of the base; and 0.001 to 0.5 equivalent of the catalyst are used, with respect to 1 equivalent of the compound represented by Formula [XXVII]. In addition, the amount of solvent to be used is from 0 to 50 L, preferably from 0.1 to 3.0 L, with respect to 1 mole of the compound represented by Formula [XXVII].

As the solvent, the base and the catalyst for use in the present process, the same ones mentioned in Process 4 of Production Method 1 can be exemplified.

After completion of the reaction, the compound represented by Formula [I] that is a desired product of the present reaction can be collected from the reaction system by a usual method and, as the case requires, purified by a manipulation such as column chromatography or recrystallization.

<Production Method 8>

(Process 19)

[Chemical Formula 18]

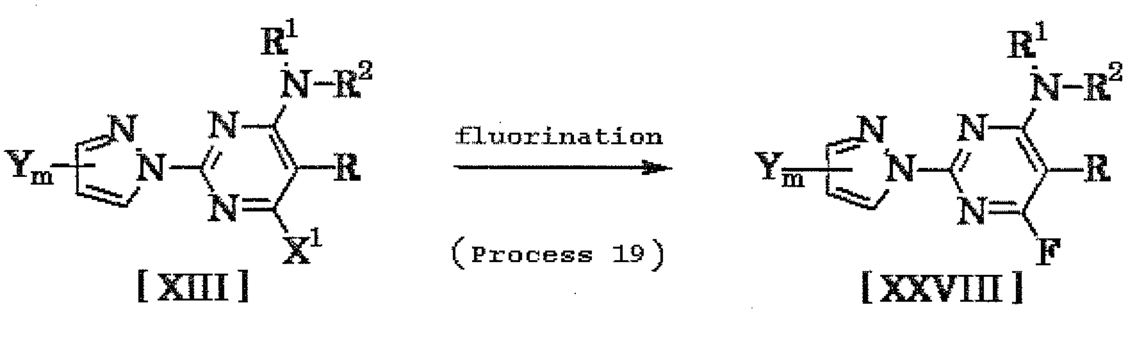


(wherein R, $R^1$, $R^2$, $X^1$, Y and m have the same meanings as defined above).

The compound represented by Formula [XXVIII] can be produced by fluorinating the compound represented by Formula [XIII] with a fluorinating agent in a suitable solvent or in the absence of a solvent.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction

amount, or the like, but is between 1 and 120 hours.

For the amount of agent to be provided in the present reaction, 1 to 20, preferably 1 to 5 equivalents of the fluorinating agent is used with respect to 1 equivalent of the compound represented by Formula [XIII]. In addition, the amount of solvent to be used is from 0 to 50 L, preferably from 0.1 to 3.0 L, with respect to 1 mole of the compound represented by Formula [XIII].

As the solvent that can be used in the present process, the same solvent mentioned in Process 4 of Production Method 1 can be exemplified.

As the fluorinating agent that can be used in the present process, potassium fluoride, sodium fluoride, cesium fluoride, or a mixture thereof can be exemplified.

After completion of the reaction, the compound represented by Formula [XXVIII] that is a desired product of the present reaction can be collected from the reaction system by a usual method and, as the case requires, purified by a manipulation such as column chromatography or recrystallization.

<Production Method 9>

(Process 20)

[Chemical Formula 19]

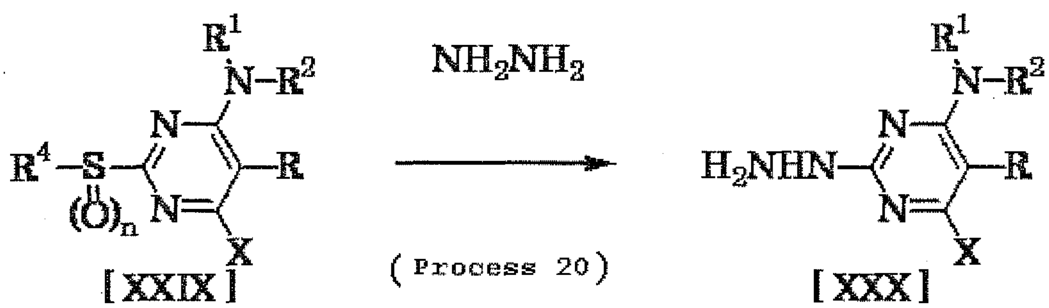


[XXIX] [XXX]

(wherein R, $R^1$, $R^2$, $R^4$ and X have the same meanings as defined above and n is an integer of 0 to 2).

The compound represented by Formula [XXX] can be produced by allowing the compound represented by Formula [XXIX] to react with hydrazine in a suitable solvent or in the absence of a solvent, in the presence or absence of a base.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 120 hours.

For the amount of agents to be provided in the present reaction, 1 to 20, preferably 1 to 5 equivalents of hydrazine; and 0 to 3, preferably 0 to 1.5 equivalents of the base are used, with respect to 1 equivalent of the compound represented by Formula [XXIX]. In addition, the amount of solvent to be used is from 0 to 50 L, preferably from 0.1 to 3.0 L, with respect to 1 mole of the compound represented by Formula [XXIX].

As the solvent and the base for use in the present process, the same ones mentioned in Process 4 of Production Method 1 can be exemplified.

After completion of the reaction, the compound represented by Formula [XXX] that is a desired product of the present reaction can be collected from the reaction system by a usual method and, as the case requires, purified by a manipulation such as column chromatography or recrystallization.

(Process 21)

[Chemical Formula 20]

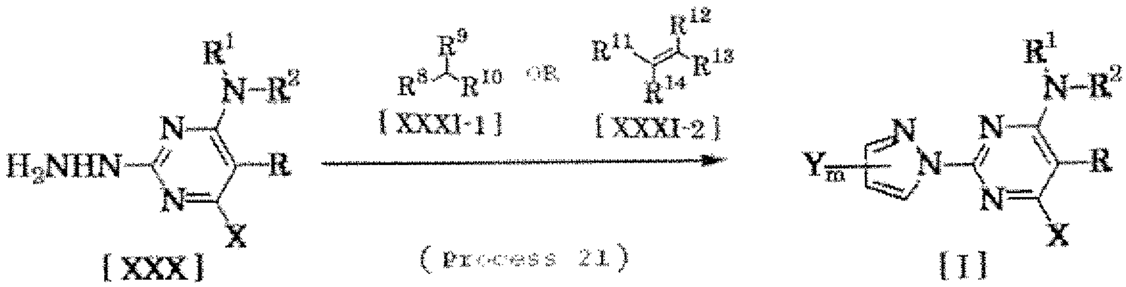


[XXX]

[I]

(wherein R, $R^1$, $R^2$, X, Y and m have the same meanings as defined above; $R^8$, $R^9$, $R^{12}$ and $R^{13}$ are each independently a halogen atom, a cyano group, a $C_{1-6}$ alkylthio group, a $C_{1-6}$ acyl group, a $C_{1-6}$ haloalkylcarbonyl group, a $C_{3-8}$ cycloalkylcarbonyl group, a carboxyl group, a $C_{1-6}$ alkoxycarbonyl group or a carbamoyl group; $R^{10}$ is a hydrogen atom, a halogen atom, a $C_{1-6}$ alkyl group, a $C_{1-6}$ haloalkyl group, a $C_{3-8}$ cycloalkyl group, a $C_{1-6}$ alkoxy group, a $C_{1-6}$ acryl group, an amino group, a nitro group, a cyano group, a hydroxyl group or a $C_{1-6}$ alkoxycarbonyl group; and $R^{11}$ and $R^{14}$ are each independently a hydrogen atom, a $C_{1-6}$ alkylthio group, a di($C_{1-6}$ alkyl)amino group or a $C_{1-6}$ alkoxy group).

The compound represented by Formula [I] can be produced by allowing the compound represented by Formula [XXX] to react with the compound represented by Formula [XXXI-1] or [XXXI-2] in a suitable solvent or in the absence of a solvent, in the presence or absence of acid or base.

The reaction temperature of the present reaction is in the arbitrarily range of from 0˚C to reflux temperature in the reaction system, preferably from 0 to 150˚C.

The reaction time of the present reaction varies according to a reaction temperature, a reactant, a reaction amount or the like, but is between 1 and 120 hours.

For the amount of agents to be provided in the present reaction, 1 to 10, preferably 1 to 3 equivalents of the compound represented by Formula [XXXI-1] or [XXXI-2]; and 0 to 5, preferably 0 to 3 equivalents of acid or base are used, with respect to 1 equivalent of the compound represented by Formula [XXX]. In addition, the amount of solvent to be used is from 0 to 50 L, preferably from 0.1 to 3.0 L, with respect to 1 mole of the compound represented by Formula [XXX].

As the solvent and the base for use in the present process, the same ones mentioned in Process 1 of Production Method 1 can be exemplified.

Examples of the acid for use in the present process may include mineral acids such as hydrochloric acid, hydrobromic acid and sulfuric acid; organic acids such as formic acid, acetic acid, methane sulfonic acid and p-toluenesulfonic acid; and the like.

After completion of the reaction, the compound represented by Formula [I] that is a desired product of the present reaction can be collected from the reaction system by a usual method and, as the case requires, purified by a manipulation such as column chromatography or recrystallization.

<Production Method 10>

(Process 22)

[Chemical Formula 21]

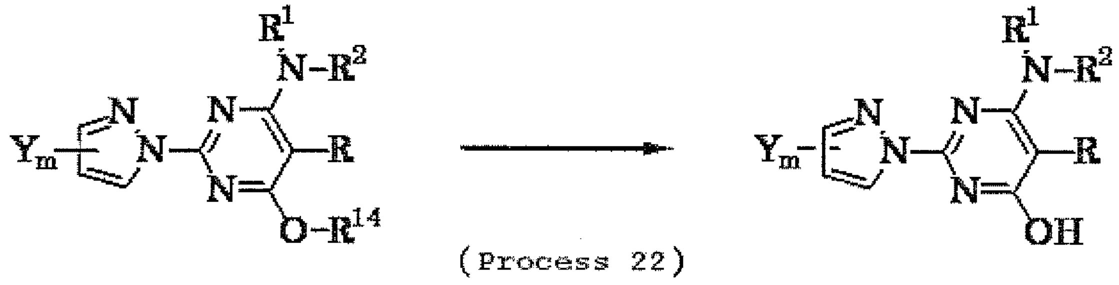


[XXXII] [XXXIII]

(wherein R, $R^1$, $R^2$, Y and m have the same meanings as defined above and $R^{14}$ is a $C_{1-6}$ alkyl group, a $C_{1-6}$ acyl group or an optionally substituted benzyl group).

The compound represented by Formula [XXXIII] can be produced by applying the compound represented by Formula [XXXII] to a hydrolysis with acid or base, or to a hydrogenolysis by a catalytic reduction method which employs the use of hydrogen gas and a suitable catalyst, in a suitable solvent or in the absence of a solvent. The compound represented by Formula [XXXII] can be produced by allowing the compound represented by Formula [XV] to react with an alcohol compound $R^{14}$-OH or a carboxylic compound $R^{14}$-C(=O)OH, according to the method described in Process 9 of Production Method 3.

The reaction temperature of the present reaction is in the arbitrarily range of from 0˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agents to be provided in the present reaction, 1 to 10, preferably 1 to 3 equivalents of acid or base; and 0.001 to 1, preferably 0.01 to 0.5 equivalents of the catalyst are used, with respect to 1 equivalent weight of the compound represented by Formula [XXXII]. In addition, the amount of solvent to be used is from 0 to 50 L, preferably from 0.1 to 3.0 L, with respect to 1 mole of the compound represented by Formula [XXXII].

As the solvent, the acid and the base for use in the present process, the same ones mentioned in Process 5 of Production Method 1 can be exemplified. As the catalyst, the same one mentioned in Process 8 of Production Method 2 can be exemplified.

After completion of the reaction, the compound represented by Formula [XXXIII] that is a desired product of the present reaction can be collected from the reaction system by a usual method and, as the case requires, purified by a manipulation such as column chromatography or recrystallization.

(Process 23)

[Chemical Formula 22]

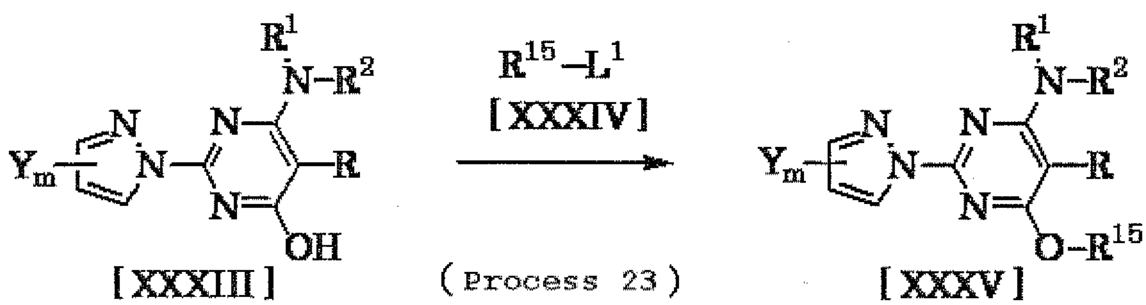


[XXXIII] (Process 23) [XXXV]

(wherein R, $R^1$, $R^2$, Y and m have the same meanings as defined above; and $R^{15}$ is a $C_{1-10}$ alkyl group, a $C_{1-6}$ haloalkyl group, a $G_{3-8}$ cycloalkyl group, a $C_{3-8}$ cycloalkyl $C_{1-3}$ alkyl group, an optionally substituted benzyl group, a $C_{2-6}$ alkenyl group or a $C_{2-6}$ alkynyl group, provided that when $R^{15}$ is a $C_{1-6}$ haloalkyl group, $L^1$ is a leaving group having a higher reactivity than that of a halogen atom remained after haloalkylation. For example, when $R^{15}$ is a $CHF_2$ group, $L^1$ is a chlorine atom or a bromine atom and when $R^{15}$ is a $CH_2CF_3$ group, $L^1$ is a chlorine atom, a bromine atom, an iodine atom, a p-toluenesulfonyloxy group, a methylsulfonyloxy group, a trifluoromethylsulfonyloxy group, or the like).

The compound represented by Formula [XXXV] can be produced by allowing the compound represented by Formula [XXXIII] to react with the compound represented by Formula [XXXIV] in a suitable solvent or in the absence of a solvent, in the presence or absence of a base.

The reaction temperature of the present reaction is in the arbitrarily range of from 0˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agents to be provided in the present reaction, 1 to 5, preferably 1 to 2 equivalents of the compound represented by Formula [XXXIV]; and 0 to 3, preferably 1 to 1.5 equivalents of the base are used, with respect to 1 equivalent of the compound represented by Formula [XXXIII]. In addition, the amount of solvent to be used is from 0 to 50 L, preferably from 0.1 to 3.0 L, with respect to 1 mole of the compound represented by Formula [XXXIII].

As the solvent and the base for use in the present process, the same ones mentioned in Process 4 of Production Method 1 can be exemplified.

After completion of the reaction, the compound represented by Formula [XXXV] that is a desired product of the present reaction can be collected from the reaction system by a usual method and, as the case requires, purified by a manipulation such as column chromatography or recrystallization.

<Intermediate Production Method 1>

(Process 24)

[Chemical Formula 23]

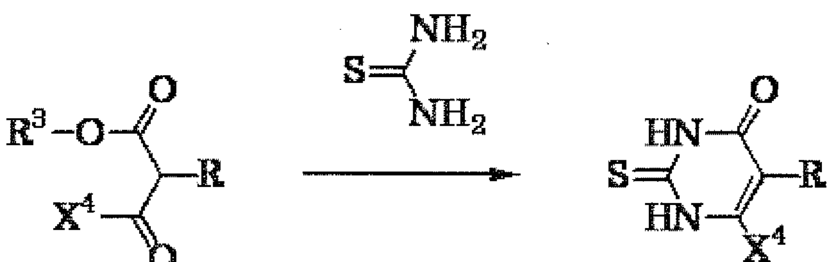

[XXXVI] (Process 24) [XXXVII]

(wherein R and $R^3$ have the same meanings as defined above and $X^4$ is a hydrogen atom, a $C_{1-10}$ alkyl group, a $C_{3-8}$ cycloalkyl group or a $C_{1-6}$ haloalkyl group).

The compound represented by Formula [XXXVII] can be produced by allowing the compound represented by Formula [XXXVI] with thiourea in a suitable solvent, in the presence of a suitable base.

The reaction temperature of the present reaction is in the arbitrarily range of from 0˚C to reflux temperature in the reaction system, preferably from 10 to 150˚C.

The reaction time of the present reaction varies according to a reaction temperature, a reactant, a reaction amount, or the like, but is between 0.5 and 120 hours.

For the amount of agents to be provided in the present reaction, 1 to 2 equivalents of the thiourea and 1 to 5 equivalents of the base are used, with respect to 1 equivalent of the compound represented by Formula [XXXVI]. In addition, the amount of solvent to be used is from 0.1 to 50 L, preferably from 0.1 to 3.0 L, with respect to 1 mole of the compound represented by Formula [XXXVI].

As the solvent and the base for use in the present process, the same ones mentioned in Process 1 of Production Method 1 can be exemplified.

After completion of the reaction, the compound represented by Formula [XXXVII] that is a desired product of

the present reaction can be used in the subsequent process without being isolated and purified, but can be also collected from the reaction system by a usual method and, as the case requires, purified by a manipulation such as column chromatography or recrystallization.

(Process 25)

[Chemical Formula 24]

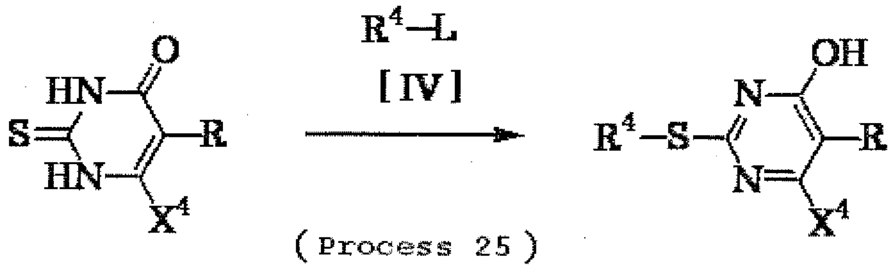


[XXXVII] [XXXVIII]

(wherein R, $R^4$, L and $X^4$ have the same meanings as defined above).
The compound represented by Formula [XXXVIII] can be produced by allowing the compound represented by Formula [XXXVII] to react with the compound represented by Formula [IV] in a suitable solvent, in the presence of a suitable base.
The reaction temperature of the present reaction is in the arbitrarily range of from 0˚C to reflux temperature in the reaction system, preferably from 10 to 150˚C.
The reaction time of the present reaction varies according to a reaction temperature, a reactant, a reaction amount, or the like, but is between 1 and 72 hours.
For the amount of agents to be provided in the present reaction, 1 to 3 equivalents of the compound represented by Formula [IV] and 1 to 3 equivalents of the base are used, with respect to 1 equivalent of the compound represented by Formula [XXXVII]. In addition, the amount of solvent to be used is from 0.1 to 50 L, preferably from 0.1 to 3.0 L, with respect to 1 mole of the compound represented by Formula [XXXVII].
As the solvent and the base for use in the present process, the same ones mentioned in Process 1 of Production Method 1 can be exemplified.
After completion of the reaction, the compound represented by Formula [XXXVIII] that is a desired product of the present reaction can be collected from the reaction system by a usual method and, as the case requires, purified by a manipulation such as column chromatography or recrystallization.

(Process 26)

[Chemical Formula 25]

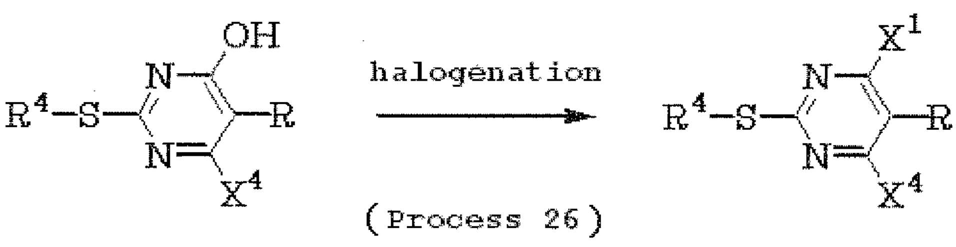


[XXXVIII] [XXXIX]

(wherein R, $R^4$, $X^1$ and $X^4$ have the same meanings as defined above).

The compound represented by Formula [XXXIX] can be produced by allowing the compound represented by Formula [XXXVIII] to react with a halogenating agent in a suitable solvent or in the absence of a solvent. Also, a suitable catalyst can be added for the production.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 72 hours.

For the amount of agents to be provided in the present reaction, 1 to 5 equivalents of the halogenating agent and 0 to 1.0 equivalent of the catalyst are used, with respect to 1 equivalent of the compound represented by Formula [XXXVIII]. In addition, the amount of solvent to be used is from 0 to 50 L, preferably from 0 to 3.0 L, with respect to 1 mole of the compound represented by Formula [XXXVIII].

As the solvent, the halogenating agent and the catalyst for use in the present process, the same ones mentioned in Process 3 of Production Method 1 can be exemplified.

After completion of the reaction, the compound represented by Formula [XXXIX] that is a desired product of the present reaction can be collected from the reaction system by a usual method and, as the case requires, purified by a manipulation such as column chromatography or recrystallization.

<Production Method 11>

(Process 27)

[Chemical Formula 26]

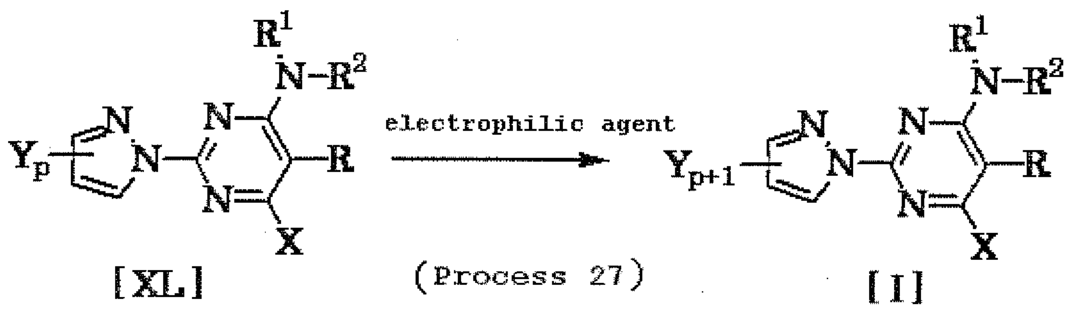


[XL] (Process 27) [I]

(wherein R, $R^1$, $R^2$, X and Y have the same meanings as defined above; and p is 0, 1, or 2, while when p is 1 or greater, $Y_{p+1}$ may be the same with or different from each other).

The compound represented by Formula [I] can be produced by allowing the compound represented by Formula [XL] to react with an electrophilic agent in a suitable solvent or in the absence of a solvent.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 120 hours.

For the amount of agent to be provided in the present reaction, 1 to 10, preferably 1 to 3 equivalents of the electrophilic agent is used, with respect to 1 equivalent of the compound represented by Formula [XL]. In addition, the amount of solvent to be used is from 0 to 50 L, preferably from 0.1 to 3.0 L, with respect to 1 mole of the compound represented by Formula [XL].

As the solvent for use in the present process, the same one mentioned in Process 4 of Production Method 1 can be exemplified.

Examples of the electrophilic agent that can be used in the present process may include a halogenating agent such as chlorine, bromine, N-bromosuccinimide, N-chlorosuccinimide, MEC-03 or MEC-31 (trade name by Daikin Ltd.), Selectfluor (trade name by Air Products Inc.), F-PLUS-B800, B500, or B300 (trade names by Tosoh F-TECH, Inc.) or sulfuryl chloride; a nitrating agent such as nitric acid, fuming nitric acid or acetyl nitrate; a chlorosulfonylation agent such as chlorosulfuric acid; a thiocyanating agent employing sodium thiocyanate or potassium thicyanate and chlorine, bromine, N-bromosuccinimide, N-chlorosuccinimide or sulfuryl chloride; and the like.

After completion of the reaction, the compound represented by Formula [I] that is a desired product of the present reaction can be collected from the reaction system by a usual method and, as the case requires, purified by a manipulation such as column chromatography or recrystallization.

<Intermediate Production Method 2>

(Process 28)

[Chemical Formula 27]

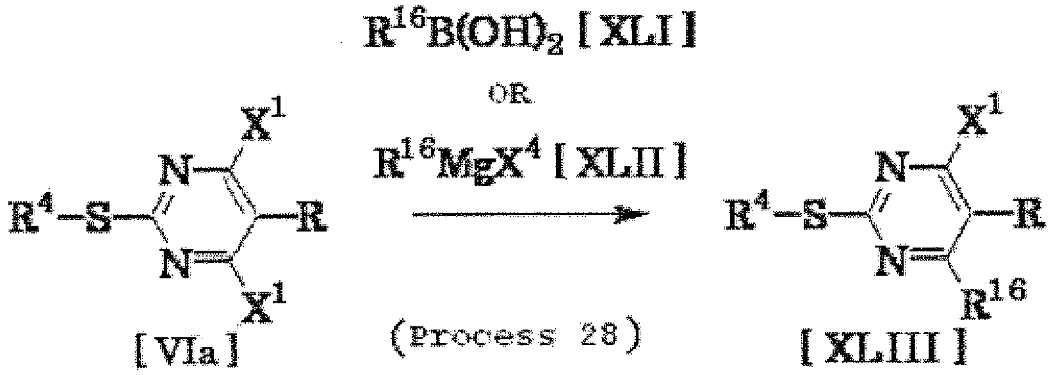


(wherein R, $R^4$ and $X^1$ have the same meanings as defined above; $R^{16}$ is a $C_{1-10}$ alkyl group, a $C_{3-8}$ cycloalkyl group, a $C_{3-8}$ cycloalkyl $C_{1-3}$ alkyl group, a $C_{2-6}$ alkynyl group or a $C_{2-6}$ alkenyl group; and $X^4$ is a halogen atom).

The compound represented by Formula [XLIII] can be produced by allowing the compound represented by Formula [VIa] to react with the compound represented by Formula [XLI] or Formula [XLII] in a suitable solvent or in the absence of a solvent, in the presence or absence of a base and a catalyst.

The reaction temperature of the present reaction is in the arbitrarily range of from 0˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agents to be provided in the present reaction, 1 to 3 equivalents of the compound represented by Formula [XLI] or Formula [XLII]; 0 to 3 equivalents of the base; and 0 to 0.5 equivalent of the catalyst are used, with respect to 1 equivalent of the compound represented by Formula [VIa]. In addition, the amount of solvent to be used is from 0 to 50 L, preferably from 0.1 to 3.0 L, with respect to 1 mole of the compound represented by Formula [VIa].

The solvent to be provided for the present reaction may be any solvent as long as it is an inert solvent not inhibiting the process of the present reaction. Examples thereof may include aliphatic hydrocarbons such as pentane, hexane, cyclohexane and petroleum ether; aromatic hydrocarbons such as toluene and xylene; ethers such as diethylether, diisopropylether, tert-butylmethylether, dioxane, 1,2-dimethoxyethane and tetrahydrofuran; alcohols such as methanol, ethanol, 1-propanol, 2-propanol, 1-butanol and tert-butanol; water; and a mixture thereof.

Examples of the base to be provided for the present reaction may include organic bases such as pyridine, triethylamine, tributylamine and 1,8-diazabicyclo[5.4.0]-7-undecene; and inorganic bases such as alkali metal hydroxide, for example, sodium hydroxide or potassium hydroxide, alkaline-earth metal hydroxide, for example, calcium hydroxide or magnesium hydroxide, alkali metal carbonates, for example, sodium carbonate or potassium carbonate and alkali metal bicarbonates, for example, sodium bicarbonate or potassium bicarbonate.

As the catalyst to be provided in the present reaction, a palladium catalyst such as palladium acetate, tetrakis (triphenylphosphine)palladium, or (diphenyl-phosphinoferrocene)palladium dichloride can be exemplified.

After completion of the reaction, the compound represented by Formula [XLIII] that is a desired product of the present reaction can be collected from the reaction system by a usual method and, as the case requires, purified by a manipulation such as column chromatography or recrystallization.

<Intermediate Production Method 3>

(Process 29)

[Chemical Formula 28]

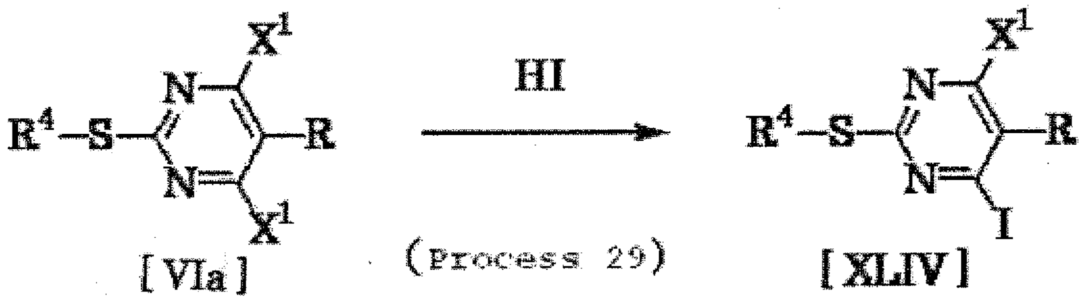


[VIa] [XLIV]

(wherein R, $R^4$ and $X^1$ have the same meanings as defined above).

The compound represented by Formula [XLIV] can be produced by iodizing the compound represented by Formula [VIa] using hydriodic acid.

The reaction temperature of the present reaction is in the arbitrarily range of from 0˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agent to be provided in the present reaction, 1 to 20, preferably 1 to 5 equivalents of the hydriodic acid is used, with respect to 1 equivalent of the compound represented by Formula [VIa].

After completion of the reaction, the compound represented by Formula [XLIV] that is a desired product of the present reaction can be collected from the reaction system by a usual method and, as the case requires, purified by a manipulation such as column chromatography or recrystallization.

(Process 30)

[Chemical Formula 29]

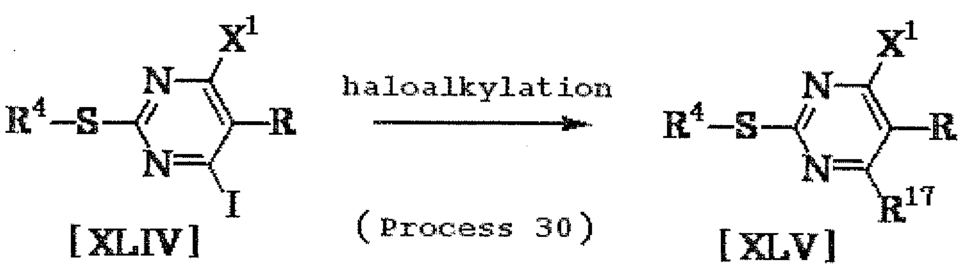


[XLIV] [XLV]

(wherein R, $R^4$ and $X^1$ have the same meanings as defined above and $R^{17}$ is a $C_{1-6}$ haloalkyl group).

The compound represented by Formula [XLV] can be produced by haloalkylating the compound represented by Formula [XLIV] using a haloalkylating agent in a suitable solvent or in the absence of a solvent, in the presence or absence of a base and a catalyst, according to a method described in Synthesis, Vol. 5, 798-803 (2005).

The reaction temperature of the present reaction is in the arbitrarily range of from 0˚C to reflux temperature in the reaction system, preferably from 0 to 180˚C.

The reaction time of the present reaction varies according to a reaction temperature, a reactant, a reaction

amount, or the like, but is between 1 and 120 hours.

For the amount of agents to be provided in the present reaction, 1 to 3 equivalents of the haloalkylating agent; 0 to 3 equivalents of the base; and 0 to 0.5 equivalent of the catalyst are used, with respect to 1 equivalent of the compound represented by Formula [XLIV]. In addition, the amount of solvent to be used is from 0 to 50 L, preferably from 0.1 to 3.0 L, with respect to 1 mole of the compound represented by Formula [XLIV].

Examples of the haloalkylating agent to be provided in the present reaction may include sodium chlorodifluoroacetate, sodium trifluoroacetate, trifluoromethyl iodide, 1,1,2,2,2-pentafluoroethyl iodide, 1,1,2,2,3,3,4,4,4-nonafluorobutyl iodide, trifluoromethyltrimethylsilane, trifluoromethyl-triethylsilane and the like.

Examples of the base to be provided in the present reaction may include potassium fluoride, sodium fluoride, cesium fluoride and the like.

Examples of the catalyst to be provided in the present reaction may include copper powder, copper iodide and the like.

The solvent to be provided in the present reaction may be any solvent as long as it is an inert solvent not inhibiting the process of the present reaction. Examples thereof may include amides such as N,N-dimethylformamide, N,N-dimethylacetoamide, N-methylpyrrolidinone and 1,3-dimethyl-2-imidazolidinone; dimethylsulfoxide; pyridine; and the like.

After completion of the reaction, the compound represented by Formula [XLV] that is a desired product of the present reaction can be collected from the reaction system by a usual method and, as the case requires, purified by a manipulation such as column chromatography or recrystallization.

<Intermediate Production Method 4>

(Process 31)

[Chemical Formula 30]

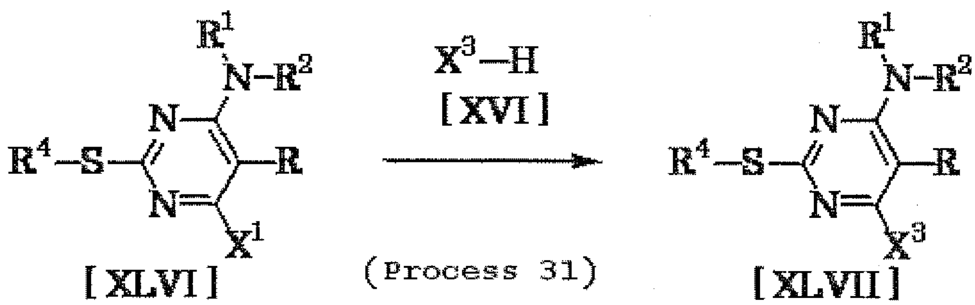


[XLVI] [XLVII]

(wherein R, $R^1$, $R^2$, $R^4$, $X^1$ and $X^3$ have the same meanings as defined above).

The compound represented by Formula [XLVII] can be produced by allowing the compound represented by Formula [XLVI] to react with the compound represented by Formula [XVI] in a suitable solvent, in the presence of a suitable base. Also, a suitable catalyst can be added for the production.

The reaction temperature of the present reaction is in the arbitrarily range of from -30˚C to reflux temperature in the reaction system, preferably from 0 to 150˚C.

The reaction time of the present reaction varies according to a reaction temperature, a reactant, a reaction amount, or the like, but is between 1 and 120 hours.

For the amount of agents to be provided in the present reaction, 1 to 3 equivalents of the compound represented by Formula [XVI]; 1 to 3 equivalents of the base; and 0 to 0.5 equivalent of the catalyst are used, with respect to 1 equivalent of the compound represented by Formula [XLVI]. In addition, the amount of solvent to be used is from 0.1 to 50 L, preferably from 0.1 to 3.0 L, with respect to 1 mole of the compound represented by Formula [XLVI].

As the solvent, the base and the catalyst, for use in the present process, the same ones mentioned in Process 9 of Production Method 3 can be exemplified.

After completion of the reaction, the compound represented by Formula [XLVII] that is a desired product of the present reaction can be collected from the reaction system by a usual method and, as the case requires, purified by a manipulation such as column chromatography or recrystallization.

<Production Method 12>

(Process 32)

[Chemical Formula 31]

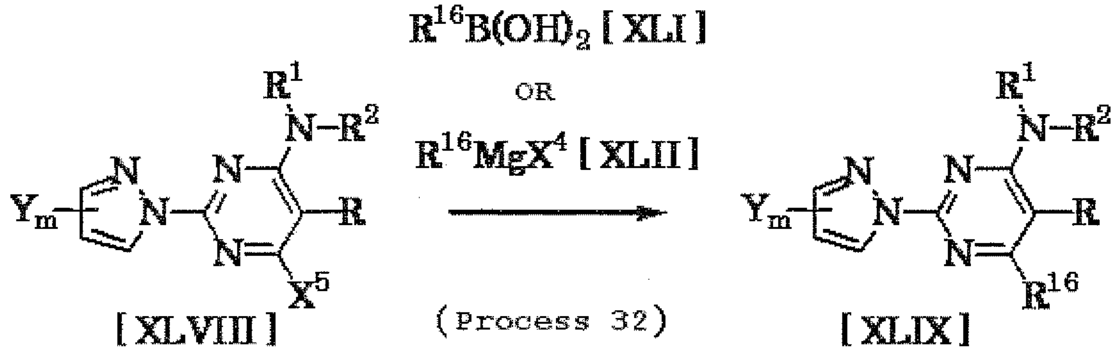


(wherein R, $R^1$, $R^2$, $R^{16}$, Y, m and $X^4$ have the same meanings as defined above and $X^5$ is a halogen atom, a $C_{1-6}$ alkylsulfonyloxy group or a $C_{1-6}$ haloalkylsulfonyloxy group).

The compound represented by Formula [XLIX] can be produced by allowing the compound represented by Formula [XLVIII] to react with the compound represented by Formula [XLI] or [XLII] in a suitable solvent or in the absence of a solvent, in the presence or absence of a base and a catalyst.

The reaction temperature of the present reaction is in the arbitrarily range of from 0°C to reflux temperature in the reaction system, preferably from 0 to 150°C.

The reaction time of the present reaction varies according to a reaction temperature, a reactant, a reaction amount, or the like, but is between 1 and 120 hours.

For the amount of agents to be provided in the present reaction, 1 to 3 equivalents of the compound represented by Formula [XLI] or [XLII]; 0 to 3 equivalents of the base; and 0 to 0.5 equivalent weight of the catalyst are used, with respect to 1 equivalent of the compound represented by Formula [XLVIII]. In addition, the amount of solvent to be used is from 0 to 50 L, preferably from 0.1 to 3.0 L, with respect to 1 mole of the compound represented by Formula [XLVIII].

As the solvent, the base and the catalyst to be provided in the present reaction, the same ones mentioned in Process 28 of Production Method 13 can be exemplified.

After completion of the reaction, the compound represented by Formula [XLIX] that is a desired product of the present reaction can be collected from the reaction system by a usual method and, as the case requires, purified by a manipulation such as column chromatography or recrystallization.

The plant disease control agent for agricultural or horticultural use according to the present invention is formed by containing the aminopyrimidine derivative represented by Formula [I] or an agriculturally acceptable salt thereof as the active ingredient.

In the case of using the compound of present application as a plant disease control agent for agricultural or horticultural use, it may be used singly or alternatively as the active ingredient in a suitable form according to its purpose.

In general, an active ingredient is diluted with an inert liquid or solid carrier and a surfactant and any others are added thereto as necessary, so as to be formed into a formulation of powder, wettable powder, emulsion, granules, or the like for a use. A ratio of the active ingredient to be blended may be suitably selected depending on the situation, but it is appropriate to be in the range of from 0.1 to 50% (by weight) in the case of powder and granule, or from 5 to 80% (by weight) in the case of emulsion and wettable powder.

Examples of the carrier to be used upon formulation may include solid carriers such as talc, bentonite, zeolite, clay, kaolin, diatomite, acid clay, white clay, white carbon, vermiculite, pearlite, pumice, calcium carbonate, slaked lime, silica sand, ammonium sulfate, urea and wooden powder; liquid carriers such as n-paraffin, isoparaffin, naphthene, isopropyl alcohol, cyclohexanol, ethylene glycol, diethylene glycol, propylene glycol, polyethylene glycol, polypropylene glycol, xylene, alkyl benzene, cyclohexane, alkylnaphthalene, fatty acid methyl ester, N-alkylpyrrolidone, isophorone, coconut oil, soybean oil and water; and the like.

Examples of the surfactant and dispersion may include sorbitan fatty acid ester, metal alkylbenzenesulfonate, metal dinaphthylmethane disulfonate, alcohol sulfate ester, alkyl aryl sulfonate, lignin sulfonate, metal dialkylsulfosuccinate, polyoxyethyleneglycolether, polyoxyethylene alkylaryl ether, a polyoxyethylenealkylaryl polymer, polyoxyethylene alkylaryl ether sulfonate, polyoxyethylene sorbitan monoalkylate, a salt of β-naphthalenesulfonate-formalin condensate, polyoxyethylene styrenated phenylethersulfate and the like.

Examples of the auxiliary agent may include carboxymethyl cellulose, alphanized starch, modified dextrin, polyethylene glycol, xanthan gum, gum arabic, silicone and the like.

Furthermore, the plant disease control agent for agricultural or horticultural use according to the invention can be mixed with or used in combination with other known active compounds such as insecticide, miticide, insect growth regulator, nematocide, fungicide, plant disease control agent, herbicide, plant growth regulator, fertilizer and soil conditioner, if needed, in addition to the compound of present application which is an active ingredient for various formulations described above.

Known fungicidal compounds which may be mixed or used in combination will be exemplified by:

benomyl, carbendazim, fuberidazole, thiabendazole, thiophanate, thiophanate-methyl, chlozolinate, iprodione, procymidone, vinclozolin, azaconazole, bitertanol, bromuconazole, cyproconazole, difenoconazole, diniconazole, epoxiconazole, fenarimol, fenbuconazole, fluquinconazole, flusilazole, flutriafol, hexaconazole, imazalil, imibenconazole, ipconazole, metconazole, myclobutanil, nuarimol, oxpoconazole fumarate, paclobutrazol, pefurazoate, penconazole, prochloraz, propiconazole, prothioconazole, pyrifenox, simeconazole, tebuconazole, tetraconazole, triadimefon, triadimenol, triflumizole, triforine, triticonazole, benalaxyl, furalaxyl, mefenoxam, metalaxyl, metalaxyl-M, ofurace, oxadixyl, aldimorph, dodemorph, fenpropidin, fenpropimorph, piperalin, spiroxamine, tridemorph, edifenphos, iprobenfos, isoprothiolane, pyrazophos, benodanil, boscalid, carboxin, fenfuram, flutolanil, furametpyr, mepronil, oxycarboxin, penthiopyrad, thifluzamide, bupirimate, dimethirimol, ethirimol, cyprodinil, mepanipyrim, pyrimethanil, diethofencarb, azoxystrobin, dimoxystrobin, enestrobin, famoxadone, fenamidone, fluoxastrobin, kresoxim-methyl, metominostrobin, orysastrobin, picoxystrobin, pyraclostrobin, trifloxystrobin, fenpiclonil, fludioxonil, quinoxyfen, biphenyl, chloroneb, dicloran, etridiazole, quintozene, tecnazene, tolclofos-methyl, fthalide, pyroquilon, tricyclazole, carpropamid, diclocymet, fenoxanil, fenhexamid, pyributicarb, polyoxin, pencycuron, cyazofamid, zoxamide, blasticidin-S, kasugamycin, streptomycin, validamycin, cymoxanil, iodocarb, propamocarb, prothiocarb, binapacryl, dinocap, ferimzone, fluazinam, TPTA(fentin acetate), TPTC(fentin chloride), TPTH(fentin hydroxide, oxolinic acid, hymexazol, octhilinone, fosetyl, phosphonic acid and a salt thereof, tecloftalam, triazoxide, flusulfamide, diclomezine, silthiofam, diflumetorim, benthiavalicarb-isopropyl, dimethomorph, flumorph, iprovalicarb, mandipropamid, oxytetracycline, methasulfocarb, chinomethionat, fluoroimide, milneb, copper hydroxide, copper octanoate, copper oxychloride, copper sulfate, cuprous oxide, mancopper, oxine-copper, sulfur, ferbam, mancozeb, maneb, metiram, propineb, thiram, zineb, ziram, captafol, captan, folpet, chlorothalonil, dichlofluanid, tolylfluanid, anilazine, dodine, guazatine, iminoctadine, dithianon, acibenzolar-S-methyl, probenazole, tiadinil, ethaboxam, cyflufenamid, proquinazid, metrafenone, fluopicolide, dazomet, difenzoquat, amisubrom, Bordeaux mixture, F-991, nabam, phenazine oxide, polycarbamate, or pyribencarb.

Known fungicidal and nematicidal compounds which may be mixed or used in combination will be exemplified by:

demeton-S-methyl, bioallethrin, bioallethrin S-cyclopentenylisomer, famphur, DDT, DNOC, EPN, XMC, acrinathrin, azadirachtin, azamethiphos, azinphos-ethyl, azinphos-methyl, acequinocyl, acetamiprid, acetoprol, acephate, azocyclotin, abamectin, amitraz, alanycarb, aldicarb, alphacypermethrin, allethrin[(1R)-isomers], d-cis-trans Allethrin, d-trans Allethrin, isocarbophos, isoxathion, isofenphos, isoprocarb, imicyafos, imidacloprid, imiprothrin, indoxacarb, esfenvalerate, ethiofencarb, ethiprole, ethion, ethiprole, etoxazole, etofenprox, ethoprophos, emamectin, endosulfan, Empenthrin, empenthrin[(EZ)-(1R)-isomers], oxamyl, oxydemeton-methyl, omethoate, cadusafos, cartap, carbaryl, carbosulfan, carbofuran, gamma-cyhalothrin, gamma-BCH(Lindane), xylylcarb, quinalphos, kinoprene, quinomethionate, chinomethionat, coumaphos, clothianidin, clofentezine, chromafenozide, chlorethoxyfos, chlordane, chlorpyrifos, chlorpyrifos-methyl, chlorfenapyr, chlorfenvinphos, chlorfluazuron, chlormephos, cyenopyrafen, cyanophos, diafenthiuron, diethofencarb, dienochlor, dicrotophos, dichlofenthion, cycloprothrin, dichlorvos, dicofol, disulfoton, dinotefuran, cyhalothrin, cyphenothrin[(1R)-trans-isomers], cyfluthrin, diflubenzuron, cyflumetofen, cyhexatin, cypermethrin, dimethylvinphos, dimethoate, tartaremetic, silafluofen, cyromazine, spinosad, spirodiclofen, spirotetramat, spiromesifen, sulfotep, zeta-cypermethrin, diazinon, tau-fluvalinate, thiacloprid, thiamethoxam, thiodicarb, thiocyclam, thiosultap-sodium, thiofanox, thiometon, tetrachlorvinphos, tetradifon, tetramethrin, tetramethrin[(1R)-isomers], depallethrin, tebupirimfos, tebufenozide, tebufenpyrad, tefluthrin, teflubenzuron, temephos, deltamethrin, terbufos, tralomethrin, transfluthrin, triazamate, triazophos, trichlorfon, tribufos, triflumuron, trimethacarb, tolfenpyrad, naled, nicotine, nitenpyram, nemadectin, novaluron, noviflumuron, hydroprene, vamidothion, parathion, parathion-methyl, halfenprox, halofenozide, bioresmethrin, bistrifluron, pyridaphenthion, hydramethylnon, bifenazate, bifenthrin, piperonyl butoxide, pymetrozine, pyraclofos, pyridafenthion, pyridaben, pyridalyl, pyriproxyfen, pirimicarb, pyrimidifen, pirimiphos-methyl, Pyrethrins(pyrethrum), fipronil, fenazaquin, fenamiphos, fenisobromolate, fenitrothion, fenoxycarb, phenothrin[(1R)-transisomer], fenobucarb, fenthion, phenthoate, fentrifanil, fenvalerate, fenpyroximate, fenbutatin oxide, fenpropathrin, butocarboxim, butoxycarboxim, buprofrzin, furathiocarb, prallethrin, fluacrypyrim, flucycloxuron, flucythrinate, flusulfamide, fluvalinate, flupyrazofos, flufenerim, flurenoxuron, Flubendiamide, flumethrin, flurimfen, prothiofos, flonicamid, propaphos, propargite, profenofos, propetamphos, propoxur, bromopropylate, beta-cyfluthrin, beta-cypermethrin, hexythiazox, hexaflumuron, heptenophos, permethrin, bensultap, benzoximate, bendiocarb, benfuracarb, borax, phoxim, phosalone, fosthiazate, phosphamidon, phosmet, formetanate, phorate, malathion, milbemectin, mecarbam, mesulfenfos, methomyl, metaflumizon, methamidophos, metham-ammonium, metham-sodium, methiocarb, methidathion, methoxychlor, methoxyfenozide, methothrin, methoprene, metolcarb, mevinphos, monocrotophos, lambda-cyhalothrin, rynaxypyr, aluminium phosphide, phosphine, lufenuron, resmethrin, lepmectin, rotenone, Bacillus sphaericus, Bacillus thuringiensis subsp. Aizawai, Bacillus thuringiensis subsp. Israelensis, Bacillus thuringiensis subsp. Kurstaki, Bacillus thuringiensis subsp. tenebrionis, CL900167, NNI-0101, RU15525, XDE-175, or ZXI8901.

Known herbicidal compounds which may be mixed or used in combination will be exemplified by:

2,3,6-TBA, 2,4-D, 2,4-DB, DNOC, EPTC, HC-252, MCPA, MCPA-thioethyl, MCPB, S-metolachlor, TCA, ioxynil, aclonifen, azafenidin, acifluorfen, azimsulfuron, asulam, acetochlor, atrazine, anilofos, amicarbazone, amidosulfuron, amitrole, aminopyralid (DE-750), amiprophos-methyl, ametryn, alachlor, alloxydim, ancymidol, iodosulfulon-methyl-sodium, isouron, isoxachlortole, isoxaflutole, isoxaben, isoproturon, imazaquin, imazapyr, imazamethabenz-methyl, imazapic, imazamox, imazethapyr, imazosulfuron, indanofan, esprocarb, ethametsulfuron-methyl, ethalfluralin, ethidimuron, ethoxysulfuron, ethofumesate, etobenzanid, oxadiazon, oxadiargyi, oxaziclomefone, oxasulfuron, oxyfluorfen, oryzalin, orbencarb, cafenstrole, carfentrazone-ethyl, karbutilate, carbetamide, quizalofop-P, quizalofop-P-ethyl, quizalofop-P-tefuryl, quizalofop-ethyl, quinclorac, quinmerac, cumyluron, glyphosate, glyphosate-trimesium(sulfosate), glufosinate-ammonium, glufosinate-sodium, clethodim, clodinafop-propargyl, clopyralid, clomazone, chlomethoxyfen, clomeprop, cloransulam-methyi, chloramben, chloridazon, chlorimuron-ethyl, chlorsulfuron, chlorthal-dimethyl, chlorthiamid, chlorpropham, chlormequat chloride, chloroxuron, chlorotoluron, chlorobromuron, cyanazine, diuron, dicamba, cycloate, cycloxydim, diclosulam, cyclosulfamuron, dichlobenil, diclofop-methyl, dichlorprop, dichlorprop-P, diquat dibromide, dithiopyr, siduron, dinitramine, cinidon-ethyl, cinosulfuron, dinoseb, dinoterb, cyhalofop-butyl, diphenamid, difenzoquat, diflufenican, diflufenzopyr, diflumetorim, simazine, dimethachlor, dimethametryn, dimethenamid, simetryn, dimepiperate, dimefuron, cinmethylin, sulcotrione, sulfentrazone, sulfosulfuron, sulfometuronmethyl, sethoxydim, terbacil, daimuron, dalapon, thiazopyr, tiocarbazil, thiobencarb, thidiazimin, thidiazuron, thifensulfuron-methyl, desmedipham, desmetryne, thenylchlor, tebutam, tebuthiuron, tepraloxydim, tefuryltrion, terbuthylazine, terbutryn, terbumeton, tembotrione, topramezone, tralkoxydim, triaziflam, triasulfuron, triallate, trietazine, triclopyr, triflusulfuron-methyl, tritosulfuron, trifluralin, trifloxy-sulruron-sodium, tribenuron-methyl, naptalam, naproanilide, napropamide, nicosulfuron, neburon, norflurazon, vernolate, paraquat dichloride, haloxyfop, haloxyfop-P, haloxyfop-P-methyl, halosulfuron-methyl, pinoxaden, picloram, picolinafen, bispyribac-sodium, bifenox, piperophos, pyraclonil, pyrasulfotole, pyrazoxyfen, pyrazosulfuron-ethyl, pyrazolynate, bilanafos, pyraflufen-ethyl, pyridafol, pyrithiobac-sodium, pyridate, pyriftalid, pyributicarb, pyribenzoxim, pyrimisulfan, primisulfuron-methyl, pyriminobac-methyl, pyroxysulam, fenuron, fenoxaprop-P-ethyl, fenoxaprop-ethyl, fenclorim, fentrazamide, phenmedipham, foramsulfuron, butachlor, butafenacil, butamifos, butylate, butralin, butroxydim, flazasulfuron, flamprop-M, fluazifop-butyl, fluazifop-P-butyl, fluazolate, fluometuron, fluometuron, fluoroglycofen-ethyl, flucarbazone-sodium, flucetosulfuron, fluthiacet-methyl, flupyrsulfuron-methyl-sodium, flufenacet, flufenpyr-ethyl, flupropanate, flupoxame, flumioxazin, flumiclorac-pentyl, flumetsulam, fluridone, flurtamone, flurprimidol, fluroxypyr, flurochloridone, pretilachlor, prodiamine, prosulfuron, prosulfocarb, propaquizafop, propachlor, propazine, propanil, propyzamide, propisochlor, propham, profluazol, propoxyoarbazone, propoxycarbazone-sodium, profoxydim, bromacil, prometryn, prometon, bromoxynil, bromofenoxim, bromobutide, florasulam, hexazinone, pethoxamid, benazolin, penoxsulam, beflubutamid, pebulate, TM435, pendimethalin, benzfendizone, bensulide, bensulfuron-methyl, benzobicyclon, benzofenap, bentazone, pentanochlor, pentoxazone, benfluralin, benfuresate, fosamine, fomesafen, forchlorfenuron, maleic hydrazide, mecoprop, mecoprop-P, mesosulfuron-methyl, mesotrione, metazachlor, methabenzthiazuron, metamitron, metamifop, methyl-dimuron, metoxuron, metosulam, metsulfuron-methyl, metobromuron, metobenzuron, metolachlor, metribuzin, mepiquat chloride, mefenacet, monolinuron, molinate, lactofen, linuron, rimsulfuron, lenacil, prohexadione-calcium, or trinexapac-ethyl.

The plant disease control agent for agricultural or horticultural use of the invention can be used directly in the form of those formulations, or by diluting the formulations, for foliage application, seed treatment, soil application, submerged application, nursery box application, or the like. The application amount varies depending on a kind of the compound to be used, target disease, growth pattern, degree of damage, environmental conditions, form of use and the like.

For example, in the case of a direct use of powder or granular formulation, the amount should be arbitrarily selected from 0.1g to 5kg, preferably from 1g to 1kg per 10 are in terms of an active ingredient.

Further, in the case of using in a liquid form of emulsion or wettable powder, the amount should be arbitrarily selected from 0.1 ppm to 10,000 ppm, preferably from 10 to 3,000 ppm.

In the case of using for a nursery box application, a long-term effect can be exhibited by providing a formulation in which an elution property of the compound is controlled.

The plant disease control agent for agricultural or horticultural use of the invention can control plant diseases caused by filamentous fungi, bacteria and virus, according to the above-described application patterns.

Next, specific plant diseases will be exemplified without being limited thereto:

Pseudoperonospora cubensis, Phytophthora melonis, Fusarium oxysporum, Pythium debaryanum, Corynespora cassiicola, Botrytis cinerea, Colletotrichum lagenarium, Sphaerotheca cucurbitae, Pseudomonas syringae, Pseudomonas solanacearum, Erysiphe graminis, Septoria nodorum, Septoria tritici, Puccinia recondite, Puccinia striiformis, Puccinia graminis, Pseudccercosporella herpotrichoides, Pyrenophora teres, Rhynchosporium secalis, Erwinia carotovora, Phytophthora infestans, Sclerotinia sclerotiorum, Cladosporium fulvum, Corynebacterium michiganense, Pyricularia oryzae, Rhizoctonia solani, Cochliobolus miyabeanus, Xanthomonas oryzae, Fusarium spp., Pythium spp., Rhizopus spp., Trichoderma sp., Burkholderia glumae, Burkholderia plantarii, Acidovorax avenae, Erwinia ananas, Venturia inaequalis, Alternaria mali, Gymnosporangium yamadae, Physalospora piricola, Alternaria kikuchiana, Phomopsis fukushii, Monilinia fructicola, Glomerella cingulata, Plasmopara viticola, Diaporthe citri, Elsinoe fawcetti and the like.

Hereinafter, production methods of the derivative of Formula [I] that can be employed in the plant disease control agent for agricultural or horticultural use according to the invention, formulation methods and applications will be described in detail with reference to Examples below. However, the present invention is not limited to these Examples in any way. In the description below, '%' means 'percent by weight'. Methods for producing Production Intermediates of the compound of the invention will also be described.

## Examples

[Example 1]

Production of 5-sec-butyl-4-chloro-6-(4-methylpiperidin-1-yl)-2-(1H-pyrazol-1-yl)pyrimidine (Inventive Compound No. 1192)

0.6g of sodium hydride (purity: 60%, 13.9 mmol) was added to 30 ml of an N,N-dimethylformamide solution containing 0.9g (12.7 mmol) of 1H-pyrazole at room temperature and the mixture was stirred for 1 hour. Further, 10 ml of an N,N-dimethylformamide solution containing 4.0g (11.6 mmol) of 5-sec-butyl-4-chloro-6-(4-methylpiperidin-1-yl)-2-methylsulfonylpyrimidine was added thereto at room temperature and the mixture was stirred for 1 hour. After confirming the completion of reaction, the reaction solution was poured into water and extracted with diethylether. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 3.7g of 5-sec-butyl-4-chloro-6-(4-methylpiperidin-1-yl)-2-(1H-pyrazol-1-yl)pyrimidine as a transparent liquid (yield: 95%).

Refractive Index ($n_D^{20}$) : 1.5721
$^1$H-NMR Data ($CDCl_3$/TMS δ (ppm)): 0.83 (3H, t, J=7.4 Hz), 1.01 (3H, d, J=6.6 Hz), 1.24-1.46 (5H, m), 1.60-1.89 (5H, m), 2.83-3.06 (3H, m), 3.71-3.75 (2H, m), 6.42-6.44 (1H, m), 7.79 (1H, bs), 8.48 (1H, d, J=2.7 Hz)

[Example 2]

Production of 5-sec-butyl-4-(4-methylpiperidin-1-yl)-2-(1H-pyrazol-1-yl)pyrimidine (Inventive Compound No. 1190)

0.3g (4.2 mmol) of anhydrous sodium acetate and 10mg of palladium carbon were added to 100 ml of a methanol solution containing 0.7g (2.1 mmol) of 5-sec-butyl-4-chloro-6-(4-methylpiperidin-1-yl)-2-(1H-pyrazol-1-yl)pyrimidine. A hydrogen gas was supplied under normal pressure and the mixture was stirred for 42 hours at room temperature. After confirming the completion of reaction, a catalyst was filtered off through celite. After concentrating thus obtained filtrate, water was added and extraction was subjected with ethyl acetate. The organic layer was dried over anhydrous magnesium sulfate, the insolubles were separated by filtration and then the solvent was distilled off under reduced pressure. Thus obtained residue was purified by silica gel column chromatography to obtain 0.5g of 5-sec-butyl-4-(4-methylpiperidin-1-yl)-2-(1H-pyrazol-1-yl)pyrimidine as a colorless transparent oily substance (yield: 86%).

Refractive Index ($n_D^{20}$): 1.5565
$^1$H-NMR Data ($CDCl_3$/TMS δ (ppm)): 0.85 (3H, t, J=7.4 Hz), 1.00 (3H, d, J=6.3 Hz), 1.29-1.44 (5H, m), 1.57-1.78 (5H,

m), 2.68-2.75 (1H, m), 2.89-3.04 (1H, m), 3.79-3.86 (2H, m), 6.42-6.43 (1H, m), 7.77 (1H, s), 8.25 (1H, s), 8.51 (1H, d, J=2.8 Hz)

[Example 3]

Production of 5-sec-butyl-6-(4-methylpiperidin-1-yl)-4-methylthio-2-(1H-pyrazol-1-yl)pyrimidine (Inventive Compound No. 1204)

0.3g (4.9 mmol) of sodium thiomethoxide was added to 10 ml of a tetrahydrofuran solution containing 1.5g (4.5 mmol) of 5-sec-butyl-4-chloro-6-(4-methylpiperidin-1-yl)-2-(1H-pyrazol-1-yl)pyrimidine and the mixture was stirred for 3 hours at room temperature. After confirming the completion of reaction, the reaction solution was poured into water and extracted with ethyl acetate. The obtained organic layer was dried over anhydrous magnesium sulfate. The insolubles were separated by filtration and then the solvent was distilled off under reduced pressure, to obtain 1.6g of 5-sec-butyl-6-(4-methylpiperidin-1-yl)-4-methylthio-2-(1H-pyrazol-1-yl)pyrimidine as a white powder (yield: quantitative).
Meiting Point (˚C): 69 to 71
$^1$H-NMR Data ($CDCl_3$/TMS δ (ppm)): 0.81 (3H, t, J=7.3 Hz), 0.99 (3H, d, J=6.4 Hz), 1.28-1.59 (6H, m), 1.74-1.96 (4H, m), 2.65 (3H, s), 2.86-3.03 (3H, m), 3.51-3.58 (2H, m), 6.42-6.43 (1H, m), 7.80 (1H, s), 8.54 (1H, d, J=1.8 Hz)

[Example 4]

Production of 5-sec-butyl-6-(4-methylpiperidin-1-yl)-4-methylsulfonyl-2-(1H-pyrazol-1-yl)pyrimidine (Inventive Compound No. 1206)

2.5g of m-chloroperbenzoic acid (purity: 70%, 10.1 mmol) was added to 100 ml of a dichloromethane solution containing 1.4g (4.1 nmol) of 5-sec-butyl-6-(4-methylpiperidin-1-yl)-4-methylthio-2-(1H-pyrazol-1-yl)pyrimidine under ice cooling and the mixture was stirred for 30 minutes. The mixture was further stirred for 72 hours at room temperature. After confirming the completion of reaction, the reaction solution was poured into water and extracted with dichloromethane. The obtained organic layer was washed with an aqueous solution of sodium bisulfite, water, an aqueous sodium bicarbonate solution and brine in the said order and then dried over anhydrous magnesium sulfate. The insolubles were separated by filtration and then the solvent was distilled off under reduced pressure, to obtain 1.3g of 5-sec-butyl-6-(4-methylpiperidin-1-yl)-4-methylsulfonyl-2-(1H-pyrazol-1-yl)pyrimidine as a pale yellow viscous substance (yield: 86%).
$^1$H-NMR Data ($CDCl_3$/TMS δ (ppm)): 0.85 (3H, t, J=7.4 Hz), 1.01 (3H, d, J=6.3 He), 1.35-1.43 (2H, m), 1,53 (3H, d, J=7.1 Hz), 1.61-1.81 (3H, m), 1.88-1.98 (2H, m), 2.99-3.07 (2H, m), 3.24-3.32 (1H, m), 3.45 (3H, s), 3.85 (2H, m), 6.46-6.48 (1H, m), 7.82 (1H, s), 8.44 (1H, d, J=2.2 Hz)

[Example 5]

Production of 5-sec-butyl-6-(4-methylpiperidin-1-yl)-2-(1H-pyrazol-1-yl)pyrimidine-4-carbonitrile (Inventive Compound No. 1215)

0.3g (5.4 mmol) of sodium cyanide was added to 10 ml of a dimethylsulfoxide solution containing 1.0g (2.7 mmol) of 5-sec-butyl-6-(4-methylpiperidin-1-yl)-4-methylsulfonyl-2-(1H-pyrazol-1-yl)pyrimidine and the mixture was stirred for 1.5 hours at room temperature. After confirming the completion of reaction, the reaction solution was poured into water and extracted with diethylether. The obtained organic layer was dried over anhydrous magnesium sulfate. The insolubles were separated by filtration and then the solvent was distilled off under reduced pressure to obtain 0.6g of 5-sec-butyl-6-(4-methylpiperidin-1-yl)-2-(1H-pyrazol-1-yl)pyrimidine-4-carbonitrile as a yellow viscous substance (yield: 63%).
Refractive Index ($n_D^{20}$): 1.5700
$^1$H-NMR Data ($CDCl_3$/TMS δ (ppm)) : 0.87 (3H, t, J=7.3 Hz), 1.02 (3H, d, J=6.6 Hz), 1.24-1.44 (2H, m), 1.56 (3H, d, J=7.1 Hz), 1.64-2.05 (5H, m), 2.74-2.82 (1H, m), 2.97-3.11 (2H, m), 3.83-3.90 (2H, m), 6.46 (1H, bs), 7.81 (1H, s), 8.49 (1H, d, J=2.5 Hz)

[Example 6]

Production of 5-sec-butyl-4-methyl-6-(4-methylpiperidin-1-yl)-2-(1H-pyrazol-1-yl)pyrimidine (Inventive Compound No. 1225)

15 ml of a 12N hydrochloric acid solution containing 1.6g (3.6 mmol) of dimethyl 2-[5-sec-butyl-6-(4-methylpiperidin-1-yl)-2-(1H-pyrazol-1-yl)pyrimidin-4-yl]malonate was stirred for 32 hours at 80˚C. After confirming the completion of reaction, the reaction solution was poured into water, neutralized with a 10% aqueous sodium hydroxide solution and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 0.1g of 5-sec-butyl-4-methyl-6-(4-methylpiperidin-1-yl)-2-(1H-pyrazol-1-yl)pyrimidine as a pale yellow oily substance (yield: 12%).

Refractive Index ($n_D^{20}$): 1.5581

$^1$H-NMR Data ($CDCl_3$/TMS δ (ppm)): 0.84 (3H, t, J=7.4 Hz), 1.00 (3H, d, J=6.6 Hz), 1.33-1.45 (5H, m), 1.64-1.78 (5H, m), 2.58 (3H, s), 2.82-3.01 (3H, m), 3.55-3.60 (2H, m), 6.41 (1H, bs), 7.76 (1H, s), 8.52 (1H, d, J=2.7 Hz)

[Example 7]

Production of 5-sec-butyl-6-chloro-2-(1H-pyrazol-1-yl)-N-(2,2,2-trifluoroethyl)pyrimidine-4-amine (Inventive Compound No. 0350)

0.6g (9.5 mmol) of 1H-pyrazole and 2.2g (15.8 mmol) of potassium carbonate were added to 20 ml of an N,N-dimethylformamide solution containing 2.7g (7.9 mmol) of 5-sec-butyl-6-chloro-2-methylsulfonyl-N-(2,2,2-trifluoroethyl) pyrimidine-4-amine at room temperature and the mixture was stirred for 8 hours at 60˚C. After confirming the completion of the reaction, the reaction solution was poured into water and extracted with diethylether. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 1.6g of 5-sec-butyl-6-chloro-2-(1H-pyrazol-1-yl)-N-(2,2,2-trifluoroethyl)pyrimidine-4-amine as a white powder (yield: 61%).

Melting Point (˚C): 137 to 139

$^1$H-NMR Data ($CDCl_3$/TMS δ (pom)): 0.92 (t, 3H), 1.35 (d, 3H), 1.75 (m, 2H), 3.42 (br, 1H), 4.38 (m, 2H), 5.23 (br, 1H), 6.45 (t, 1H), 7.80 (d, 1H), 8.46 (d, 1H)

[Example 8]

Production of 5-sec-butyl-6-fluoro-2-(1H-pyrazol-1-yl)-N-(2,2,2-trifluoroethyl)pyrimidine-4-amine (Inventive Compound No. 0349)

0.2g (4.2 mmol) of potassium fluoride was added to 10 ml of a dimethylsulfoxide solution containing 0.7g (2.1 mmol) of 5-sec-butyl-6-chloro-2-(1H-pyrazol-1-yl)-N-(2,2,2-trifluoroethyl)pyrimidine-4-amine at room temperature and the mixture was stirred for 18 hours at 150˚C. After confirming the completion of reaction, the reaction solution was poured into water and extracted with diethylether. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 0.4g of 5-sec-butyl-6-fluoro-2-(1H-pyrazol-1-yl)-N-(2,2,2-trifluoroethyl)pyrimidine-4-amine as a pale yellow powder (yield: 58%).

Melting Point (˚C): 132 to 134

$^1$H-NMR Data ($CDCl_3$/TMS δ (ppm)): 0.90 (t, 3H), 1.34 (d, 3H), 1.75 (m, 2H), 2.66 (m, 1H), 4.37 (m, 2H), 5.25 (br, 1H), 6.46 (t, 1H), 7.79 (d, 1H), 8.45 (d, 1H)

[Example 9]

Production of 5-sec-butyl-N,N-diethylamino-6-methyl-2-(1H-pyrazol-1-yl)pyrimidine-4-amine (Inventive Compound No. 0953)

0.9g (13.2 mmol) of diethylamine was added to 10 ml of an N,N-dimethylformamide solution containing 0.5g (2.0 mmol) of 5-sec-butyl-4-chloro-6-methyl-2-(1H-pyrazol-1-yl)pyrimidine at room temperature and the mixture was stirred for 2 days at 80˚C. After confirming the completion of reaction, the reaction solution was poured into water and extracted with diethylether. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 0.5g of 5-sec-butyl-N,N-diethylamino-6-methyl-2-(1H-pyrazol-1-yl)pyrimidine-4-amine as an orange oily substance (yield: 84%).

Refractive Index ($n_D^{20}$): 1.5560

$^1$H-NMR Data ($CDCl_3$/TMS δ (ppm)): 0.80 (t, 3H), 1.18 (t, 6H), 1.39 (d, 3H), 1.74 (m, 2H), 2.59 (s, 3H), 3.01 (m, 1H), 3.25 (m, 2H), 3.44 (m, 2H), 6.42 (t, 1H), 7.77 (d, 1H), 8.49 (d, 1H)

[Example 10]

Production of 5-sec-butyl-6-methylthio-2-(1H-pyrazol-1-yl)-N-(2,2,2-trifluoroethyl)pyrimidine-4-amine (Inventive Compound No. 0361)

A sodium methylmercaptan solution (content: 15%, 157.3 mmol) was added to 100 ml of a tetrahydrofuran solution containing 10.5g (31.5 mmol) of 5-sec-butyl-6-chloro-2-(1H-pyrazol-1-yl)-N-(2,2,2-trifluoroethyl)pyrimidine-4-amine at room temperature and the mixture was stirred for 3 days. After confirming the completion of reaction, the reaction solution was poured into water and extracted with ethyl acetate. The obtained organic layer was washed with water and brine in this order and then dried over anhydrous magnesium sulfate. The insolubles were separated by filtration and then the solvent was distilled off under reduced pressure. Thus obtained residue was purified by silica gel column chromatography to obtain 10.6g of 5-sec-butyl-6-methylthio-2-(1H-pyrazol-1-yl)-N-(2,2,2-trifluoroethyl)pyrimidine-4-amine as a colorless transparent viscous substance (yield: 97.2%).

Refractive Index ($n_D^{20}$): 1.5559

$^1$H-NMR Data ($CDCl_3$/TMS δ (ppm)): 0.91 (t, 3H), 1.32 (d, 3H), 1.73 (m, 2H), 2.62 (s, 3H), 3.14 (br, 1H), 4.38 (m, 2H), 4.88 (br, 1H), 6.44 (t, 1H), 7.80 (s, 1H), 8.52 (d, 1H)

[Example 11]

Production of 5-sec-butyl-6-methylsulfonyl-2-(1H-pyrazol-1-yl)-N-(2,2,2-trifluoroethyl)pyrimidine-4-amine (Inventive Compound No. 0363)

16.6g of m-chloroperbenzoic acid (purity: 70%, 67.5 mmol) was added to 100 ml of a chloroform solution containing 10.6g (30.7 mmol) of 5-sec-butyl-6-methylthio-2-(1H-pyrazol-1-yl)-N-(2,2,2-trifluoroethyl)pyrimidine-4-amine under ice cooling and the mixture was stirred for 30 minutes. Then, the mixture was further stirred overnight at room temperature. After confirming the completion of reaction, the reaction solution was poured into water and extracted with chloroform. The obtained organic layer was washed with an aqueous solution of sodium bisulfite, water, an aqueous sodium bicarbonate solution, water and brine in this order and then dried over anhydrous magnesium sulfate. The insolubles were separated by filtration and then the solvent was distilled off under reduced pressure. Thus obtained residue was purified by silica gel column chromatography to obtain 9.2g of 5-sec-butyl-6-methylsulfonyl-2-(1H-pyrazol-1-yl)-N-(2,2,2-trifluoroethyl)pyrimidine-4-amine as a white crystal (yield: 79.3%).

Melting Point (˚C): 52 to 55

$^1$H-NMR Data ($CDCl_3$/TMS δ (ppm)): 0.96 (t, 3H), 1.38 (d, 3H), 1.77 (m, 2H), 3.46 (s, 3H), 4.02 (m, 1H), 4.45 (m, 2H), 5.48 (br, 1H), 6.49 (t, 1H), 7.83 (s, 1H), 8.42 (d, 1H)

[Example 12]

Production of 6-benzyloxy-5-sec-butyl-2-(1H-pyrazol-1-yl)-N-(2,2,2-trifluoroethyl)pyrimidine-4-amine (Inventive Compound No. 0357)

A mixture of 1.6g (4.2 mmol) of 5-sec-butyl-6-methylsulfonyl-2-(1H-pyrazol-1-yl)-N-(2,2,2-trifluoroethyl)pyrimidine-4-amine, 0.7g (6.4 mmol) of sodium bicarbonate and 10 ml of benzyl alcohol was stirred for 12 hours at 150˚C. After confirming the completion of reaction, the reaction solution was poured into water and extracted with ethyl acetate. The obtained organic layer was washed with water and brine in this order and then dried over anhydrous magnesium sulfate. The insolubles were separated by filtration and then the solvent was distilled off under reduced pressure. Thus obtained residue was purified by silica gel column chromatography to obtain a mixture of 6-benzyloxy-5-sec-butyl-2-(1H-pyrazol-1-yl)-N-(2,2,2-trifluoroethyl)pyrimidine-4-amine and benzyl alcohol.

[Example 13]

Production of 5-sec-butyl-2-(1H-pyrazol-1-yl)-6-(2,2,2-trifluoroethylamino)pyrimidin-4-ol (Inventive Compound No. 0353)

The mixture of 6-benzyloxy-5-sec-butyl-2-(1H-pyrazol-1-yl)-N-(2,2,2-trifluoroethyl)pyrimidine-4-amine and benzyl alcohol, which was obtained in Example 12, was dissolved in 20 ml of methanol and 0.1g of palladium carbon was added at room temperature. A hydrogen gas was supplied to the reaction solution under normal pressure and the solution was stirred overnight at room temperature. After confirming the completion of reaction, the insolubles were separated by filtration. After concentrating thus obtained filtrate, water was added and extraction with ethyl acetate was

subjected. The obtained organic layer was dried over anhydrous magnesium sulfate. The insolubles were separated by filtration and then the solvent was distilled off under reduced pressure. Thus obtained residue was purified by silica gel column chromatography to obtain 0.8g of 5-sec-butyl-2-(1H-pyrazol-1-yl)-6-(2,2,2-trifluoroethylamino)pyrimidin-4-ol as a white powder (yield: 64.9%).

Melting Point (˚C) : 141 to 143

$^1$H-NMR Data ($CDCl_3$/TMS δ (ppm)): 0.90 (t, 3H), 1.31 (d, 3H), 1.70 (m, 1H), 1.86 (m, 1H), 2.82 (br, 1H), 4.22 (m, 2H), 4.84 (br, 1H), 6.51 (s, 1H), 7.74 (d, 1H), 8.33 (d, 1H)

[Example 14]

Production of 5-sec-butyl-6-difluoromethoxy-2-(1H-pyrazol-1-yl)-N-(2,2,2-trifluoroethyl)pyrimidine-4-amine (Inventive Compound No. 0358)

0.4g (3.0 mmol) of anhydrous potassium carbonate was added to 10 ml of an N,N-dimethylformamide solution containing 0.63g (2.0 mmol) of 5-sec-butyl-2-(1H-pyrazol-1-yl)-6-(2,2,2-trifluoroethylamino)pyrimidin-4-ol at room temperature. At 50˚C, the mixture was stirred for 1 hour while introducing an excessive amount of chlorodifluoromethane to the reaction solution. After confirming the completion of reaction, the reaction solution was poured into water and extracted with ethyl acetate. The obtained organic layer was washed with water and brine in this order and then dried over anhydrous magnesium sulfate. The insolubles were separated by filtration and then the solvent was distilled off under reduced pressure. Thus obtained residue was purified by silica gel column chromatography to obtain 0.35g of 5-sec-butyl-6-difluoromethoxy-2-(1H-pyrazol-1-yl)-N-(2,2,2-trifluoroethyl)pyrimidine-4-amine as a white powder (yield: 47.9%).

Melting Point (˚C): 94 to 95

$^1$H-NMR Data ($CDCl_3$/TMS δ (ppm)): 0.88 (t, 3H), 1.33 (d, 3H), 1.76 (m, 2H), 2.83 (m, 1H), 4.37 (m, 2H), 5.24 (br, 1H), 6.45 (dd, 1H), 7.64 (t, 1H, J=72.5 Hz), 7.80 (d, 1H), 8.43 (d, 1H)

[Example 13]

Production of ethyl 5-amino-1-[5-sec-butyl-4-chloro-6-(2,2,2-trifluoroethylamino)pyrimidin-2-yl]-1H-pyrazol-4-carboxylate (Inventive Compound No. 0601)

0.6g (3.8 mmol) of ethyl ethoxymethylenecyanoacetate was added to 10 ml of an ethanol solution containing 1.0g (3.2 mmol) of 5-sec-butyl-6-chloro-2-hydrazinyl-N-(2,2,2-trifluoroethyl)pyrimidine-4-amine at room temperature and the mixture was stirred for 27 hours under reflux. After confirming the completion of reaction, the solvent was distilled off under reduced pressure. The reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 0.9g of ethyl 5-amino-1-[5-sec-butyl-4-chloro-6-(2,2,2-trifluoroethylamino)pyrimidin-2-yl]-1H-pyrazol-4-carboxylate as a white powder (yield: 63%).

Melting Point (˚C): 173 to 174

$^1$H-NMR Data ($CDCl_3$/TMS δ (ppm)): 0.91 (t, 3H), 1.34-1.39 (m, 6H), 1.73-1.78 (m, 2H), 3.39 (br, 1H), 4.26-4.44 (m, 4H), 5.27 (br, 1H), 7.29 (br, 2H), 7.82 (s, 1H)

[Example 16]

Production of 5-sec-butyl-6-chloro-2-(4-chloro-1H-pyrazol-1-yl)-N-(2,2,2-trifluoroethyl)pyrimidine-4-amine (Inventive Compound No. 0434)

0.4g (3.3 mmol) of N-chlorosuccinimide was added to 10 ml of an acetonitrile solution containing 1.0g (3.0 mmol) of 5-sec-butyl-6-chloro-2-(1H-pyrazol-1-yl-)-N-(2,2,2-trifluoroethyl)pyrimidine-4-amine at room temperature and the mixture was stirred for 2 hours under reflux. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 0.8g of 5-sec-butyl-6-chloro-2-(4-chloro-1H-pyrazol-1-yl)-N-(2,2,2-trifluoroethyl)pyrimidine-4-amine as a white powder (yield: 76%).

Melting Point (˚C) : 173 to 174

$^1$H-NMR Data ($CDCl_3$/TMS δ (ppm)): 0.91 (t, 3H), 1.35 (d, 3H), 1.75 (m, 2H), 3.40 (br, 1H), 4.35 (m, 2H), 5.28 (br, 1H),

7.71 (s, 1H), 8.42 (s, 1H)

[Example 17]

Production of 5-sec-butyl-N-(1-methylethyl)-2-(1H-pyrazol-1-yl)-6-trifluoromethylpyrimidine-4-amine (Inventive Compound No. 0240)

0.4g (4.0 mmol) of isopropylamine hydrochloride and 0.4g (4.0 mmol) of triethylamine were added to 20 ml of a tetrahydrofuran solution containing 0.4g (1.3 mmol) of 5-sec-butyl-4-chioro-2-(1H-pyrazol-1-yl)-6-trifluoromethylpyrimidine that can be obtained in Reference Example 19 which will be described below, at room temperature and the mixture was stirred overnight at 60˚C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was dissolved in 20 ml of ethanol and 0.2g (2.4 mmol) of anhydrous sodium acetate and 40mg of 10% palladium carbon were added thereto. A hydrogen gas was supplied under normal pressure and the mixture was stirred overnight at room temperature. After confirming the completion of reaction, the catalyst was removed by filtration and thus obtained filtrate was concentrated. Water was added to the residue and it was extracted with ethyl acetate. The obtained organic layer was dried over anhydrous magnesium sulfate. The insolubles were separated by filtration and then the solvent was distilled off under reduced pressure. Thus obtained residue was purified by silica gel column chromatography to obtain 0.26g of 5-sec-butyl-N-(1-methylethyl)-2-(1H-pyrazol-1-yl)-6-trifluoromethylpyrimidine-4-amine as a colorless crystal (yield: 59%).
Melting Point (˚C): 85 to 87
$^1$H-NMR Data ($CDCl_3$/TMS δ (ppm)): 0.89 (t, 3H), 1.32 (d, 9H), 1.73 (m, 2H), 3.25 (m, 1H), 4.54 (m, 1H), 5.06 (br, 1H), 6.43 (dd, 1H), 7.80 (d, 1H), 8.52 (d, 1H)

[Example 18]

Production of 5-sec-butyl-6-ethyl-2-(1H-pyrazol-1-yl)-N-(2,2,2-trifluorcethyl)pyrimidine-4-amine (Inventive Compound No. 0385)

0.4g (6.6 mmol) of 1H-pyrazole and 0.9g (6.6 mmol) of potassium carbonate were added to 30 ml of an N,N-dimethylformamide solution containing 1.1g (3.3 mmol) of 5-sec-butyl-6-ethyl-2-methylsulfonyl-N-(2,2,2-trifluoroethyl) pyrimidine-4-amine at room temperature and the mixture was stirred for 4 days at 80˚C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 1.0g of 5-sew-butyl-6-ethyl-2-(1H-pyrazol-1-yl)-N-(2,2,2-trifluoroethyl)pyrimidine-4-amine as a white powder (yield: 89%).
Melting Point (˚C): 89 to 90
$^1$H-NMR Data ($CDCl_3$/TMS δ (ppm)): 0.91 (t, 3H), 1.26-1.35 (m, 6H), 1.66-1.78 (m, 2H), 2.76-2.89 (m, 2H), 3.10 (br, 1H), 5.00 (br, 1H), 6.43 (t, 1H), 7.78 (d, 1H), 8.51 (d, 1H)

[Example 19]

Production of 6-bromo-5-sec-butyl-2-(1H-pyrazol-1-yl)-N-(2,2,2-trifluoroethyl)pyrimidine-4-amine (Inventive Compound No. 0351)

0.5g (7.1 mmol) of 1H-pyrazole and 1.6g (11.9 mmol) of anhydrous potassium carbonate were added to 30 ml of an N,N-dimethylformamide solution containing 2.3g (5.9 mmol) of 6-bromo-5-sec-butyl-2-methylsulfonyl-N-(2,2,2-trifluoroethyl)pyrimidine-4-amine at room temperature and the mixture was stirred for 24 hours at 50˚C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 1.4g of 6-bromo-5-sec-butyl-2-(1H-pyrazol-1-yl)-N-(2,2,2-trifluoroethyl)pyrimidine-4-amine as a white powder (yield: 62%).
Melting Point (˚C): 136 to 139
$^1$H-NMR Data ($CDCl_3$/TMS δ (ppm)): 0.93 (t, 3H), 1.33 (d, 3H), 1.69-1.79 (m, 2H), 3.50 (br, 1H), 4.32-4.43 (m, 2H), 5.22 (br, 1H), 6.45 (q, 1H), 7.79 (d, 1H), 8.45 (d, 1H)

[Example 20]

Production of 5-sec-butyl-6-cyclopropyl-2-(1H-pyrazol-1-yl)-N-(2,2,2-trifluoroethyl)pyrimidine-4-amine (Inventive Compound No. 0387)

0.1g (0.1 mmol) of tetrakis(triphenylphosphine)palladium, 0.2g (2.0 mmol) of cyclopropylboronic acid, 0.3g (2.5 mmol) of anhydrous sodium carbonate and 5 ml of water were added to 20 ml of a toluene solution containing 0.6g (1.7 mmol) of 5-sec-butyl-6-bromo-2-(1H-pyrazol-1-yl)-N-(2,2,2-trifluoroethyl)pyrimidine-4-amine and the mixture was stirred for 26 hours under reflux. The reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 0.3g of 5-sec-butyl-6-cycloprapyl-2-($^1$H-pyrazol-1-yl)-N-(2,2,2-trifluoroethyl)pyrimidine-4-amine as a pale yellow powder (yield: 49%).

Melting Point (˚C): 84 to 85

$^1$H-NMR Data ($CDCl_3$/TMS δ (ppm) : 0.89-1.03 (m, 5H), 1.18-1.37 (m, 5H), 1.73-1.83 (m, 2H), 2.10-2.19 (m, 1H), 3.30 (br, 1H), 4.37-4.43 (m, 2H), 4.98 (br, 1H), 6.40 (t, 1H), 7.77 (t, 1H), 8.47 (d, 1H)

[Example 21]

Production of 5-sec-butyl-$N^4$, $N^4$-diethyl-2-(1H-pyrazol-1-yl)-$N^6$-(2,2,2-trifluoroethyl)pyrimidine-4,6-diamine (Inventive Compound No. 0371)

0.3g (2.4 mmol) of anhydrous potassium carbonate and 0.1g (1.6 mmol)) of 1H-pyrazole were added to 10 ml of 1,3-dimethyl-2-imidazolidinone solution containing 0.6g (1.6 mmol) of 5-sec-butyl-$N^4$,$N^4$-diethyl-2-methylsulfonyl-$N^6$-(2,2,2-trifluoroethyl)pyrimidine-4,6-diamine and the mixture was stirred for 6 hours at 150˚C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 0.2g of 5-sec-butyl-$N^4$,$N^4$-diethyl-2-(1H-pyrazol-1-yl)-$N^6$-(2,2,2-trifluoroethyl)pyrimidine-4,6-diamine as a yellowish brown viscous liquid (yield: 38%).

Refractive Index ($n_D^{20}$):1.5280

$^1$H-NMR Data ($CDCl_3$/TMS δ (ppm)): 0.83 (t, 3H), 1.14 (t, 6H), 1.34 (d, 3H), 1.66 (m, 2H), 3.15 (m, 2H), 3.36 (m, 2H), 4.49-4.28 (m, 2H), 4.79 (br, 1H), 6.40 (m, 1H), 7.78 (d, 1H), 8.49 (d, 1H)

Next, physical properties of the compounds of present application synthesized according to above-mentioned Examples 1 to 21 are shown in Tables 41 to 50.

[Table 41]

| Compound No. | M.P. (˚C) OR R.I.($n^D_{20}$) | |
|---|---|---|
| 0002 | M.P. | 146-148 |
| 0006 | M.P. | 141-144 |
| 0009 | M.P. | 42-44 |
| 0010 | M.P. | 78-80 |
| 0013 | M.P. | 53-54 |
| 0014 | M.P. | 91-92 |
| 0018 | M.P. | 131-133 |
| 0022 | M.P. | 148-151 |
| 0025 | R.I. | 1.5758 |
| 0026 | R.I. | 1.5888 |
| 0029 | R.I. | 1.5870 |
| 0030 | M.P. | 90-91 |
| 0035 | M.P. | 140-142 |
| 0047 | M.P. | 149-151 |
| 0049 | M.P. | 140-142 |
| 0133 | M.P. | 64-65 |

(continued)

| Compound No. | M.P. (˚C) OR R.I.($n^D_{20}$) | |
|---|---|---|
| 0135 | R.I. | 1.5766 |
| 0147 | M.P. | 89-90 |
| 0149 | M.P. | 107-108 |
| 0155 | M.P. | 175-178 |
| 0159 | M.P. | 139-141 |
| 0162 | R.I. | 1.5691 |
| 0163 | M.P. | 63-65 |
| 0166 | M.P. | 55-56 |
| 0167 | M.P. | 78-79 |
| 0171 | M.P. | 168-169 |
| 0175 | M.P. | 120-123 |
| 0178 | R.I. | 1.5631 |
| 0179 | M.P. | 41-42 |
| 0182 | R.I. | 1.5699 |
| 0183 | R.I. | 1.5696 |
| 0186 | R.I. | 1.5542 |
| 0187 | M.P. | 132-133 |
| 0190 | R.I. | 1.5717 |
| 0191 | M.P. | 164-165 |
| 0194 | M.P. | 105-107 |
| 0195 | M.P. | 138-140 |
| M.P.: Melting Point<br>R.I.: Refractive Index | | |

[Table 42]

| Compound No. | M.P.(˚C) OR R.I.($n^B_{20}$) | |
|---|---|---|
| 0198 | M.P. | 110-111 |
| 0199 | M.P. | 150-151 |
| 0200 | R.I. | 1.5709 |
| 0211 | R.I. | 1.5740 |
| 0222 | M.P. | 116-117 |
| 0240 | M.P. | 85-87 |
| 0241 | M.P. | 120-121 |
| 0242 | M.P. | 106-107 |
| 0245 | R.I. | 1.5449 |
| 0246 | M.P. | 48-50 |
| 0249 | M.P. | 99-101 |
| 0250 | M.P. | 113-114 |
| 0253 | M.P. | 88-90 |
| 0254 | R.I. | 1.5668 |
| 0259 | M.P. | 93-95 |
| 0300 | R.I. | 1.5555 |
| 0301 | R.I | 1.5642 |
| 0304 | M.P. | 91-93 |
| 0305 | R.I. | 1.5608 |
| 0308 | M.P. | 89-90 |
| 0309 | R.I. | 1.5639 |

(continued)

| Compound No. | M.P.(˚C) OR R.I.($n^B_{20}$) | |
|---|---|---|
| 0312 | R.I. | 1.5335 |
| 0313 | R.I. | 1.5587 |
| 0316 | M.P. | 104-107 |
| 0317 | M.P. | 125-127 |
| 0325 | R.I. | 1.5569 |
| 0328 | M.P. | 138-139 |
| 0329 | M.P. | 117-119 |
| 0332 | M.P. | 110-112 |
| 0333 | M.P. | 93-95 |
| 0336 | M.P. | 87-90 |
| 0337 | M.P. | 107-109 |
| 0341 | M.P. | 114-117 |
| 0348 | M.P. | 147-149 |
| 0349 | M.P. | 132-134 |
| 0350 | M.P. | 137-139 |
| 0351 | M.P. | 136-139 |
| M.P.: Melting Point<br>R.I.: Refractive Index | | |

[Table 43]

| Compoun No. | M.P. (˚C) 0R R.I. ($n^D_{20}$) | |
|---|---|---|
| 0353 | M.P. | 141-143 |
| 0354 | M.P. | 95-96 |
| 0355 | M.P. | 97-98 |
| 0358 | M.P. | 94-95 |
| 0360 | M.P. | 104-105 |
| 0361 | R.I. | 1.5559 |
| 0363 | M.P. | 52-55 |
| 0367 | R.I. | 1.5312 |
| 0368 | R.I. | 1.5289 |
| 0370 | M.P. | 97-99 |
| 0371 | R.I. | 1.5280 |
| 0372 | M.P. | 193-195 |
| 0374 | M.P. | 102-103 |
| 0376 | M.P. | 178-180 |
| 0377 | M.P. | 124-127 |
| 0379 | M.P. | 173-174 |
| 0380 | M.P. | 84-85 |
| 0381 | M.P. | 92-94 |
| 0382 | M.P. | 88-90 |
| 0383 | R.I. | 1.5236 |
| 0384 | R.I. | 1.5342 |
| 0385 | M.P. | 89-90 |
| 0386 | M.P. | 94-96 |
| 0387 | M.P. | 84-85 |
| 0389 | M.P. | 50-52 |
| 0391 | M.P. | 109-110 |

(continued)

| Compoun No. | M.P. (˚C) 0R R.I. ($n^D_{20}$) | |
|---|---|---|
| 0392 | M.P. | 141-143 |
| 0393 | M.P. | 47-48 |
| 0394 | M.P. | 67-70 |
| 0395 | M.P. | 87-89 |
| 0401 | M.P. | 189-191 |
| 0403 | M.P. | 177-178 |
| 0410 | M.P. | 154-155 |
| 0422 | M.P. | 198-200 |
| 0434 | M.P. | 173-174 |
| 0435 | M.P. | 157-160 |
| 0436 | M.P. | 170-172 |
| M.P.: Melting Point<br>R.I.: Refractive Index | | |

[Table 44]

| Compound No. | M.P. (˚C) OR R.I. ($n^D_{20}$) | |
|---|---|---|
| 0437 | M.P. | 180-182 |
| 0462 | M.P. | 133-134 |
| 0489 | M.P. | 143-144 |
| 0504 | M.P. | 213-216 |
| 0507 | M.P. | 181-182 |
| 0508 | M.P. | 192-193 |
| 0510 | M.P. | 189-190 |
| 0628 | M.P. | 253-255 |
| 0536 | M.P. | 203-206 |
| 0601 | M.P. | 173-174 |
| 0614 | M.P. | 188-189 |
| 0710 | M.P. | 50-52 |
| 0721 | R.I. | 1.5458 |
| 0723 | M.P. | 111-113 |
| 0732 | M.P. | 58-60 |
| 0764 | M.P. | 50-62 |
| 0768 | M.P. | 150-153 |
| 0772 | M.P. | 83-85 |
| 0775 | M.P. | 101-102 |
| 0776 | M.P. | 97-99 |
| 0780 | R.I. | 1.5781 |
| 0784 | M.P. | 33-35 |
| 0791 | M.P. | 78-79 |
| 0792 | M.P. | 77-79 |
| 0796 | R.I. | 1.5643 |
| 0799 | R.I. | 1.5609 |
| 0800 | R.I. | 1.5647 |
| 0812 | M.P. | 81-83 |
| 0816 | M.P. | 57-59 |
| 0820 | M.P. | 103-105 |
| 0824 | M.P. | 176-177 |

(continued)

| Compound No. | M.P. (˚C) OR R.I. ($n^D_{20}$) | |
|---|---|---|
| 0828 | M.P. | 173-174 |
| 0836 | R.I. | 1.5566 |
| 0840 | R.I. | 1.5511 |
| 0844 | R.I. | 1.5432 |
| 0860 | M.P. | 109-111 |
| 0864 | M.P. | 99-100 |
| M.P.: Melting Point<br>R.I.: Refractive Index | | |

[Table 45]

| Compound No | M.P. (˚C) OR R.I. ($D^D_{20}$) | |
|---|---|---|
| 0868 | M.P. | 181-183 |
| 0900 | R.I. | 1.5822 |
| 0901 | R.I. | 1.5678 |
| 0902 | R.I. | 1.5720 |
| 0903 | M.P. | 137-138 |
| 0904 | M.P. | 115-116 |
| 0906 | M.P. | 95-98 |
| 0907 | R.I. | 1.5760 |
| 0908 | R.I. | 1.5862 |
| 0911 | R.I. | 1.5742 |
| 0912 | M.P. | 88-90 |
| 0913 | M.P. | 143-144 |
| 0015 | R.I. | 1.5650 |
| 0916 | R.I. | 1.5734 |
| 0919 | R.I. | 1.5615 |
| 0920 | R.I. | 1.5461 |
| 0921 | R.I. | 1.5681 |
| 0934 | M.P. | 87-88 |
| 0943 | M.P. | 95-98 |
| 0953 | R.I. | 1.5560 |
| 0961 | R.I. | 1.5273 |
| 0962 | R.I. | 1.5585 |
| 0963 | R.I. | 1.5755 |
| 0966 | R.I. | 1.5500 |
| 0967 | R.I. | 1.5642 |
| 0970 | R.I. | 1.5468 |
| 0971 | R.I. | 1.5630 |
| 0974 | M.P. | 98-99 |
| 0975 | R.I. | 1.5674 |
| 0979 | R.I. | 1.5556 |
| 0986 | R.I. | 1.5498 |
| 0987 | R.I. | 1.5640 |
| 0990 | R.I. | 1.5407 |
| 0991 | R.I. | 1.5573 |
| 0994 | R.I. | 1.5427 |
| 0996 | R.I. | 1.5538 |

(continued)

| Compound No | M.P. (°C) OR R.I. ($D^D_{20}$) | |
|---|---|---|
| 0998 | M.P. | 65-67 |
| M.P.: Melting Point<br>R.I.: Refractive Index | | |

[Table 46]

| Compound No. | M.P. (°C) OR R.I. ($R^D_{20}$) | |
|---|---|---|
| 1000 | M.P. | 79-81 |
| 1008 | M.P. | 33-36 |
| 1030 | M.P. | 111-113 |
| 1031 | M.P. | 131-132 |
| 1058 | R.I. | 1.5730 |
| 1060 | R.I. | 1.5718 |
| 1061 | R.I. | 1.5631 |
| 1062 | M.P. | 87-90 |
| 1064 | R.I. | 1.5563 |
| 1065 | R.I. | 1.5670 |
| 1068 | M.P. | 134-136 |
| 1070 | R.I. | 1.5671 |
| 1071 | M.P. | 123-125 |
| 1074 | R.I. | 1.5528 |
| 1075 | M.P. | 78-79 |
| 1079 | R.I. | 1.5661 |
| 1081 | M.P. | 100-102 |
| 1082 | M.P. | 86-88 |
| 1085 | R.I. | 1.5676 |
| 1086 | R.I. | 1.5652 |
| 1087 | R.I. | 1.5753 |
| 1089 | R.I. | 1.5629 |
| 1090 | M.P. | 87-90 |
| 1093 | R.I. | 1.5562 |
| 1094 | R.I. | 1.5645 |
| 1097 | M.P. | 61-63 |
| 1098 | R.I. | 1.5893 |
| 1101 | M.P. | 70-71 |
| 1102 | R.I. | 1.5810 |
| 1106 | M.P. | 133-135 |
| 1110 | M.P. | 102-104 |
| 1115 | M.P. | 96-98 |
| 1160 | R.I. | 1.5247 |
| 1163 | M.P. | 129-130 |
| 1167 | M.P. | 89-91 |
| 1175 | R.I. | 1.6073 |
| 1178 | M.P. | 61-64 |
| M.P.: Melting Point<br>R.I.: Refractive Index | | |

[Table 47]

| Compound No | M.P.(˚C) OR R.I. ($n^D_{20}$) | |
|---|---|---|
| 1179 | M.P. | 99-101 |
| 1182 | R.I. | 1.5617 |
| 1183 | R.I. | 1.5671 |
| 1186 | R.I. | 1.5598 |
| 1187 | R.I. | 1.5650 |
| 1190 | R.I. | 1.5565 |
| 1191 | R.I. | 1.5533 |
| 1192 | R.I. | 1.5721 |
| 1193 | R.I. | 1.5888 |
| 1195 | M.P. | 147-149 |
| 1196 | R.I. | 1.5503 |
| 1197 | R.I. | 1.5465 |
| 1199 | M.P. | 90-93 |
| 1204 | M.P. | 69-71 |
| 1211 | R.I. | 1.5621 |
| 1216 | R.I | 1.5700 |
| 1222 | M.P. | 160-162 |
| 1225 | R.I. | 1.5581 |
| 1233 | R.I. | 1.5344 |
| 1239 | R.I. | 1.5690 |
| 1243 | R.I. | 1.5623 |
| 1247 | R.I. | 1.5251 |
| 1254 | R.I. | 1.5707 |
| 1255 | R.I. | 1.5729 |
| 1259 | R.I. | 1.5610 |
| 1263 | R.I. | 1.6000 |
| 1267 | M.P. | 65-68 |
| 1271 | M.P. | 89-92 |
| 1275 | R.I. | 1.5741 |
| 1279 | M.P. | 152-153 |
| 1283 | M.P. | 121-124 |
| 1286 | R.I. | 1.5600 |
| 1287 | R.I. | 1.5707 |
| 1290 | R.I. | 1.5653 |
| 1291 | M.P. | 66-68 |
| 1311 | M.P. | 83-85 |
| 1315 | M.P. | 168-169 |
| M.P.: Melting Point<br>R.I.: Refractive Index | | |

[Table 48]

| Compound No. | M.P. (˚C) OR R.I. ($n^D_{20}$)) | |
|---|---|---|
| 1318 | R.I. | 1.5557 |
| 1319 | R.I. | 1.5629 |
| 1322 | M.P. | 56-58 |
| 1323 | R.I. | 1.5741 |

(continued)

| Compound No. | M.P. (˚C) OR R.I. ($n^D_{20}$)) | |
|---|---|---|
| 1328 | M.P. | 103-104 |
| 1342 | M.P. | 103-105 |
| 1426 | M.P. | 65-67 |
| 1428 | R.I. | 1.5670 |
| 1440 | M.P. | 61-63 |
| 1442 | M.P. | 71-72 |
| 1448 | M.P. | 127-128 |
| 1462 | M.P. | 81-83 |
| 1455 | R.I. | 1.5513 |
| 1466 | R.I. | 1.5731 |
| 1459 | M.P. | 64-66 |
| 1460 | M.P. | 93-95 |
| 1497 | M.P. | 111-113 |
| 1511 | M.P. | 152-155 |
| 1633 | M.P. | 183-184 |
| 1664 | M.P. | 68-70 |
| 1576 | M.P. | 83-86 |
| 1595 | M.P. | 55-57 |
| 1597 | M.P. | 94-96 |
| 1609 | M.P. | 80-83 |
| 1611 | M.P. | 91-93 |
| 1617 | M.P. | 141-143 |
| 1624 | R.I. | 1.5728 |
| 1625 | R.I. | 1.5760 |
| 1628 | M.P. | 117-119 |
| 1629 | M.P. | 118-119 |
| 1633 | M.P. | 123-126 |
| 1637 | M.P. | 146-147 |
| 1640 | R.I. | 1.5700 |
| 1641 | R.I. | 1.5830 |
| 1644 | R.I. | 1.5798 |
| 1645 | R.I. | 1.5877 |
| 1651 | R.I. | 1.5513 |
| M.P.: Melting Point<br>R.I.: Refractive Index | | |

[Table 49]

| Compound No. | M.P. (˚C) OR R.I. ($n^D_{20}$) | |
|---|---|---|
| 1661 | M.P. | 119-122 |
| 1737 | M.P. | 106-108 |
| 1829 | M.P. | 151-152 |
| 1833 | R.I. | 1.6217 |
| 1845 | M.P. | 118-120 |
| 1849 | R.I. | 1.5917 |
| 1850 | M.P. | 141-143 |
| 1862 | M.P. | 96-98 |
| 1898 | R.I. | 1.6052 |

(continued)

| Compound No. | M.P. (°C) OR R.I. ($n^D_{20}$) | |
|---|---|---|
| 1906 | M.P. | 107-109 |
| 1914 | R.I. | 1.5904 |
| 1918 | M.P. | 55-57 |
| 1994 | R.I. | 1.5803 |
| 1996 | M.P. | 163-170 |
| 2011 | M.P. | 95-97 |
| 0398 | M.P. | 145-146 |
| 0454 | M.P. | 144-145 |
| 0455 | M.P. | 81-83 |
| 0458 | M.P. | 168-169 |
| 0459 | M.P. | 125-127 |
| 0461 | M.P. | 253-255 |
| 0464 | M.P. | 249-250 |
| 0465 | M.P. | 123-125 |
| 0481 | M.P. | 175-178 |
| 0505 | M.P. | 203-204 |
| 0509 | M.P. | 157-160 |
| 0511 | M.P. | 55-58 |
| 0529 | M.P. | 188-190 |
| 0531 | M.P. | 147-149 |
| 0533 | M.P. | 202-204 |
| 0537 | M.P. | 168-170 |
| 0538 | M.P. | 174-176 |
| 0539 | M.P. | 107-110 |
| 0548 | M.P. | 180-181 |
| 0555 | M.P. | 163-164 |
| 0556 | M.P. | 165-167 |
| 0557 | M.P. | 1.4794 |
| 0615 | M.P. | 46-49 |
| 0698 | M.P. | 193-194 |
| 0702 | M.P. | 221-222 |
| 1958 | R.I. | 1.5581 |
| M.P.: Melting Point<br>R.I.: Refractive Index | | |

[Table 50]

| Compound No. | $^1$H-NMR data($CDCl_3$/TMS δ(ppm)) |
|---|---|
| 0232 | 0.88(t, 3H, J=7.4Hz), 1.26-1.31(m, 3H), 1.65-1.79(m,2H), 2.48(s, 3H), 4.35-4.50(m, 1H), 4.63(br, 1H), 6.39-6.41(m, 1H), 7.76-7.76(m, 1H), 8.49-8.58(m, 1H) |
| 0373 | 0.88(t, 3H, J=7.4Hz), 1.34(d, 3H, J=7.2Hz), 1.63-1.79(m, 2H), 4.10-4.15(m, 1H). 4.37-4.46(m, 2H), 6.45(br, 1H), 6.49-6.50(m, 1H), 7.84(s, 1H), 8.53(d, 1H, J=2.8Hz) |
| 0708 | 0.91-0.97(m,3H), 1.28-1.32(m, 3H), 1.47(d, 3H, J=6.9Hz), 1.62-1.78(m, 2H), 2.43-2.63(m, 1H), 4.79-4.83(br, 1H), 5.22- 5.35(m, 2H), 6.46-6.45(m, 1H), 7.79(s, 1H), 8.48(d.,1H, J=2.1Hz) |
| 0760 | 0.89(3H, t, J=7.6Hz), 1.31(3H, d, J=7.3Hz), 1.69-1.73(2H, m), 2.49-2.60(2H, m), 3.34-3.51(1H, br), 3.89-3.93(2H, m), 5.34(1H, br), 6.44-6.45(1H, m), 7.78(1H, t), 8.45-8.49(1H. m) |

(continued)

| Compound No. | $^1$H-NMR data($CDCl_3$/TMS δ(ppm)) |
|---|---|
| 0788 | 0.91(3H. t, J=7.3Hz 1.33(3H, d, J=7.2 Hz), 1.69-1.78(2H, m), 3.36(1H, brs), 3.78(2H, dt, J=9.1, 5.2 Hz), 3.89(3H, t, J=4.8 Hz), 5.60(1.H, br), 6.43(1H, dd, J=2.5, 1.7Hz), 7.77(1H, a), 8.47(1H, d, J=2.2Hz,) |
| 0827 | 0.91(3H, t, J=7.3Hz), 1.26(3H, d, J=6.8Hz), 1.58-1.77(2H, m), 2.48-2.57(1H, m), 2.67(2H, t, J=6.1Hz), 3.86-3.93(2H, m), 5.69(1H, br), 6.44-6.47(1H, m), 7.77(1H, t), 8.08(1H, s), 8.47(1H, d) |
| 1155 | 0.91(3H, t, J=7.4 Hz), 1.30(3H, d, J=7.7 Hz), 1.58-1.71(2H, m), 2.39-2.53(2H, m), 2.88-2.94(1H, m), 3.95-4.09(4H, m), 6.44(1H, s), 7.79(1H, s), 8.25(1H, s), 8.48(1H, d, J=2.5 Hz) |
| 1156 | 0.83(3H, t, J=7.5 Hz, 1.48(3H, J=7.1 Hz), 1.88-1.97(2H, m), 2.39-2.49(2H, m), 2.83-2.92(m, m), 3.80-3.93(m, m), 3.98-4.17(2H, m, 6.45(1H, dd, J=2.6 1.6 Hz), 7.79(1H, d, J=0.5 Hz), 8.46(1H, d, J=2.7 Hz) |
| 1159 | 0.92(3H, t, J=7.4 Hz), 1.82(3H, d, J=6.8 Hz), 1.58-1.75(2H, m), 2.77-1159 2.83(1H, m), 4.09-4.30 (4H, m), 6.46(1H, d, J=2.5 Hz, 7.81(1H, s), 8.33(1H, s), 8.47(1H, d, J=2.5 Hz) |
| 1206 | 0.85(3H, t, J=7.4Hz), 1.01(3H, d J=6.3Hz, 1.35-1.43(2H,m), 1.53(3H, d) 1206 J=7.1Hz), 1.61-1.81 (3H, m), 1.88-1.98(2H,m), 2.99-3.07(2H, m), 3.24-3.33(1H, m), 3.45(3H, s), 3.85(2H, m), 6.46-6.48 (1H, m), 7.82(1H, s), 8.44(1H, d, J=2.2Hz) |

(Intermediate Production Method)

<Reference Example 1>

Production of 5-sec-butyl-2-mercaptopyrimidine-4,6-diol

69.2g (203.4 mmol) of a 20% sodium ethoxide-ethanol solution was added to 100 ml of an ethanol solution containing 20.0g (92.5 mmol) of diethyl sec-butylmalonate and 7.7g (101.7 mmol) of thiourea at room temperature and the mixture was stirred under reflux for 6 hours. A 5-sec-butyl-2-mercaptopyrimidine-4,6-diol production was confirmed with a gas chromatograph and a gas chromatograph mass spectrometer.

<Peference Example 2>

Production of 5-sec-b-butyl-2-methylthiopyrimidine-4,6-diol

14.4g (101.7 mmol) of methyl iodide was added to the reaction solution of Reference Example 1 at room temperature and the mixture was stirred for 14 hours at room temperature. After confirming the completion of reaction, the solvent was distilled off under reduced pressure. To the obtained residue, water was added and pH was adjusted to 2 using concentrated hydrochloric acid. A precipitated crystal was filtered, washed with water and then dried to obtain 17.5g of 5-sec-butyl-2-methylthiopyrimidine-4,6-diol as a milky white crystal (yield: 88%).

$^1$H-NMR Data (DMSO-d6/TMS δ (ppm)): 0.73 (3H, t, J=7.4 Hz), 1.11 (3H, d, J=7.1 Hz), 1.39-1.53 (1H, m), 1.62-1.77 (1H, m), 2.46 (3H, s), 2.74-2.86 (1H, m), 11.5 (2H, br).

<Reference Example 3>

Production of 5-sec-butyl-4,6-dichloro-2-methylthiopyrimidine

11.5g (74.9 mmol) of phosphorus oxychloride and 3.0g (24.9 mmol) of N,N-dimethylaniline were added to 5.0g (25.0 mmol) of 5-sec-butyl-2-methylthiopyrimidine-4,6-diol and the mixture was stirred for 2 hours at 100°C. After confirming the completion of reaction, the reaction solution was poured into water and extracted with dichloromethane. The obtained organic layer was washed with water and then with a saturated aqueous sodium bicarbonate solution and dried over anhydrous magnesium sulfate. The insolubles were separated by filtration and then the solvent was distilled off under reduced pressure, to obtain 5.9g of 5-sec-butyl-4,6-dichloro-2-methylthiopyrimidine (yield: 99%).

$^1$H-NMR Data ($CDCl_3$/TMS δ (ppm)): 0.86 (3H, t, J=7.4 Hz), 1.36 (3H, d, J=7.1 Hz)), 1.67-1.83 (1H, m), 1.89-2.04 (1H, m), 2.55 (3H, s), 3.38-3.51 (1H, m)

<Reference Example 4>

Production of 5-sec-butyl-4-chloro-6-(4-methylpiperidin-1-yl)-2-methylthiopyrimidine

4.0g (39.2 mmol) of triethylamine was added to 35 ml of a tetrahydrofuran solution containing 4.5g (17.8 mmol) of 5-sec-butyl-4,6-dichloro-2-methylthiopyrimidine. Thereto, 1.9g (19.6 mmol) of 4-methylpiperidine was further added under ice cooling and the mixture was stirred for 14 hours at room temperature. After confirming the completion of reaction, the reaction solution was poured into water and extracted with ethyl acetate. The obtained organic layer was washed with 5% hydrochloric acid, a saturated aqueous sodium bicarbonate solution and a saturated aqueous sodium chloride solution and then dried over anhydrous magnesium sulfate. The insolubles were separated by filtration and then the solvent was distilled off under reduced pressure, to obtain 5.7g of 5-sec-butyl-4-ohloro-6-(4-methylpiperidin-1-yl)-2-methylthiopyrimidine as a pale yellow viscous substance (yield: quantitative).
Refractive Index ($n_D^{20}$): 1.5537
$^1$H-NMR Data ($CDCl_3$/TMS $\delta$ (ppm)): 0.81 (3H, t, J=7.4 Hz), 0.98 (3H, d, J=6.6 Hz), 1.25-1.40 (5H, m), 1.57-1.90 (5H, m), 2.50 (3H, s), 2.75-2.95 (3H, m), 3.58-3.63 (2H, m)

<Reference Example 5>

Production of 5-sec-butyl-4-chloro-6-(4-methylpiperidin-1-yl)-2-methylsulfonylpyrimidine

9.2g of m-chloroperbenzoic acid (purity: 70%, 53.4 mmol) was added to 200 ml of a dichloromethane solution containing 5.7g (17.8 mmol) of 5-sec-butyl-4-chloro-6-(4-methylpiperidin-1-yl)-2-methylthiopyrimidine under ice cooling and the mixture was stirred for 30 minutes. Then, the mixture was further stirred for 7 hours at room temperature. After confirming the completion of reaction, the reaction solution was poured into water and extracted with dichloromethane. The obtained organic layer was washed with an aqueous solution of sodium bisulfite, water, an aqueous sodium bicarbonate solution and brine in this order and then dried over anhydrous magnesium sulfate. The insolubles were separated by filtration and then the solvent was distilled off under reduced pressure. Thus obtained residue was purified by silica gel column chromatography to obtain 5.9g of 5-sec-butyl-4-chloro-6-(4-methylpiperidin-1-yl)-2-methylsulfonylpyrimidine as a white powder (yield: 96%).
Melting Point (˚C) : 88 to 90
$^1$H-NMR Data ($CDCl_3$/TMS $\delta$ (ppm)): 0.84 (3H, t, J=7.4 Hz), 1.00 (3H, d, J=6.6 Hz), 1.22-1.45 (5H, m), 1.56-1.96 (5H, m), 2.77-2.85 (1H, m), 2.94-3.06 (2H, m), 3.29 (3H, s), 3.79-3.83 (2H, m)

<Reference Example 6>

Production of dimethyl 2-[5-sec-butyl-6-(4-methylpiperidin-1-yl)-2-(1H-pyrazol-1-yl)pyrimidin-4-yl]malonate

1.1g (8.5 mmol) of dimethyl malonate was added to 20 ml of a tetrahydrofuran solution containing 0.3g of sodium hydride (purity: 60%, 6.4 mmol) under ice cooling and the mixture was stirred for 1 hour at room temperature. Thereto, 10 ml of a tetrahydrofuran solution containing 1.6g (4.2 mmol) of 5-sec-butyl-6-(4-methylpiperidin-1-yl)-4-methylsulfonyl-2-(1H-pyrazol-1-yl)pyrimidine was further added dropwise under ice cooling. Thereafter, the mixture was stirred for 5 hours at 80˚C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. The obtained residue was purified by silica gel column chromatography to obtain 1.8g of dimethyl 2-[5-sec-butyl-6-(4-methylpiperidin-1-yl)-2-(1H-pyrazol-1-yl)pyrimidin-4-yl]malonate (yield: quantitative).
$^1$H-NMR Data ($CDCl_3$/TMS $\delta$ (ppm)): 0.88 (3H, t, J=7.5 Hz), 1.01 (3H, d, J=6.6 Hz),1.32-1.43 (4H, m), 1.51-1.95 (10H, m), 2.91-3.07 (2H, m), 3.58-3.71 (2H, m), 3.78 (3H, s), 5.12 (1H, s), 6.38-6.39 (1H, m), 7.78 (1H, s), 8.47 (1H, d, J=2.8 Hz)

<Reference Example 7>

Production of tert-butyl N-(5-sec-butyl-6-chloro-2-methylthiopyrimidon-4-yl)-N-2,2,2-trifluoroethylcarbamate

8.3g (41.6 mmol) of tert-butyl 2,2,2-trifluoroethylcarbamate was added to 40 ml of an N,N-dimethylformamide solution containing 1.3g of sodium hydride (purity: 60%, 33.3 mmol) under ice cooling and the mixture was stirred for 1 hour. Thereto, 7.0g (27.7 mmol) of 5-sec-butyl-4,6-dichloro-2-methylthiopyrimidine was further added at room temperature and the mixture was stirred overnight. After confirming the completion of reaction, the reaction solution was poured

into water and extracted with ethyl acetate. The obtained organic layer was washed with water and then dried over anhydrous magnesium sulfate. The insoluble were separated by filtration and the solvent was distilled off under reduced pressure. The obtained residue was purified by silica gel column chromatography to obtain 7.2g of ter-butyl N-(5-sec-butyl-6-chloro-2-methylthiopyrimidin-4-yl)-N-2,2,2-trifluoroethylcarbamate as a pale orange oily substance (yield: 63%).

Refractive Index ($n_D^{20}$): 1.4888
$^1$H-NMR Data ($CDCl_3$/TMS δ (ppm)): 0.94 (br, 3H), 1.24-1.59 (m, 14H), 2.54 (s, 3H), 2.77 (br, 1H), 4.46 (br, 2H)

<Reference Example 8>

Production of 5-sec-butyl-6-chloro-2-methylthio-N-(2,2,2-trifluoroethyl)pyrimidine-4-amine

7.7g (67.3 mmol) of trifluoroacetic acid was added to 20 ml of a dichloromethane solution containing 3.7g (9.0 mmol) of tert-butyl N-(5-sec-butyl-6-chloro-2-methylthiopyrimidin-4-yl)-N-2,2,2-trifluoroethylcarbamate at room temperature and the mixture was stirred overnight. After confirming the completion of reaction, the reaction solution was poured into water and extracted with dichloromethane. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 2.6g of 5-sec-butyl-6-chloro-2-methylthio-N-2,2,2-trifluoroethylpyrimidine-4-amine as a white powder (yield: 91%).

Melting Point (˚C): 82 to 83
$^1$H-NMR Data ($CDCl_3$/TMS δ (ppm)): 0.89 (t, 3H), 1.29 (d, 3H), 1.70 (m, 2H), 2.50 (s, 3H), 3.29 (br, 1H), 4.28 (m, 2H), 5.02 (br, 1H)

<Reference Example 9>

Production of 5-sec-butyl-6-chloro-2-methylsulfonyl-N-2,2,2-trifluoroethylpyrimidine-4-amine

7.9g of m-chloroperbenzoic acid (purity: 70%, 32.2 mmol) was added to 200 ml of a chloroform solution containing 2.9g (9.2 mmol) of 5-sec-butyl-6-chloro-2-methylthio-N-2,2,2-trifluoroethylpyrimidine-4-amine under ice cooling and the mixture was stirred for 30 minutes. Then, the mixture was further stirred for 1.5 hours at room temperature. After confirming the completion of reaction, the reaction solution was poured into water and extracted with dichloromethane. The obtained organic layer was washed with an aqueous solution of sodium bisulfite, water, an aqueous sodium bicarbonate solution and brine in this order and then dried over anhydrous magnesium sulfate. The insolubles were separated by filtration and then the solvent was distilled off under reduced pressure. Thus obtained residue was purified by silica gel column chromatography to obtain 2.74g of 5-sec-butyl-6-chloro-2-methylsulfonyl-N-2,2,2-trifluoroethylpyrimidine-4-amine as a white crystal (yield: 86%).

Melting Point (˚C): 107 to 109
$^1$H-NMR Data ($CDCl_3$/TMS δ (ppm)): 0.92 (t, 3H), 1.35 (d, 3H), 1.77 (m, 2H), 3.29 (s, 3H), 3.44 (br, 1H), 4.34 (m, 2H), 5.42 (br, 1H)

<Reference Example 10>

Production of 5-sec-butyl-4-hydroxy-2-mercapto-6-methylpyrimidine

To 100 my of an ethanol solution containing 2.7g (14.5 mmol) of ethyl 2-sec-butyl-3-oxobutanate synthesized according to a method disclosed in US No. 6348618 and 1.2g (15.9 mmol) of thiourea, 9.9g (29.0 mmol) of a 20% sodium ethoxide-ethanol solution was added at room temperature and the mixture was stirred for 4 hours under reflux. A 5-sec-butyl-4-hydroxy-2-mercapto-6-methylpyrimidine production was confirmed with a gas chromatograph and a gas chromatograph mass spectrometer.

<Reference Example 11>

Production of 5-sec-butyl-4-hydroxy-6-methyl-2-methylthiopyrimidine

2.3g (15.9 mmol) of methyl iodide was added to the reaction solution of Reference Example 10 at room temperature and the mixture was stirred for 24 hours at room temperature. After confirming the completion of reaction, the solvent was distilled off under reduced pressure. To thus obtained residue, water was added, pH was adjusted to 2 using concentrated hydrochloric acid and extraction was subjected using n-hexane. The obtained organic layer was dried over anhydrous magnesium sulfate, the insolubles were separated by filtration and then the solvent was distilled

off under reduced pressure. Thus obtained residue was purified by silica gel column chromatography to obtain 0.5g of 5-sec-butyl-4-hydroxy-6-methyl-2-methylthiopyrimidine as a pale yellow powder (yield: 16%).

Melting Point (˚C) : 98 to 100

$^1$H-NMR Data ($CDCl_3$/TMS δ (ppm)): 0.83 (t, 3H), 1.29 (d, 3H), 1.66 (m, 1H), 1.95 (m, 1H), 2.31 (s, 3H), 2.56 (s, 3H), 2.75 (m, 1H), 11.78 (br, 1H)

<Reference Example 12>

Production of 5-sec-butyl-4-chloro-6-methyl-2-methylthiopyrimidine

Phosphorus oxychloride (6.5g, 42.4 mmol) and N,N-dimethylaniline (0.5g, 4.2 mmol) were added to 3.0g (14.1 mmol) of 5-sec-butyl-4-hydroxy-6-methyl-2-methylthiopyrimidine and the mixture was stirred for 2 hours at 100˚C. After confirming the completion of reaction, the reaction solution was poured into water and extracted with dichloromethane. The obtained organic layer was washed with water and then washed with a saturated aqueous sodium bicarbonate solution and dried over anhydrous magnesium sulfate. The insolubles were separated by filtration and then the solvent was distilled off under reduced pressure. Thus obtained residue was purified by silica gel column chromatography to obtain 3.0g of 5-sec-butyl-4-chloro-6-methyl-2-methylthiopyrimidine as a yellow transparent oily substance (yield: 92%).

Refractive Index ($n_D^{20}$): 1.5613

$^1$H-NMR Data ($CDCl_3$/TMS δ (ppm)): 0.85 (t, 3H), 1.33 (d, 3H), 1.75 (m, 1H), 1.87 (m, 1H), 2.53 (s, 3H), 2.54 (s, 3H), 3.19 (br, 1H)

<Reference Example 13>

Production of 5-sec-butyl-4-chlorc-6-methyl-2-methylsulfonylpyrimidine

7.5g of m-chloroperbenzoic acid (purity: 70%, 30.3 mmol) was added to 200 ml of a dichloromethane solution containing 2.8g (12.1 mmol) of 5-sec-butyl-4-chloro-6-methyl-2-methylthiopyrimidine under ice cooling and the mixture was stirred for 30 minutes. Then, the mixture was further stirred for 2 hours at room temperature. After confirming the completion of reaction, the reaction solution was poured into water and extracted with dichloromethane. The obtained organic layer was washed with an aqueous solution of sodium bisulfite, water, an aqueous sodium bicarbonate solution and brine in this order and then dried over anhydrous magnesium sulfate. The insolubles were separated by filtration and then the solvent was distilled off under reduced pressure, to obtain 3.3g of 5-sec-butyl-4-chloro-6-methyl-2-methylsulfonylpyrimidine as a colorless transparent oily substance (yield: quantitative).

Refractive Index ($n_D^{20}$): 1.5368

$^1$H-NMR Data ($CDCl_3$/TMS δ (ppm)): 0.88 (t, 3H), 1.39 (d, 3H), 1.82 (m, 1H), 1.90 (m, 1H), 2.73 (s, 3H), 3.35 (s, 3H), 3.35 (br, 1H)

<Reference Example 14>

Production of 5-sec-butyl-4-chloro-6-methyl-2-(1H-pyrazol-1-yl)pyrimdine

0.9g (12.9 mmol) of pyrazole was added to 20 ml of a tetrahydrofuran solution containing 0.5g of sodium hydride (purity: 60%, 12.9 mmol) at 0˚C and the mixture was stirred for 30 minutes at room temperature. To 20 ml of a tetrahydrofuran solution containing 3.1g (11.7 mmol) of 5-sec-butyl-4-chloro-6-methyl-2-methylsulfonylpyrimidine, a solution prepared in advance was added dropwise at -70˚C and the mixture was stirred for 10 minutes at the same temperature. After confirming the completion of reaction, 100 ml of water was added to the reaction solution and the reaction solution was extracted with diethylether. The obtained organic layer was washed with water and dried over anhydrous magnesium sulfate. The insoluble were separated by filtration and then the solvent was distilled off under reduced pressure, to obtain 3.17g of 5-sec-butyl-4-chloro-6-methyl-2-(1H-pyrazol-1-yl)pyrimidine as a yellow transparent oily substance (yield: quantitative).

Refractive Index ($n_D^{20}$): 1.5611

$^1$H-NMR Data ($CDCl_3$/TMS δ (ppm)): 0.87 (t, 3H), 1.39 (d, 3H), 2.00-1.77 (m, 2H), 2.68 (s, 3H), 3.35 (br, 1H), 6.48 (s, 1H), 7.81 (s, 1H), 8.54 (d, 1H)

<Reference Example 15>

Production of 5-sec-butyl-6-chloro-2-hydrazinyl-N-(2,2,2-trifluoroethyl)pyrimidine-4-amine

0.2g (4.0 mmol) of hydrazine monohydrate was added to 40 ml of an ethanol solution containing 1.3g (3.6 mmol) of 5-sec-butyl-6-chloro-2-methylsulfonyl-N-(2,2,2-trifluoroethyl)pyrimidine-4-amine at room temperature and the mixture was stirred for 4 hours under reflux. After confirming the completion of reaction, the solvent was distilled off under reduced pressure. To the resultant, water was added and the mixture was extracted with chloroform. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 1.1g of 5-sec-butyl-6-chloro-2-hydrazinyl-N-(2,2,2-trifluoroethyl)pyrimdine-4-gamine as a pale yellow oily substance (yield: 98%).

Refractive Index ($n_D^{20}$): 1.5136

$^1$H-NMR Data ($CDCl_3$/TMS δ (ppm)): 0.87 (t, 3H), 1.28 (d, 3H), 1.65-1.70 (m, 2H), 3.27 (br, 1H), 3.86 (br, 1H), 4.18-4.29 (m, 2H), 4.96 (br, 1H), 6.03 (br, 1H)

<Reference Example 16>

Production of 5-sec-butyl-4-chloro-6-iodo-2-methylthiopyrimidine

5.0g (19.9 mmol) of 5-sec-butyl-4,6-dichloro-2-methylthiopyrimidine was added to 30 ml of 55% hydroiodic acid and the mixture was stirred for 2 hours at room temperature. The reaction solution was poured into water, neutralized with a saturated sodium bicarbonate solution and extracted with diethylether. The obtained organic layer was washed with water and then dried over anhydrous magnesium sulfate. The insolubles were separated by filtration and then the solvent was distilled off under reduced pressure, to obtain 6.0g of crude 5-sec-butyl-4-chloro-6-iodo-2-methylthiopyrimidine (yield: 81%).

<Reference Example 17>

Production of 5-sec-butyl-4-chloro-2-methylthio-6-trifluoromethylpyrimidine

9.1g (64.0 mmol) of trifluoromethyltrimethylsilane, 1.5g (25.6 mmol) of potassium fluoride and 4.9g (25.6 mmol) of copper iodide were added to 50 ml of an N-methyl-2-pyrrolidinone solution containing 8.8g (25.6 mmol) of crude 5-sec-butyl-4-chloro-6-iodo--2-methylthiopyrimidine at room temperature and the mixture was stirred for 1 hour at 60°C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure, to obtain 6.0g of crude 5-sec-butyl-4-chloro-2-methylthio-6-trifluoromethylpyrimidine (yield: 83%).

<Reference Example 18>

Production of 5-sec-butyl-4-chloro-2-methylsulfonyl-6-trifluoromethylpyrimidine

5.7g of m-chloroperbenzoic acid (purity: 70%, 23.4 mmol) was added to 30 ml of a dichloromethane solution containing 3.0g (10.6 mmol) of crude 5-sec-butyl-4-chloro-2-methylthio-6-trifluoromethylpyrimidine under ice cooling and the mixture was stirred for 30 minutes. Then, the mixture was further stirred for 1 hour at room temperature. After confirming the completion of reaction, the reaction solution was poured into water and extracted with dichloromethane. The obtained organic layer was washed with an aqueous solution of sodium bisulfite, water, an aqueous sodium bicarbonate solution, water and brine in this order and then dried over anhydrous magnesium sulfate. The insolubles were separated by filtration and then the solvent was distilled off under reduced pressure, to obtain 1.8g of crude 5-sec-butyl-4-chloro-2-methylsulfonyl-6-trifluoromethylpyrimidine (yield: 54%).

<Reference Example 19>

Production of 5-sec-butyl-4-chloro-2-(1H-pyrazol-1-yl)-6-trifluoromethylpyrimidine

0.41g (6.0 mmol) of 1H-pyrazole was added to 20 ml of a tetrahydrofuran solution containing 0.26g of sodium hydride (purity: 60%, 5.7 mmol) at room temperature and the mixture was stirred for 30 minutes. The reaction solution

was cooled to -78˚C, 1.8g (5.7 mmol) of crude 5-sec-butyl-4-chloro-2-methylsulfonyl-6-trifluoromethylpyrimidine was added thereto and the mixture was stirred for 10 minutes. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure, to obtain 0.90g of crude 5-sec-butyl-4-chloro-2-(1H-pyrazol-1-yl)-6-trifluoromethylpyrimidine (yield: 52%).

<Reference Example 20>

Production of 5-sec-butyl-4-chloro-6-ethyl-2-methylthiopyrimidine

0.2g (0.2 mmol) of [1,1-bis(diphenylphosphino)ferrocene]dichloropalladium complexed with dichloromethane was added to 50 ml of a tetrahydrofuran solution containing 3.0g (24.4 mmol) of 5-sec-butyl-4,6-dichloro-2-methylthiopyrimidine at room temperature and thereto 36.6 ml (1.00 mol/l, 36.6 mmol) of a tetrahydrofuran solution of ethyl magnesium bromide was further added dropwise at 45˚C. The mixture was stirred for 2 hours at the same temperature. After confirming the completion of reaction, an aqueous solution of saturated ammonium chloride was added under ice cooling, the reaction mixture was heated to room temperature and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 5.2g of 5-sec-butyl-4-chloro-6-ethyl-2-methylthiopyrimidine (yield: 87%).

$^1$H-NMR Data ($CDCl_3$/TMS δ (ppm)): 0.85 (t, 3H), 1.24-1.37 (m, 6H), 1.76-1.82 (m, 3H), 2.55 (s, 3H), 2.81 (q, 2H)

<Reference Example 21>

Production of 5-sec-butyl-6-ethyl-2-methylthio-N-(2,2,2-trifluoroethyl)pyrimidine-4-amine

2.1g (21.7 mmol) of 2,2,2-trifluoroethylamine and a catalytic amount of sodium p-toluenesulfinate were added to 20 ml of a 1,3-dimethyl-2-imidazolidinone solution containing 1.8g (7.2 mmol) of 5-sec-butyl-4-chloro-6-ethyl-2-methylthiopyrimidine. In a sealed tube, the mixture was stirred for 56 hours at 150˚C. After confirming the completion of reaction, the reaction solution was poured into water and extracted with ethyl acetate. The obtained organic layer was washed with water and then dried over anhydrous magnesium sulfate. The insolables were separated by filtration and then the solvent was distilled off under reduced pressure. Thus obtained residue was purified by silica gel column chromatography to obtain 1.7g of 5-sec-butyl-6-ethyl-2-methylthio-N-(2,2,2-trifluoroethyl)pyrimidine-4-amine (yield: 75%).

$^1$H-NMR Data ($CDCl_3$/TMS δ (ppm)): 0.88 (t, 3H), 1.19-1.31 (m, 6H), 1.59-1.75 (m, 2H), 2.61-2.74 (m, 1H), 3.01 (br, 1H), 4.23-4.34 (m, 2H), 4.80 (br, 1H)

<Reference Example 22>

Production of 5-sec-butyl-6-ethyl-2-methylsulfonyl-N-(2,2,2-trifluoroethyl)pyrimidine-4-amine

3.3g of m-chloroperbenzoic acid (purity: 70%, 13.5 mmol) was added to 100 ml of a dichloromethane solution containing 1.7g (5.4 mmol) of 5-sec-butyl-6-ethyl-2-methylthio-N-(2,2,2-trifluoroethyl)pyrimidine-4-amine under ice cooling and the mixture was stirred for 30 minutes. Then, the mixture was further stirred at room temperature for 24 hours. After confirming the completion of reaction, the reaction solution was poured into water and extracted with dichloromethane. The obtained organic layer was washed with an aqueous solution of sodium bisulfite, water, an aqueous sodium bicarbonate solution and brine in this order and the dried over anhydrous magnesium sulfate. The insolubles were separated by filtration and then the solvent was distilled off under reduced pressure. Thus obtained residue was purified by silica gel column chromatography to obtain 1.12g of 5-sec-butyl-6-ethyl-2-methylsulfonyl-N-(2,2,2-trifluoroethyl)pyrimidine-4-amine (yield: 61%).

$^1$H-NMR Data ($CDCl_3$/TMS δ (ppm)): 0.91 (t, 3H), 1.24-1.36 (m, 6H), 1.65-1.79 (m, 2H), 2.78-2.86 (m, 2H), 3.02 (br, 1H), 3.29 (s, 3H), 4.27-4.38 (m, 2H), 5.18 (br, 1H)

<Reference Example 23>

Production of 5-sec-butyl-4,6-dibromo-2-methylthiopyrimidine

13.4g (46.7 mmol) of phosphorus oxybromide was added to 40 ml of a chlorobenzene solution containing 5.0g

(23.3 mmol) of 5-sec-butyl-2-methylthiopyrimidine-4,6-diol and the mixture was stirred for 3 hours under reflux. After confirming the completion of reaction, the reaction solution was poured into water and extracted with dichloromethane. The obtained organic layer was washed with water and then washed with a saturated aqueous sodium bicarbonate solution and then dried over anhydrous magnesium sulfate. The insolubles were separated by filtration and then the solvent was distilled off under reduced pressure. Thus obtained residue was purified by silica gel column chromatography to obtain 2.1g of 5-sec-butyl-4,6-dibromo-2-methylthiopyrimidine (yield: 26%).

$^1$H-NMR Data ($CDCl_3$/TMS δ (ppm)): 0.86 (t, 3H), 1.37 (d, 3H), 1.70-1.81 (m, 1H), 2.00-2.10 (m, 1H), 2.55 (s, 3H), 3.46-3.51 (m, 1H)

<Reference Example 24>

Production of 6-bromo-5-sec-butyl-2-methylthio-N-(2,2,2-trifuoroethyl)pyrimidine-4-amine

1.8g (18.5 mmol) of 2,2,2-trifluoroethylamine was added to 10 ml of a 1,3-dimethyl-2-imidazolidinone solution containing 2.1g (6.2 mmol) of 5-sec-butyl-4,6-dibromo-2-methylthiopyrimidine. In a sealed tube, the mixture was stirred for 10 hours at 120°C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 2.2g of 6-bromo-5-sec-butyl-2-methylthio-N-(2,2,2-trifluoroethyl)pyrimidine-4-amine (yield: 99%).

$^1$H-NMR Data ($CDCl_3$/TMS δ (ppm)): 0.91 (t, 3H), 1.28 (d, 3H), 1.66-1.71 (m, 2H), 2.49 (s, 3H), 3.40 (br, 1H), 4.21-4.32 (m, 2H), 4.99 (br, 1H)

<Reference Example 25>

Production of 6-bromo-5-sec-butyl-2-methylsulfonyl-N-(2,2,2-trifluoroethyl)pyrimidine-4-amine

3.8g of m-chloroperbenzoic acid (purity: 70%, 15.2 mmol) was added to 60 ml of a dichloromethane solution containing 2.2g (6.1 mmol) of 6-bromo-5-sec-butyl-2-methylthio-N-(2,2,2-trifluoroethyl)pyrimidine-4-amine under ice cooling and the mixture was stirred for 30 minutes. Then the mixture was further stirred at room temperature for 2 hours. After confirming the completion of reaction, the reaction solution was poured into water and extracted with dichloromethane. The obtained organic layer was washed with an aqueous solution of sodium bisulfite, water, an aqueous sodium bicarbonate solution and brine in this order and then dried over anhydrous magnesium sulfate. The insolubles were separated by filtration and then the solvent was distilled off under reduced pressure, to obtain 2.32g of 6-bromo-5-sec-butyl-2-methylsulfonyl-N-(2,2,2-trifluorcethyl)pyrimidine-4-aimine (yield: 98%).

$^1$H-NMR Data ($CDCl_3$/TMS δ (ppm)): 0.94 (t, 3H), 1.33 (d, 3H), 1.73-1.78 (m, 2H), 3.29 (s, 3H), 3.52 (br, 1H), 4.28-4.39 (m, 2H), 5.39 (br, 1H)

<Reference Example 26>

Production of 5-sec-butyl-$N^4$,$N^4$-diethyl-2-methylthio-$N^6$-(2,2,2-trifluoroethyl)pyrimidine-4,6-diamine

1.1g (15.0 mmol) of diethylamine and a catalytic amount of sodium p-toluenesulfinate were added to 10 ml of a 1,3-dimethyl-2-imidazolidinone solution containing 1.0g (3.0 mmol) of 5-sec-butyl-6-chloro-2-methylthio-N-(2,2,2-trifluoroethyl)pyrimidine-4-amine. In a sealed tube, the mixture was stirred for 23 hours at 150°C. After confirming the completion of reaction, the reaction solution was poured into water and extracted with ethyl acetate. The obtained organic layer was washed with water and then dried over anhydrous magnesium sulfate. The insolubles were separated by filtration and then the solvent was distilled off under reduced pressure. Thus obtained residue was purified by silica gel column chromatography to obtain 1.1g of 5-sec-butyl-$N^4$,$N^4$-diethyl-2-methylthio-$N^6$-(2,2,2-trifluoroethyl)pyrimidine-4,6-diamine (yield: quantitative).

<Reference Example 27>

Production of 5-sec-butyl-$N^4$,$N^4$-diethyl-2-methylsulfonyl-$N^6$-(2,2,2-trifluoroethyl)pyrimidine-4,6-diamine

1.9g of m-chloroperbenzoic acid (purity: 70%, 7.5 mmol) was added, under ice cooling, to 50 ml of a chloroform solution containing 1.1g(3.0 mmol) of 5-sec-butyl-$N^4$,$N^4$-diethyl-2-methylthio-$N^6$-(2,2,2-trifluoroethyl)pyrimidine-4,6-diamine obtained in Reference Example 26 and the mixture was stirred for 30 minutes. Then, the mixture was further

stirred overnight at room temperature. After confirming the completion of reaction, the reaction solution was poured into water and extracted with chloroform. The organic layer was washed with an aqueous solution of sodium bisulfite, water, an aqueous sodium bicarbonate solution and brine in this order and then dried over anhydrous magnesium sulfate. The insolubles were separated by filtration and then the solvent was distilled off under reduced pressure. Thus obtained residue was purified by silica gel column chromatography to obtain 0.6g of 5-sec-butyl-$N^4$,$N^4$-diethyl-2-methylsulfonyl-$N^6$-(2,2,2-trifluoroethyl)pyrimidine-4,6-diamine (yield: 52%).

$^1$H-NMR Data ($CDCl_3$/TMS $\delta$ (ppm)): 0.83 (t, 3H), 1.15 (t, 6H), 1.30 (d,2H), 1.66 (m, 2H), 3.02 (q, 1H), 3.17 (m, 2H), 3.24 (s, 3H), 3.34 (m, 2H), 4.30 (m, 2H), 4.91 (br, 1H)

Next, a formulation preparing method will be described in detail with reference to representative Formulation Examples. The type and blending ratio of the compound and additive are not limited thereto and they can be changed in a wide range. In the description below, 'parts' means 'parts by weight'.

[Formulation Example 1] Wettable Powder

0.5 parts of polyoxyethyleneoctylphenyl ether, 0.5 parts of a sodium salt of β-naphthalene sulfonic acid formalin condensate, 20 parts of diatomite and 69 parts of clay were mixed with 10 parts of Compound No. 0259 and the mixture was crushed to obtain wettable powder.

[Formulation Example 2] Wettable Powder

50 parts of the compound of Compound No. 0350, 45 parts of diatomite, 2 parts of sodium dinaphthylmethanedisulfonate and 3 parts of sodium lignin sulfonate were homogeneously mixed and crushed to obtain wettable powder.

[Formulation Example 3] Flowable

0.2 parts of xanthan gum was dissolved in 75.8 parts of water. Thereto, in addition to 13 parts of the compound of Compound No. 0147, 4 parts of polyoxyethylene styrenated phenyl ether sulfate and 7 parts of ethylene glycol, silicone AF-128N (produced by Asahi Chemical Industry Co., Ltd.) was also added by 130 ppm with respect to the total amount and they were mixed for 30 minutes with a high-speed stirrer. Thereafter, the mixture was crushed using a wet pulverizer to obtain a flowable.

[Formulation Example 4] Emulsion

60 parts of a mixture of equal parts of xylene and isophorone and 10 parts of a surfactant obtained as a mixture of polyoxyethylene sorbitan alkylate, a polyoxyethylene alkylaryl polymer and alkylaryl sulfonate, were added to 30 parts of the compound of Compound No. 0919 and these were well stirred to obtain an emulsion.

[Formulation Example 5] Granule

To 5 parts of the compound of Compound No. 1215, 85 parts of a filler obtained by mixing talc and bentonite in a 1:3 ratio, 5 parts of white carbon and 5 parts of a surfactant obtained as a mixture of polyoxyethylene sorbitan alkylate, a polyoxyethylene alkylaryl polymer and alkylaryl sulfonate, there added 10 parts of water and the mixture was well kneaded to give a paste form. This paste product was pushed out through a sieve having a diameter of 0.7 mm, it was then dried and cut in a length of 0.5 to 1 mm, to obtain a Granule.

With compounds shown in Tables 1 to 40, various formulations can be produced in the same manner according to Formulation Examples 1 to 5.

Next, effects exhibited by the compound of the invention will be described with reference to Test Examples.

[Test Example 1] Test on protective effect against Pyricularia oryzae

18 rice seeds (variety: Aichi Asahi) were sown in each clay pot having a diameter of 7.5 cm and allowed to grow for 2 to 4 weeks in a greenhouse. The wettable powder prepared according to Formulation Example 1 was diluted in water in the manner for an active ingredient concentration to be 500 ppm, a spreader (Kumiten) was added for the dilution factor to be 3,000 times and the resultant agent was sprayed to rice plants at a four-leaf stage in the amount of 20 ml per 1 pot. After air drying, the rice plants were inoculated by spraying a conidia suspension of Pyricularia oryzae and incubated in a moist chamber at 25˚C (relative humidity of 100%) until a development of disease. 5 days after the innoculation, the number of lesion on a leaf that had been on the top at the time of spraying the agent was counted and a control level (%) was calculated using the expression shown below.

According to this test, compounds providing a 100% control level were Compound Nos.:

0009, 0010, 0018, 0022, 0025, 0026, 0029, 0035, 0049, 0133, 0135, 0147, 0159, 0162, 0163, 0166, 0175, 0178, 0179, 0186, 0194, 0195, 0198, 0199, 0200, 0222, 0240, 0242, 0246, 0249, 0250, 0254, 0259, 0301, 0305, 0309, 0312, 0313, 0317, 0328, 0329, 0336, 0337, 0341, 0349, 0350, 0351, 0354, 0358, 0361, 0370, 0372, 0384, 0385, 0387, 0389, 0391, 0392, 0434, 0435, 0436, 0710, 0732, 0760, 0764, 0768, 0776, 0780, 0792, 0800, 0828, 0844, 0860, 0864, 0868, 0907, 0916, 0919, 0921, 0943, 0962, 0963, 0966, 0967, 0971, 0975, 0979, 0986, 0987, 0990, 0991, 0994, 0995, 0998, 1000, 1008, 1031, 1058, 1060, 1061, 1062, 1065, 1068, 1071, 1082, 1085, 1086, 1087, 1090, 1093, 1094, 1106, 1110, 1115, 1156, 1160, 1179, 1187, 1191, 1196, 1239, 1283, 1286, 1287, 1291, 1311, 1315, 1318, 1319, 1323, 1328, 1342, 1426, 1428, 1442, 1452, 1455, 1456, 1459, 1460, 1497, 1511, 1554, 1576, 1595, 1597, 1617, 1624, 1625, 1628, 1633, 1640, 1641, 1657, 1898 and the like.
Herein, Comparative Compound 1 did not exhibit a control effect.

Hereinbelow, an expression to calculate a control level (%) is shown.

[Expression 1]

$$\text{Control Level } (\%) = \left(1 - \frac{\text{average lesion number on treated area}}{\text{average lesion number on untreated area}}\right) \times 100$$

Comparative Compound 1 mentioned above is Compound No. 1-1693 disclosed in JP-A No. 2005-232081. A structure thereof is shown below.

[Chemical Formula 32]

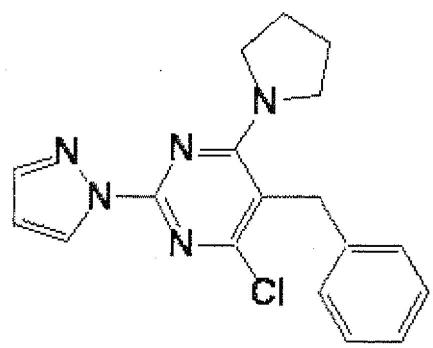

Compound No. 1-1693 in JP-A No. 2005-232081

<Test Example 2> Test on protective effect against Rhizoctonia solani

15 rice seeds (variety: Kinmaze) were sown in each clay pot having a diameter of 7.5 cm and allowed to grow for 3 weeks in a greenhouse. The wettable powder prepared according to Formulation Example 1 was diluted in water in the manner for an active ingredient concentration to be 500 ppm, a spreader (Kumiten) was added for the dilution factor to be 3,000 times and the resultant agent was sprayed to rice at a two and a half to three-leaf stage in the amount of 20 ml per 1 pot. After air drying, the plants were inoculated by uniformly covering the surface of the soil with Rhizoctonia solani cultured in a rice-husk bran medium and incubated in a moist chamber at 30˚C (relative humidity of 100%) until a development of disease. 5 days after the innoculation, disease development indexes of total pots were examined according to the standard described below and a control level (%) was calculated using the expression shown below.

According to this test, compounds providing a 100% control level were Compound Nos.:

0006, 0010, 0018, 0035, 0049, 0135, 0159, 0163, 0186, 0190, 0191, 0194, 0195, 0199, 0200, 0222, 0232, 0241, 0242, 0246, 0249, 0250, 0254, 0259, 0301, 0305, 0309, 0312, 0313, 0328, 0329, 0333, 0336, 0337, 0341, 0348, 0349, 0350, 0351, 0354, 0361, 0367, 0368, 0370, 0372, 0373, 0377, 0382, 0384, 0387, 0389, 0391, 0393, 0395, 0401, 0434, 0435, 0436, 0437, 0536, 0708, 0710, 0732, 0760, 0764, 0768, 0772, 0775, 0776, 0780, 0788, 0792, 0796, 0800, 0812, 0824, 0828, 0840, 0844, 0860, 0864, 0868, 0904, 0908, 0933, 0915, 0916, 0920, 0921, 0943, 0953, 0962, 0963, 0967, 0971, 0975, 0979, 0998, 1000, 1008, 1031, 1058, 1060, 1062, 1065, 1068, 1071, 1082, 1085, 1087, 1090, 1094, 1098, 1102, 1106, 1110, 1115, 1156, 1167, 1179, 1187, 1190, 1191, 1192, 1204, 1215, 1255, 1263, 1283, 1290, 1315, 1318, 1319, 1328, 1342, 1426, 1428, 1442, 1452, 1497, 1511, 1533, 1554, 1576, 1595, 1597, 1617, 1625, 1633, 1637, 1640, 1641, 1833, 1849, 1898, 1906, 1914, 1994, 2011 and the like. Herein, Comparative Compound 1 did not exhibit a control effect and Comparative Compound 2 showed a control level of 50%.

The standard for disease development index is as follows:

[Table 51]

| Disease Development index | |
|---|---|
| 0: | Disease Development is not recognized |
| 1: | Infected height is less than 25% of that in untreated area |
| 2: | Infected height is 25% or more to less than 50% of that in untreated area |
| 3: | Infected height is 50% or more to less than 75% of that in untreated area |
| 4: | Infected height is 75% or more of that in untreated area |

Hereinbelow, an expression to calculate a control level (%) is shown.

[Expression 2]

$$\text{Control Level}\ (\%) = \left(1 - \frac{\text{average disease development index of treated area}}{\text{average disease development index of untreated area}}\right) \times 100$$

Herein, Comparative Compounds 1 and 2 mentioned above are Compound No. 1-1693 disclosed in JP-A No. 2005-232081 and Compound No. 182 disclosed in JP-A No. S54-115384, respectively. A structure of Comparative Compound 1 is shown as above and a structure of Comparative Compound 2 is shown below.

[Chemical Formula 33]

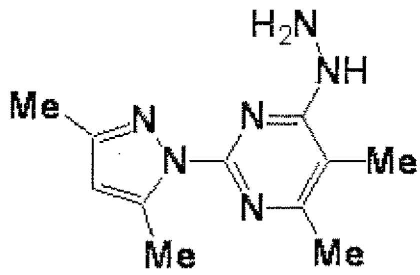

Compound No. 182 in JP-A No. S54-115384

<Test Example 3> Test on protective effect against Pseudoperonospora cubensis

4 cucumber seeds (variety: Sagami-Hanziro) were sown in each plastic cup having a diameter of 5.5 cm and allowed to grow for 7 days in a greenhouse. The wettable powder prepared according to Formulation Example 1 was diluted in water in the manner for an active ingredient concentration to be 500 ppm, a spreader (Kumiten) was added for the dilution factor to be 3,000 times and the resultant agent was sprayed to cucumber seedling the seed leaf of which is opened in the amount of 20 ml per 1 cup. After air drying, the plants were inoculated by spraying with a conidia suspension of Pseudoperonospora cubensis. The inoculated plants were immediately put in a moist chamber at 20°C (relative humidity of 100%) for 24 hours. Thereafter, the plants were transferred to a greenhouse. 6 days after, disease development indexes of cotyledons for total pots were examined according to the standard described below, a disease severity was determined using an expression shown below and a control level (%) was calculated using another expression shown below.

According to this test, compounds providing a 100% control level were Compound Nos.:

0002, 0006, 0009, 0010, 0013, 0014, 0018, 0022, 0025, 0026, 0029, 0035, 0049, 0133, 0135, 0147, 0155, 0159, 0162, 0163, 0166, 0178, 0179, 0182, 0186, 0187, 0190, 0191, 0194, 0195, 0200, 0222, 0232, 0241, 0242, 0246, 0249, 0250, 0259, 0300, 0301, 0304, 0305, 0308, 0309, 0313, 0316, 0325, 0328, 0329, 0336, 0341, 0348, 0349, 0350, 0351, 0358, 0361, 0368, 0370, 0371, 0372, 0384, 0385, 0387, 0389, 0393, 0394, 0401, 0435, 0436, 0437, 0708, 0710, 0721, 0732, 0760, 0764, 0768, 0772, 0775, 0776, 0791, 0799, 0812, 0824, 0827, 0828, 0860, 0864, 0907, 0908, 0915, 0921, 0943, 0953, 0962, 0963, 0966, 0967, 0970, 0971, 0974, 0975, 0986, 0987, 0990, 0994, 1000, 1008, 1058, 1071, 1075, 1081, 1082, 1085, 1086, 1087, 1090, 1094, 1097, 1098, 1101, 1110, 1115, 1178, 1182, 1183, 1186, 1187, 1190, 1197, 1215, 1225, 1263, 1279, 1286, 1287, 1290, 1291, 1318, 1342, 1426, 1440, 1452, 1455, 1456, 1459, 1511, 1533, 1554, 1576, 1595, 1624, 1628, 1637, 1641, 1657, 1829, 1906 and the like. Comparative Compound 1 did not exhibit a control effect.

The standard for disease development index is as follows:

[Table 52]

| Disease Development Index | |
|---|---|
| 0: | Disease Development is not recognized |
| 1: | disease development area of less than 25% |
| 2: | disease development area of 25% or more to less than 50% |
| 3: | disease development area of 50% or more to less than 75% |
| 4: | disease development area of 75% or more |

Hereinbelow, an expression to calculate a disease severity (%) is shown.

[Expression 3]

$$\text{disease severity} = \left( \frac{n0 \times 0 + n1 \times 1 + n2 \times 2 + n3 \times 3 + n4 \times 4}{4 \times N} \right) \times 100$$

provided that,

N: total number of examined leaves
n0: number of leaves of disease development index 0
n1: number of leaves of disease development index 1
n2: number of leaves of disease development index 2
n3: number of leaves of disease development index 3
n4: number of leaves of disease development index 4

Hereinbelow, an expression to calculate a control level (%) is shown.

[Expression 4]

$$\text{control level } (\%) = \left(1 - \frac{\text{disease severity of treated area}}{\text{disease severity of untreated area}}\right) \times 100$$

Comparative Compound 1 mentioned above is Compound No. 1-1693 disclosed in JP-A No. 2005-232081 mentioned before.

<Test Example 4> Test on protective effect against Botrytis cinerea

4 cucumber seeds (variety: Sagami-Hanziro) were sown in each plastic cup having a diameter of 5.5 cm and allowed to grow for 7 days in a greenhouse. The wettable powder prepared according to Formulation Example 1 was diluted in water in the manner for an active ingredient concentration to be 500 ppm, a spreader (Kumiten) was added for the dilution factor to be 3,000 times and the resultant agent was sprayed to cucumber seedling the seed leaf of which is opened in the amount of 20 ml per 1 cup. After air drying, a sterilized paper disc was immersed in a conidia suspension of Botrytis cinerea and laid on an upper side of a cucumber cotyledon for innoculation and thereafter cared in a moist chamber at 20˚C (relative humidity of 100%) until a development of disease. 2 days after, disease development indexes of total pots were examined according to the standard in Test Example 3, a disease severity was determined using the expression in Test Example 3 and a control level (%) was calculated using the expression in Test Example 3.
According to this test, compounds providing a 100% control level were Compound Nos.:

0049, 0195, 0200, 0222, 0242, 0250, 0254, 0259, 0309, 0313, 0349, 0350, 0351, 0372, 0401, 0434, 0435, 0436, 0437, 0710, 0732, 0776, 0860, 0864, 0943, 1000, 1008, 1086, 1087, 1090, 1097, 1115, 1328, 1342, 1554, 1576, 1845 and the like. Comparative Compound 1 did not exhibit a control effect and Comparative Compound 2 showed a control level of 25%.

Herein, Comparative Compounds 1 and 2 mentioned above are Compound No. 1-1693 disclosed in JP-A No. 2005-232081 and Compound No. 182 disclosed in JP-A No. S54-115384, respectively.

<Test Example 5> Test on protective effect against Erysiphe graminis

10 wheat seeds (variety: Norin No. 61) were sown in each plastic cup having a diameter of 5.5 cm and allowed to grow for 8 days in a greenhouse. The wettable powder prepared according to Formulation Example 1 was diluted in water in the manner for an active ingredient concentration to be 500 ppm, a spreader (Kumiten) was added for the dilution factor to be 3,000 times and the resultant agent was sprayed to wheat at a one and a half to two-leaf stage in the amount of 20 ml per 1 cup. After air drying, the plants were inoculated by equally sprinkling with conidia of Erysiphe graminis by using a midget duster or the like for innoculation and then incubated in a greenhouse until a development of disease. 7 days after, disease development indexes of first leaves for total pots were examined according to the standard in Test Example 3, a disease severity was determined using the expression in Test Example 3 and a control level (%) was calculated using the expression in Test Example 3.
According to this test, compounds providing a 100% control level were Compound Nos.:

0010, 0018, 0022, 0025, 0026, 0029, 0035, 0133, 0135, 0149, 0162, 0163, 0166, 0167, 0178, 0179, 0183, 0186, 0187, 0194, 0199, 0200, 0211, 0222, 0232, 0240, 0249, 0259, 0305, 0309, 0312, 0313, 0317, 0333, 0341, 0349, 0350, 0351, 0354, 0355, 0358, 0361, 0370, 0371, 0373, 0384, 0385, 0386, 0387, 0389, 0391, 0392, 0401, 0434, 0437, 0708, 0710, 0721, 0732, 0760, 0764, 0768, 0792, 0796, 0800, 0844, 0864, 0900, 0901, 0902, 0906, 0907, 0915, 0916, 0919, 0920, 0921, 0934, 0943, 0953, 0961, 0962, 0963, 0966, 0967, 0970, 0971, 0975, 0979, 0986, 0987, 0990, 0991, 0994, 0995, 0998, 1000, 1008, 1030, 1058, 1060, 1061, 1062, 1064, 1065, 1087, 1089, 1093, 1094, 1097, 1098, 1101, 1102, 1115, 1156, 1160, 1178, 1179, 1182, 1183, 1186, 1187, 1190, 1191, 1192, 1196, 1197, 1204, 1215, 1225, 1233, 1239, 1243, 1254, 1255, 1259, 1286, 1287, 1290, 1291, 1311, 1318, 1322, 1328, 1342, 1426, 1428, 1440, 1442, 1448, 1452, 1455, 1456, 1497, 1511, 1554, 1576, 1595, 1597, 1624, 1625, 1633, 1640, 1641, 1645, 1657, 1661, 1898, 1906, 1914 and the like.

<Test Example 6> Test on protective effect against Septoria nodorum

10 wheat seeds (variety: Norin No. 61) were sown in each plastic cup having a diameter of 5.5 cm and allowed to grow for 9 days in a greenhouse. The wettable powder prepared according to Formulation Example 1 was diluted in water in the manner for an active ingredient concentration to be 500 ppm, a spreader (Kumiten) was added for the dilution factor to be 3,000 times and the resultant agent was sprayed to wheat at a two-leaf stage in the amount of 20 ml per 1 cup. After air drying, the plants were inoculated by spraying with a pycnidiospore suspension of Septoria nodorum. The inoculated plants were immediately put in a moist chamber at 25˚C (relative humidity of 100%) for 48 hours. Thereafter, the plants were transferred to a greenhouse. 9 days after, disease development indexes of first leaves for total pots were examined according to the standard in Test Example 3, a disease severity was determined using the expression in Test Example 3 and a control level (%) was calculated using the expression in Test Example 3.
According to this test, compounds providing a 100% control level were Compound Nos.:

0002, 0006, 0009, 0010, 0014, 0018, 0025, 0029, 0049, 0133, 0147, 0162, 0163, 0166, 0167, 0178, 0179, 0182, 0186, 0187, 0191, 0194, 0198, 0232, 0241, 0242, 0245, 0249, 0253, 0259, 0300, 0312, 0313, 0316, 0328, 0329, 0332, 0341, 0349, 0350, 0351, 0367, 0384, 0385, 0401, 0710, 0732, 0764, 0772, 0775, 0776, 0791, 0799, 0812, 0860, 0868, 0907, 0908, 0915, 0916, 0919, 0921, 0953, 0962, 0963, 0966, 0967, 0970, 0979, 0990, 0994, 1000, 1008, 1062, 1065, 1087, 1085, 1087, 1093, 1115, 1178, 1182, 1183, 1186, 1187, 1190, 1225, 1263, 1286, 1290, 1318, 1322, 1342, 1426, 1452, 1459, 1511, 1533, 1595, 1640, 1641, 1644, 1898, 1906, 1918, 1794 and the like. Comparative Compound 1 did not exhibit a control effect and Comparative Compound 2 showed a control level of 75%.

Herein, Comparative Compounds 1 and 2 mentioned above are Compound No. 1-1693 disclosed in JP-A No. 2005-232081 and Compound No. 182 disclosed in JP-A No. S54-115384, respectively.

## Claims

**1.** A plant disease control agent for agricultural or horticultural use, which is **characterized by** containing as an active ingredient one or more compounds selected from aminopyrimidine derivatives represented by General Formula [I]:

[Chemical Formula 1]

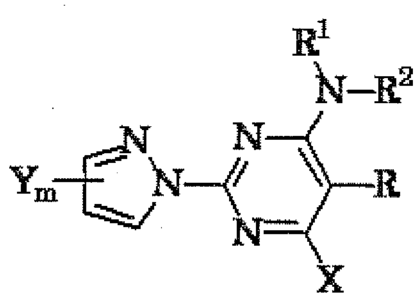

[I]

[wherein

R is a $C_{1-10}$ alkyl group, a $C_{3-8}$ cycloalkyl group, a $C_{3-8}$ cycloalkyl $C_{1-3}$ alkyl group, a $C_{1-6}$ haloalkyl group, a $C_{2-6}$ alkenyl group, a $C_{2-6}$ alkynyl group, a $C_{3-8}$ cycloalkenyl group, a $c_{1-6}$ acyl group, a $C_{1-6}$ hydroxylalkyl group, a $C_{1-6}$ alkoxy $c_{1-6}$ alkyl group, a 1,3-dioxolan-2-yl group or a 1,3-dioxan-2-yl group;
$R^1$ and $R^2$ are each independently a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono ($C_{1-6}$ alkyl) aminocarbonyl group, a di ($C_{1-6}$ alkyl) aminocarbonyl group, a $C_{1-6}$ alkylsulfonyl group, a $C_{3-6}$ haloalkylsulfonyl group, a sulfamoyl group, a mono($C_{1-6}$ alkyl) aminosulfonyl group, or a di($C_{1-6}$ alkyl) aminosulfonyl group,
while $R^1$ and $R^2$ may form a 5-membered or 6-membered ring (the 5-membered or 6-membered ring may be substituted with one or more substituents selected from Substituent Group α) with an atom arbitrarily selected from the group consisting of a carbon atom, an oxygen atom, a nitrogen atom and a sulfur atom, together with the nitrogen atom to which $R^1$ and $R^2$ are bonded;
X is a hydrogen atom or a substituent selected from Substituent Group α;
Y is a substituent selected from Substituent Group α; and
m is an integer from 0 to 3,
while Substituent Group α being defined as follows:

"Substituent Group α":

a halogen atom, a $C_{1-10}$ alkyl group, a $C_{3-8}$ cycloalkyl group, a $C_{3-8}$ cycloalkyl $C_{1-3}$ alkyl group, a $C_{2-6}$ alkynyl group, a $C_{2-6}$ alkenyl group, a $C_{1-10}$ alkoxy group, a $C_{3-6}$ alkoxy $C_{1-3}$ alkyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkynyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, an amino group, a nitro group, a hydroxyl group, a $C_{1-6}$ hydroxylalkyl group, a mono($C_{1-6}$ alkyl) amino group, a di($C_{1-6}$ alkyl)amino group, a $C_{1-6}$ acyl group, a carboxyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a thiol group, a thiocyanate group, a tri($C_{1-6}$ alkyl)silyl group, an optionally substituted benzyloxy group, a hydroxyiminomethyl group, a $C_{1-6}$ alkoxyiminomethyl group and an optionally substituted phenyl group]

and agriculturally acceptable salts thereof.

**2.** An aminopyrimidine derivative represented by General Formula [I]:

[Chemical Formula 2]

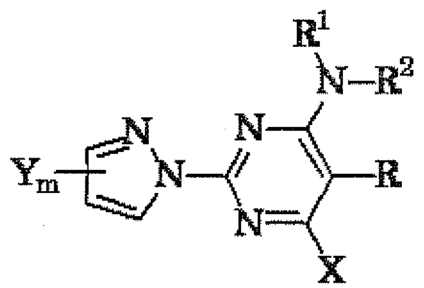

[I]

[wherein
R is a $C_{2-10}$ alkyl group, a $C_{3-8}$ cycloalkyl group, a $C_{3-8}$ cycloalkyl $C_{1-3}$ alkyl group, a $C_{1-6}$ haloalkyl group, a $C_{2-6}$ alkenyl group, a $C_{2-6}$ alkynyl group, a $C_{3-8}$ cycloalkenyl group, a $C_{1-6}$ acyl group, a $C_{1-6}$ hydroxylalkyl group, a $C_{1-6}$ alkoxy $C_{1-6}$ alkyl group, a 1,3-dioxolan-2-yl group or a 1,3-dioxan-2-yl group;
$R^1$ and $R^2$ are each independently a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a car-

bamoyl group, a mono($C_{1-6}$ alkyl)aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group, or a di($C_{1-6}$ alkyl) aminosulfonyl group,
while $R^1$ and $R^2$ may form a 5-membered or 6-membered ring (the 5-membered or 6-membered ring may be substituted with one or more substituents selected from Substituent Group α) with an atom arbitrarily selected from the group consisting of a carbon atom, an oxygen atom, a nitrogen atom and a sulfur atom, together with the nitrogen atom to which $R^1$ and $R^2$ are bonded;
X is a hydrogen atom or a Substituent selected from Substituent Group α;
Y is a substituent selected from Substituent Group α; and
m is an integer from 0 to 3,
while Substituent Group α being defined as follows:

"Substituent Group α":

a halogen atom, a $C_{1-10}$ alkyl group, a $C_{3-8}$ cycloalkyl group, a $C_{3-8}$ cycloalkyl $C_{1-3}$ alkyl group, a $C_{2-6}$ alkynyl group, a $C_{2-6}$ alkenyl group, a $C_{1-10}$ alkoxy group, a $C_{1-6}$ alkoxy $C_{1-3}$ alkyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkynyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, an amino group, a nitro group, a hydroxyl group, a $C_{1-6}$ hydroxylalkyl group, a mono($C_{1-6}$ alkyl)amino group, a di($C_{1-6}$ alkyl)amino group, a $C_{1-6}$ acyl group, a carboxyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a thiol group, a thiocyanate group, a tri($C_{1-6}$ alkyl)silyl group, an optionally substituted benzyloxy group, a hydroxyiminomethyl group, a $C_{1-6}$ alkoxyiminomethyl group and an optionally substituted phenyl group]

or an agriculturally acceptable salt thereof.

**3.** The aminopyrimidine derivative or an agriculturally acceptable salt thereof according to Claim 2, wherein, in General Formula [I],
$R^1$ is a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl) aminocarbonyl group, a $C_{1-6}$ alkylsulfonyl group, a $C_{1-6}$ haloalkylsulfonyl group, a sulfamoyl group, a mono($C_{1-6}$ alkyly)aminosulfonyl group or a di($C_{1-6}$ alkyl) aminosulfonyl group and
$R^2$ is a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl) aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring may be substituted with one or more substituents selected from Substituent Group α) with an atom arbitrarily selected from the group consisting of a carbon atom, an oxygen atom, a nitrogen atom and a sulfur atom, together with the nitrogen atom to which $R^1$ and $R^2$ are bonded.

**4.** The aminopyrimidine derivative or an agriculturally acceptable salt thereof according to Claim 2, wherein, in General Formula [I],
$R^1$ is a $C_{1-10}$ alkyl group which may be substituted with one or more substituents selected from Substituent Group β, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group and
$R^2$ is a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl) aminocarbonyl group,

a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring is independently substituted with 1 to 4 halogen atoms or/and a $C_{1-6}$ haloalkyl group) with an atom arbitrarily selected from the group consisting of a carbon atom, an oxygen atom, a nitrogen atom and a sulfur atom, together with the nitrogen atom to which $R^1$ and $R^2$ are bonded,
where Substituent Group β being defined as follows:

"Substituent Group β":

a halogen atom, a $C_{2-6}$ alkynyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkenyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, a nitro group, a $C_{1-6}$ acyl group, a carboxyl group, a $C_{1-6}$ alkoxycarbonyl group, a carbamoyl group, a mono($C_{1-6}$ alkyl) aminocarbonyl group, a di($C_{1-6}$ alkyl)aminocarbonyl group and a tri($C_{1-6}$ alkyl)silyl group.

**5.** The aminopyrimidine derivative or an agriculturally acceptable salt thereof according to Claim 2,
wherein, in General Formula [I],
$R^1$ is a hydrogen atom, a $C_{1-10}$ alkyl group which may be substituted with one or more substituents selected from Substituent Group α, a $C_{1-10}$ alkoxy group which may be substituted with one or more substituents selected from Substituent Group α, a $C_{3-8}$ cycloalkyl group, a $C_{2-6}$ alkynyl group, a $C_{2-6}$ alkenyl group, a $C_{1-6}$ acyl group, a hydroxyl group, a $C_{1-6}$ haloalkylcarbonyl group, a $C_{2-6}$ alkoxycarbonyl group, a carbamoyl group, a mono($C_{1-6}$ alkyl)aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group and
$R^2$ is a hydrogen atom, a $C_{1-10}$ alkyl group which may be substituted with one or more substituents selected from Substituent Group β, a $C_{1-10}$ alkoxy group which may be substituted with one or more substituents selected from Substituent Group α, a $C_{2-6}$ alkynyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl) aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring is independently substituted with 1 to 4 halogen atoms or/and a $C_{1-6}$ haloalkyl group) with an atom arbitrarily selected from the group consisting of a carbon atom, an oxygen atom, a nitrogen atom and a sulfur atom, together with the nitrogen atom to which $R^1$ and $R^2$ are bonded,
where Substituent Group P being defined as follows:

"Substituent Group β:

a halogen atom, a $C_{2-6}$ alkynyl group, a $C_{3-8}$ cycloalkyloxy group, a $C_{3-8}$ cycloalkyl $C_{1-3}$ alkyloxy group, a $C_{1-6}$ haloalkoxy group, a $C_{2-6}$ alkynyloxy group, a $C_{2-6}$ alkenyloxy group, a $C_{1-6}$ haloalkyl group, a $C_{1-6}$ alkylthio group, a $C_{1-6}$ alkylsulfinyl group, a $C_{1-6}$ alkylsulfonyl group, a $C_{1-6}$ haloalkylthio group, a $C_{1-6}$ haloalkylsulfinyl group, a $C_{1-6}$ haloalkylsulfonyl group, a cyano group, a nitro group, a $C_{1-6}$ acyl group, a carboxyl group, a $C_{1-6}$ alkoxycarbonyl group, a carbamoyl group, a mono($C_{1-6}$ alkyl)aminocarbonyl group, a di($C_{1-6}$ alkyl)aminocarbonyl group and a tri($C_{1-6}$ alkyl)silyl group.

**6.** The aminopyrimidine derivative or an agriculturally acceptable salt thereof according to Claim 5,
wherein, in General Formula [I],
$R^2$ is a $C_{1-10}$ alkyl group which may be substituted with one or more substituents selected from Substituent Group β, a $C_{1-10}$ alkoxy group which may be substituted with one or more substituents selected from Substituent Group α, a $C_{2-6}$ alkynyl group, a $C_{1-6}$ acyl group, a $C_{1-6}$ haloalkylcarbonyl group, a $C_{1-6}$ alkoxycarbonyl group, a carbamoyl group, a mono($C_{1-6}$ alkyl) aminocarbonyl group, a di($C_{1-6}$ alkyl)aminocarbonyl group, a $C_{1-6}$ alkylsulfonyl group, a $C_{1-6}$ haloalkylsulfonyl group, a sulfamoyl group, a mono($C_{1-6}$ alkyl)aminosulfonyl group or a di($C_{1-6}$ alkyl)aminosulfonyl group,
while $R^1$ and $R^2$ may form a 5-membered or 6-membered ring (the 5-membered or 6-membered ring is independently substituted with 1 to 4 halogen atoms or/and a $C_{1-6}$ haloalkyl group) with an atom arbitrarily selected from the group consisting of a carbon atom, an oxygen atom, a nitrogen atom and a sulfur atom, together with the nitrogen atom to which $R^1$ and $R^2$ are bonded.

**7.** A plant disease control agent for agricultural or horticultural use, which is **characterized by** containing as an active ingredient one or more compounds selected from the aminopyrimidine derivative as described in any one of Claims 2 to 6 and an agriculturally acceptable salt thereof.

**8.** A method of using an agent, which includes applying an effective amount of one or more compounds selected from the aminopyrimidine derivative according to any one of claims 2 to 6 and an agriculturally acceptable salt thereof to target useful crops or soil, for protecting the useful crops from plant disease.

## INTERNATIONAL SEARCH REPORT

International application No.
PCT/JP2007/050674

A. CLASSIFICATION OF SUBJECT MATTER
*C07D403/04*(2006.01)i, *A01N43/54*(2006.01)i, *A01P3/00*(2006.01)i, *C07D403/14*(2006.01)i

According to International Patent Classification (IPC) or to both national classification and IPC

B. FIELDS SEARCHED

Minimum documentation searched (classification system followed by classification symbols)
C07D403/04, A01N43/54, A01P3/00, C07D403/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 2005/19207 A1 (NIPPON SODA CO., LTD.), 03 March, 2005 (03.03.05), Claims 1 to 2 & US 2005/38041 A1 | 1-8 |
| Y | JP 54-115384 A (Hokko Chemical Industry Co., Ltd.), 07 September, 1979 (07.09.79), Claims 1 to 2 (Family: none) | 1-8 |
| Y | JP 2005-232081 A (Bayer Cropscience AG.), 02 September, 2005 (02.09.05), Claim 1 & WO 2005/79798 A1 | 1-8 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>02 February, 2007 (02.02.07) | Date of mailing of the international search report<br>13 February, 2007 (13.02.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No. PCT/JP2007/050674

C (Continuation). DOCUMENTS CONSIDERED TO BE RELEVANT

| Category* | Citation of document, with indication, where appropriate, of the relevant passages | Relevant to claim No. |
|---|---|---|
| Y | JP 55-36402 A (Hokko Chemical Industry Co., Ltd.), 14 March, 1980 (14.03.80), Claims 1 to 2 (Family: none) | 1-8 |
| Y | JP 54-147921 A (Hokko Chemical Industry Co., Ltd.), 19 November, 1979 (19.11.79), Claim 1 (Family: none) | 1-8 |
| A | JP 62-404 A (Takeda Chemical Industries, Ltd.), 06 January, 1987 (06.01.87), Full text (Family: none) | 1-8 |
| A | JP 10-130265 A (Hokko Chemical Industry Co., Ltd.), 19 May, 1998 (19.05.98), Full text (Family: none) | 1-8 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S54115384 A
- JP S55036402 A
- WO 2002074753 A
- WO 2004103978 A
- JP 2005232081 A
- US 6348618 B


### Non-patent literature cited in the description


- *Revista de Chimie,* 1987, vol. 38 (8), 674-679
- *Synthesis,* 2005, vol. 5, 798-803